# EUROPEAN PATENT SPECIFICATION

(11) **EP 3 046 572 B1**
(45) Date of publication and mention of the grant of the patent: **30.07.2025**
(21) Application number: 14846407.6
(22) Date of filing: 17.09.2014
(51) Int. Cl.: A61K 47/64, A61K 49/00, A61K 38/00, C12N 9/96, A61K 9/19, A61K 47/26, C07K 14/435, G01N 33/574, A61K 9/00, A61P 35/00

(54) **CHLOROTOXIN CONJUGATES AND METHODS OF USE THEREOF**
CHLORTOXINKONJUGATE UND VERFAHREN ZUR VERWENDUNG DAVON
CONJUGUÉS DE CHLOROTOXINE ET PROCÉDÉS POUR LES UTILISER

(30) Priority: 17.09.2013 US 201361879096 P; 17.09.2013 US 201361879108 P; 07.05.2014 US 201461990101 P
(43) Date of publication of application: 27.07.2016
(73) Proprietor: Blaze Bioscience, Inc., Seattle, WA 98109 (US)
(72) Inventor: JOCHHEIM, Claudia, Seattle, WA 98177 (US); MILLER, Dennis, M., Woodinville, WA 98072 (US); NAIRN, Natalie, Seattle, WA 98115 (US); STROUD, Mark, Seattle, WA 98119 (US); BYRNES-BLAKE, Kelly, Sultan, WA 98294 (US); HANSEN, Stacey, J., Renton, WA 98058 (US); NOVAK, Julia, E., Suquamish, WA 98392 (US); WISS, Valorie, R., Moscow, ID 83843 (US)
(74) Representative: MacLean, Martin Robert
(86) International application number: PCT/US2014/056177
(87) International publication number: WO 2015/042202

(56) References cited:
- WO-A1-2012/039741
- WO-A2-2011/142858
- US-A- 5 968 479
- US-A1- 2009 028 788
- US-A1- 2013 028 836
- PRAMOD V BUTTE ET AL: "Near-infrared imaging of brain tumors using the Tumor Paint BLZ-100 to achieve near-complete resection of brain tumors", NEUROSURGICAL FOCUS, 1 February 2014 (2014-02-01), United States, pages E1, XP055372779, Retrieved from the Internet <URL:http://thejns.org/doi/pdf/10.3171/2013.11.FOCUS13497> DOI: 10.3171/2013.11.FOCUS13497
- DANA M MIZRAHI ET AL: "Synthesis, fluorescence and biodistribution of a bone-targeted near-infrared conjugate", EUROPEAN JOURNAL OF MEDICINAL CHEMISTRY, EDITIONS SCIENTIFIQUE ELSEVIER, PARIS, FR, vol. 46, no. 10, 28 August 2011 (2011-08-28), pages 5175 - 5183, XP028390973, ISSN: 0223-5234, [retrieved on 20110831], DOI: 10.1016/J.EJMECH.2011.08.040

## Description

### STATEMENT AS TO FEDERALLY SPONSORED RESEARCH

This invention was made with the support of the United States government by the National Cancer Institute, National Institutes of Health, Department of Health and Human Services, under Contract No. HHSN261201200054C.

### BACKGROUND OF THE INVENTION

For many types of cancer, the precision of surgical resection directly influences patient prognosis. Unfortunately, intra-operative identification of tumor margins or small foci of cancer cells remains imprecise or depends on surgical judgment. Thus, the extent of surgical resection is constrained by the requirement to avoid harming vital structures.
WO 2011/142858 A2 describes chlorotoxin variants, conjugates, and methods for their use. Butte et al (2014), Neurosurg Focus, 3 6, E1, 1-8 describes near-infrared imaging of brain tumors using the Tumor Pain BLZ-100. US 5,968,479 A describes a diagnostic marker. Mizrahi et al (2011), European Journal of Medicinal Chemistry, 46, 5175-5183 describes synthesis, fluorescence and biodistribution of a bone-targeted near-infrared conjugate. US 2009/028788 A1 describes compounds having RD targeting moieties. US 2013/028836 A1 describes chlorotoxin polypeptides and conjugates and uses thereof.

Despite the advances in the development of probes for targeting and imaging tumors, there exists a need for a probe that allows for intra-operative visualization of cancerous tissues.

### SUMMARY OF THE INVENTION

In various aspects, the presently described compounds further comprise a detectable label, which can be used for the detection of the peptide-label conjugate and the cancerous cells to which they are bound.

The present compounds have a structure of Formula (III), or a pharmaceutically acceptable salt thereof: 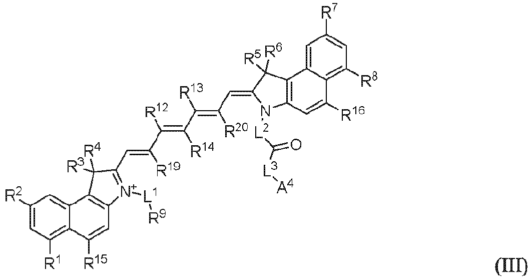 wherein:
R¹, R², R³, R⁴, R⁵, R⁶, R⁷, R⁸ , R¹⁵, and R¹⁶ are each independently selected from hydrogen, C₁-C₆ alkyl, C₁-C₆ alkylene-COOH, sulfonate, -COOH, -SO₂-NH₂, or C₁-C₆ alkoxy;
R⁹ is sulfonate;
L¹ is C₃-C₆ alkylene;
L² is C₁-C₁₀ alkylene;
L³ is a bond, -O-, -NR¹⁰-, -NR¹⁰-C₁-C₆ alkylene-, -O-NR¹⁰-, -NR¹⁰-C₁-C₆ alkylene- (O-C₁-C₆ alkylene)ₙ-, -NR¹⁰-L⁴-, -NR¹⁰-C₁-C₆ alkylene-NR¹¹-(C (= O)-C₁-C₆ alkylene-O-)ₘ-, or -NR¹⁰-C₁-C₆ alkylene-NR¹⁰-C₁-C₆ alkylene-NR¹⁰-C₁-C₆ alkylene-;
L⁴ is a bond, -heterocyclyl-, or -heterocyclyl-C₁-C₆ alkylene-;
R¹⁰ is hydrogen or C₁-C₆ alkyl;
R¹¹ is hydrogen or C₁-C₆ alkyl;
R¹² and R¹³ are independently selected from hydrogen, C₁-C₆ alkyl, or R¹² and R¹³ are joined together along with the other atoms to which they are attached to form a 5-membered or 6-membered carbocyclic or heterocyclic ring;
R¹⁴ is hydrogen or C₁-C₆ alkylene, -(L⁵)-aryl, -(L⁵)-heteroaryl, -NR¹⁷ R¹⁸, R¹⁴ and R¹⁹ are joined together along with the other atoms to which they are attached to form a 5-membered or 6-membered carbocyclic or heterocyclic ring, or R¹⁴ and R²⁰ are joined together along with the other atoms to which they are attached to form a 5-membered or 6-membered carbocyclic or heterocyclic ring;
L⁵ is a bond, C₁-C₁₀ alkylene, -O-, or -NR¹⁰-;
R¹⁷ and R¹⁸ are each independently hydrogen or aryl;
R¹⁹ and R²⁰ are independently selected from hydrogen, C₁-C₆ alkyl, R¹⁴ and R¹⁹ are joined together along with the other atoms to which they are attached to form a 5-membered or 6-membered carbocyclic or heterocyclic ring, or R¹⁴ and R²⁰ are joined together along with the other atoms to which they are attached to form a 5-membered or 6-membered carbocyclic or heterocyclic ring;
n is 0, 1, 2, or 3;
m is 0, 1, 2, or 3;
p is 0, 1, 2, or 3;
q is 0, 1, 2, or 3; and
A⁴ is a polypeptide having at least 90% sequence identity with MCMPCFTTDHQMARRCDDCCGGRGRGKCYGPQCLCR or a fragment thereof, wherein the fragment of A⁴ comprises at least 25 amino acid residues.

In various aspects, the present disclosure provides a kit comprising a vessel configured to contain a fluid; any of the claimed compounds and compositions; and an elastomeric closure affixed to the vessel.

In various aspects, the present disclosure provides a claimed composition, wherein when the composition is intravenously administering to a human subject at a dose of from 1 mg to 30 mg, the composition produces in the human subject an average maximum compound blood plasma concentration (average Cₘₐₓ) of at least from 110 ng/mL to 240 ng/mL per each 1 mg dosage of the compound administered.

In various aspects, the present disclosure provides a claimed compound for use in a method of administering to a human subject, the method comprising: intravenously administering to the human subject a dose of from 1 mg to 30 mg of the compound; and producing in the human subject an average maximum compound blood plasma concentration (average Cₘₐₓ) of at least from 110 ng/mL to 240 ng/mL per each 1 mg dosage of the compound administered.

In various aspects, the present disclosure provides a claimed compound for use in a method of detecting a cancer cell in a human subject, the method comprising: intravenously administering to the human subject a dose of from 1 mg to 30 mg of the compound; producing in the human subject an average maximum compound blood plasma concentration (average Cₘₐₓ) of at least from 110 ng/mL to 240 ng/mL per each 1 mg dosage of the compound administered; and detecting the presence or absence of the detectable label in the human subject, wherein the presence of the detectable label indicates the presence of the cancer cell.

In various aspects, the present disclosure provides a claimed compound for use in a method of diagnosing cancer in a human subject, the method comprising: intravenously administering to the human subject a dose of from 1 mg to 30 mg of the compound; producing in the human subject an average maximum compound blood plasma concentration (average Cₘₐₓ) of at least from 110 ng/mL to 240 ng/mL per each 1 mg dosage of the compound administered; and detecting the presence or absence of the detectable label in the human subject, wherein the presence of the detectable label indicates a diagnosis of cancer.

In various aspects, the present disclosure provides a claimed compound for use in a method of treating cancer in a human subject, the method comprising: intravenously administering to the human subject a dose of from 1 mg to 30 mg of the compound conjugated to a therapeutic agent; producing in the human subject an average maximum compound blood plasma concentration (average Cₘₐₓ) of at least from 110 ng/mL to 240 ng/mL per each 1 mg dosage of the compound administered; and reducing or improving a symptom or condition associated with cancer in the human subject. In some aspects, the human subject is in need thereof. In some aspects, the methods comprise administering a therapeutically effective dose of the compound to the human subject

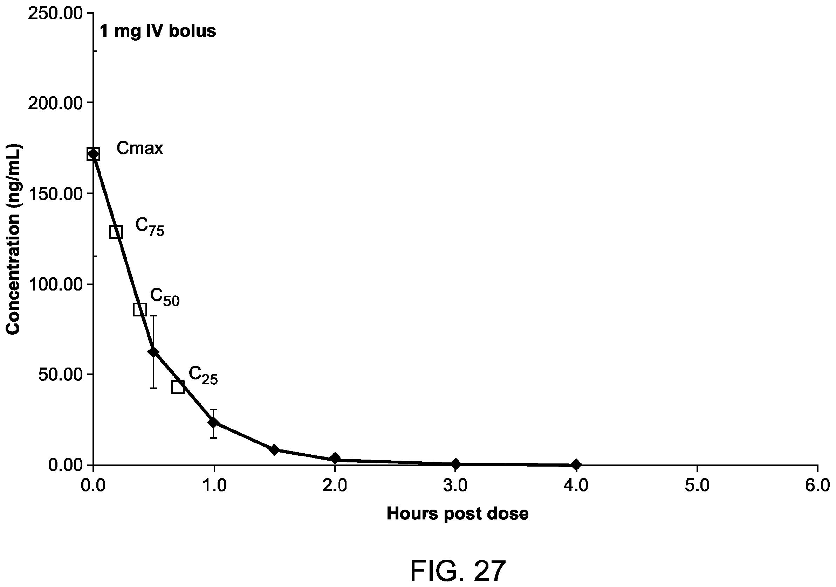

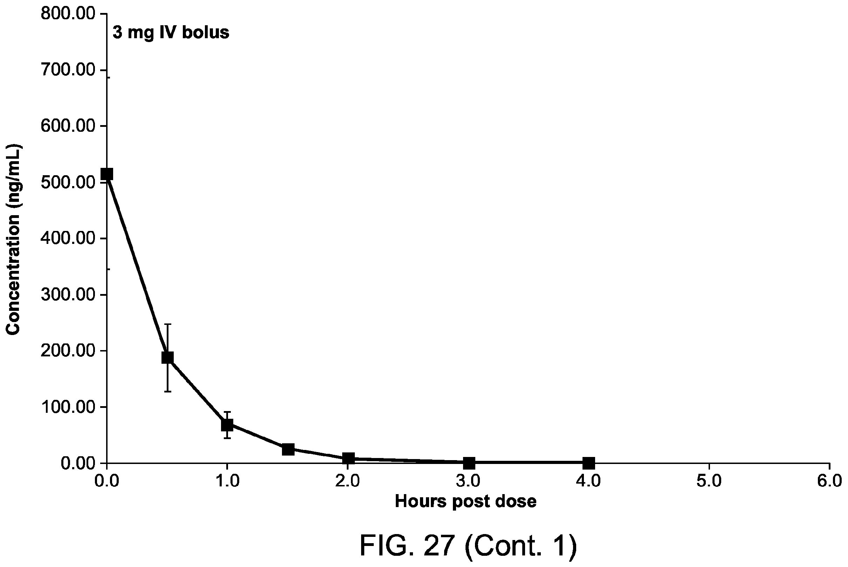

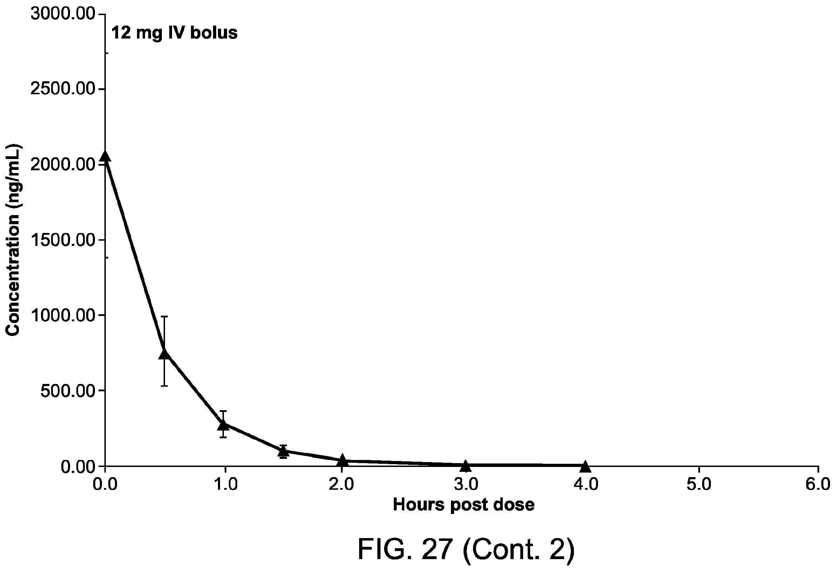

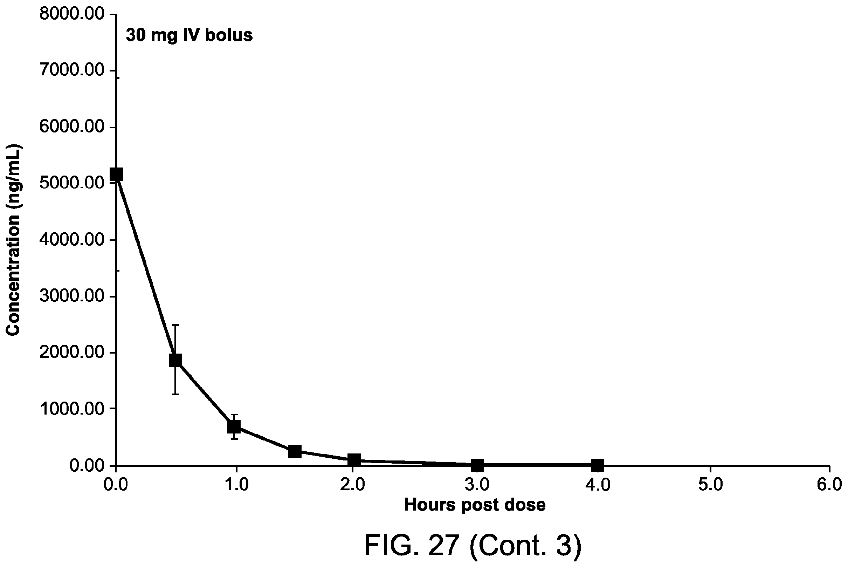

In various aspects, the present disclosure provides a claimed compound for use in a method of administering to a human subject, the method comprising: administering to the human subject a dose of from 1 mg to 30 mg of the compound; and producing in the human subject pharmacokinetic profile of FIG. 27.

In various aspects, the present disclosure provides a claimed compound for use in a method for detecting a cancer cell in a subject, the method comprising: administering the compound; and detecting the presence or absence of the compound in the subject, wherein the presence of the compound indicates the presence of a cancer cell.

In various aspects, the present disclosure provides a claimed compound for use in a method of administering to a subject, the method comprising administering a therapeutically effective amount of the compound to the subject.

In various aspects, the present disclosure provides a claimed compound for use in a method of treating a subject in need thereof, the method comprising administering to the subject the compound further comprising a therapeutic agent in an amount sufficient to treat cancer in the subject. In certain aspects, the therapeutic agent is a cytotoxic agent.

In one embodiment, the chlorotoxin conjugate comprises a chemotherapeutic, an anti-cancer agent, or an anti-cancer drug. In another embodiment, the chlorotoxin conjugate comprising the chemotherapeutic, an anti-cancer agent, or an anti-cancer drug is administered after the central or primary tumor is detected during surgery. In a further embodiment, the central or primary tumor is detected with a chlorotoxin conjugated to a labeling agent.

In another aspect is provided a chlorotoxin conjugate as elaimed for use in a method of inhibiting, preventing, minimizing, shrinking, or killing cells, or preventing metastasis in residual tumor cells in a tumor bed in an individual, comprising the step of administering the chlorotoxin conjugate to the individual wherein the chlorotoxin conjugate binds to the residual tumor cells in a tumor bed; and whereby the residual tumor cells in the tumor bed are inhibited, prevented, minimized, shrinked, or killed cells, or metastasize is prevented. In one embodiment, the chlorotoxin conjugate comprises a chemotherapeutic, an anti-cancer agent, or an anti-cancer drug. In another embodiment, the chlorotoxin conjugate comprising the chemotherapeutic, an anticancer agent, or an anti-cancer drug is admmistered after the central or primary tumor is detected during surgery. In a further embodiment, the central or primary tumor is detected with a chlorotoxin conjugated to a labeling agent.

In another aspect the invention provides, a compound as claimed conjugated to a chemotherapeutic, an anti-cancer agent, or an anti-cancer drug for use in a method of administering the same to an individual to treat, inhibit, prevent, minimize, shrink, or kill cells, or prevent metastasis in cells that are identified with a chlorotoxin conjugated to a labeling agent. In some embodiments the anti-cancer agent include antibodies, polypeptides, polysaccharides, and nucleic acids. In an embodiment, the chlorotoxin conjugate is administered about 1 day before the surgery. In another embodiment, the chlorotoxin conjugate is administered about 2 days before surgery. In another embodiment, the chlorotoxin conjugate is administered in multiple sub-doses. In an embodiment, the chlorotoxin conjugate is administered in about 2 sub-doses, 3 sub-doses, 4 sub-doses, or more sub-doses. In another embodiment, the chlorotoxin conjugate comprising the chemotherapeutic, an anti-cancer agent, or an anticancer drug is administered after the central or primary tumor is detected during surgery. In a further embodiment, the central or primary tumor is detected with a chlorotoxin conjugated to a labeling agent.

In one aspect, the invention provides a chlorotoxin conjugate as claimed for use in a method of detecting soft-tissue sarcoma in an individual, comprising the steps of: a) administering the chlorotoxin conjugate to the individual wherein the chlorotoxin conjugate binds to the soft-tissue sarcoma; and b) imaging, visualizing, or analyzing the bound chlorotoxin conjugate. In an embodiment, the detecting comprises *in vivo*, or *ex vivo* detecting. In another embodiment, the imaging visualizing, or analyzing comprises visualizing the chlorotoxin conjugate. In a further embodiment, the imaging, visualizing, or analyzing comprises in *vivo*, or *ex vivo* imaging. visualizing, or analyzing. In some embodiments, the visualizing comprises optically imaging the sarcoma. In some embodiments a plot is made of the fluorescent intensity of the chlorotoxin conjugate.

In an aspect, a chlorotoxin conjugate comprising one or more labeling agents is used in detecting the soft-tissue sarcoma. In an embodiment, the labeling agent comprises a fluorescent moiety. In a further embodiment, the fluorescent moiety comprises a near infrared fluorescent moiety. In another embodiment, the labeling agent comprises a radionuclide. In another embodiment, the soft-tissue sarcoma is selected from the group consisting of: trachea, fat tissue tumors, muscle tissue tumors, skeletal muscle sarcomas, rhabdomyosarcomas, peripheral nerve tumors, fibrous tissue tumors, myxofibrosarcomas, fibromatosis, joint tissue tumors, tumors of blood vessels and lymph vessels, angiosarcomas, gastrointestinal stromal tumors, alveolar soft part sarcoma, dermatofibrosarcoma protuberans (DFSP), desmoplastic small round cell tumour, epithelioid sarcoma, extra skeletal myxoid chondrosarcoma, and giant cell fibroblastoma (GCF).

In another aspect a chlorotoxin conjugate is used to detect soft-tissue sarcoma in subcutaneous fatty tissue. In an embodiment, the detecting comprises imaging, visualizing, or analyzing the chlorotoxin conjugate during or related to surgery, surgical resection, or intraoperative imaging and resection. In another embodiment, the sarcoma, or a portion thereof, is removed during or related to surgery.

The invention provides a chlorotoxin conjugate as claimed for use in a method of detecting cutaneous squamous cell carcinoma in an individual, comprising the steps of: a) administering the chlorotoxin conjugate to the individual, wherein the chlorotoxin conjugate binds to the cutaneous squamous cell carcinoma; and b) imaging, visualizing, or analyzing the bound chlorotoxin conjugate. In an embodiment, the detecting comprises in vivo, or ex vivo detection. In another embodiment, the imaging, visualizing, or analyzing comprises visualizing the chlorotoxin conjugate. In another embodiment, the imaging, visualizing, or analyzing comprises in vivo, or ex vivo imaging, visualizing, or analyzing.

In another aspect, the invention provides a chlorotoxin conjugate as claimed for use in a method of using a chlorotoxin conjugate to optically image cutaneous squamous cell carcinoma. In an embodiment, the chlorotoxin conjugate comprises one or more labeling agents. In another embodiment, the labeling agent comprises a fluorescent moiety. In a further embodiment, the fluorescent moiety comprises a near infrared fluorescent moiety. In another embodiment, the labeling agent comprises a radionuclide. In some embodiments, the detecting comprises imaging, visualizing, or analyzing the chlorotoxin conjugate during or related to surgery, surgical resection, or intraoperative imaging and resection. In some embodiments, the cutaneous squamous cell carcinoma, or a portion thereof, is removed during or related to surgery. In some embodiments the fluorescent intensity of the chlorotoxin conjugate is made.

In an aspect the invention provides a chlorotoxin conjugate as claimed for use in a method of detecting a low-grade tumor in an individual, comprising the steps of: a) administering the chlorotoxin conjugate to the individual wherein the chlorotoxin conjugate binds to the low-grade tumor; and b) imaging, visualizing, or analyzing the bound chlorotoxin conjugate. In an embodiment, the detecting comprises in vivo, or ex vivo detecting. In some embodiments, the imaging, visualizing, or analyzing comprises vivo, or ex vivo imaging, visualizing, or analyzing.

The invention provides a chlorotoxin conjugate as claimed for use in a method of using a chlorotoxin conjugate to optically image a low-grade tumor. In an embodiment, the chlorotoxin conjugate comprises one or more labeling agents. In an embodiment, the labeling agent comprises a fluorescent moiety. In an embodiment, the fluorescent moiety comprises a near infrared fluorescent moiety. In another embodiment, the labeling agent comprises a radionuclide. In another embodiment, the detecting is performed during or related to surgery or resection. In a further embodiment, the low-grade tumor, or a portion thereof, is removed during or related to surgery, surgical resection, or intraoperative imaging and resection. In some embodiments, the low-grade tumor is selected from the group consisting of: a) a low-grade tumor in or from brain tissue; b) a low-grade tumor in or from subcutaneous fatty tissue; c) a low-grade tumor in or from breast or mammary tissue, and d) a low-grade tumor in or from lung tissue. In some embodiments a plot is made of the fluorescent intensity of the chlorotoxin conjugate.

In an embodiment, the chlorotoxin conjugate comprises one or more labeling agents. In an embodiment, the labeling agent comprises a fluorescent moiety. In an embodiment, the fluorescent moiety comprises a near infrared fluorescent moiety. In another embodiment, the labeling agent comprises a radionuclide. In an embodiment, the detecting comprises *in vivo,* or *ex vivo* detection. In an embodiment, the imaging, visualizing, or analyzing comprises *in vivo,* or *ex vivo* imaging, visualizing, or analyzing. In some embodiments a plot is made of the fluorescent intensity of the chlorotoxin conjugate.

In another aspect, is provided a chlorotoxin conjugate as claimed for use in a method for using a chlorotoxin conjugate to detect residual cancer in the tumor bed of an individual following removal of a primary or central tumor in breast cancer surgery, comprising: a) administering the chlorotoxin conjugate to the individual wherein the chlorotoxin conjugate binds to residual cancer; and b) imaging, visualizing, or analyzing the bound chlorotoxin conjugate. In an embodiment, the chlorotoxin conjugate comprises one or more labeling agents. In a further embodiment, the labeling agent comprises a fluorescent moiety. In another embodiment, the fluorescent moiety comprises a near infrared fluorescent moiety. In another embodiment, the labeling agent comprises a radionuclide. In some embodiments the detecting is performed during or related to surgery or resection. In further embodiments, the residual cancer, or a portion thereof, is removed during or related to surgery, surgical resection, or intraoperative imaging and resection.

In an embodiment, the chlorotoxin conjugate comprises one or more labeling agents. In a further embodiment, the labeling agent comprises a fluorescent moiety. In another embodiment, the fluorescent moiety comprises a near infrared fluorescent moiety. In another embodiment, the labeling agent comprises a radionuclide. In an aspect, the invention provides, a chlorotoxin conjugate as claimed for use in a method for detecting a tumor in an individual comprising the steps of a) administering the chlorotoxin conjugate to the individual wherein the chlorotoxin conjugate binds to the tumor; and b) imaging, visualizing, or analyzing the bound chlorotoxin conjugate wherein the chlorotoxin conjugate is administered in an amount of between about 0.9 mg/m² to about 1.1 mg/m² or in an amount of between about 3 mg to about 6 mg. In an embodiment, the detecting comprises *in vivo,* or *ex vivo* detection. In some embodiments, the imaging, visualizing, or analyzing comprises *in vivo,* or *ex vivo* imaging, visualizing, or analyzing.

The invention provides a chlorotoxin conjugate as claimed for use in a method of using a chlorotoxin conjugate to optically image a tumor, in an embodiment, the chlorotoxin conjugate comprises one or more labeling agents. In a further embodiment, the labeling agent comprises a fluorescent moiety. In a further embodiment, the fluorescent moiety comprises a near infrared fluorescent moiety. In another embodiment, the labeling agent comprises a radionuclide. In some embodiments the detecting is performed during or related to surgery or resection. In some, embodiments, the tumor, of a portion thereof, is removed during or related to surgery surgical resection, or intraoperative imaging and resection.

The invention provides a chlorotoxin conjugate as claimed for use in a method of administering a chlorotoxin conjugate to an individual to detect soft-tissue sarcoma, low-grade tumor, cutaneous squamous cell carcinoma, of cells therefrom in tumors of skin or breast, and lung and mammary cancers. In an embodiment, the chlorotoxin conjugate comprises one or more labeling agents. In an embodiment, the labeling agent comprises a fluorescent moiety. In an embodiment, the fluorescent moiety comprises a near infrared fluorescent moiety. In another embodiment, the labeling agent comprises a radionuclide. In another embodiment, the detecting is performed during or related to surgery of resection. In a further embodiment, the soft-tissue sarcoma, low-grade tumor, cutaneous squamous cell carcinoma, or cells therefrom, in tumors of skin or breast, and lung and mammary cancers, of a portion thereof is removed during or related to surgery, surgical resection, or intraoperative imaging and resection. In other embodiments, the chlorotoxin conjugate is administered in an amount of between about 0.9 mg/m² to about 1.1 mg/m² or in an amount of between about 3 mg to about 6 mg. In an embodiment, the chlorotoxin conjugate is administered about 1 day before the surgery. In another embodiment, the chlorotoxin conjugate is administered about 2 days before surgery. In another embodiment, the chlorotoxin conjugate is administered in multiple sub-doses. In an embodiment, the chlorotoxin conjugate is administered in about 2 sub-doses, 3 sub-doses, 4 sub-doses, or more sub-doses. In an embodiment, the detecting comprises *in vivo*, or *ex vivo* detection. In some embodiments, the imaging, visualizing, or analyzing comprises *in vivo,* or *ex vivo* imaging, visualizing, or analyzing. In some embodiments a plot is made of the fluorescent intensity of the chlorotoxin conjugate.

In another aspect, is provided a chlorotoxin conjugate as claimed for use in a method for using a chlorotoxin conjugate to detect residual cancer in the tumor bed of an individual following removal of a primary or central tumor in breast cancer surgery, comprising: a) administering the chlorotoxin conjugate to the individual wherein the chlorotoxin conjugate binds to residual cancer; and b) imaging, visualizing, or analyzing the bound chlorotoxin conjugate. In an embodiment, the chlorotoxin conjugate comprises one or more labeling agents. In a further embodiment, the labeling agent comprises a fluorescent moiety. In another embodiment, the fluorescent moiety comprises a near infrared fluorescent moiety. In another embodiment, the labeling agent comprises a radionuclide. In some embodiments the detecting is performed during or related to surgery or resection. In further embodiments, the residual cancer, or a portion thereof, is removed during or related to surgery. surgical resection, or intraoperative imaging and resection.

In an embodiment, the chlorotoxin conjugate comprises one or more labeling agents. In an embodiment, the labeling agent comprises a fluorescent moiety. In an embodiment, the fluorescent moiety comprises a near infrared fluorescent moiety. In another embodiment, the labeling agent comprises a radionuclide. In another embodiment, the detecting is performed during or related to surgery or resection. In an embodiment, the chlorotoxin conjugate is administered about 1 day before the surgery. In another embodiment, the chlorotoxin conjugate is administered about 2 days before surgery. In another embodiment, the chlorotoxin conjugate is administered in multiple sub-doses. In an embodiment, the chlorotoxin conjugate is administered in about 2 sub-doses, 3 sub-doses, 4 sub-doses, or more sub-doses. In an embodiment, the detecting comprises vivo, or *ex vivo* detection. In some embodiments, the imaging, visualizing, or analyzing comprises *in vivo,* or *ex vivo* imaging, visualizing, or analyzing. In some embodiments a plot is made of the fluorescent intensity of the chlorotoxin conjugate.

The invention further provides a chlorotoxin conjugate as claimed for use in a method for detecting soft tissue sarcoma in an individual comprising, administering the chlorotoxin conjugate to the individual, wherein the chlorotoxin conjugate comprises a detectable agent, binding the chlorotoxin conjugate to the soft tissue sarcoma, and detecting the bound chlorotoxin conjugate, wherein an elevated level of bound chlorotoxin conjugate indicates the presence of soft tissue sarcoma.

### BRIEF DESCRIPTION OF THE DRAWINGS

The novel features of the invention are set forth with particularity in the appended claims. A better understanding of the features and advantages of the present invention will be obtained by reference to the following detailed description that sets forth illustrative embodiments, in which the principles of the invention are utilized, and the accompanying drawings of which:
FIG. 1 shows SDS-PAGE of chlorotoxin conjugate formulations after 14 days at room temperature.
FIG. 2 shows SDS-PAGE of chlorotoxin conjugate formulations after 5 days at 40 °C (and t0).
FIG. 3 shows SDS-PAGE of chlorotoxin conjugate formulations after 3 days at room temperature in the light or 3X F/T. Samples are after 3 days at room temperature in the light unless marked as F/T
FIG. 4 shows total peak area of RP-HPLC chromatograms for chlorotoxin conjugate formulations with no lyophilization or lyophilization with fast or slow freezing.
FIG. 5 shows FTIR-derived secondary structures obtained for chlorotoxin conjugate lyophilized with slow versus fast freezing (left) and in a liquid formulation (right).
FIG. 6 shows SDS-PAGE analysis of various lyophilized formulations.

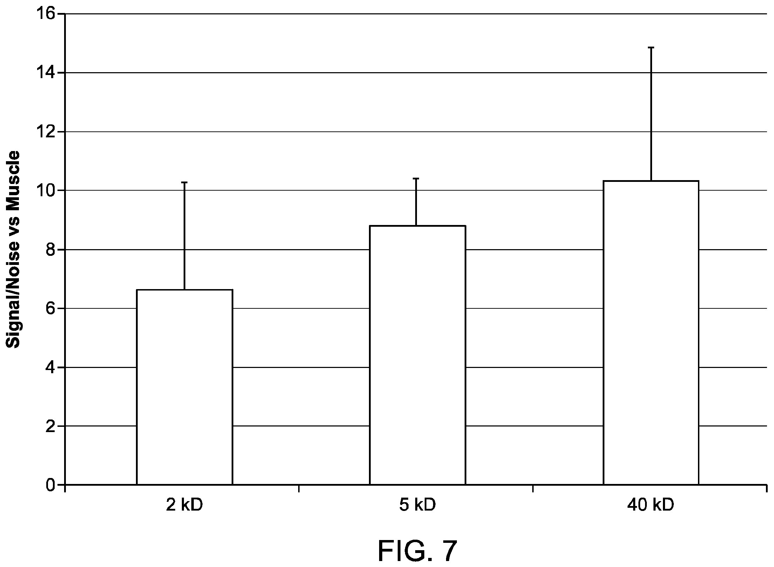

FIG. 7 shows signal-to-noise ratios (SNRs) of chlorotoxin conjugates in U87 flank tumors 24 hours after injection.
FIG. 8 shows fluorescent images of Compound 76-2kD (left), Compound 76-5kD (middle) and Compound 76-40kD (right).
FIG. 9A shows a fluorescent image of Compound 76-5kD. FIG. 9B shows an H&E stain of the tissue sample.
FIG. 10 shows biodistribution analysis of Compound 76-5kD. FIG. 10A shows integrated intensity for sections from liver, kidney, spleen, heart and brain. FIGS. 10B and 10C show representative images from liver and kidney, respectively.
FIG. 11 shows an Odyssey fluorescent image of Compound 76-5kD (top) and H&E stain (bottom) of heart tissue.
FIG. 12A SNR (tumor/muscle) for different doses of chlorotoxin conjugate (n = 2-7; sample size is shown inside each column; error = standard deviation).
FIG. 12B shows signal from a subset of tumor and muscle samples. Representative muscle and tumor images for the 6 nmol and 20 nmol dose groups 1 and 3 days post injection are shown below the graph.
FIG. 13 shows biodistribution of select tissues using the IVIS Spectrum imaging system. FIG. 13A represents tissues one day after injection. FIG. 13B shows tissues imaged three days post injection. (B- brain, H- heart, K- kidney, S- skin, L- liver). FIG. 13C shows fluorescent signal in tissues for the 2 nmol and 20 nmol groups one and three days after injection.
FIG. 14 shows whole body live animal imaging of chlorotoxin conjugate from six hours to three days after injection.
FIG. 15A shows *ex vivo* imaging of chlorotoxin conjugate 1 day after injection using the IVIS Spectrum. FIG. 15B shows *ex vivo* imaging of the brain from orthotopic mouse 2. FIG. 15C shows *ex vivo* Odyssey imaging of the brain and skull from orthotopic mouse 1.
   FIG. 16 shows fluorescence intensity in gross tumors, grouped by tumor type. Regions of interest (ROI) were drawn on Odyssey scans of gross tumors. ROIs were the same size across the data set.
   FIG. 17 shows *ex vivo* imaging of a canine soft tissue sarcoma. Patient 13 was a 7-year-old female Standard Poodle who presented with a subcutaneous hemangiopericytoma, a type of soft tissue sarcoma. She was treated with BLZ-100 at 0.94 mg/m², and surgery was performed 48 hours later. White light (A, C) and Odyssey near-infrared (B, D) images are shown of gross tumor and adjacent normal fat (A, B) and uninvolved skin (C, D). Gross ratios of tumor to fat were 257:1 to 127:1, and gross ratios of tumor to skin were 89:1 to 33:1.
   FIG. 18 shows a determination of the chlorotoxin conjugate dose at which tumor to background ratio is maximal. Sections of tumor and non-tumor tissue were scored by a histopathologist who was blinded to the fluorescence data. Total fluorescence in 2x2 mm grid squares was measured, and each square was designated tumor or non-tumor by overlaying the fluorescence image with the H&E image scored by the histopathologist. The average of tumor and non-tumor squares was used to compute the tumor to background ratio (TBR), shown at right of each data point.
FIG. 19 shows a box & whiskers plot of gross tumor intensity for soft-tissue sarcomas (all subtypes) and carcinomas (including adenocarcinoma and squamous cell carcinoma). Tissues were labeled with BLZ-100.
FIG. 20 shows signal and image analysis of tumor versus normal cortical tissue in SmoA1 mouse brain.
FIG. 21 shows small foci of fluorescence that correspond to small clusters of tumor cells highlighted in the H&E stained slide.
FIG. 22 shows the mean (SD) of serum BLZ-100 concentration vs. time profiles following a single intravenous bolus of 0.02 mg BLZ-100 administered to female mice.
FIG. 23 shows the mean (SD) of serum BLZ-100 concentration vs. time profiles following a single intravenous bolus of 0.03 or 0.3 mg dose to male rats.
FIG. 24 shows the individual serum BLZ-100 concentration vs. time profiles following a single intravenous bolus dose of 0.6 mg to male monkeys.
FIG. 25 shows the mean (±SD) BLZ-100 serum concentrations (ng/mL) summarized by dose following a single intravenous bolus dose to male and female rats.
FIG. 26 shows the mean (±SD) BLZ-100 serum concentrations (ng/mL) summarized by dose following a single intravenous bolus dose to male and female monkeys.
FIG. 27 shows chlorotoxin conjugate serum concentration vs time for human subjects.

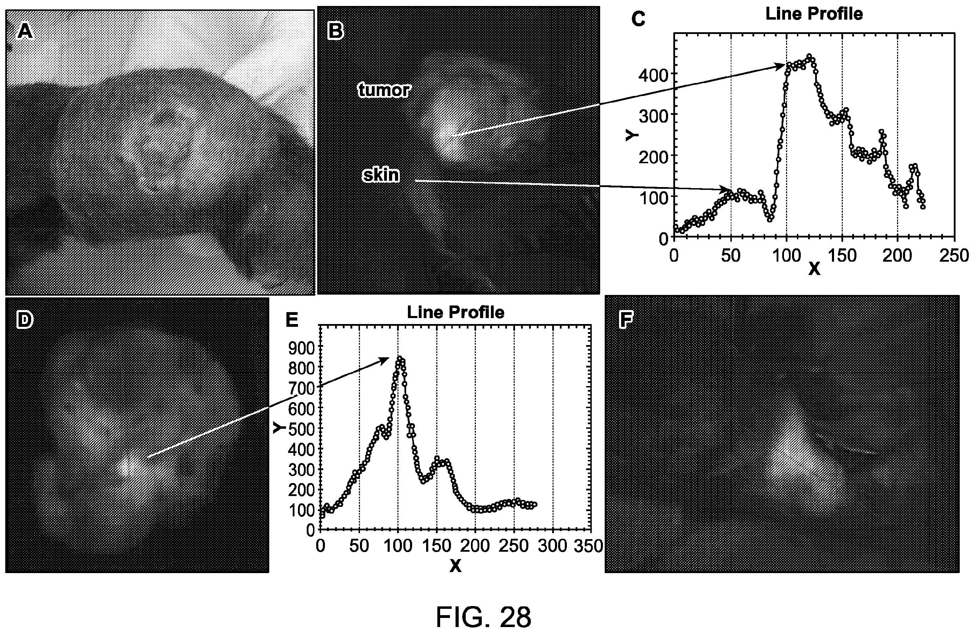

FIG. 28 shows intraoperative imaging of a soft-tissue sarcoma (patient 19). (A) White light preoperative image of gross tumor showing ulcerated and grossly swollen peritumoral skin. (B) NIR image of tumor *in situ.* (C) Plot of fluorescence intensity along the line drawn through the image in panel B. (D) NIR image of excised tumor. (E) Plot of fluorescence intensity along the line drawn through the image in panel D. (F) NIR image of peritumoral skin, surrounding uninvolved skin, and tumor bed. Peritumoral skin is 6-fold more intense than uninvolved skin. There is no residual fluorescence in the tumor bed. Tissues were labeled with BLZ-100.

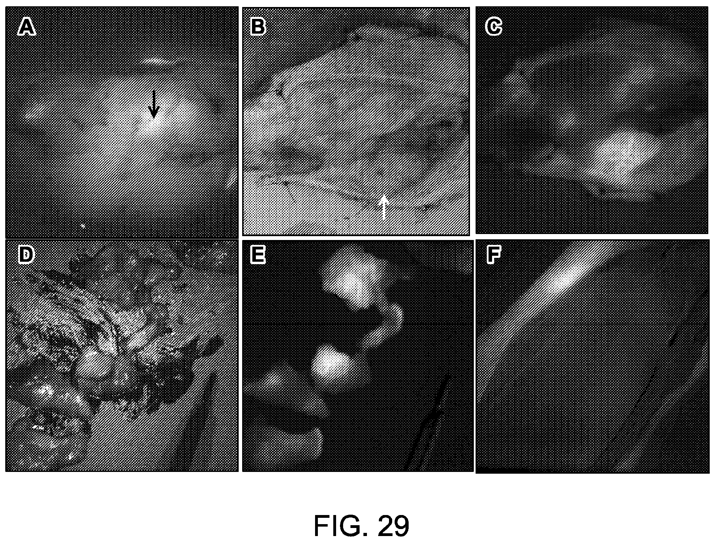

FIG. 29 shows intraoperative imaging of a mammary carcinoma (patient 22). (A) NIR image of fluorescence from the primary mass (arrow) imaged from the bottom of the resected tissue, taken immediately post-excision. This aspect had a margin of about 0.5 cm of normal tissue. Panels B (fluorescence) and C (overlay) show the primary mass from the skin side, after the skin was opened and a slice removed for further imaging. The small fluorescent patches were originally part of the primary mass, but were separated by the removal of the slice. Panels D and E show the gross appearance and fluorescence overlay of the pieces submitted for further imaging. The contrast between gross tumor and adjacent tissue is about 2.5-fold. (F) Overlay image of the tumor bed, showing lack of residual fluorescence in the muscle wall. Tissues were labeled with BLZ-100.

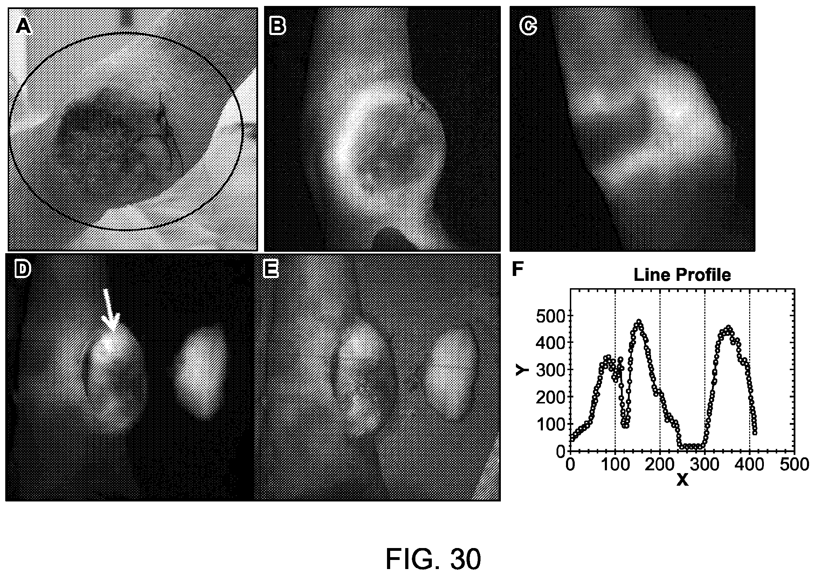

FIG. 30 shows intraoperative imaging of a cutaneous squamous cell carcinoma (patient 23). (A) White light preoperative image of tumor site, showing grossly ulcerated and swollen peritumoral skin. Panels B and C show preoperative NIR fluorescence images of the tumor from the top (B) and side (C). NIR fluorescence (D) and overlay (E) are shown following removal of the tail. The mass at right is a section of central tumor removed for further analysis. The skin is retracted, and the remaining gross tumor (arrow) is revealed. Fluorescence intensity is similar in the central tumor and remaining gross tumor, while peritumoral skin has somewhat lower fluorescence intensity (F). Tissues were labeled with BLZ-100.
   FIG. 31 shows intraoperative imaging of a thyroid carcinoma (patient 20). (Top) White light images of excised lymph node, thyroid mass, and tumor bed. (Middle) NIR fluorescence signal overlaid on white light images. (Bottom) Monochrome NIR images. The lymph node and thyroid mass each have a section removed for further analysis, shown side-by-side with the bulk tissue.
   FIG. 32 shows a box & whiskers plots of fluorescence intensity in grid squares for each tissue section analyzed. T, tumor. NT, adjacent non-tumor tissue. PS, peritumoral skin. S, uninvolved skin. For the cutaneous tumors, the NT consisted of underlying dermis, subcutaneous fat, and adjacent dermis/epidermis. Tissues were labeled with a chlorotoxin conjugate compound.
   FIG. 33 shows imaging of a brain tumor labeled with BLZ-100. (A) NIR fluorescence image of tumor *in situ.* T, tumor. M, nasal mucosa. B, normal brain. Panels B and C show fluorescence images of 30 micron sections from two of the tumor pieces. Panels D and E show H&E stains of the sections in B and C, respectively.

### DETAILED DESCRIPTION

The present disclosure provides compositions and methods for the detection and/or treatment of cancers. The compositions described herein comprise peptide conjugates comprising a detectable label, which are suitable for the detection and treatment of various cancers. In certain aspects, the compositions are provided in combination with a pharmaceutically acceptable carrier, which can be administered to a subject by any parenteral route of administration. The compositions described herein give rise to a pharmacokinetic profile when administered intravenously to a human subject. Following administration of the compositions described herein, the conjugates bind selectively to cancer cells. The cancer cells can then be detected, for example, by imaging or other visualization or detection method suitable for detecting the detectable label of the peptide conjugate. In further aspects, the presently described compositions can be used to treat cancer by way of a therapeutic agent, which is attached to the conjugate and which acts on the cancer cells following binding by the peptide portion of the conjugate. These and other aspects are described in detail herein.

The invention will best be understood by reference to the following detailed description of the aspects and embodiments of the invention, taken in conjunction with the accompanying drawings and figures. The discussion below is descriptive, illustrative and exemplary and is not to be taken as limiting the scope defined by any appended claims.

As used in the specification and appended claims, unless specified to the contrary, the following terms have the meaning indicated.

As used herein and in the appended claims, the singular forms "a," "and," and "the" include plural referents unless the context clearly dictates otherwise. Thus, for example, reference to "a compound" includes a plurality of such compounds, reference to "an agent" includes a plurality of such agents, and reference to "the cell" includes reference to one or more cells (or to a plurality of cells) and equivalents thereof known to those skilled in the art, and so forth. When ranges are used herein for physical properties, such as molecular weight, or chemical properties, such as chemical formulae, all combinations and subcombinations of ranges and specific embodiments therein are intended to be included. The term "about" when referring to a number or a numerical range means that the number or numerical range referred to is an approximation within experimental variability (or within statistical experimental error), and thus the number or numerical range may vary between 1% and 15% of the stated number or numerical range. The term "comprising" (and related terms such as "comprise" or "comprises" or "having" or "including") is not intended to exclude that in other certain embodiments, for example, an embodiment of any composition of matter, composition, method, or process, or the like, described herein, may "consist of" or "consist essentially of" the described features.

"Cyano" refers to the -CN radical.

"Nitro" refers to the -NO₂ radical.

"Oxa" refers to the -O- radical.

"Oxo" refers to the = O radical.

"Thioxo" refers to the = S radical.

"Imino" refers to the = N-H radical.

"Hydrazino" refers to the = N-NH₂ radical.

"Alkyl" refers to a straight or branched hydrocarbon chain radical consisting solely of carbon and hydrogen atoms, containing no unsaturation, having from one to fifteen carbon atoms (e.g., C₁-C₁₅ alkyl). In certain embodiments, an alkyl comprises one to thirteen carbon atoms (e.g., C₁-C₁₃ alkyl). In certain embodiments, an alkyl comprises one to eight carbon atoms (e.g., C₁-C₈ alkyl). In other embodiments, an alkyl comprises five to fifteen carbon atoms (e.g., C₅-C₁₅ alkyl). In other embodiments, an alkyl comprises five to eight carbon atoms (e.g., C₅-C₈ alkyl). The alkyl is attached to the rest of the molecule by a single bond, for example, methyl (Me), ethyl (Et), n-propyl, 1-methylethyl (iso-propyl), n-butyl, n-pentyl, 1,1-dimethylethyl (t-butyl), 3-methylhexyl, 2-methylhexyl, and the like. Unless stated otherwise specifically in the specification, an alkyl group is optionally substituted by one or more of the following substituents: halo, cyano, nitro, oxo, thioxo, trimethylsilanyl, -OR^{a},-SR^{a}, -OC(O)-R^{a}, -N(R^{a})₂, -C(O)R^{a}, -C(O)OR^{a}, -C(O)N(R^{a})₂, -N(R^{a})C(O)OR^{a}, -N(R^{a})C(O)R^{a}, -N(R^{a})S(O)ₜR^{a} (where t is 1 or 2), -S(O)ₜOR^{a} (where t is 1 or 2) and -S(O)ₜN(R^{a})₂ (where t is 1 or 2) where each R^{a} is independently hydrogen, alkyl, fluoroalkyl, carbocyclyl, carbocyclylalkyl, aryl, aralkyl, heterocyclyl, heterocyclylalkyl, heteroaryl or heteroarylalkyl.

"Alkenyl" refers to a straight or branched hydrocarbon chain radical group consisting solely of carbon and hydrogen atoms, containing at least one double bond, and having from two to twelve carbon atoms. In certain embodiments, an alkenyl comprises two to eight carbon atoms. In other embodiments, an alkenyl comprises two to four carbon atoms. The alkenyl is attached to the rest of the molecule by a single bond, for example, ethenyl (*i.e.,* vinyl), prop-1-enyl (*i.e.,* allyl), but-1-enyl, pent-1-enyl, penta-1,4-dienyl, and the like. Unless stated otherwise specifically in the specification, an alkenyl group is optionally substituted by one or more of the following substituents: halo, cyano, nitro, oxo, thioxo, trimethylsilanyl, -OR^{a},-SR^{a}, -OC(O)-R^{a}, -N(R^{a})₂, -C(O)R^{a}, -C(O)OR^{a}, -C(O)N(R^{a})₂, -N(R^{a})C(O)OR^{a}, -N(R^{a})C(O)R^{a}, -N(R^{a})S(O)ₜR^{a} (where t is 1 or 2), -S(O)ₜOR^{a} (where t is 1 or 2) and -S(O)ₜN(R^{a})₂ (where t is 1 or 2) where each R^{a} is independently hydrogen, alkyl, fluoroalkyl, carbocyclyl, carbocyclylalkyl, aryl, aralkyl, heterocyclyl, heterocyclylalkyl, heteroaryl or heteroarylalkyl.

"Alkynyl" refers to a straight or branched hydrocarbon chain radical group consisting solely of carbon and hydrogen atoms, containing at least one triple bond, having from two to twelve carbon atoms. In certain embodiments, an alkynyl comprises two to eight carbon atoms. In other embodiments, an alkynyl has two to four carbon atoms. The alkynyl is attached to the rest of the molecule by a single bond, for example, ethynyl, propynyl, butynyl, pentynyl, hexynyl, and the like. Unless stated otherwise specifically in the specification, an alkynyl group is optionally substituted by one or more of the following substituents: halo, cyano, nitro, oxo, thioxo, trimethylsilanyl, -OR^{a},-SR^{a}, -OC(O)-R^{a}, -N(R^{a})₂, -C(O)R^{a}, -C(O)OR^{a}, -C(O)N(R^{a})₂, -N(R^{a})C(O)OR^{a}, -N(R^{a})C(O)R^{a}, -N(R^{a})S(O)ₜR^{a} (where t is 1 or 2), -S(O)ₜOR^{a} (where t is 1 or 2) and -S(O)ₜN(R^{a})₂ (where t is 1 or 2) where each R^{a} is independently hydrogen, alkyl, fluoroalkyl, carbocyclyl, carbocyclylalkyl, aryl, aralkyl, heterocyclyl, heterocyclylalkyl, heteroaryl or heteroarylalkyl.

"Alkylene" or "alkylene chain" refers to a straight or branched divalent hydrocarbon chain linking the rest of the molecule to a radical group, consisting solely of carbon and hydrogen, containing no unsaturation and having from one to twelve carbon atoms, for example, methylene, ethylene, propylene, n-butylene, and the like. The alkylene chain is attached to the rest of the molecule through a single bond and to the radical group through a single bond. The points of attachment of the alkylene chain to the rest of the molecule and to the radical group can be through one carbon in the alkylene chain or through any two carbons within the chain. Unless stated otherwise specifically in the specification, an alkylene chain is optionally substituted by one or more of the following substituents: halo, cyano, nitro, aryl, cycloalkyl, heterocyclyl, heteroaryl, oxo, thioxo, trimethylsilanyl, -OR^{a},-SR^{a}, -OC(O)-R^{a}, -N(R^{a})₂, -C(O)R^{a}, -C(O)OR^{a}, -C(O)N(R^{a})₂, -N(R^{a})C(O)OR^{a}, -N(R^{a})C(O)R^{a}, -N(R^{a})S(O)ₜR^{a} (where t is 1 or 2), -S(O)ₜOR^{a} (where t is 1 or 2) and -S(O)ₜN(R^{a})₂ (where t is 1 or 2) where each R^{a} is independently hydrogen, alkyl, fluoroalkyl, carbocyclyl, carbocyclylalkyl, aryl, aralkyl, heterocyclyl, heterocyclylalkyl, heteroaryl or heteroarylalkyl.

"Alkenylene" or "alkenylene chain" refers to a straight or branched divalent hydrocarbon chain linking the rest of the molecule to a radical group, consisting solely of carbon and hydrogen, containing at least one double bond and having from two to twelve carbon atoms, for example, ethenylene, propenylene, n-butenylene, and the like. The alkenylene chain is attached to the rest of the molecule through a double bond or a single bond and to the radical group through a double bond or a single bond. The points of attachment of the alkenylene chain to the rest of the molecule and to the radical group can be through one carbon or any two carbons within the chain. Unless stated otherwise specifically in the specification, an alkenylene chain is optionally substituted by one or more of the following substituents: halo, cyano, nitro, aryl, cycloalkyl, heterocyclyl, heteroaryl, oxo, thioxo, trimethylsilanyl, -OR^{a},-SR^{a}, -OC(O)-R^{a}, -N(R^{a})₂, -C(O)R^{a}, -C(O)OR^{a}, -C(O)N(R^{a})₂, -N(R^{a})C(O)OR^{a}, -N(R^{a})C(O)R^{a}, -N(R^{a})S(O)ₜR^{a} (where t is 1 or 2), -S(O)ₜOR^{a} (where t is 1 or 2) and -S(O)ₜN(R^{a})₂ (where t is 1 or 2) where each R^{a} is independently hydrogen, alkyl, fluoroalkyl, cycloalkyl, cycloalkylalkyl, aryl (optionally substituted with one or more halo groups), aralkyl, heterocyclyl, heterocyclylalkyl, heteroaryl or heteroarylalkyl, and where each of the above substituents is unsubstituted unless otherwise indicated.

"Aryl" refers to a radical derived from an aromatic monocyclic or multicyclic hydrocarbon ring system by removing a hydrogen atom from a ring carbon atom. The aromatic monocyclic or multicyclic hydrocarbon ring system contains only hydrogen and carbon from six to eighteen carbon atoms, where at least one of the rings in the ring system is fully unsaturated, *i.e.,* it contains a cyclic, delocalized (4n+2) π-electron system in accordance with the Hückel theory. Aryl groups include, but are not limited to, groups such as phenyl, fluorenyl, and naphthyl. Unless stated otherwise specifically in the specification, the term "aryl" or the prefix "ar-" (such as in "aralkyl") is meant to include aryl radicals optionally substituted by one or more substituents independently selected from alkyl, alkenyl, alkynyl, halo, fluoroalkyl, cyano, nitro, optionally substituted aryl, optionally substituted aralkyl, optionally substituted aralkenyl, optionally substituted aralkynyl, optionally substituted carbocyclyl, optionally substituted carbocyclylalkyl, optionally substituted heterocyclyl, optionally substituted heterocyclylalkyl, optionally substituted heteroaryl, optionally substituted heteroarylalkyl, -R^{b}-OR^{a}, -R^{b}-OC(O)-R^{a}, -R^{b}-N(R^{a})₂, -R^{b}-C(O)R^{a}, -R^{b}-C(O)OR^{a}, -R^{b}-C(O)N( R^{a})₂, -R^{b}-O-R^{c}-C(O)N(R^{a})₂, -R^{b}-N(R^{a})C(O)OR^{a}, -R^{b}-N(R^{a})C(O)R^{a}, -R^{b}-N(R^{a})S(O)ₜR^{a} (where t is 1 or 2), -R^{b}-S(O)ₜOR^{a} (where t is 1 or 2) and -R^{b}-S(O)ₜN(R^{a})₂ (where t is 1 or 2), where each R^{a} is independently hydrogen, alkyl, fluoroalkyl, cycloalkyl, cycloalkylalkyl, aryl (optionally substituted with one or more halo groups), aralkyl, heterocyclyl, heterocyclylalkyl, heteroaryl or heteroarylalkyl, each R^{b} is independently a direct bond or a straight or branched alkylene or alkenylene chain, and R^{c} is a straight or branched alkylene or alkenylene chain, and where each of the above substituents is unsubstituted unless otherwise indicated.

"Aralkyl" refers to a radical of the formula -R^{c}-aryl where R^{c} is an alkylene chain as defined above, for example, benzyl, diphenylmethyl and the like. The alkylene chain part of the aralkyl radical is optionally substituted as described above for an alkylene chain. The aryl part of the aralkyl radical is optionally substituted as described above for an aryl group.

"Aralkenyl" refers to a radical of the formula -R^{d}-aryl where R^{d} is an alkenylene chain as defined above. The aryl part of the aralkenyl radical is optionally substituted as described above for an aryl group. The alkenylene chain part of the aralkenyl radical is optionally substituted as defined above for an alkenylene group.

"Aralkynyl" refers to a radical of the formula -R^{e}-aryl, where R^{e} is an alkynylene chain as defined above. The aryl part of the aralkynyl radical is optionally substituted as described above for an aryl group. The alkynylene chain part of the aralkynyl radical is optionally substituted as defined above for an alkynylene chain.

"Carbocyclyl" refers to a stable non-aromatic monocyclic or polycyclic hydrocarbon radical consisting solely of carbon and hydrogen atoms, which may include fused or bridged ring systems, having from three to fifteen carbon atoms. In certain embodiments, a carbocyclyl comprises three to ten carbon atoms. In other embodiments, a carbocyclyl comprises five to seven carbon atoms. The carbocyclyl is attached to the rest of the molecule by a single bond. Carbocyclyl may be saturated, (*i.e.,* containing single C-C bonds only) or unsaturated (*i.e.,* containing one or more double bonds or triple bonds.) A fully saturated carbocyclyl radical is also referred to as "cycloalkyl." Examples of monocyclic cycloalkyls include, *e.g*., cyclopropyl, cyclobutyl, cyclopentyl, cyclohexyl, cycloheptyl, and cyclooctyl. An unsaturated carbocyclyl is also referred to as "cycloalkenyl." Examples of monocyclic cycloalkenyls include, *e.g*., cyclopentenyl, cyclohexenyl, cycloheptenyl, and cyclooctenyl. Polycyclic carbocyclyl radicals include, for example, adamantyl, norbornyl (*i.e.,* bicyclo[2.2.1]heptanyl), norbornenyl, decalinyl, 7,7-dimethyl-bicyclo[2.2.1]heptanyl, and the like. Unless otherwise stated specifically in the specification, the term "carbocyclyl" is meant to include carbocyclyl radicals that are optionally substituted by one or more substituents independently selected from alkyl, alkenyl, alkynyl, halo, fluoroalkyl, oxo, thioxo, cyano, nitro, optionally substituted aryl, optionally substituted aralkyl, optionally substituted aralkenyl, optionally substituted aralkynyl, optionally substituted carbocyclyl, optionally substituted carbocyclylalkyl, optionally substituted heterocyclyl, optionally substituted heterocyclylalkyl, optionally substituted heteroaryl, optionally substituted heteroarylalkyl, -R^{b}-OR^{a}, -R^{b}-SR^{a}, -R^{b}-OC(O)-R^{a}, -R^{b}-N(R^{a})₂, -R^{b}-C(O)R^{a}, -R^{b}-C(O)OR^{a}, -R^{b} -C(O)N(R^{a})₂, -R^{b}-O-R^{c}-C(O)N(R^{a})₂, -R^{b}-N(R^{a})C(O)OR^{a}, -R^{b}-N(R^{a})C(O)R^{a}, -R^{b}-N(R^{a})S(O)ₜR ^{a} (where t is 1 or 2), -R^{b}-S(O)ₜOR^{a} (where t is 1 or 2) and -R^{b}-S(O)ₜN(R^{a})₂ (where t is 1 or 2), where each R^{a} is independently hydrogen, alkyl, fluoroalkyl, cycloalkyl, cycloalkylalkyl, aryl, aralkyl, heterocyclyl, heterocyclylalkyl, heteroaryl or heteroarylalkyl, each R^{b} is independently a direct bond or a straight or branched alkylene or alkenylene chain, and R^{c} is a straight or branched alkylene or alkenylene chain, and where each of the above substituents is unsubstituted unless otherwise indicated.

"Carbocyclylalkyl" refers to a radical of the formula -R^{c}-carbocyclyl where R^{c} is an alkylene chain as defined above. The alkylene chain and the carbocyclyl radical is optionally substituted as defined above.

"Halo" or "halogen" refers to bromo, chloro, fluoro or iodo substituents.

"Fluoroalkyl" refers to an alkyl radical, as defined above, that is substituted by one or more fluoro radicals, as defined above, for example, trifluoromethyl, difluoromethyl, 2,2,2-trifluoroethyl, 1-fluoromethyl-2-fluoroethyl, and the like. The alkyl part of the fluoroalkyl radical is optionally substituted as defined above for an alkyl group.

"Heterocyclyl" refers to a stable 3- to 18-membered non-aromatic ring radical that comprises two to twelve carbon atoms and from one to six heteroatoms selected from nitrogen, oxygen and sulfur. Unless stated otherwise specifically in the specification, the heterocyclyl radical is a monocyclic, bicyclic, tricyclic or tetracyclic ring system, which may include fused or bridged ring systems. The heteroatoms in the heterocyclyl radical may be optionally oxidized. One or more nitrogen atoms, if present, are optionally quaternized. The heterocyclyl radical is partially or fully saturated. The heterocyclyl may be attached to the rest of the molecule through any atom of the ring(s). Examples of such heterocyclyl radicals include, but are not limited to, dioxolanyl, thienyl[1,3]dithianyl, decahydroisoquinolyl, imidazolinyl, imidazolidinyl, isothiazolidinyl, isoxazolidinyl, morpholinyl, octahydroindolyl, octahydroisoindolyl, 2-oxopiperazinyl, 2-oxopiperidinyl, 2-oxopyrrolidinyl, oxazolidinyl, piperidinyl, piperazinyl, 4-piperidonyl, pyrrolidinyl, pyrazolidinyl, quinuclidinyl, thiazolidinyl, tetrahydrofuryl, trithianyl, tetrahydropyranyl, thiomorpholinyl, thiamorpholinyl, 1-oxo-thiomorpholinyl, and 1,1-dioxo-thiomorpholinyl. Unless stated otherwise specifically in the specification, the term "heterocyclyl" is meant to include heterocyclyl radicals as defined above that are optionally substituted by one or more substituents selected from alkyl, alkenyl, alkynyl, halo, fluoroalkyl, oxo, thioxo, cyano, nitro, optionally substituted aryl, optionally substituted aralkyl, optionally substituted aralkenyl, optionally substituted aralkynyl, optionally substituted carbocyclyl, optionally substituted carbocyclylalkyl, optionally substituted heterocyclyl, optionally substituted heterocyclylalkyl, optionally substituted heteroaryl, optionally substituted heteroarylalkyl, -R^{b}-OR^{a}, -R^{b}-SR^{a}, -R^{b}-OC(O)-R^{a}, -R^{b}-N(R^{a})₂, -R^{b}-C(O)R^{a}, -R^{b}-C(O)OR^{a}, -R^{b} -C(O)N(R^{a})₂, -R^{b}-O-R^{c}-C(O)N(R^{a})₂, -R^{b}-N(R^{a})C(O)OR^{a}, -R^{b}-N(R^{a})C(O)R^{a}, -R^{b}-N(R^{a})S(O)ₜR ^{a} (where t is 1 or 2), -R^{b}-S(O)ₜOR^{a} (where t is 1 or 2) and -R^{b}-S(O)ₜN(R^{a})₂ (where t is 1 or 2), where each R^{a} is independently hydrogen, alkyl, fluoroalkyl, cycloalkyl, cycloalkylalkyl, aryl, aralkyl, heterocyclyl, heterocyclylalkyl, heteroaryl or heteroarylalkyl, each R^{b} is independently a direct bond or a straight or branched alkylene or alkenylene chain, and R^{c} is a straight or branched alkylene or alkenylene chain, and where each of the above substituents is unsubstituted unless otherwise indicated.

"*N*-heterocyclyl" or "N-attached heterocyclyl" refers to a heterocyclyl radical as defined above containing at least one nitrogen and where the point of attachment of the heterocyclyl radical to the rest of the molecule is through a nitrogen atom in the heterocyclyl radical. An N-heterocyclyl radical is optionally substituted as described above for heterocyclyl radicals. Examples of such *N*-heterocyclyl radicals include, but are not limited to, 1-morpholinyl, 1-piperidinyl, 1-piperazinyl, 1-pyrrolidinyl, pyrazolidinyl, imidazolinyl, and imidazolidinyl.

"*C*-heterocyclyl" or "C-attached heterocyclyl" refers to a heterocyclyl radical as defined above containing at least one heteroatom and where the point of attachment of the heterocyclyl radical to the rest of the molecule is through a carbon atom in the heterocyclyl radical. A *C*-heterocyclyl radical is optionally substituted as described above for heterocyclyl radicals. Examples of such *C*-heterocyclyl radicals include, but are not limited to, 2-morpholinyl, 2- or 3- or 4-piperidinyl, 2-piperazinyl, 2- or 3-pyrrolidinyl, and the like.

"Heterocyclylalkyl" refers to a radical of the formula -R^{c}-heterocyclyl where R^{c} is an alkylene chain as defined above. If the heterocyclyl is a nitrogen-containing heterocyclyl, the heterocyclyl is optionally attached to the alkyl radical at the nitrogen atom. The alkylene chain of the heterocyclylalkyl radical is optionally substituted as defined above for an alkylene chain. The heterocyclyl part of the heterocyclylalkyl radical is optionally substituted as defined above for a heterocyclyl group.

"Heteroaryl" refers to a radical derived from a 3- to 18-membered aromatic ring radical that comprises two to seventeen carbon atoms and from one to six heteroatoms selected from nitrogen, oxygen and sulfur. As used herein, the heteroaryl radical may be a monocyclic, bicyclic, tricyclic or tetracyclic ring system, wherein at least one of the rings in the ring system is fully unsaturated, *i.e.,* it contains a cyclic, delocalized (4n+2) π-electron system in accordance with the Hückel theory. Heteroaryl includes fused or bridged ring systems. The heteroatom(s) in the heteroaryl radical is optionally oxidized. One or more nitrogen atoms, if present, are optionally quaternized. The heteroaryl is attached to the rest of the molecule through any atom of the ring(s). Examples of heteroaryls include, but are not limited to, azepinyl, acridinyl, benzimidazolyl, benzindolyl, 1,3-benzodioxolyl, benzofuranyl, benzooxazolyl, benzo[d]thiazolyl, benzothiadiazolyl, benzo[b][1,4]dioxepinyl, benzo[b][1,4]oxazinyl, 1,4-benzodioxanyl, benzonaphthofuranyl, benzoxazolyl, benzodioxolyl, benzodioxinyl, benzopyranyl, benzopyranonyl, benzofuranyl, benzofuranonyl, benzothienyl (benzothiophenyl), benzothieno[3,2-d]pyrimidinyl, benzotriazolyl, benzo[4,6]imidazo[1,2-a]pyridinyl, carbazolyl, cinnolinyl, cyclopenta[d]pyrimidinyl, 6,7-dihydro-5H-cyclopenta[4,5]thieno[2,3-d]pyrimidinyl, 5,6-dihydrobenzo[h]quinazolinyl, 5,6-dihydrobenzo[h]cinnolinyl, 6,7-dihydro-5H-benzo[6,7]cyclohepta[1,2-c]pyridazinyl, dibenzofuranyl, dibenzothiophenyl, furanyl, furanonyl, furo[3,2-c]pyridinyl, 5,6,7,8,9,10-hexahydrocycloocta[d]pyrimidinyl, 5,6,7,8,9,10-hexahydrocycloocta[d]pyridazinyl, 5,6,7,8,9,10-hexahydrocycloocta[d]pyridinyl,isothiazolyl, imidazolyl, indazolyl, indolyl, indazolyl, isoindolyl, indolinyl, isoindolinyl, isoquinolyl, indolizinyl, isoxazolyl, 5,8-methano-5,6,7,8-tetrahydroquinazolinyl, naphthyridinyl, 1,6-naphthyridinonyl, oxadiazolyl, 2-oxoazepinyl, oxazolyl, oxiranyl, 5,6,6a,7,8,9,10,10a-octahydrobenzo[h]quinazolinyl, 1-phenyl-1*H*-pyrrolyl, phenazinyl, phenothiazinyl, phenoxazinyl, phthalazinyl, pteridinyl, purinyl, pyrrolyl, pyrazolyl, pyrazolo[3,4-d]pyrimidinyl, pyridinyl, pyrido[3,2-d]pyrimidinyl, pyrido[3,4-d]pyrimidinyl, pyrazinyl, pyrimidinyl, pyridazinyl, pyrrolyl, quinazolinyl, quinoxalinyl, quinolinyl, isoquinolinyl, tetrahydroquinolinyl, 5,6,7,8-tetrahydroquinazolinyl, 5,6,7,8-tetrahydrobenzo[4,5]thieno[2,3-d]pyrimidinyl, 6,7,8,9-tetrahydro-5H-cyclohepta[4,5]thieno[2,3-d]pyrimidinyl, 5,6,7,8-tetrahydropyrido[4,5-c]pyridazinyl, thiazolyl, thiadiazolyl, triazolyl, tetrazolyl, triazinyl, thieno[2,3-d]pyrimidinyl, thieno[3,2-d]pyrimidinyl, thieno[2,3-c]pridinyl, and thiophenyl (*i.e.* thienyl). Unless stated otherwise specifically in the specification, the term "heteroaryl" is meant to include heteroaryl radicals as defined above which are optionally substituted by one or more substituents selected from alkyl, alkenyl, alkynyl, halo, fluoroalkyl, haloalkenyl, haloalkynyl, oxo, thioxo, cyano, nitro, optionally substituted aryl, optionally substituted aralkyl, optionally substituted aralkenyl, optionally substituted aralkynyl, optionally substituted carbocyclyl, optionally substituted carbocyclylalkyl, optionally substituted heterocyclyl, optionally substituted heterocyclylalkyl, optionally substituted heteroaryl, optionally substituted heteroarylalkyl, -R^{b}-OR^{a}, -R^{b}-SR^{a}, -R^{b}-OC(O)-R^{a}, -R^{b}-N(R^{a})₂, -R^{b}-C(O)R^{a}, -R^{b}-C(O)OR^{a}, -R^{b} -C(O)N(R^{a})₂, -R^{b}-O-R^{c}-C(O)N(R^{a})₂, -R^{b}-N(R^{a})C(O)OR^{a}, -R^{b}-N(R^{a})C(O)R^{a}, -R^{b}-N(R^{a})S(O)ₜR ^{a} (where t is 1 or 2), -R^{b}-S(O)ₜOR^{a} (where t is 1 or 2) and -R^{b}-S(O)ₜN(R^{a})₂ (where t is 1 or 2), where each R^{a} is independently hydrogen, alkyl, fluoroalkyl, cycloalkyl, cycloalkylalkyl, aryl, aralkyl, heterocyclyl, heterocyclylalkyl, heteroaryl or heteroarylalkyl, each R^{b} is independently a direct bond or a straight or branched alkylene or alkenylene chain, and R^{c} is a straight or branched alkylene or alkenylene chain, and where each of the above substituents is unsubstituted unless otherwise indicated.

"*N*-heteroaryl" refers to a heteroaryl radical as defined above containing at least one nitrogen and where the point of attachment of the heteroaryl radical to the rest of the molecule is through a nitrogen atom in the heteroaryl radical. An N-heteroaryl radical is optionally substituted as described above for heteroaryl radicals.

"*C*-heteroaryl" refers to a heteroaryl radical as defined above and where the point of attachment of the heteroaryl radical to the rest of the molecule is through a carbon atom in the heteroaryl radical. A C-heteroaryl radical is optionally substituted as described above for heteroaryl radicals.

"Heteroarylalkyl" refers to a radical of the formula -R^{c}-heteroaryl, where R^{c} is an alkylene chain as defined above. If the heteroaryl is a nitrogen-containing heteroaryl, the heteroaryl is optionally attached to the alkyl radical at the nitrogen atom. The alkylene chain of the heteroarylalkyl radical is optionally substituted as defined above for an alkylene chain. The heteroaryl part of the heteroarylalkyl radical is optionally substituted as defined above for a heteroaryl group.

The compounds, or their pharmaceutically acceptable salts may contain one or more asymmetric centers and may thus give rise to enantiomers, diastereomers, and other stereoisomeric forms that may be defined, in terms of absolute stereochemistry, as (*R*)- or (*S*)- or, as (D)- or (L)- for amino acids. When the compounds described herein contain olefinic double bonds or other centers of geometric asymmetry, and unless specified otherwise, it is intended that the compounds include both *E* (or *trans*) and Z (*cis*) geometric isomers. Likewise, all possible isomers, as well as their racemic and optically pure forms, and all tautomeric forms are also intended to be included.

A "stereoisomer" refers to a compound made up of the same atoms bonded by the same bonds but having different three-dimensional structures, which are not interchangeable. It is therefore contemplated that various stereoisomers and mixtures thereof and includes "enantiomers," which refers to two stereoisomers whose molecules are nonsuperimposeable mirror images of one another.

A "tautomer" refers to a proton shift from one atom of a molecule to another atom of the same molecule. The compounds presented herein may exist as tautomers. Tautomers are compounds that are interconvertible by migration of a hydrogen atom, accompanied by a switch of a single bond and adjacent double bond. In solutions where tautomerization is possible, a chemical equilibrium of the tautomers will exist. The exact ratio of the tautomers depends on several factors, including temperature, solvent, and pH. Some examples of tautomeric pairs include:

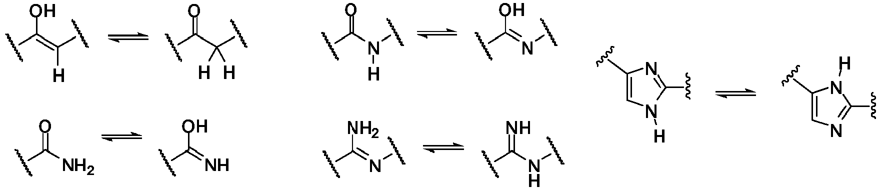

"Optional" or "optionally" means that a subsequently described event or circumstance may or may not occur and that the description includes instances when the event or circumstance occurs and instances in which it does not.

"Pharmaceutically acceptable salt" includes both acid and base addition salts. A pharmaceutically acceptable salt of any one of the alkoxyphenyl-linked amine derivative compounds described herein is intended to encompass any and all pharmaceutically suitable salt forms. Preferred pharmaceutically acceptable salts of the compounds described herein are pharmaceutically acceptable acid addition salts and pharmaceutically acceptable base addition salts.

"Pharmaceutically acceptable acid addition salt" refers to those salts which retain the biological effectiveness and properties of the free bases, which are not biologically or otherwise undesirable, and which are formed with inorganic acids such as hydrochloric acid, hydrobromic acid, sulfuric acid, nitric acid, phosphoric acid, hydroiodic acid, hydrofluoric acid, phosphorous acid, and the like. Also included are salts that are formed with organic acids such as aliphatic mono- and dicarboxylic acids, phenyl-substituted alkanoic acids, hydroxy alkanoic acids, alkanedioic acids, aromatic acids, aliphatic and. aromatic sulfonic acids, etc. and include, for example, acetic acid, trifluoroacetic acid, propionic acid, glycolic acid, pyruvic acid, oxalic acid, maleic acid, malonic acid, succinic acid, fumaric acid, tartaric acid, citric acid, benzoic acid, cinnamic acid, mandelic acid, methanesulfonic acid, ethanesulfonic acid, p-toluenesulfonic acid, salicylic acid, and the like. Exemplary salts thus include sulfates, pyrosulfates, bisulfates, sulfites, bisulfites, nitrates, phosphates, monohydrogenphosphates, dihydrogenphosphates, metaphosphates, pyrophosphates, chlorides, bromides, iodides, acetates, trifluoroacetates, propionates, caprylates, isobutyrates, oxalates, malonates, succinate suberates, sebacates, fumarates, maleates, mandelates, benzoates, chlorobenzoates, methylbenzoates, dinitrobenzoates, phthalates, benzenesulfonates, toluenesulfonates, phenylacetates, citrates, lactates, malates, tartrates, methanesulfonates, and the like. Also contemplated are salts of amino acids, such as arginates, gluconates, and galacturonates (see, for example, Berge S.M. et al., "Pharmaceutical Salts," Journal of Pharmaceutical Science, 66: 1-19 (1997)).

Acid addition salts of basic compounds may be prepared by contacting the free base forms with a sufficient amount of the desired acid to produce the salt according to methods and techniques with which a skilled artisan is familiar.

"Pharmaceutically acceptable base addition salt" refers to those salts that retain the biological effectiveness and properties of the free acids, which are not biologically or otherwise undesirable. These salts are prepared from addition of an inorganic base or an organic base to the free acid. Pharmaceutically acceptable base addition salts may be formed with metals or amines, such as alkali and alkaline earth metals or organic amines. Salts derived from inorganic bases include, but are not limited to, sodium, potassium, lithium, ammonium, calcium, magnesium, iron, zinc, copper, manganese, aluminum salts and the like. Salts derived from organic bases include, but are not limited to, salts of primary, secondary, and *tert*iary amines, substituted amines including naturally occurring substituted amines, cyclic amines and basic ion exchange resins, for example, isopropylamine, trimethylamine, diethylamine, triethylamine, tripropylamine, ethanolamine, diethanolamine, 2-dimethylaminoethanol, 2-diethylaminoethanol, dicyclohexylamine, lysine, arginine, histidine, caffeine, procaine, *N,N-*dibenzylethylenediamine, chloroprocaine, hydrabamine, choline, betaine, ethylenediamine, ethylenedianiline, *N*-methylglucamine, glucosamine, methylglucamine, theobromine, purines, piperazine, piperidine, *N*-ethylpiperidine, polyamine resins and the like. See Berge et al., *supra.*

As used herein, "treatment" or "treating," or "palliating" or "ameliorating" are used interchangeably herein. These terms refers to an approach for obtaining beneficial or desired results including but not limited to therapeutic benefit and/or a prophylactic benefit. By "therapeutic benefit" is meant eradication or amelioration of the underlying disorder being treated. Also, a therapeutic benefit is achieved with the eradication or amelioration of one or more of the physiological symptoms associated with the underlying disorder such that an improvement is observed in the patient, notwithstanding that the patient may still be afflicted with the underlying disorder. For prophylactic benefit, the compositions may be administered to a patient at risk of developing a particular disease, or to a patient reporting one or more of the physiological symptoms of a disease, even though a diagnosis of this disease may not have been made.

"Prodrug" is meant to indicate a compound that may be converted under physiological conditions or by solvolysis to a biologically active compound described herein. Thus, the term "prodrug" refers to a precursor of a biologically active compound that is pharmaceutically acceptable. A prodrug may be inactive when administered to a subject, but is converted *in vivo* to an active compound, for example, by hydrolysis. The prodrug compound often offers advantages of solubility, tissue compatibility or delayed release in a mammalian organism (*see,* e.g., Bundgard, H., Design of Prodrugs (1985), pp. 7-9, 21-24 (Elsevier, Amsterdam).

A discussion of prodrugs is provided in Higuchi, T., et al., "Pro-drugs as Novel Delivery Systems," A.C.S. Symposium Series, Vol. 14, and in Bioreversible Carriers in Drug Design, ed. Edward B. Roche, American Pharmaceutical Association and Pergamon Press, 1987.

The term "prodrug" is also meant to include any covalently bonded carriers, which release the active compound *in vivo* when such prodrug is administered to a mammalian subject. Prodrugs of an active compound, as described herein, may be prepared by modifying functional groups present in the active compound in such a way that the modifications are cleaved, either in routine manipulation or *in vivo,* to the parent active compound. Prodrugs include compounds wherein a hydroxy, amino or mercapto group is bonded to any group that, when the prodrug of the active compound is administered to a mammalian subject, cleaves to form a free hydroxy, free amino or free mercapto group, respectively. Examples of prodrugs include, but are not limited to, acetate, formate and benzoate derivatives of alcohol or amine functional groups in the active compounds and the like.

### Conjugate Compounds

The present disclosure provides compounds that selectively bind to cancerous cells and tissues. In various aspects, the compounds of the present disclosure comprise a peptide portion and a detectable agent conjugated together.

In various aspects of the present disclosure, the peptide portions of the compounds described herein have certain features in common with the native chlorotoxin (CTX) peptide. The native chlorotoxin peptide was originally isolated from the scorpion *Leiurus quinquestriatus.* Chlorotoxin is a 36 amino acid peptide that selectively binds to cancerous cells. The peptide portions of the present compounds have advantageously retained at least some of the cancer-cell binding activity of chlorotoxin. The cancer-cell binding activity of chlorotoxin provides certain advantages for the detection and treatment of cancer because it facilitates the selective localization of detectable agents and therapeutic agents to the cancer cells for the detection and treatment of cancer.

Table 1 below sets forth certain polypeptide sequences for use with the present disclosure. Citrulline is designated as"Cit" in the sequences.

**Table 1. Exemplary peptide sequences suitable for use in the compounds of the present disclosure. Cit = Citrulline.**

| **SEQ ID NO.** | **Polypeptide Sequence** |
|---|---|
| 1 | MCMPCFTTDHQMARKCDDCCGGKGRGKCYGPQCLCR |
| 2 | MCMPCFTTDHQMARACDDCCGGKGRGKCYGPQCLCR |
| 3 | MCMPCFTTDHQMARRCDDCCGGKGRGKCYGPQCLCR |
| 4 | MCMPCFTTDHQMARKCDDCCGGAGRGKCYGPQCLCR |
| 5 | MCMPCFTTDHQMARACDDCCGGAGRGKCYGPQCLCR |
| 6 | MCMPCFTTDHQMARRCDDCCGGAGRGKCYGPQCLCR |
| 7 | MCMPCFTTDHQMARKCDDCCGGRGRGKCYGPQCLCR |
| 8 | MCMPCFTTDHQMARACDDCCGGRGRGKCYGPQCLCR |
| 9 | MCMPCFTTDHQMARRCDDCCGGRGRGKCYGPQCLCR |
| 10 | MCMPCFTTDHQMARKCDDCCGGKGRGACYGPQCLCR |
| 11 | MCMPCFTTDHQMARACDDCCGGKGRGACYGPQCLCR |
| 12 | MCMPCFTTDHQMARRCDDCCGGKGRGACYGPQCLCR |
| 13 | MCMPCFTTDHQMARKCDDCCGGAGRGACYGPQCLCR |
| 14 | MCMPCFTTDHQMARACDDCCGGAGRGACYGPQCLCR |
| 15 | MCMPCFTTDHQMARRCDDCCGGAGRGACYGPQCLCR |
| 16 | MCMPCFTTDHQMARKCDDCCGGRGRGACYGPQCLCR |
| 17 | MCMPCFTTDHQMARACDDCCGGRGRGACYGPQCLCR |
| 18 | MCMPCFTTDHQMARRCDDCCGGRGRGACYGPQCLCR |
| 19 | MCMPCFTTDHQMARKCDDCCGGKGRGRCYGPQCLCR |
| 20 | MCMPCFTTDHQMARACDDCCGGKGRGRCYGPQCLCR |
| 21 | MCMPCFTTDHQMARRCDDCCGGKGRGRCYGPQCLCR |
| 22 | MCMPCFTTDHQMARKCDDCCGGAGRGRCYGPQCLCR |
| 23 | MCMPCFTTDHQMARACDDCCGGAGRGRCYGPQCLCR |
| 24 | MCMPCFTTDHQMARRCDDCCGGAGRGRCYGPQCLCR |
| 25 | MCMPCFTTDHQMARKCDDCCGGRGRGRCYGPQCLCR |
| 26 | MCMPCFTTDHQMARACDDCCGGRGRGRCYGPQCLCR |
| 27 | MCMPCFTTDHQMARRCDDCCGGRGRGRCYGPQCLCR |
| 28 | MCMPCFTTDHQMARRCDDCCGGRGRGRCYGPQCLCR |
| 29 | KCMPCFTTDHQMARRCDDCCGGRGRGRCYGPQCLCR |
| 30 | ACAPCFTTDHQAARRCDDCCGGRGRGRCYGPQCLCR |
| 31 | KCAPCFTTDHQAARRCDDCCGGRGRGRCYGPQCLCR |
| 32 | MCMPCFTTDHQMAR(Cit)CDDCCGG(Cit)GRGKCYGPQCLCR |
| 33 | MCMPCFTTDHQMAR(Cit)CDDCCGG(Cit)GRG(Cit)CYGPQCLCR |
| 34 | KCMPCFTTDHQMAR(Cit)CDDCCGG(Cit)GRG(Cit)CYGPQCLCR |
| 35 | ACAPCFTTDHQAAR(Cit)CDDCCGG(Cit)GRGKCYGPQCLCR |
| 36 | ACAPCFTTDHQAAR(Cit)CDDCCGG(Cit)GRG(Cit)CYGPQCLCR |
| 37 | KCAPCFTTDHQAAR(Cit)CDDCCGG(Cit)GRG(Cit)CYGPQCLCR |
| 38 | MCMPCFTTDHQMARKCDDCCGGKGRGKCYGPQCLCRGAGAAGG |
| 39 | MCMPCFTTDHQMARACDDCCGGKGRGKCYGPQCLCRGAGAAGG |
| 40 | MCMPCFTTDHQMARRCDDCCGGKGRGKCYGPQCLCRGAGAAGG |
| 41 | MCMPCFTTDHQMARKCDDCCGGAGRGKCYGPQCLCRGAGAAGG |
| 42 | MCMPCFTTDHQMARACDDCCGGAGRGKCYGPQCLCRGAGAAGG |
| 43 | MCMPCFTTDHQMARRCDDCCGGAGRGKCYGPQCLCRGAGAAGG |
| 44 | MCMPCFTTDHQMARKCDDCCGGRGRGKCYGPQCLCRGAGAAGG |
| 45 | MCMPCFTTDHQMARACDDCCGGRGRGKCYGPQCLCRGAGAAGG |
| 46 | MCMPCFTTDHQMARRCDDCCGGRGRGKCYGPQCLCRGAGAAGG |
| 47 | MCMPCFTTDHQMARKCDDCCGGKGRGACYGPQCLCRGAGAAGG |
| 48 | MCMPCFTTDHQMARACDDCCGGKGRGACY GPQCLCRGAGAAGG |
| 49 | MCMPCFTTDHQMARRCDDCCGGKGRGACYGPQCLCRGAGAAGG |
| 50 | MCMPCFTTDHQMARKCDDCCGGAGRGACYGPQCLCRGAGAAGG |
| 51 | MCMPCFTTDHQMARACDDCCGGAGRGACYGPQCLCRGAGAAGG |
| 52 | MCMPCFTTDHQMARRCDDCCGGAGRGACYGPQCLCRGAGAAGG |
| 53 | MCMPCFTTDHQMARKCDDCCGGRGRGACYGPQCLCRGAGAAGG |
| 54 | MCMPCFTTDHQMARACDDCCGGRGRGACYGPQCLCRGAGAAGG |
| 55 | MCMPCFTTDHQMARRCDDCCGGRGRGACYGPQCLCRGAGAAGG |
| 56 | MCMPCFTTDHQMARKCDDCCGGKGRGRCYGPQCLCRGAGAAGG |
| 57 | MCMPCFTTDHQMARACDDCCGGKGRGRCYGPQCLCRGAGAAGG |
| 58 | MCMPCFTTDHQMARRCDDCCGGKGRGRCYGPQCLCRGAGAAGG |
| 59 | MCMPCFTTDHQMARKCDDCCGGAGRGRCYGPQCLCRGAGAAGG |
| 60 | MCMPCFTTDHQMARACDDCCGGAGRGRCYGPQCLCRGAGAAGG |
| 61 | MCMPCFTTDHQMARRCDDCCGGAGRGRCYGPQCLCRGAGAAGG |
| 62 | MCMPCFTTDHQMARKCDDCCGGRGRGRCYGPQCLCRGAGAAGG |
| 63 | MCMPCFTTDHQMARACDDCCGGRGRGRCYGPQCLCRGAGAAGG |
| 64 | MCMPCFTTDHQMARRCDDCCGGRGRGRCYGPQCLCRGAGAAGG |
| 65 | MCMPCFTTDHQMARRCDDCCGGRGRGRCYGPQCLCRGAGAAGG |
| 66 | KCMPCFTTDHQMARRCDDCCGGRGRGRCYGPQCLCRGAGAAGG |
| 67 | ACAPCFTTDHQAARRCDDCCGGRGRGRCYGPQCLCRGAGAAGG |
| 68 | KCAPCFTTDHQAARRCDDCCGGRGRGRCYGPQCLCRGAGAAGG |
| 69 | MCMPCFTTDHQMAR(Cit)CDDCCGG(Cit)GRGKCYGPQCLCRGAGAAGG |
| 70 | MCMPCFTTDHQMAR(Cit)CDDCCGG(Cit)GRG(Cit)CYGPQCLCRGAGAAGG |
| 71 | KCMPCFTTDHQMAR(Cit)CDDCCGG(Cit)GRG(Cit)CYGPQCLCRGAGAAGG |
| 72 | ACAPCFTTDHQAAR(Cit)CDDCCGG(Cit)GRGKCYGPQCLCRGAGAAGG |
| 73 | ACAPCFTTDHQAAR(Cit)CDDCCGG(Cit)GRG(Cit)CYGPQCLCRGAGAAGG |
| 74 | KCAPCFTTDHQAAR(Cit)CDDCCGG(Cit)GRG(Cit)CYGPQCLCRGAGAAGG |
| 75 | MCMPCFTTDHQMVRKCDDCCGGKGRGKCYGPQCLCR |
| 76 | MCMPCFTTDHQMVRVCDDCCGGKGRGKCYGPQCLCR |
| 77 | MCMPCFTTDHQMVRRCDDCCGGKGRGKCYGPQCLCR |
| 78 | MCMPCFTTDHQMVRKCDDCCGGVGRGKCYGPQCLCR |
| 79 | MCMPCFTTDHQMVRVCDDCCGGVGRGKCYGPQCLCR |
| 80 | MCMPCFTTDHQMVRRCDDCCGGVGRGKCYGPQCLCR |
| 81 | MCMPCFTTDHQMVRKCDDCCGGRGRGKCYGPQCLCR |
| 82 | MCMPCFTTDHQMVRVCDDCCGGRGRGKCYGPQCLCR |
| 83 | MCMPCFTTDHQMVRRCDDCCGGRGRGKCYGPQCLCR |
| 84 | MCMPCFTTDHQMVRKCDDCCGGKGRGVCYGPQCLCR |
| 85 | MCMPCFTTDHQMVRVCDDCCGGKGRGVCYGPQCLCR |
| 86 | MCMPCFTTDHQMVRRCDDCCGGKGRGVCYGPQCLCR |
| 87 | MCMPCFTTDHQMVRKCDDCCGGVGRGVCYGPQCLCR |
| 88 | MCMPCFTTDHQMVRVCDDCCGGVGRGVCYGPQCLCR |
| 89 | MCMPCFTTDHQMVRRCDDCCGGVGRGVCYGPQCLCR |
| 90 | MCMPCFTTDHQMVRKCDDCCGGRGRGVCYGPQCLCR |
| 91 | MCMPCFTTDHQMVRVCDDCCGGRGRGVCYGPQCLCR |
| 92 | MCMPCFTTDHQMVRRCDDCCGGRGRGVCYGPQCLCR |
| 93 | MCMPCFTTDHQMVRKCDDCCGGKGRGRCYGPQCLCR |
| 94 | MCMPCFTTDHQMVRVCDDCCGGKGRGRCYGPQCLCR |
| 95 | MCMPCFTTDHQMVRRCDDCCGGKGRGRCYGPQCLCR |
| 96 | MCMPCFTTDHQMVRKCDDCCGGVGRGRCYGPQCLCR |
| 97 | MCMPCFTTDHQMVRVCDDCCGGVGRGRCYGPQCLCR |
| 98 | MCMPCFTTDHQMVRRCDDCCGGVGRGRCYGPQCLCR |
| 99 | MCMPCFTTDHQMVRKCDDCCGGRGRGRCYGPQCLCR |
| 100 | MCMPCFTTDHQMVRVCDDCCGGRGRGRCYGPQCLCR |
| 101 | MCMPCFTTDHQMVRRCDDCCGGRGRGRCYGPQCLCR |
| 102 | MCMPCFTTDHQMVRRCDDCCGGRGRGRCYGPQCLCR |
| 103 | KCMPCFTTDHQMVRRCDDCCGGRGRGRCYGPQCLCR |
| 104 | VCVPCFTTDHQVVRRCDDCCGGRGRGRCYGPQCLCR |
| 105 | KCVPCFTTDHQVVRRCDDCCGGRGRGRCYGPQCLCR |
| 106 | MCMPCFTTDHQMVR(Cit)CDDCCGG(Cit)GRGKCYGPQCLCR |
| 107 | MCMPCFTTDHQMVR(Cit)CDDCCGG(Cit)GRG(Cit)CYGPQCLCR |
| 108 | KCMPCFTTDHQMVR(Cit)CDDCCGG(Cit)GRG(Cit)CYGPQCLCR |
| 109 | VCVPCFTTDHQVVR(Cit)CDDCCGG(Cit)GRGKCYGPQCLCR |
| 110 | VCVPCFTTDHQVVR(Cit)CDDCCGG(Cit)GRG(Cit)CYGPQCLCR |
| 111 | KCVPCFTTDHQVVR(Cit)CDDCCGG(Cit)GRG(Cit)CYGPQCLCR |
| 112 | MCMPCFTTDHQMVRKCDDCCGGKGRGKCYGPQCLCRGAGAAGG |
| 113 | MCMPCFTTDHQMVRVCDDCCGGKGRGKCYGPQCLCRGAGAAGG |
| 114 | MCMPCFTTDHQMVRRCDDCCGGKGRGKCYGPQCLCRGAGAAGG |
| 115 | MCMPCFTTDHQMVRKCDDCCGGVGRGKCYGPQCLCRGAGAAGG |
| 116 | MCMPCFTTDHQMVRVCDDCCGGVGRGKCYGPQCLCRGAGAAGG |
| 117 | MCMPCFTTDHQMVRRCDDCCGGVGRGKCYGPQCLCRGAGAAGG |
| 118 | MCMPCFTTDHQMVRKCDDCCGGRGRGKCYGPQCLCRGAGAAGG |
| 119 | MCMPCFTTDHQMVRVCDDCCGGRGRGKCYGPQCLCRGAGAAGG |
| 120 | MCMPCFTTDHQMVRRCDDCCGGRGRGKCYGPQCLCRGAGAAGG |
| 121 | MCMPCFTTDHQMVRKCDDCCGGKGRGVCYGPQCLCRGAGAAGG |
| 122 | MCMPCFTTDHQMVRVCDDCCGGKGRGVCYGPQCLCRGAGAAGG |
| 123 | MCMPCFTTDHQMVRRCDDCCGGKGRGVCYGPQCLCRGAGAAGG |
| 124 | MCMPCFTTDHQMVRKCDDCCGGVGRGVCYGPQCLCRGAGAAGG |
| 125 | MCMPCFTTDHQMVRVCDDCCGGVGRGVCYGPQCLCRGAGAAGG |
| 126 | MCMPCFTTDHQMVRRCDDCCGGVGRGVCYGPQCLCRGAGAAGG |
| 127 | MCMPCFTTDHQMVRKCDDCCGGRGRGVCYGPQCLCRGAGAAGG |
| 128 | MCMPCFTTDHQMVRVCDDCCGGRGRGVCYGPQCLCRGAGAAGG |
| 129 | MCMPCFTTDHQMVRRCDDCCGGRGRGVCYGPQCLCRGAGAAGG |
| 130 | MCMPCFTTDHQMVRKCDDCCGGKGRGRCYGPQCLCRGAGAAGG |
| 131 | MCMPCFTTDHQMVRVCDDCCGGKGRGRCYGPQCLCRGAGAAGG |
| 132 | MCMPCFTTDHQMVRRCDDCCGGKGRGRCYGPQCLCRGAGAAGG |
| 133 | MCMPCFTTDHQMVRKCDDCCGGVGRGRCYGPQCLCRGAGAAGG |
| 134 | MCMPCFTTDHQMVRVCDDCCGGVGRGRCYGPQCLCRGAGAAGG |
| 135 | MCMPCFTTDHQMVRRCDDCCGGVGRGRCYGPQCLCRGAGAAGG |
| 136 | MCMPCFTTDHQMVRKCDDCCGGRGRGRCYGPQCLCRGAGAAGG |
| 137 | MCMPCFTTDHQMVRVCDDCCGGRGRGRCYGPQCLCRGAGAAGG |
| 138 | MCMPCFTTDHQMVRRCDDCCGGRGRGRCYGPQCLCRGAGAAGG |
| 139 | MCMPCFTTDHQMVRRCDDCCGGRGRGRCYGPQCLCRGAGAAGG |
| 140 | KCMPCFTTDHQMVRRCDDCCGGRGRGRCYGPQCLCRGAGAAGG |
| 141 | VCVPCFTTDHQVVRRCDDCCGGRGRGRCYGPQCLCRGAGAAGG |
| 142 | KCVPCFTTDHQVVRRCDDCCGGRGRGRCYGPQCLCRGAGAAGG |
| 143 | MCMPCFTTDHQMVR(Cit)CDDCCGG(Cit)GRGKCYGPQCLCRGAGAAGG |
| 144 | MCMPCFTTDHQMVR(Cit)CDDCCGG(Cit)GRG(Cit)CYGPQCLCRGAGAAGG |
| 145 | KCMPCFTTDHQMVR(Cit)CDDCCGG(Cit)GRG(Cit)CYGPQCLCRGAGAAGG |
| 146 | VCVPCFTTDHQVVR(Cit)CDDCCGG(Cit)GRGKCYGPQCLCRGAGAAGG |
| 147 | VCVPCFTTDHQVVR(Cit)CDDCCGG(Cit)GRG(Cit)CYGPQCLCRGAGAAGG |
| 148 | KCVPCFTTDHQVVR(Cit)CDDCCGG(Cit)GRG(Cit)CYGPQCLCRGAGAAGG |
| 149 | MCMPCFTTDHQMLRKCDDCCGGKGRGKCYGPQCLCR |
| 150 | MCMPCFTTDHQMLRLCDDCCGGKGRGKCYGPQCLCR |
| 151 | MCMPCFTTDHQMLRRCDDCCGGKGRGKCYGPQCLCR |
| 152 | MCMPCFTTDHQMLRKCDDCCGGLGRGKCYGPQCLCR |
| 153 | MCMPCFTTDHQMLRLCDDCCGGLGRGKCYGPQCLCR |
| 154 | MCMPCFTTDHQMLRRCDDCCGGLGRGKCYGPQCLCR |
| 155 | MCMPCFTTDHQMLRKCDDCCGGRGRGKCYGPQCLCR |
| 156 | MCMPCFTTDHQMLRLCDDCCGGRGRGKCYGPQCLCR |
| 157 | MCMPCFTTDHQMLRRCDDCCGGRGRGKCYGPQCLCR |
| 158 | MCMPCFTTDHQMLRKCDDCCGGKGRGLCYGPQCLCR |
| 159 | MCMPCFTTDHQMLRLCDDCCGGKGRGLCYGPQCLCR |
| 160 | MCMPCFTTDHQMLRRCDDCCGGKGRGLCYGPQCLCR |
| 161 | MCMPCFTTDHQMLRKCDDCCGGLGRGLCYGPQCLCR |
| 162 | MCMPCFTTDHQMLRLCDDCCGGLGRGLCYGPQCLCR |
| 163 | MCMPCFTTDHQMLRRCDDCCGGLGRGLCYGPQCLCR |
| 164 | MCMPCFTTDHQMLRKCDDCCGGRGRGLCYGPQCLCR |
| 165 | MCMPCFTTDHQMLRLCDDCCGGRGRGLCYGPQCLCR |
| 166 | MCMPCFTTDHQMLRRCDDCCGGRGRGLCYGPQCLCR |
| 167 | MCMPCFTTDHQMLRKCDDCCGGKGRGRCYGPQCLCR |
| 168 | MCMPCFTTDHQMLRLCDDCCGGKGRGRCYGPQCLCR |
| 169 | MCMPCFTTDHQMLRRCDDCCGGKGRGRCYGPQCLCR |
| 170 | MCMPCFTTDHQMLRKCDDCCGGLGRGRCYGPQCLCR |
| 171 | MCMPCFTTDHQMLRLCDDCCGGLGRGRCYGPQCLCR |
| 172 | MCMPCFTTDHQMLRRCDDCCGGLGRGRCYGPQCLCR |
| 173 | MCMPCFTTDHQMLRKCDDCCGGRGRGRCYGPQCLCR |
| 174 | MCMPCFTTDHQMLRLCDDCCGGRGRGRCYGPQCLCR |
| 175 | MCMPCFTTDHQMLRRCDDCCGGRGRGRCYGPQCLCR |
| 176 | MCMPCFTTDHQMLRRCDDCCGGRGRGRCYGPQCLCR |
| 177 | KCMPCFTTDHQMLRRCDDCCGGRGRGRCYGPQCLCR |
| 178 | LCLPCFTTDHQLLRRCDDCCGGRGRGRCYGPQCLCR |
| 179 | KCLPCFTTDHQLLRRCDDCCGGRGRGRCYGPQCLCR |
| 180 | MCMPCFTTDHQMLR(Cit)CDDCCGG(Cit)GRGKCYGPQCLCR |
| 181 | MCMPCFTTDHQMLR(Cit)CDDCCGG(Cit)GRG(Cit)CYGPQCLCR |
| 182 | KCMPCFTTDHQMLR(Cit)CDDCCGG(Cit)GRG(Cit)CYGPQCLCR |
| 183 | LCLPCFTTDHQLLR(Cit)CDDCCGG(Cit)GRGKCYGPQCLCR |
| 184 | LCLPCFTTDHQLLR(Cit)CDDCCGG(Cit)GRG(Cit)CYGPQCLCR |
| 185 | KCLPCFTTDHQLLR(Cit)CDDCCGG(Cit)GRG(Cit)CYGPQCLCR |
| 186 | MCMPCFTTDHQMLRKCDDCCGGKGRGKCYGPQCLCRGAGAAGG |
| 187 | MCMPCFTTDHQMLRLCDDCCGGKGRGKCYGPQCLCRGAGAAGG |
| 188 | MCMPCFTTDHQMLRRCDDCCGGKGRGKCYGPQCLCRGAGAAGG |
| 189 | MCMPCFTTDHQMLRKCDDCCGGLGRGKCYGPQCLCRGAGAAGG |
| 190 | MCMPCFTTDHQMLRLCDDCCGGLGRGKCYGPQCLCRGAGAAGG |
| 191 | MCMPCFTTDHQMLRRCDDCCGGLGRGKCYGPQCLCRGAGAAGG |
| 192 | MCMPCFTTDHQMLRKCDDCCGGRGRGKCYGPQCLCRGAGAAGG |
| 193 | MCMPCFTTDHQMLRLCDDCCGGRGRGKCYGPQCLCRGAGAAGG |
| 194 | MCMPCFTTDHQMLRRCDDCCGGRGRGKCYGPQCLCRGAGAAGG |
| 195 | MCMPCFTTDHQMLRKCDDCCGGKGRGLCYGPQCLCRGAGAAGG |
| 196 | MCMPCFTTDHQMLRLCDDCCGGKGRGLCYGPQCLCRGAGAAGG |
| 197 | MCMPCFTTDHQMLRRCDDCCGGKGRGLCYGPQCLCRGAGAAGG |
| 198 | MCMPCFTTDHQMLRKCDDCCGGLGRGLCYGPQCLCRGAGAAGG |
| 199 | MCMPCFTTDHQMLRLCDDCCGGLGRGLCYGPQCLCRGAGAAGG |
| 200 | MCMPCFTTDHQMLRRCDDCCGGLGRGLCYGPQCLCRGAGAAGG |
| 201 | MCMPCFTTDHQMLRKCDDCCGGRGRGLCYGPQCLCRGAGAAGG |
| 202 | MCMPCFTTDHQMLRLCDDCCGGRGRGLCYGPQCLCRGAGAAGG |
| 203 | MCMPCFTTDHQMLRRCDDCCGGRGRGLCYGPQCLCRGAGAAGG |
| 204 | MCMPCFTTDHQMLRKCDDCCGGKGRGRCYGPQCLCRGAGAAGG |
| 205 | MCMPCFTTDHQMLRLCDDCCGGKGRGRCYGPQCLCRGAGAAGG |
| 206 | MCMPCFTTDHQMLRRCDDCCGGKGRGRCYGPQCLCRGAGAAGG |
| 207 | MCMPCFTTDHQMLRKCDDCCGGLGRGRCYGPQCLCRGAGAAGG |
| 208 | MCMPCFTTDHQMLRLCDDCCGGLGRGRCYGPQCLCRGAGAAGG |
| 209 | MCNVCFTTDHQMLRRCDDCCGGLGRGRCYGPQCLCRGAGAAGG |
| 210 | MCMPCFTTDHQMLRKCDDCCGGRGRGRCYGPQCLCRGAGAAGG |
| 211 | MCMPCFTTDHQMLRLCDDCCGGRGRGRCYGPQCLCRGAGAAGG |
| 212 | MCMPCFTTDHQMLRRCDDCCGGRGRGRCYGPQCLCRGAGAAGG |
| 213 | MCMPCFTTDHQMLRRCDDCCGGRGRGRCYGPQCLCRGAGAAGG |
| 214 | KCMPCFTTDHQMLRRCDDCCGGRGRGRCYGPQCLCRGAGAAGG |
| 215 | LCLPCFTTDHQLLRRCDDCCGGRGRGRCYGPQCLCRGAGAAGG |
| 216 | KCLPCFTTDHQLLRRCDDCCGGRGRGRCYGPQCLCRGAGAAGG |
| 217 | MCMPCFTTDHQMLR(Cit)CDDCCGG(Cit)GRGKCYGPQCLCRGAGAAGG |
| 218 | MCMPCFTTDHQMLR(Cit)CDDCCGG(Cit)GRG(Cit)CYGPQCLCRGAGAAGG |
| 219 | KCMPCFTTDHQMLR(Cit)CDDCCGG(Cit)GRG(Cit)CYGPQCLCRGAGAAGG |
| 220 | LCLPCFTTDHQLLR(Cit)CDDCCGG(Cit)GRGKCYGPQCLCRGAGAAGG |
| 221 | LCLPCFTTDHQLLR(Cit)CDDCCGG(Cit)GRG(Cit)CYGPQCLCRGAGAAGG |
| 222 | KCLPCFTTDHQLLR(Cit)CDDCCGG(Cit)GRG(Cit)CYGPQCLCRGAGAAGG |
| 223 | GCGPCFTTDHQGARKCDDCCGGKGRGKCYGPQCLCR |
| 224 | GCGPCFTTDHQGARACDDCCGGKGRGKCYGPQCLCR |
| 225 | GCGPCFTTDHQGARRCDDCCGGKGRGKCYGPQCLCR |
| 226 | GCGPCFTTDHQGARKCDDCCGGAGRGKCYGPQCLCR |
| 227 | GCGPCFTTDHQGARACDDCCGGAGRGKCYGPQCLCR |
| 228 | GCGPCFTTDHQGARRCDDCCGGAGRGKCYGPQCLCR |
| 229 | GCGPCFTTDHQGARKCDDCCGGRGRGKCYGPQCLCR |
| 230 | GCGPCFTTDHQGARACDDCCGGRGRGKCYGPQCLCR |
| 231 | GCGPCFTTDHQGARRCDDCCGGRGRGKCYGPQCLCR |
| 232 | GCGPCFTTDHQGARKCDDCCGGKGRGACYGPQCLCR |
| 233 | GCGPCFTTDHQGARACDDCCGGKGRGACYGPQCLCR |
| 234 | GCGPCFTTDHQGARRCDDCCGGKGRGACYGPQCLCR |
| 235 | GCGPCFTTDHQGARKCDDCCGGAGRGACYGPQCLCR |
| 236 | GCGPCFTTDHQGARACDDCCGGAGRGACYGPQCLCR |
| 237 | GCGPCFTTDHQGARRCDDCCGGAGRGACYGPQCLCR |
| 238 | GCGPCFTTDHQGARKCDDCCGGRGRGACYGPQCLCR |
| 239 | GCGPCFTTDHQGARACDDCCGGRGRGACYGPQCLCR |
| 240 | GCGPCFTTDHQGARRCDDCCGGRGRGACYGPQCLCR |
| 241 | GCGPCFTTDHQGARKCDDCCGGKGRGRCYGPQCLCR |
| 242 | GCGPCFTTDHQGARACDDCCGGKGRGRCYGPQCLCR |
| 243 | GCGPCFTTDHQGARRCDDCCGGKGRGRCYGPQCLCR |
| 244 | GCGPCFTTDHQGARKCDDCCGGAGRGRCYGPQCLCR |
| 245 | GCGPCFTTDHQGARACDDCCGGAGRGRCYGPQCLCR |
| 246 | GCGPCFTTDHQGARRCDDCCGGAGRGRCYGPQCLCR |
| 247 | GCGPCFTTDHQGARKCDDCCGGRGRGRCYGPQCLCR |
| 248 | GCGPCFTTDHQGARACDDCCGGRGRGRCYGPQCLCR |
| 249 | GCGPCFTTDHQGARRCDDCCGGRGRGRCYGPQCLCR |
| 250 | GCGPCFTTDHQGARRCDDCCGGRGRGRCYGPQCLCR |
| 251 | KCGPCFTTDHQGARRCDDCCGGRGRGRCYGPQCLCR |
| 252 | ACAPCFTTDHQAARRCDDCCGGRGRGRCYGPQCLCR |
| 253 | KCAPCFTTDHQAARRCDDCCGGRGRGRCYGPQCLCR |
| 254 | GCGPCFTTDHQGAR(Cit)CDDCCGG(Cit)GRGKCYGPQCLCR |
| 255 | GCGPCFTTDHQGAR(Cit)CDDCCGG(Cit)GRG(Cit)CYGPQCLCR |
| 256 | KCGPCFTTDHQGAR(Cit)CDDCCGG(Cit)GRG(Cit)CYGPQCLCR |
| 257 | ACAPCFTTDHQAAR(Cit)CDDCCGG(Cit)GRGKCYGPQCLCR |
| 258 | ACAPCFTTDHQAAR(Cit)CDDCCGG(Cit)GRG(Cit)CYGPQCLCR |
| 259 | KCAPCFTTDHQAAR(Cit)CDDCCGG(Cit)GRG(Cit)CYGPQCLCR |
| 260 | ACAPCFTTDHQAARKCDDCCGGKGRGKCYGPQCLCR |
| 261 | ACAPCFTTDHQAARACDDCCGGKGRGKCYGPQCLCR |
| 262 | ACAPCFTTDHQAARRCDDCCGGKGRGKCYGPQCLCR |
| 263 | ACAPCFTTDHQAARKCDDCCGGAGRGKCYGPQCLCR |
| 264 | ACAPCFTTDHQAARACDDCCGGAGRGKCYGPQCLCR |
| 265 | ACAPCFTTDHQAARRCDDCCGGAGRGKCYGPQCLCR |
| 266 | ACAPCFTTDHQAARKCDDCCGGRGRGKCYGPQCLCR |
| 267 | ACAPCFTTDHQAARACDDCCGGRGRGKCYGPQCLCR |
| 268 | ACAPCFTTDHQAARRCDDCCGGRGRGKCYGPQCLCR |
| 269 | ACAPCFTTDHQAARKCDDCCGGKGRGACYGPQCLCR |
| 270 | ACAPCFTTDHQAARACDDCCGGKGRGACYGPQCLCR |
| 271 | ACAPCFTTDHQAARRCDDCCGGKGRGACYGPQCLCR |
| 272 | ACAPCFTTDHQAARKCDDCCGGAGRGACYGPQCLCR |
| 273 | ACAPCFTTDHQAARACDDCCGGAGRGACYGPQCLCR |
| 274 | ACAPCFTTDHQAARRCDDCCGGAGRGACYGPQCLCR |
| 275 | ACAPCFTTDHQAARKCDDCCGGRGRGACYGPQCLCR |
| 276 | ACAPCFTTDHQAARACDDCCGGRGRGACYGPQCLCR |
| 277 | ACAPCFTTDHQAARRCDDCCGGRGRGACYGPQCLCR |
| 278 | ACAPCFTTDHQAARKCDDCCGGKGRGRCYGPQCLCR |
| 279 | ACAPCFTTDHQAARACDDCCGGKGRGRCYGPQCLCR |
| 280 | ACAPCFTTDHQAARRCDDCCGGKGRGRCYGPQCLCR |
| 281 | ACAPCFTTDHQAARKCDDCCGGAGRGRCYGPQCLCR |
| 282 | ACAPCFTTDHQAARACDDCCGGAGRGRCYGPQCLCR |
| 283 | ACAPCFTTDHQAARRCDDCCGGAGRGRCYGPQCLCR |
| 284 | ACAPCFTTDHQAARKCDDCCGGRGRGRCYGPQCLCR |
| 285 | ACAPCFTTDHQAARACDDCCGGRGRGRCYGPQCLCR |
| 286 | ACAPCFTTDHQAARRCDDCCGGRGRGRCYGPQCLCR |
| 287 | ACAPCFTTDHQAARRCDDCCGGRGRGRCYGPQCLCR |
| 288 | KCAPCFTTDHQAARRCDDCCGGRGRGRCYGPQCLCR |
| 289 | ACAPCFTTDHQAARRCDDCCGGRGRGRCYGPQCLCR |
| 290 | KCAPCFTTDHQAARRCDDCCGGRGRGRCYGPQCLCR |
| 291 | ACAPCFTTDHQAAR(Cit)CDDCCGG(Cit)GRGKCYGPQCLCR |
| 292 | ACAPCFTTDHQAAR(Cit)CDDCCGG(Cit)GRG(Cit)CYGPQCLCR |
| 293 | KCAPCFTTDHQAAR(Cit)CDDCCGG(Cit)GRG(Cit)CYGPQCLCR |
| 294 | ACAPCFTTDHQAAR(Cit)CDDCCGG(Cit)GRGKCYGPQCLCR |
| 295 | ACAPCFTTDHQAAR(Cit)CDDCCGG(Cit)GRG(Cit)CYGPQCLCR |
| 296 | KCAPCFTTDHQAAR(Cit)CDDCCGG(Cit)GRG(Cit)CYGPQCLCR |
| 297 | ICIPCFTTDHQIARKCDDCCGGKGRGKCYGPQCLCR |
| 298 | ICIPCFTTDHQIARACDDCCGGKGRGKCYGPQCLCR |
| 299 | ICIPCFTTDHQIARRCDDCCGGKGRGKCYGPQCLCR |
| 300 | ICIPCFTTDHQIARKCDDCCGGAGRGKCYGPQCLCR |
| 301 | ICIPCFTTDHQIARACDDCCGGAGRGKCYGPQCLCR |
| 302 | ICIPCFTTDHQIARRCDDCCGGAGRGKCYGPQCLCR |
| 303 | ICIPCFTTDHQIARKCDDCCGGRGRGKCYGPQCLCR |
| 304 | ICIPCFTTDHQIARACDDCCGGRGRGKCYGPQCLCR |
| 305 | ICIPCFTTDHQIARRCDDCCGGRGRGKCYGPQCLCR |
| 306 | ICIPCFTTDHQIARKCDDCCGGKGRGACYGPQCLCR |
| 307 | ICIPCFTTDHQIARACDDCCGGKGRGACYGPQCLCR |
| 308 | ICIPCFTTDHQIARRCDDCCGGKGRGACYGPQCLCR |
| 309 | ICIPCFTTDHQIARKCDDCCGGAGRGACYGPQCLCR |
| 310 | ICIPCFTTDHQIARACDDCCGGAGRGACYGPQCLCR |
| 311 | ICIPCFTTDHQIARRCDDCCGGAGRGACYGPQCLCR |
| 312 | ICIPCFTTDHQIARKCDDCCGGRGRGACYGPQCLCR |
| 313 | ICIPCFTTDHQIARACDDCCGGRGRGACYGPQCLCR |
| 314 | ICIPCFTTDHQIARRCDDCCGGRGRGACYGPQCLCR |
| 315 | ICIPCFTTDHQIARKCDDCCGGKGRGRCYGPQCLCR |
| 316 | ICIPCFTTDHQIARACDDCCGGKGRGRCYGPQCLCR |
| 317 | ICIPCFTTDHQIARRCDDCCGGKGRGRCYGPQCLCR |
| 318 | ICIPCFTTDHQIARKCDDCCGGAGRGRCYGPQCLCR |
| 319 | ICIPCFTTDHQIARACDDCCGGAGRGRCYGPQCLCR |
| 320 | ICIPCFTTDHQIARRCDDCCGGAGRGRCYGPQCLCR |
| 321 | ICIPCFTTDHQIARKCDDCCGGRGRGRCYGPQCLCR |
| 322 | ICIPCFTTDHQIARACDDCCGGRGRGRCYGPQCLCR |
| 323 | ICIPCFTTDHQIARRCDDCCGGRGRGRCYGPQCLCR |
| 324 | ICIPCFTTDHQIARRCDDCCGGRGRGRCYGPQCLCR |
| 325 | KCIPCFTTDHQIARRCDDCCGGRGRGRCYGPQCLCR |
| 326 | ACAPCFTTDHQAARRCDDCCGGRGRGRCYGPQCLCR |
| 327 | KCAPCFTTDHQAARRCDDCCGGRGRGRCYGPQCLCR |
| 328 | ICIPCFTTDHQIAR(Cit)CDDCCGG(Cit)GRGKCYGPQCLCR |
| 329 | ICIPCFTTDHQIAR(Cit)CDDCCGG(Cit)GRG(Cit)CYGPQCLCR |
| 330 | KCIPCFTTDHQIAR(Cit)CDDCCGG(Cit)GRG(Cit)CYGPQCLCR |
| 331 | ACAPCFTTDHQAAR(Cit)CDDCCGG(Cit)GRGKCYGPQCLCR |
| 332 | ACAPCFTTDHQAAR(Cit)CDDCCGG(Cit)GRG(Cit)CYGPQCLCR |
| 333 | KCAPCFTTDHQAAR(Cit)CDDCCGG(Cit)GRG(Cit)CYGPQCLCR |
| 334 | TCTPCFTTDHQTARKCDDCCGGKGRGKCYGPQCLCR |
| 335 | TCTPCFTTDHQTARACDDCCGGKGRGKCYGPQCLCR |
| 336 | TCTPCFTTDHQTARRCDDCCGGKGRGKCYGPQCLCR |
| 337 | TCTPCFTTDHQTARKCDDCCGGAGRGKCYGPQCLCR |
| 338 | TCTPCFTTDHQTARACDDCCGGAGRGKCYGPQCLCR |
| 339 | TCTPCFTTDHQTARRCDDCCGGAGRGKCYGPQCLCR |
| 340 | TCTPCFTTDHQTARKCDDCCGGRGRGKCYGPQCLCR |
| 341 | TCTPCFTTDHQTARACDDCCGGRGRGKCYGPQCLCR |
| 342 | TCTPCFTTDHQTARRCDDCCGGRGRGKCYGPQCLCR |
| 343 | TCTPCFTTDHQTARKCDDCCGGKGRGACYGPQCLCR |
| 344 | TCTPCFTTDHQTARACDDCCGGKGRGACYGPQCLCR |
| 345 | TCTPCFTTDHQTARRCDDCCGGKGRGACYGPQCLCR |
| 346 | TCTPCFTTDHQTARKCDDCCGGAGRGACYGPQCLCR |
| 347 | TCTPCFTTDHQTARACDDCCGGAGRGACYGPQCLCR |
| 348 | TCTPCFTTDHQTARRCDDCCGGAGRGACYGPQCLCR |
| 349 | TCTPCFTTDHQTARKCDDCCGGRGRGACYGPQCLCR |
| 350 | TCTPCFTTDHQTARACDDCCGGRGRGACYGPQCLCR |
| 351 | TCTPCFTTDHQTARRCDDCCGGRGRGACYGPQCLCR |
| 352 | TCTPCFTTDHQTARKCDDCCGGKGRGRCYGPQCLCR |
| 353 | TCTPCFTTDHQTARACDDCCGGKGRGRCYGPQCLCR |
| 354 | TCTPCFTTDHQTARRCDDCCGGKGRGRCYGPQCLCR |
| 355 | TCTPCFTTDHQTARKCDDCCGGAGRGRCYGPQCLCR |
| 356 | TCTPCFTTDHQTARACDDCCGGAGRGRCYGPQCLCR |
| 357 | TCTPCFTTDHQTARRCDDCCGGAGRGRCYGPQCLCR |
| 358 | TCTPCFTTDHQTARKCDDCCGGRGRGRCYGPQCLCR |
| 359 | TCTPCFTTDHQTARACDDCCGGRGRGRCYGPQCLCR |
| 360 | TCTPCFTTDHQTARRCDDCCGGRGRGRCYGPQCLCR |
| 361 | TCTPCFTTDHQTARRCDDCCGGRGRGRCYGPQCLCR |
| 362 | KCTPCFTTDHQTARRCDDCCGGRGRGRCYGPQCLCR |
| 363 | ACAPCFTTDHQAARRCDDCCGGRGRGRCYGPQCLCR |
| 364 | KCAPCFTTDHQAARRCDDCCGGRGRGRCYGPQCLCR |
| 365 | TCTPCFTTDHQTAR(Cit)CDDCCGG(Cit)GRGKCYGPQCLCR |
| 366 | TCTPCFTTDHQTAR(Cit)CDDCCGG(Cit)GRG(Cit)CYGPQCLCR |
| 367 | KCTPCFTTDHQTAR(Cit)CDDCCGG(Cit)GRG(Cit)CYGPQCLCR |
| 368 | ACAPCFTTDHQAAR(Cit)CDDCCGG(Cit)GRGKCYGPQCLCR |
| 369 | ACAPCFTTDHQAAR(Cit)CDDCCGG(Cit)GRG(Cit)CYGPQCLCR |
| 370 | KCAPCFTTDHQAAR(Cit)CDDCCGG(Cit)GRG(Cit)CYGPQCLCR |
| 371 | VCVPCFTTDHQVARKCDDCCGGKGRGKCYGPQCLCR |
| 372 | VCVPCFTTDHQVARACDDCCGGKGRGKCYGPQCLCR |
| 373 | VCVPCFTTDHQVARRCDDCCGGKGRGKCYGPQCLCR |
| 374 | VCVPCFTTDHQVARKCDDCCGGAGRGKCYGPQCLCR |
| 375 | VCVPCFTTDHQVARACDDCCGGAGRGKCYGPQCLCR |
| 376 | VCVPCFTTDHQVARRCDDCCGGAGRGKCYGPQCLCR |
| 377 | VCVPCFTTDHQVARKCDDCCGGRGRGKCYGPQCLCR |
| 378 | VCVPCFTTDHQVARACDDCCGGRGRGKCYGPQCLCR |
| 379 | VCVPCFTTDHQVARRCDDCCGGRGRGKCYGPQCLCR |
| 380 | VCVPCFTTDHQVARKCDDCCGGKGRGACYGPQCLCR |
| 381 | VCVPCFTTDHQVARACDDCCGGKGRGACYGPQCLCR |
| 382 | VCVPCFTTDHQVARRCDDCCGGKGRGACYGPQCLCR |
| 383 | VCVPCFTTDHQVARKCDDCCGGAGRGACYGPQCLCR |
| 384 | VCVPCFTTDHQVARACDDCCGGAGRGACYGPQCLCR |
| 385 | VCVPCFTTDHQVARRCDDCCGGAGRGACYGPQCLCR |
| 386 | VCVPCFTTDHQVARKCDDCCGGRGRGACYGPQCLCR |
| 387 | VCVPCFTTDHQVARACDDCCGGRGRGACYGPQCLCR |
| 388 | VCVPCFTTDHQVARRCDDCCGGRGRGACYGPQCLCR |
| 389 | VCVPCFTTDHQVARKCDDCCGGKGRGRCYGPQCLCR |
| 390 | VCVPCFTTDHQVARACDDCCGGKGRGRCYGPQCLCR |
| 391 | VCVPCFTTDHQVARRCDDCCGGKGRGRCYGPQCLCR |
| 392 | VCVPCFTTDHQVARKCDDCCGGAGRGRCYGPQCLCR |
| 393 | VCVPCFTTDHQVARACDDCCGGAGRGRCYGPQCLCR |
| 394 | VCVPCFTTDHQVARRCDDCCGGAGRGRCYGPQCLCR |
| 395 | VCVPCFTTDHQVARKCDDCCGGRGRGRCYGPQCLCR |
| 396 | VCVPCFTTDHQVARACDDCCGGRGRGRCYGPQCLCR |
| 397 | VCVPCFTTDHQVARRCDDCCGGRGRGRCYGPQCLCR |
| 398 | VCVPCFTTDHQVARRCDDCCGGRGRGRCYGPQCLCR |
| 399 | KCVPCFTTDHQVARRCDDCCGGRGRGRCYGPQCLCR |
| 400 | ACAPCFTTDHQAARRCDDCCGGRGRGRCYGPQCLCR |
| 401 | KCAPCFTTDHQAARRCDDCCGGRGRGRCYGPQCLCR |
| 402 | VCVPCFTTDHQVAR(Cit)CDDCCGG(Cit)GRGKCYGPQCLCR |
| 403 | VCVPCFTTDHQVAR(Cit)CDDCCGG(Cit)GRG(Cit)CYGPQCLCR |
| 404 | KCVPCFTTDHQVAR(Cit)CDDCCGG(Cit)GRG(Cit)CYGPQCLCR |
| 405 | ACAPCFTTDHQAAR(Cit)CDDCCGG(Cit)GRGKCYGPQCLCR |
| 406 | ACAPCFTTDHQAAR(Cit)CDDCCGG(Cit)GRG(Cit)CYGPQCLCR |
| 407 | KCAPCFTTDHQAAR(Cit)CDDCCGG(Cit)GRG(Cit)CYGPQCLCR |
| 408 | LCLPCFTTDHQLARKCDDCCGGKGRGKCYGPQCLCR |
| 409 | LCLPCFTTDHQLARACDDCCGGKGRGKCYGPQCLCR |
| 410 | LCLPCFTTDHQLARRCDDCCGGKGRGKCYGPQCLCR |
| 411 | LCLPCFTTDHQLARKCDDCCGGAGRGKCYGPQCLCR |
| 412 | LCLPCFTTDHQLARACDDCCGGAGRGKCYGPQCLCR |
| 413 | LCLPCFTTDHQLARRCDDCCGGAGRGKCYGPQCLCR |
| 414 | LCLPCFTTDHQLARKCDDCCGGRGRGKCYGPQCLCR |
| 415 | LCLPCFTTDHQLARACDDCCGGRGRGKCYGPQCLCR |
| 416 | LCLPCFTTDHQLARRCDDCCGGRGRGKCYGPQCLCR |
| 417 | LCLPCFTTDHQLARKCDDCCGGKGRGACYGPQCLCR |
| 418 | LCLPCFTTDHQLARACDDCCGGKGRGACYGPQCLCR |
| 419 | LCLPCFTTDHQLARRCDDCCGGKGRGACYGPQCLCR |
| 420 | LCLPCFTTDHQLARKCDDCCGGAGRGACYGPQCLCR |
| 421 | LCLPCFTTDHQLARACDDCCGGAGRGACYGPQCLCR |
| 422 | LCLPCFTTDHQLARRCDDCCGGAGRGACYGPQCLCR |
| 423 | LCLPCFTTDHQLARKCDDCCGGRGRGACYGPQCLCR |
| 424 | LCLPCFTTDHQLARACDDCCGGRGRGACYGPQCLCR |
| 425 | LCLPCFTTDHQLARRCDDCCGGRGRGACYGPQCLCR |
| 426 | LCLPCFTTDHQLARKCDDCCGGKGRGRCYGPQCLCR |
| 427 | LCLPCFTTDHQLARACDDCCGGKGRGRCYGPQCLCR |
| 428 | LCLPCFTTDHQLARRCDDCCGGKGRGRCYGPQCLCR |
| 429 | LCLPCFTTDHQLARKCDDCCGGAGRGRCYGPQCLCR |
| 430 | LCLPCFTTDHQLARACDDCCGGAGRGRCYGPQCLCR |
| 431 | LCLPCFTTDHQLARRCDDCCGGAGRGRCYGPQCLCR |
| 432 | LCLPCFTTDHQLARKCDDCCGGRGRGRCYGPQCLCR |
| 433 | LCLPCFTTDHQLARACDDCCGGRGRGRCYGPQCLCR |
| 434 | LCLPCFTTDHQLARRCDDCCGGRGRGRCYGPQCLCR |
| 435 | LCLPCFTTDHQLARRCDDCCGGRGRGRCYGPQCLCR |
| 436 | KCLPCFTTDHQLARRCDDCCGGRGRGRCYGPQCLCR |
| 437 | ACAPCFTTDHQAARRCDDCCGGRGRGRCYGPQCLCR |
| 438 | KCAPCFTTDHQAARRCDDCCGGRGRGRCYGPQCLCR |
| 439 | LCLPCFTTDHQLAR(Cit)CDDCCGG(Cit)GRGKCYGPQCLCR |
| 440 | LCLPCFTTDHQLAR(Cit)CDDCCGG(Cit)GRG(Cit)CYGPQCLCR |
| 441 | KCLPCFTTDHQLAR(Cit)CDDCCGG(Cit)GRG(Cit)CYGPQCLCR |
| 442 | ACAPCFTTDHQAAR(Cit)CDDCCGG(Cit)GRGKCYGPQCLCR |
| 443 | ACAPCFTTDHQAAR(Cit)CDDCCGG(Cit)GRG(Cit)CYGPQCLCR |
| 444 | KCAPCFTTDHQAAR(Cit)CDDCCGG(Cit)GRG(Cit)CYGPQCLCR |
| 445 | SCSPCFTTDHQSARKCDDCCGGKGRGKCYGPQCLCR |
| 446 | SCSPCFTTDHQSARACDDCCGGKGRGKCYGPQCLCR |
| 447 | SCSPCFTTDHQSARRCDDCCGGKGRGKCYGPQCLCR |
| 448 | SCSPCFTTDHQSARKCDDCCGGAGRGKCYGPQCLCR |
| 449 | SCSPCFTTDHQSARACDDCCGGAGRGKCYGPQCLCR |
| 450 | SCSPCFTTDHQSARRCDDCCGGAGRGKCYGPQCLCR |
| 451 | SCSPCFTTDHQSARKCDDCCGGRGRGKCYGPQCLCR |
| 452 | SCSPCFTTDHQSARACDDCCGGRGRGKCYGPQCLCR |
| 453 | SCSPCFTTDHQSARRCDDCCGGRGRGKCYGPQCLCR |
| 454 | SCSPCFTTDHQSARKCDDCCGGKGRGACYGPQCLCR |
| 455 | SCSPCFTTDHQSARACDDCCGGKGRGACYGPQCLCR |
| 456 | SCSPCFTTDHQSARRCDDCCGGKGRGACYGPQCLCR |
| 457 | SCSPCFTTDHQSARKCDDCCGGAGRGACYGPQCLCR |
| 458 | SCSPCFTTDHQSARACDDCCGGAGRGACYGPQCLCR |
| 459 | SCSPCFTTDHQSARRCDDCCGGAGRGACYGPQCLCR |
| 460 | SCSPCFTTDHQSARKCDDCCGGRGRGACYGPQCLCR |
| 461 | SCSPCFTTDHQSARACDDCCGGRGRGACYGPQCLCR |
| 462 | SCSPCFTTDHQSARRCDDCCGGRGRGACYGPQCLCR |
| 463 | SCSPCFTTDHQSARKCDDCCGGKGRGRCYGPQCLCR |
| 464 | SCSPCFTTDHQSARACDDCCGGKGRGRCYGPQCLCR |
| 465 | SCSPCFTTDHQSARRCDDCCGGKGRGRCYGPQCLCR |
| 466 | SCSPCFTTDHQSARKCDDCCGGAGRGRCYGPQCLCR |
| 467 | SCSPCFTTDHQSARACDDCCGGAGRGRCYGPQCLCR |
| 468 | SCSPCFTTDHQSARRCDDCCGGAGRGRCYGPQCLCR |
| 469 | SCSPCFTTDHQSARKCDDCCGGRGRGRCYGPQCLCR |
| 470 | SCSPCFTTDHQSARACDDCCGGRGRGRCYGPQCLCR |
| 471 | SCSPCFTTDHQSARRCDDCCGGRGRGRCYGPQCLCR |
| 472 | SCSPCFTTDHQSARRCDDCCGGRGRGRCYGPQCLCR |
| 473 | KCSPCFTTDHQSARRCDDCCGGRGRGRCYGPQCLCR |
| 474 | ACAPCFTTDHQAARRCDDCCGGRGRGRCYGPQCLCR |
| 475 | KCAPCFTTDHQAARRCDDCCGGRGRGRCYGPQCLCR |
| 476 | SCSPCFTTDHQSAR(Cit)CDDCCGG(Cit)GRGKCYGPQCLCR |
| 477 | SCSPCFTTDHQSAR(Cit)CDDCCGG(Cit)GRG(Cit)CYGPQCLCR |
| 478 | KCSPCFTTDHQSAR(Cit)CDDCCGG(Cit)GRG(Cit)CYGPQCLCR |
| 479 | ACAPCFTTDHQAAR(Cit)CDDCCGG(Cit)GRGKCYGPQCLCR |
| 480 | ACAPCFTTDHQAAR(Cit)CDDCCGG(Cit)GRG(Cit)CYGPQCLCR |
| 481 | KCAPCFTTDHQAAR(Cit)CDDCCGG(Cit)GRG(Cit)CYGPQCLCR |

Chlorotoxin conjugates comprise a chlorotoxin and a labeling agent or detectable label. In an embodiment, chlorotoxin is a variant comprising at least

90%, 92%, 93%, 94%, 95%, 96%, 97%, 98%, or 99% identical to the sequence of the natural peptide of chlorotoxin. In another embodiment, the present disclosure provides a chlorotoxin having the following amino acid sequence: MCMPCFTTDHQMARKCDDCCGGKGRGKCYGPQCLCR. In a further embodiment, the present disclosure provides chlorotoxin variants comprising at least

90%, 92%, 93%, 94%, 95%, 96%, 97%, 98%, or 99% identical to the following amino acid sequence:
MCMPCFTTDHQMARKCDDCCGGKGRGKCYGPQCLCR. In another embodiment, the chlorotoxin is a chlorotoxin or variant thereof comprising at least 90%, 92%, 93%, 94%, 95%, 96%, 97%, 98%, or 99% sequence identity to the sequence of MCMPCFTTDHQMARXCDDCCGGXGRGXCYGPQCLCR, wherein X is selected from K, A and R. In another embodiment, the chlorotoxin is a chlorotoxin or variant of thereof comprising at least 90% sequence identity to the sequence of MCMPCFTTDHQMARXCDDCCGGXGRGXCYGPQCLCR, wherein X is selected from K, A and R.

In another embodiment, the compound is BLZ-100 (also referred to herein as Compound 16), which is a chlorotoxin variant comprising the sequence of MCMPCFTTDHQMARXCDDCCGGXGRGXCYGPQCLCR, wherein X15 and X23 are arginine and X27 is lysine conjugated to a cyanine fluorescent label. The peptide can be further cross-linked by four disulfide bonds formed among the cysteine residues present in the sequence.

In some aspects, the peptide is a variant of the natural peptide of chlorotoxin but retains all eight cysteine residues of the natural peptide, enabling cross-linking by up to four disulfide bonds. Conservation of cysteine residues helps to preserve the secondary structure, charge distribution, isolelectric point (pI) and other features of the natural chlorotoxin peptide because of the disulfide bonds that form between the cysteine residues.

In some aspects, the chlorotoxin peptide variant retains all eight cysteine residues of the natural peptide and has at least 90%, 92%, 93%, 94%, 95%, 96%, 97%, 98%, or 99% sequence identity with the native chlorotoxin peptide.

In some aspects, the chlorotoxin peptide variant has eight cysteine residues positioned so that the distances between pairs of cysteines is the same as the distances between pairs of cysteines found in the natural peptide, and the chlorotoxin peptide variant has at least 90%, 92%, 93%, 94%, 95%, 96%, 97%, 98%, or 99% sequence identity with the native chlorotoxin peptide.

In some aspects, the chlorotoxin peptide variant has eight cysteine residues positioned so that the distances between pairs of cysteines is functionally equivalent or functionally similar to the distances between pairs of cysteines found in the natural peptide, and the chlorotoxin peptide variant has at least 90%, 92%, 93%, 94%, 95%, 96%, 97%, 98%, or 99% sequence identity with the native chlorotoxin peptide.

In some aspects, the chlorotoxin peptide variant has eight cysteine residues positioned so that the distances between pairs of cysteines allows for secondary structure and isolectric point of the native chlorotoxin peptide to be preserved, and the chlorotoxin peptide variant has at least 90%, 92%, 93%, 94%, 95%, 96%, 97%, 98%, or 99% sequence identity with the native chlorotoxin peptide.

In some aspects, the chlorotoxin peptide variant has eight cysteine residues positioned so that the distances between pairs of cysteines is sufficient to allow disulfide bonds to form, and the chlorotoxin peptide variant has at least 90%, 92%, 93%, 94%, 95%, 96%, 97%, 98%, or 99% sequence identity with the native chlorotoxin peptide.

In some aspects, one or more methionines of the chlorotoxin peptide variant are replaced with other amino acids. In some aspects, one or more methionines of the chlorotoxin peptide variant are replaced with other amino acids selected from glycine, alanine, Isoleucine, Threonine, Valine, Leucine, Serine or a combination thereof.

In some embodiments, the chlorotoxin can be a chlorotoxin variant. Chlorotoxin and chlorotoxin variants have are further described in PCT Patent Application Publication Numbers WO2006115633 and WO201 1 142858.

In one embodiment, the peptide can have the following formula: H-Met-Cys-Met-Pro-Cys-Phe-Thr-Thr-Asp-His-Gln-Met-Ala-Arg-Xaa-Cys-Asp-Asp-Cys-Cys-Gly-Gly-Xaa-Gly-Arg-Gly-Xaa-Cys-Tyr-Gly-Pro-Gln-Cys-Leu-Cys-Arg-OH acetate salt (disulfide bonds, air oxidized), wherein Xaa is Arg, Ala, or Lys.

In another embodiment, the all peptide can have the following formula: H-Met-Cys-Met-Pro-Cys-Phe-Thr-Thr-Asp-His-Gln-Met-Ala-Arg-Xaa-Cys-Asp-Asp-Cys-Cys-Gly-Gly-Xaa-Gly-Arg-Gly-Lys-Cys-Tyr-Gly-Pro-Gln-Cys-Leu-Cys-Arg-OH acetate salt (disulfide bonds, air oxidized), wherein Xaa is Arg, or Ala.

In another embodiment, the peptide can have the following formula: H-Met-Cys-Met-Pro-Cys-Phe-Thr-Thr-Asp-His-Gln-Met-Ala-Arg-Arg-Cys-Asp-Asp-Cys-Cys-Gly-Gly-Arg-Gly-Arg-Gly-Lys-Cys-Tyr-Gly-Pro-Gln-Cys-Leu-Cys-Arg-OH acetate salt (disulfide bonds, air oxidized).

In another embodiment, the peptide can have the following formula: H-Met-Cys-Met-Pro-Cys-Phe-Thr-Thr-Asp-His-Gln-Met-Ala-Arg-Arg-Cys-Asp-Asp-Cys-Cys-Gly-Gly-Ala-Gly-Arg-Gly-Lys-Cys-Tyr-Gly-Pro-Gln-Cys-Leu-Cys-Arg-OH acetate salt (disulfide bonds, air oxidized).

In another embodiment, the peptide can have the following formula: H-Met-Cys-Met-Pro-Cys-Phe-Thr-Thr-Asp-His-Gln-Met-Ala-Arg-Ala-Cys-Asp-Asp-Cys-Cys-Gly-Gly-Arg-Gly-Arg-Gly-Lys-Cys-Tyr-Gly-Pro-Gln-Cys-Leu-Cys-Arg-OH acetate salt (disulfide bonds, air oxidized).

In another embodiment, the peptide can have the following formula: H-Met-Cys-Met-Pro-Cys-Phe-Thr-Thr-Asp-His-Gln-Met-Ala-Arg-Ala-Cys-Asp-Asp-Cys-Cys-Gly-Gly-Ala-Gly-Arg-Gly-Lys-Cys-Tyr-Gly-Pro-Gln-Cys-Leu-Cys-Arg-OH acetate salt (disulfide bonds, air oxidized).

In certain embodiments, the chlorotoxin and chlorotoxin variants can be conjugated to moieties, such as detectable labels (e.g., dyes) that can be detected (e.g., visualized) in a subject. In some embodiments, the chlorotoxin and/or chlorotoxin variants can be conjugated to detectable labels to enable tracking of the bio-distribution of a conjugated peptide. The detectable labels can include fluorescent dyes. Non-limiting examples of fluorescent dyes that could be used as a conjugating molecule in the present disclosure include rhodamine, rhodol, fluorescein, thiofluorescein, amino fluorescein, carboxyfluorescein, chlorofluorescein, methylfluorescein, sulfofluorescein, aminorhodol, carboxyrhodol, chlororhodol, methylrhodol, sulforhodol; aminorhodamine, carboxyrhodamine, chlororhodamine, methylrhodamine, sulforhodamine, and thiorhodamine, cyanine, indocarbocyanine, oxacarbocyanine, thiacarbocyanine, merocyanine, a cyanine dye (e.g., cyanine 2, cyanine 3, cyanine 3.5, cyanine 5, cyanine 5.5, cyanine 7), oxadiazole derivatives, pyridyloxazole, nitrobenzoxadiazole, benzoxadiazole, pyrene derivatives, cascade blue, oxazine derivatives, Nile red, Nile blue, cresyl violet, oxazine 170, acridine derivatives, proflavin, acridine orange, acridine yellow, arylmethine derivatives, auramine, xanthene dyes, sulfonated xanthenes dyes, Alexa Fluors (e.g., Alexa Fluor 594, Alexa Fluor 633, Alexa Fluor 647, Alexa Fluor 700), crystal violet, malachite green, tetrapyrrole derivatives, porphyrin, phtalocyanine, and bilirubin. Some other example dyes include near-infrared dyes, such as, but not limited to, Cy5.5, indocyanine green (ICG), DyLight 750 or IRdye 800. In some embodiments, near infrared dyes can include cyanine dyes.

Chemotherapueutics, anti-cancer drugs, and anti-cancer agents, include, but are not limited to: radioisotopes, toxins, enzymes, sensitizing drugs, nucleic acids, including interfering RNAs, antibodies, anti-angiogenic agents, cisplatin, anti-metabolites, mitotic inhibitors, growth factor inhibitors, paclitaxel, temozolomide, topotecan, fluorouracil, vincristine, vinblastine, procarbazine, decarbazine, altretamine, methotrexate, mercaptopurine, thioguanine, fludarabine phosphate, cladribine, pentostatin, cytarabine, azacitidine, etoposide, teniposide, irinotecan, docetaxel, doxorubicin, daunorubicin, dactinomycin, idarubicin, plicamycin, mitomycin, bleomycin, tamoxifen, flutamide, leuprolide, goserelin, aminogluthimide, anastrozole, amsacrine, asparaginase, mitoxantrone, mitotane and amifostine, and their equivalents, as well as photo-ablation.

As used herein, the terms "about" and "approximately," in reference to a number, is used herein to include numbers that fall within a range of 10%, 5%, or 1% in either direction (greater than or less than) the number unless otherwise stated or otherwise evident from the context (except where such number would exceed 100% of a possible value).

Suitable diagnostic agents include agents that provide for the detection by fluorescence methods as well as methods other than fluorescence imaging. Other suitable diagnostic agents include radiolabels (e.g., radio isotopically labeled compounds) such as ¹²⁵ I, ¹⁴C, and ³¹P, among others; and magnetic resonance imaging agents.

Suitable targeting agents include antibodies, polypeptides, polysaccharides, and nucleic acids.

In another aspect of the invention, compositions that include the modified chlorotoxin peptide conjugates are provided. The composition can include a pharmaceutically acceptable carrier or diluent for delivery of the modified chlorotoxin peptide conjugate. Suitable pharmaceutically acceptable carriers or diluents include saline or dextrose for injection.

In various aspects, the presently described compounds further comprise a detectable label, which can be used for the detection of the peptide-label conjugate and the cancerous cells to which they are bound.

In various aspects, the presently described compounds further comprise a detectable label, which can be used for the detection of the peptide-label conjugate and the cancerous cells to which they are bound.

The present compounds have a structure of Formula (III), or a pharmaceutically acceptable salt thereof: 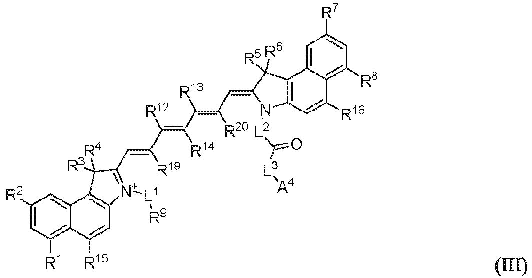 wherein:
R¹, R², R³, R⁴, R⁵, R⁶, R⁷, R⁸ , R¹⁵, and R¹⁶ are each independently selected from hydrogen, C₁-C₆ alkyl, C₁-C₆ alkylene-COOH, sulfonate, -COOH, -SO₂-NH₂, or C₁-C₆ alkoxy;
R⁹ is sulfonate;
L¹ is C₃-C₆ alkylene;
L² is C₁-C₁₀ alkylene;
L³ is a bond, -O-, -NR¹⁰-, -NR¹⁰-C₁-C₆ alkylene-, -O-NR¹⁰-, -NR¹⁰-C₁-C₆ alkylene- (O-C₁-C₆ alkylene)ₙ-, -NR¹⁰-L⁴-, -NR¹⁰-C₁-C₆ alkylene-NR¹¹-(C (= O)-C₁-C₆ alkylene-O-)ₘ-, or -NR¹⁰-C₁-C₆ alkylene-NR¹⁰-C₁-C₆ alkylene-NR¹⁰-C₁-C₆ alkylene-;
L⁴ is a bond, -heterocyclyl-, or -heterocyclyl-C₁-C₆ alkylene-;
R¹⁰ is hydrogen or C₁-C₆ alkyl;
R¹¹ is hydrogen or C₁-C₆ alkyl;
R¹² and R¹³ are independently selected from hydrogen, C₁-C₆ alkyl, or R¹² and R¹³ are joined together along with the other atoms to which they are attached to form a 5-membered or 6-membered carbocyclic or heterocyclic ring;
R¹⁴ is hydrogen or C₁-C₆ alkylene, -(L⁵)-aryl, -(L⁵)-heteroaryl, -NR¹⁷ R¹⁸, R¹⁴ and R¹⁹ are joined together along with the other atoms to which they are attached to form a 5-membered or 6-membered carbocyclic or heterocyclic ring, or R¹⁴ and R²⁰ are joined together along with the other atoms to which they are attached to form a 5-membered or 6-membered carbocyclic or heterocyclic ring;
L⁵ is a bond, C₁-C₁₀ alkylene, -O-, or -NR¹⁰-;
R¹⁷ and R¹⁸ are each independently hydrogen or aryl;
R¹⁹ and R²⁰ are independently selected from hydrogen, C₁-C₆ alkyl, R¹⁴ and R¹⁹ are joined together along with the other atoms to which they are attached to form a 5-membered or 6-membered carbocyclic or heterocyclic ring, or R¹⁴ and R²⁰ are joined together along with the other atoms to which they are attached to form a 5-membered or 6-membered carbocyclic or heterocyclic ring;
n is 0, 1, 2, or 3;
m is 0, 1, 2, or 3;
p is 0, 1, 2, or 3;
q is 0, 1, 2, or 3; and
A⁴ is a polypeptide having at least 90% sequence identity with MCMPCFTTDHQMARRCDDCCGGRGRGKCYGPQCLCR or a fragment thereof , wherein the fragment of A⁴ comprises at least 25 amino acid residues.

In certain aspects, R³, R⁴, R⁵, and R⁶ are each independently C₁-C₆ alkyl. In some aspects, R³, R⁴, R⁵, and R⁶ are each independently methyl. In certain aspects, R¹, R², R⁷, R⁸, R¹⁵, and R¹⁶ are each independently selected from hydrogen or sulfonate. In further aspects, R¹, R², R⁷, R⁸, R¹⁵, and R¹⁶ are each independently hydrogen. In some aspects, R¹², R¹³, R¹⁴, R¹⁹, R²⁰ are each independently hydrogen.

In certain aspects, R¹² and R¹³ join together along with the atoms to which they are attached to form a six-membered carbocyclic ring. In other aspects, R¹² and R¹³ join together along with the atoms to which they are attached to form a five-membered carbocyclic ring. In certain aspects, R¹⁴ and R¹⁹ join together along with the atoms to which they are attached to form a six-membered carbocyclic ring. In some aspects, R¹⁴ and R²⁰ join together along with the atoms to which they are attached to form a six-membered carbocyclic ring. In certain aspects, L¹ is C₃-C₆ alkylene. In other aspects, L¹ is C₃-C₅ alkylene. In still other aspects, L¹ is propylene. In still other aspects, L¹ is butylene. In other aspects, L¹ is pentylene. In some aspects, L² is C₃-C₆ alkylene. In other aspects, L² is propylene. In still other aspects, L² is butylene. In other aspects, L² is pentylene. R⁹ is sulfonate. In some aspects, R¹⁴ is hydrogen. In other aspects, R¹⁴ is -(L⁵)-aryl.

In some aspects, R¹ is hydrogen. In certain aspects, R² is hydrogen. In some aspects, R³ is methyl. In certain aspects, R⁴ is methyl. In some aspects, R⁵ is methyl. In certain aspects R⁶ is methyl. In some aspects, R⁷ is hydrogen. In certain aspects, R⁸ is hydrogen. In some aspects, R¹² is hydrogen. In certain aspects, R¹³ is hydrogen. In some aspects, R¹⁴ is hydrogen. In certain aspects, R¹⁹ is hydrogen. In some aspects, R²⁰ is hydrogen. In certain aspects, R¹⁰ is hydrogen. In some aspects, R¹¹ is hydrogen.

In some aspects, R¹⁷ and R¹⁸ are independently phenyl. In some aspects, L³ is selected from a bond, -O-, -NR¹⁰-, -NR¹⁰-C₁-C₆ alkylene-, -O-NR¹⁰-, or -NR¹⁰-L⁴-. In further aspects, L³ is a bond.

In some aspects, L⁴ is -heterocyclyl- or -heterocyclyl-C₁-C₆ alkylene-. In further aspects, L⁴ is -piperizinyl-(C₁-C₆ alkylene)-. In still further aspects, L⁴ is

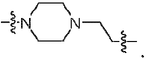

In some aspects, p is 1. In certain aspects, q is 1.

In some aspects, the compound has the structure of any one of Formulas (VII), (VIII), (IX), (XI), (XII), or (XIV): 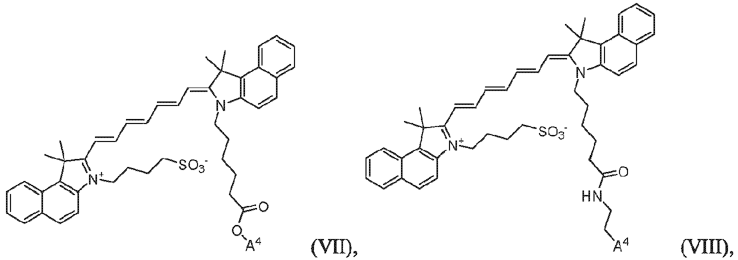 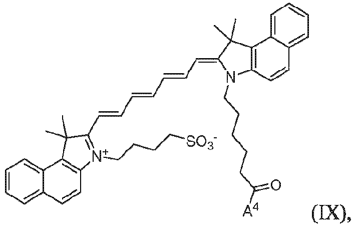 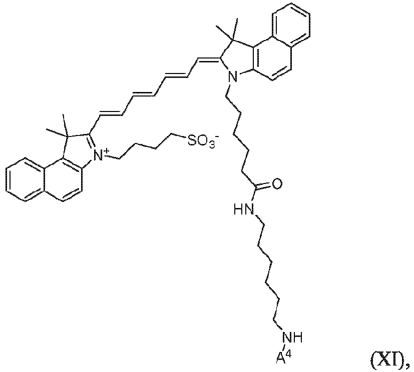 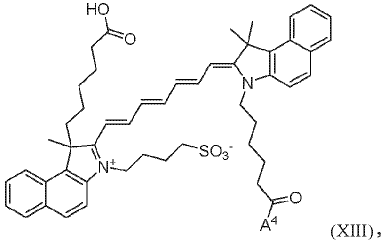 or

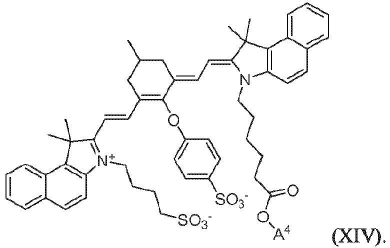

In still further aspects, A⁴ is a polypeptide having at least 92% sequence identity with MCMPCFTTDHQMARRCDDCCGGRGRGKCYGPQCLCR or a fragment thereof. In still further aspects, A⁴ is a polypeptide having at least 95% sequence identity with MCMPCFTTDHQMARRCDDCCGGRGRGKCYGPQCLCR or a fragment thereof. In still further aspects, A⁴ is a polypeptide having at least 97% sequence identity with MCMPCFTTDHQMARRCDDCCGGRGRGKCYGPQCLCR or a fragment thereof. In still further aspects, A⁴ is a polypeptide having 100% sequence identity with MCMPCFTTDHQMARRCDDCCGGRGRGKCYGPQCLCR or a fragment thereof. In still further aspects, A⁴ is a polypeptide having the sequence MCMPCFTTDHQMARRCDDCCGGRGRGKCYGPQCLCR or a fragment thereof.

The fragment of A⁴ has a length of at least 25 amino acid residues. In further aspects, the fragment of A⁴ has a length of at least 27 amino acid residues. In still further aspects, the fragment of A⁴ has a length of at least 29 amino acid residues. In still further aspects, the fragment of A⁴ has a length of at least 31 amino acid residues. In still further aspects, the fragment of A⁴ has a length of at least 33 amino acid residues.

The fragment thereof may have the tumor cell binding affinity of native chlorotoxin. The fragment thereof may have the tumor cell binding affinity of native chlorotoxin wherein one of A¹, A², A³, A⁴, or A⁵ has a sequence selected from SEQ ID NOS: 1-481.

In some aspects, the polypeptide comprises at least one lysine amino acid residue. In certain aspects, the polypeptide comprises a single lysine amino acid residue. In some aspects, the polypeptide comprises one, two, or three lysine amino acid residues. In some aspects, the polypeptide comprises a lysine residue at the position corresponding to K-27 of native chlorotoxin. In some aspects, the polypeptide comprises a lysine residue at the position corresponding to K-23 of native chlorotoxin. In some aspects, the polypeptide comprises a lysine residue at the position corresponding to K-15 of native chlorotoxin.

In some aspects, one or more of the amino acids of the polypeptide is substituted with a non-naturally occurring amino acid residue. In further aspects the non-naturally occurring amino acid residue is a citrulline amino acid residue. In still further aspects, L³ is attached to A⁴ at a citrulline amino acid residue of the polypeptide.

In some aspects, L³ is attached to A⁴ at a lysine amino acid residue of the polypeptide. In certain aspects, L³ is attached to A⁴ at the N-terminus of the polypeptide. In some aspects, L³ is attached to A⁴ at the C-terminus of the polypeptide. In some aspects, the R³ is attached to A¹ at a lysine amino acid residue of the peptide, a citrulline amino acid residue of the polypeptide, the N-terminus of the polypeptide, or the C-terminus of the polypeptide. In some aspects, the R⁵ is attached to A² at a lysine amino acid residue of the polypeptide, a citrulline amino acid residue of the polypeptide, the N-terminus of the polypeptide, or the C-terminus of the polypeptide. In some aspects, the R⁹ is attached to A³ at a lysine amino acid residue of the polypeptide, a citrulline amino acid residue of the polypeptide, the N-terminus of the polypeptide, or the C-terminus of the polypeptide. In some aspects, the aryl is attached to A⁵ at a lysine amino acid residue of the polypeptide, a citrulline amino acid residue of the polypeptide, the N-terminus of the polypeptide, or the C-terminus of the polypeptide.

In some aspects, the compound has the structure of any one of the compounds found in Tables 2-13 that are encompassed by claim 1.

In some aspects, the compound is conjugated to polyethylene glycol (PEG), hydroxyethyl starch, polyvinyl alcohol, a water soluble polymer, a zwitterionic water soluble polymer, a water soluble poly(amino acid), an albumin derivative, or a fatty acid.

In some aspects, the polypeptide has an isoelectric point of from 7.5 to 9.0. In some aspects, the polypeptide has an isoelectric point of from 8.0 to 9.0. In some aspects, the polypeptide has an isoelectric point of from 8.5 to 9.0. In some aspects, the polypeptide is basic and has an isoelectric point of greater than 7.5.

In some aspects, the polypeptide comprises at least eight cysteine amino acid residues. In some aspects, the polypeptide comprises eight cysteine amino acid residues. In some aspects, the polypeptide comprises four disulfide bonds. In some aspects, the polypeptide comprises from six to seven cysteine amino acid residues. In some aspects, the polypeptide comprises three disulfide bonds. In some aspects, the spacing between the cysteine amino acid residues in the polypeptide is essentially the same as in native chlorotoxin. In some aspects, the distribution of charge on the surface of the polypeptide is essentially the same as in native chlorotoxin.

In some aspects, one or more of the methionine amino acid residues is replaced with an amino acid residue selected from isoleucine, threonine, valine, leucine, serine, glycine, alanine, or a combination thereof.

In some aspects, the compound is capable of passing across the blood brain barrier. In some aspects, the compound further comprises a therapeutic agent attached to A. In further aspects, the therapeutic agent is a cytotoxic agent.

The compounds of the invention are as defined in claim 1. Certain compounds are provided below in Tables 2 to 13, including both the peptide portion (indicated by A) and the detectable label portion.

**Table 2. Compounds.**

| A = MCMPCFTTDHQMARRCDDCCGGRGRGKCYGPQCLCR (SEQ ID NO: 9) (attached at K-27) | | | |
|---|---|---|---|
| **No.** | **Structure** | **No.** | **Strucure** |
| 1 | 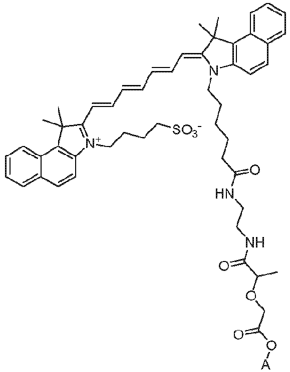 | 31 | 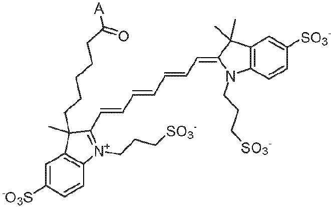 |
| 2 | 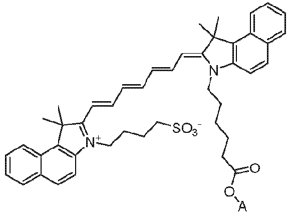 | 32 | 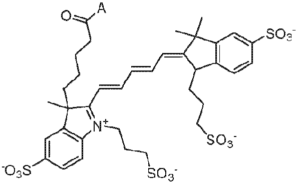 |
| 3 | 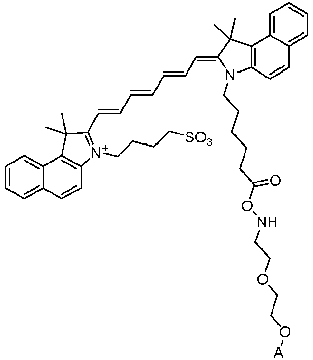 | 33 | 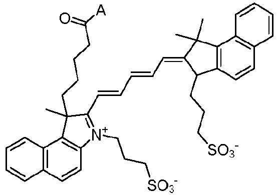 |
| 4 | 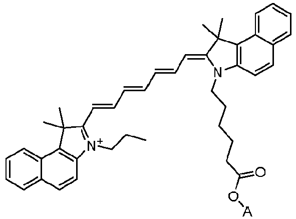 | 34 | 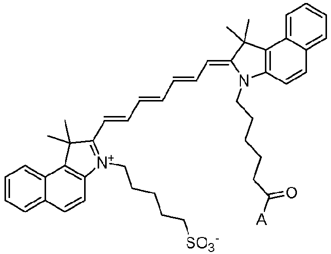 |
| 5 | 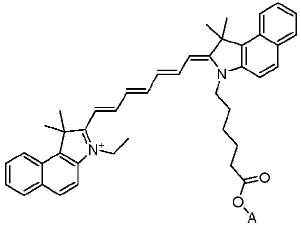 | 35 | 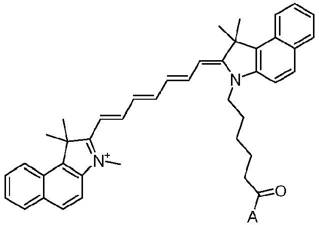 |
| 6 | 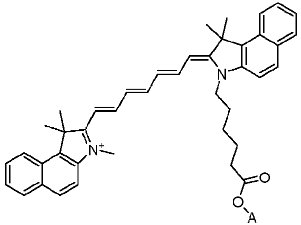 | 36 | 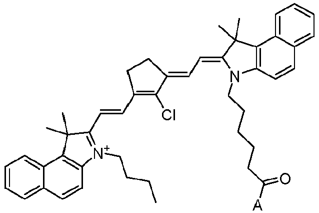 |
| 7 | 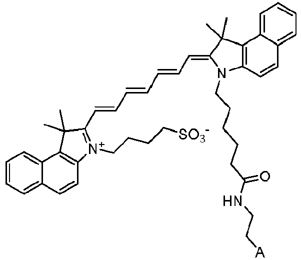 | 37 | 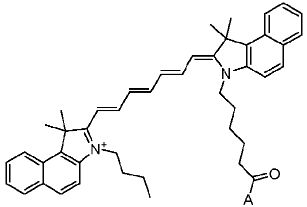 |
| 8 | 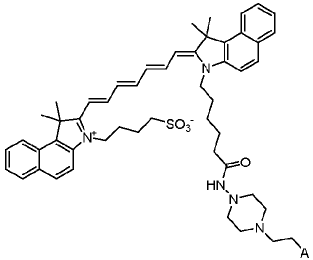 | 38 | 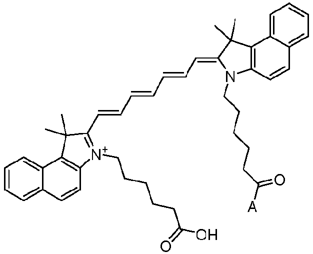 |
| 9 | 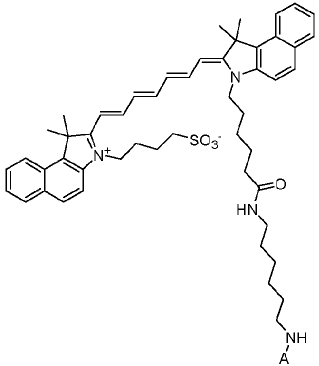 | 39 | 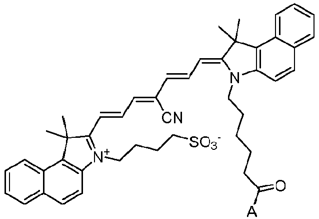 |
| 10 | 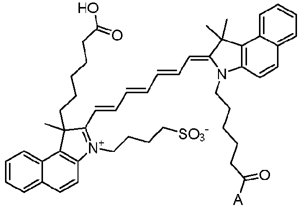 | 40 | 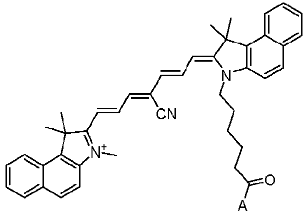 |
| 11 | 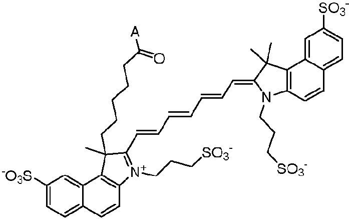 | 41 | 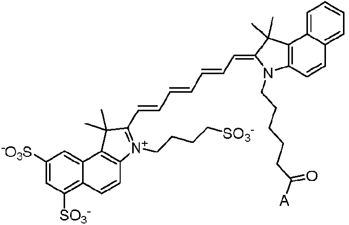 |
| 12 | 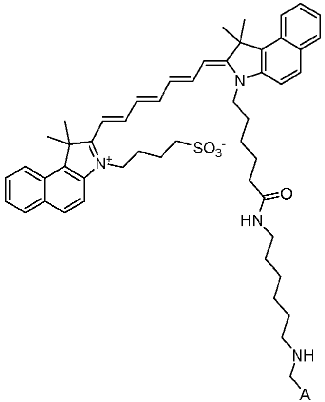 | 42 | 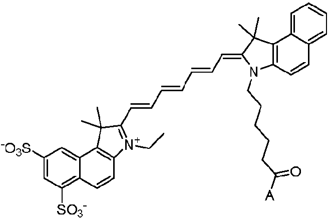 |
| 13 | 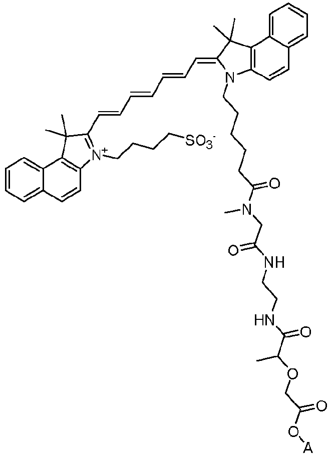 | 43 | 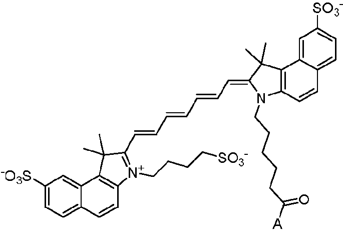 |
| 14 | 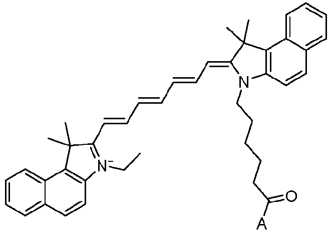 | 44 | 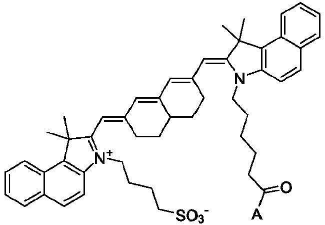 |
| 15 | 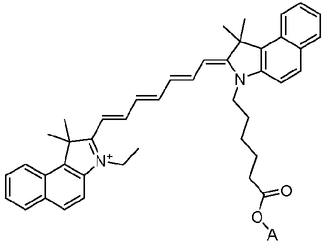 | 45 | 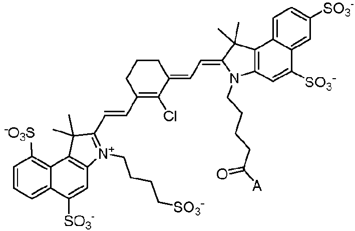 |
| 16 | 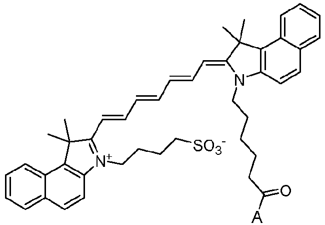 | 46 | 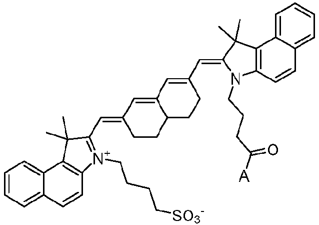 |
| 17 | 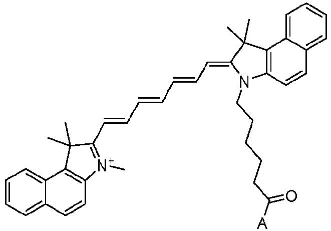 | 47 | 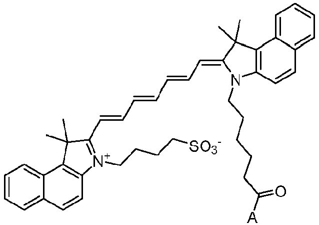 |
| 18 | 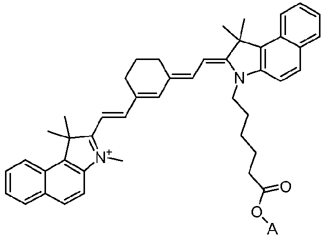 | 48 | 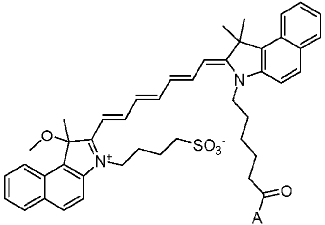 |
| 19 | 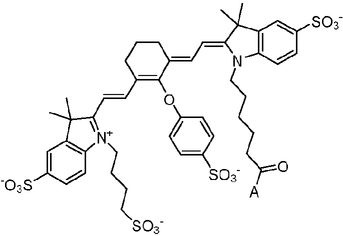 | 49 | 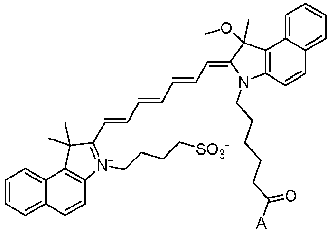 |
| 20 | 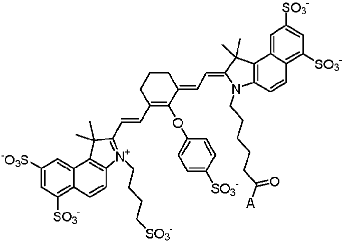 | 50 | 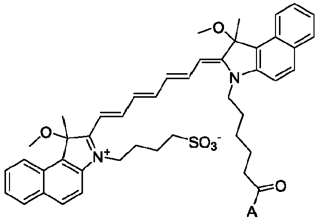 |
| 21 | 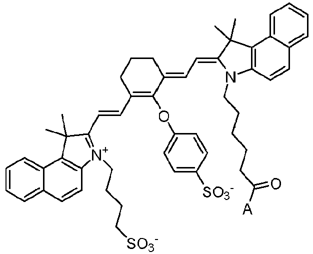 | 51 | 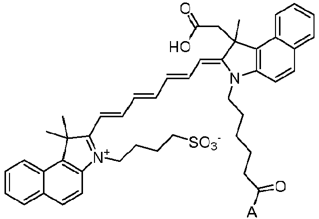 |
| 22 | 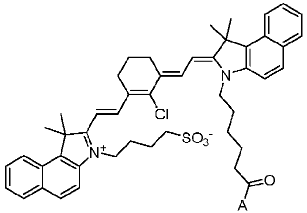 | 52 | 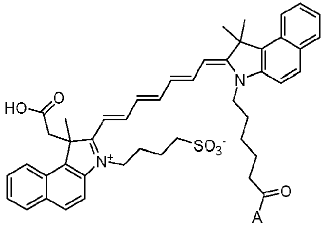 |
| 23 | 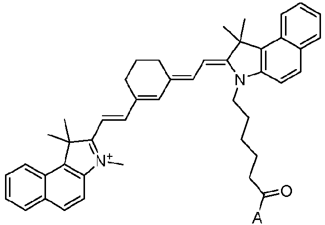 | 53 | 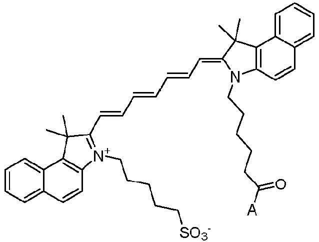 |
| 24 | 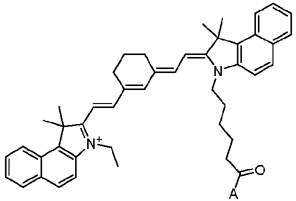 | 54 | 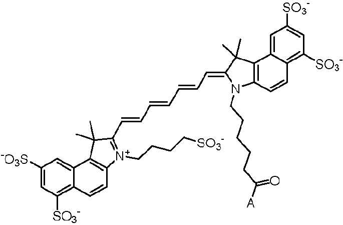 |
| 25 | 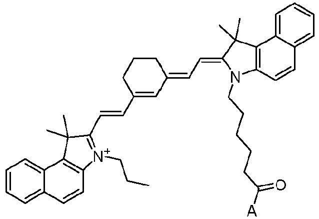 | 55 | 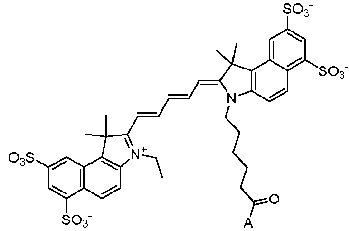 |
| 26 | 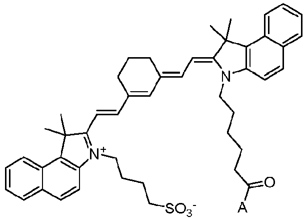 | 56 | 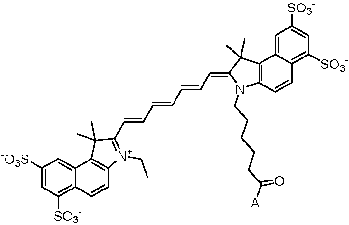 |
| 27 | 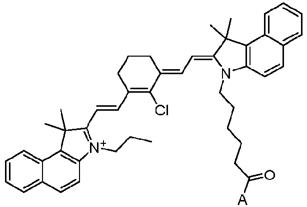 | 57 | 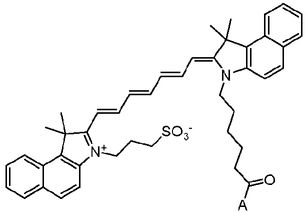 |
| 28 | 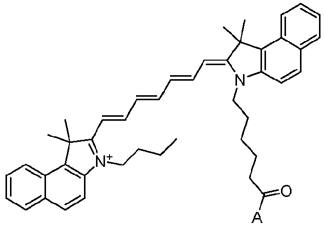 | 58 | 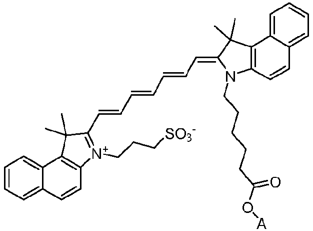 |
| 29 | 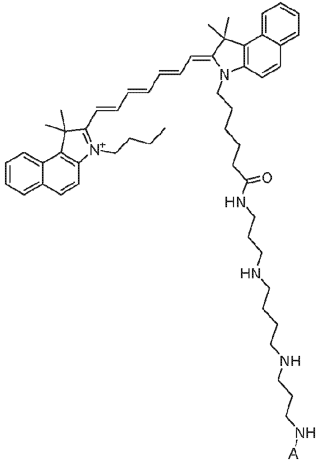 | 59 | 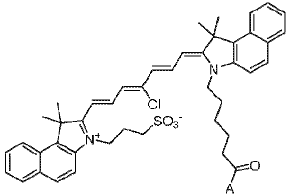 |
| 30 | 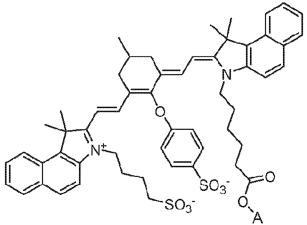 | 60 | 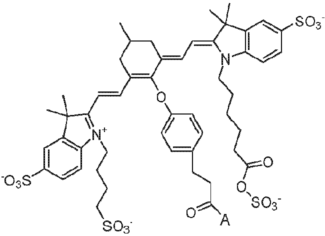 |

**Table 3. Compounds.**

| A = MCMPCFTTDHQMARACDDCCGGAGRGKCYGPQCLCR (SEQ ID NO: 5) (attached at K-27) | | | |
|---|---|---|---|
| **No.** | **Structure** | **No.** | **Strucure** |
| 61 | 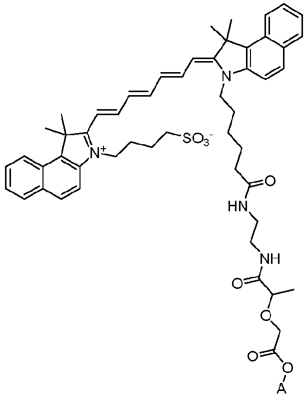 | 91 | 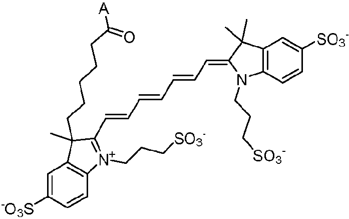 |
| 62 | 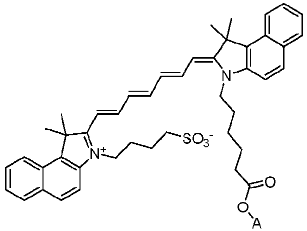 | 92 | 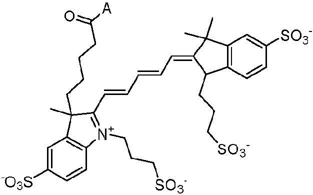 |
| 63 | 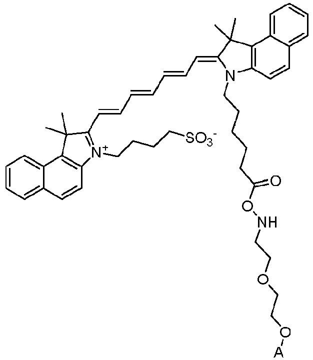 | 93 | 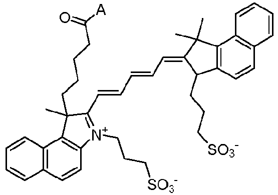 |
| 64 | 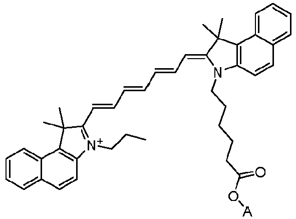 | 94 | 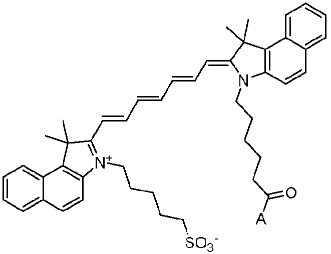 |
| 65 | 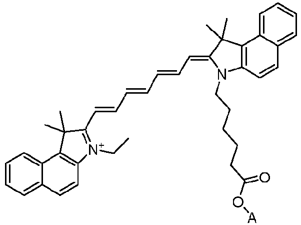 | 95 | 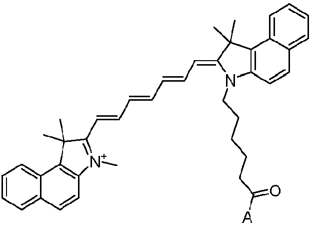 |
| 66 | 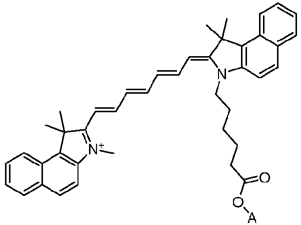 | 96 | 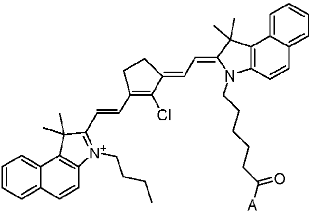 |
| 67 | 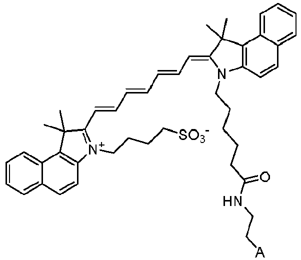 | 97 | 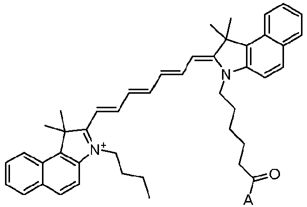 |
| 68 | 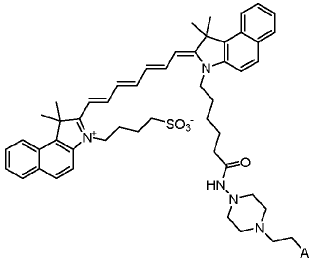 | 98 | 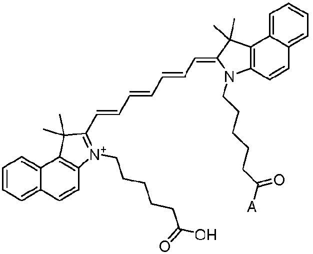 |
| 69 | 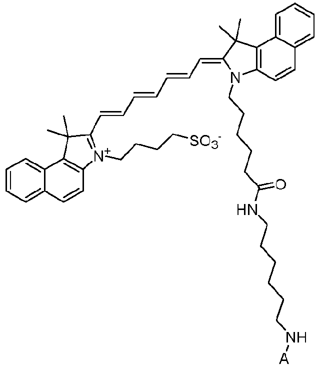 | 99 | 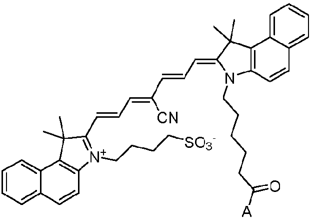 |
| 70 | 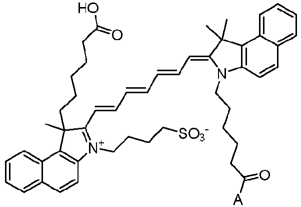 | 100 | 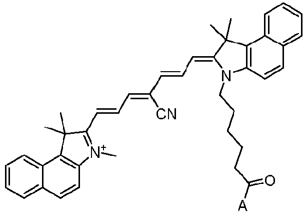 |
| 71 | 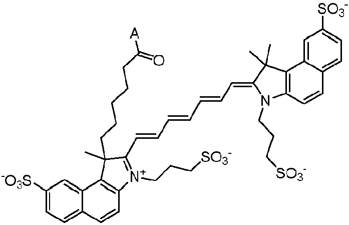 | 101 | 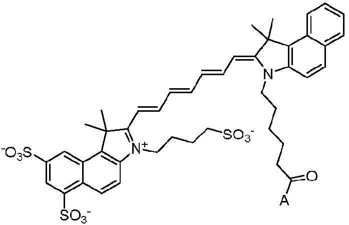 |
| 72 | 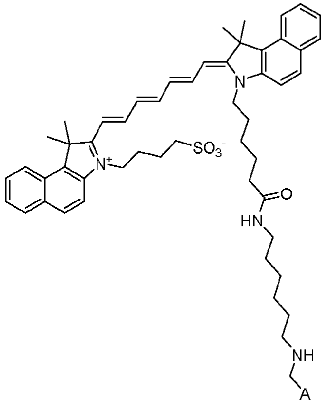 | 102 | 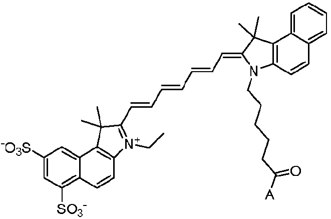 |
| 73 | 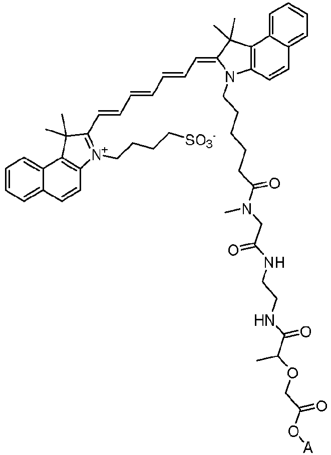 | 103 | 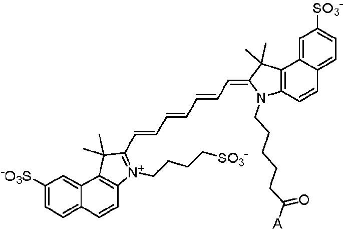 |
| 74 | 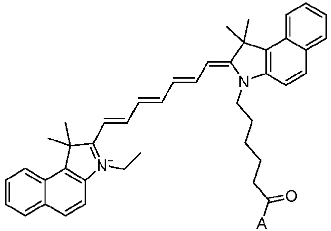 | 104 | 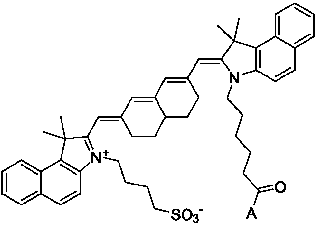 |
| 75 | 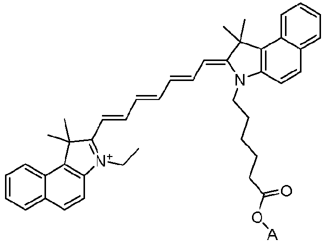 | 105 | 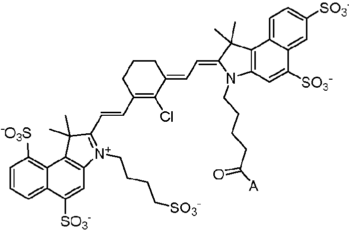 |
| 76 | 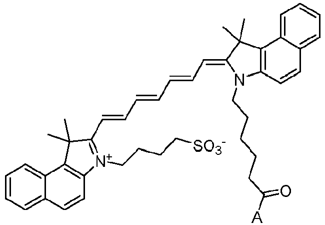 | 106 | 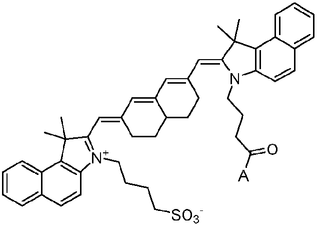 |
| 77 | 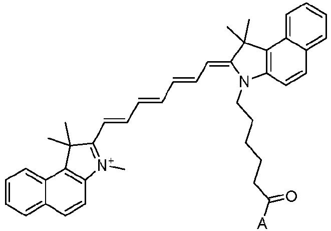 | 107 | 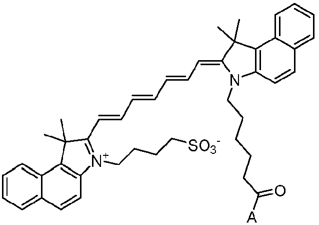 |
| 78 | 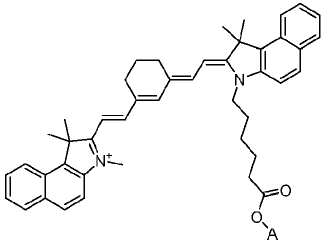 | 108 | 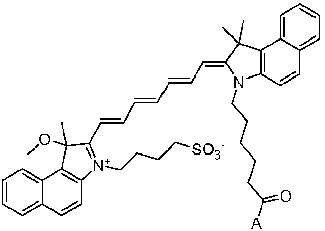 |
| 79 | 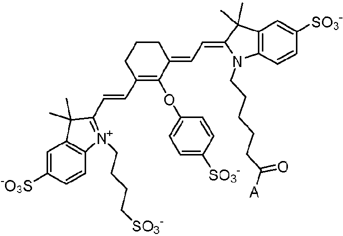 | 109 | 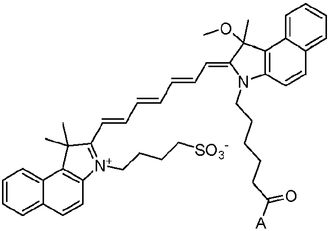 |
| 80 | 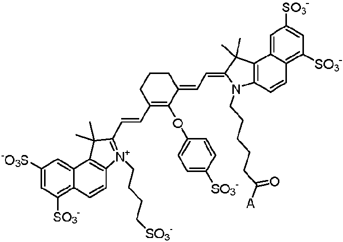 | 110 | 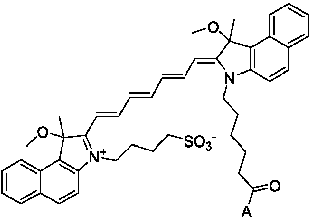 |
| 81 | 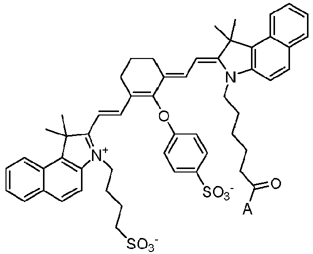 | 111 | 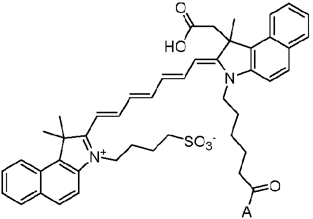 |
| 82 | 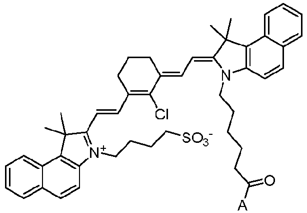 | 112 | 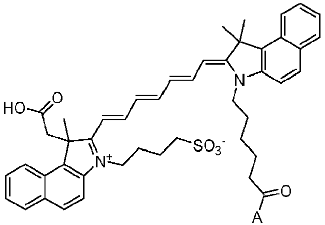 |
| 83 | 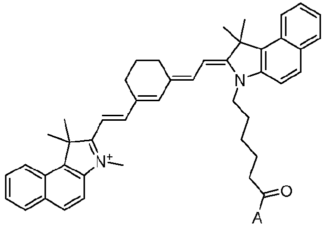 | 113 | 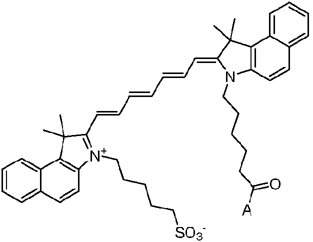 |
| 84 | 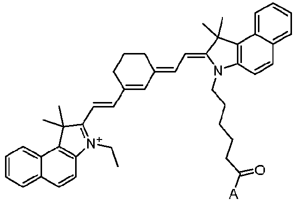 | 114 | 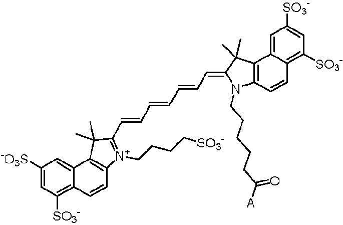 |
| 85 | 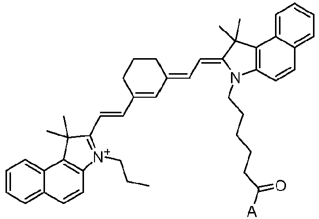 | 115 | 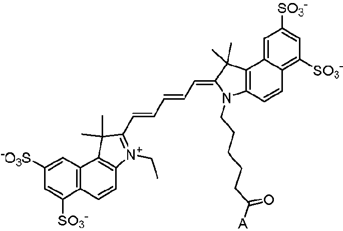 |
| 86 | 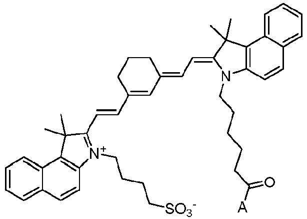 | 116 | 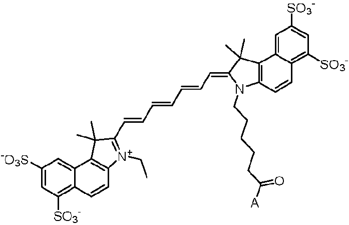 |
| 87 | 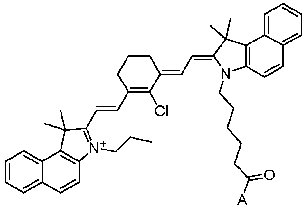 | 117 | 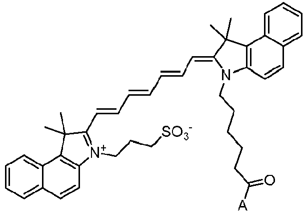 |
| 88 | 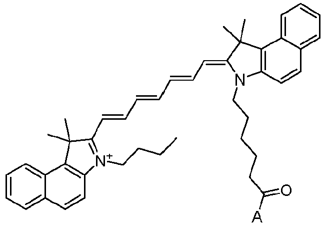 | 118 | 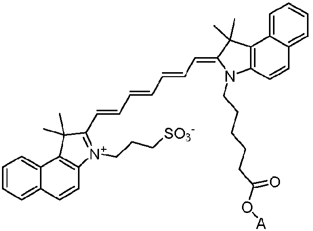 |
| 89 | 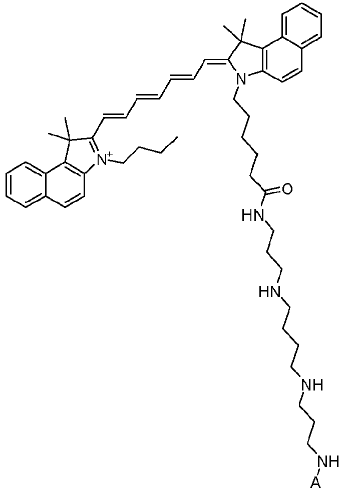 | 119 | 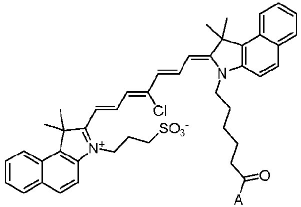 |
| 90 | 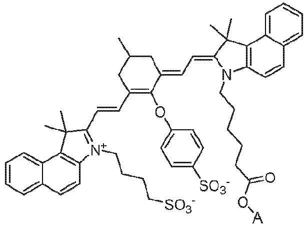 | 120 | 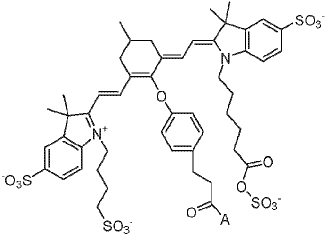 |

**Table 4. Compounds.**

| A = MCMPCFTTDHQMARRCDDCCGGAGRG**K**CYGPQCLCR (SEQ ID NO: 6) (attached at K-27) | | | |
|---|---|---|---|
| **No.** | **Structure** | **No.** | **Strucure** |
| 121 | 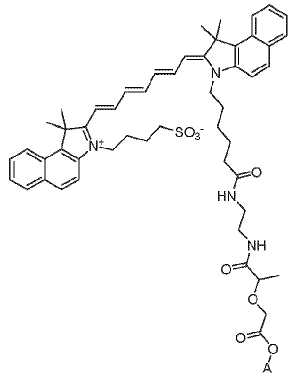 | 151 | 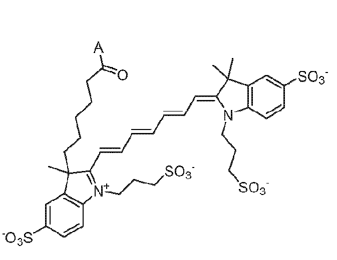 |
| 122 | 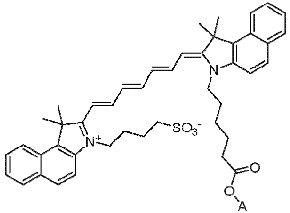 | 152 | 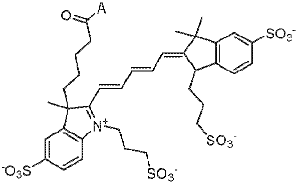 |
| 123 | 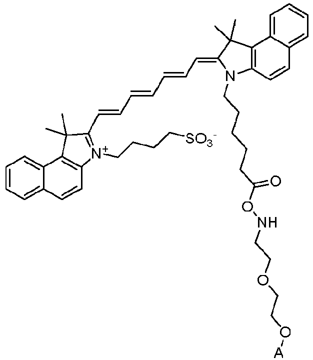 | 153 | 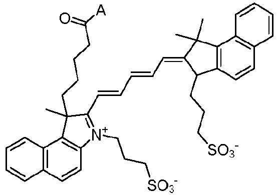 |
| 124 | 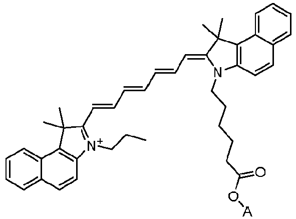 | 154 | 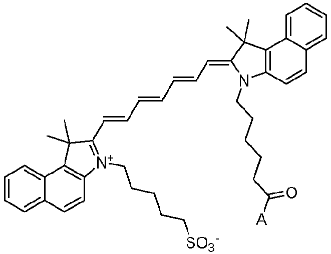 |
| 125 | 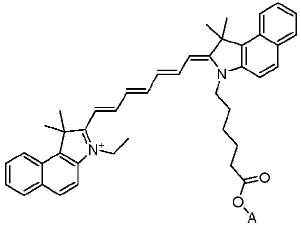 | 155 | 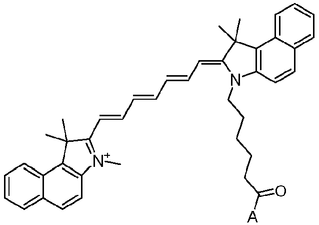 |
| 126 | 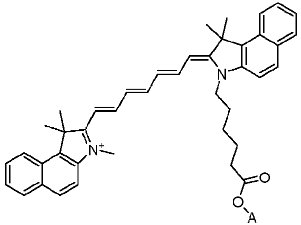 | 156 | 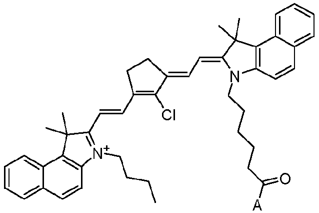 |
| 127 | 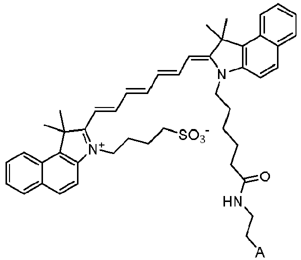 | 157 | 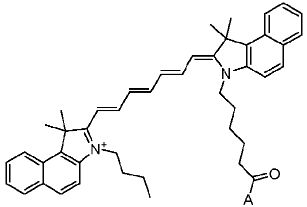 |
| 128 | 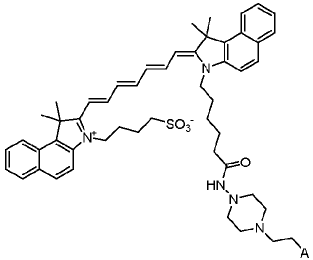 | 158 | 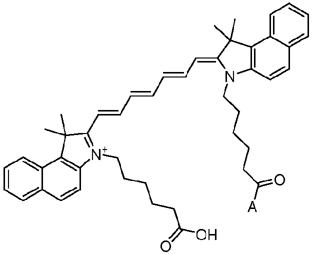 |
| 129 | 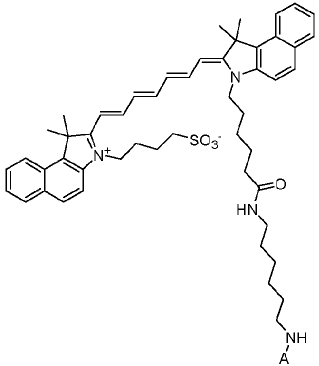 | 159 | 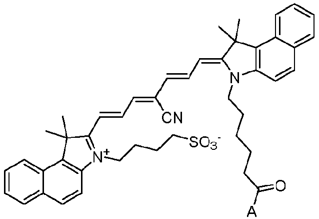 |
| 130 | 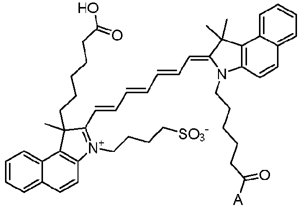 | 160 | 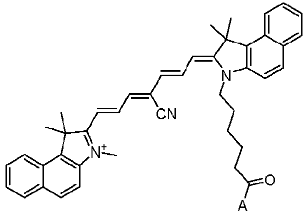 |
| 131 | 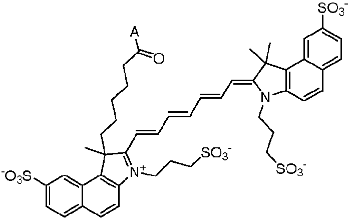 | 161 | 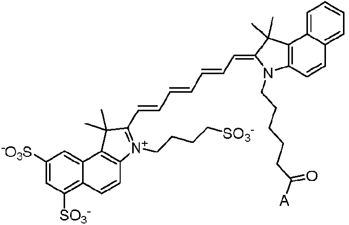 |
| 132 | 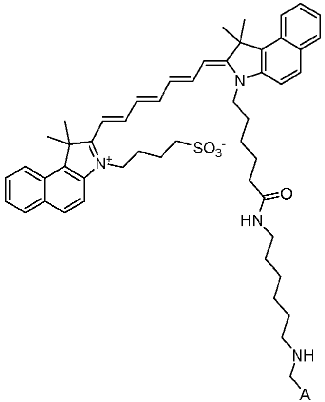 | 162 | 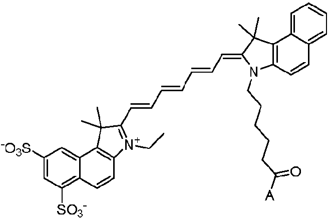 |
| 133 | 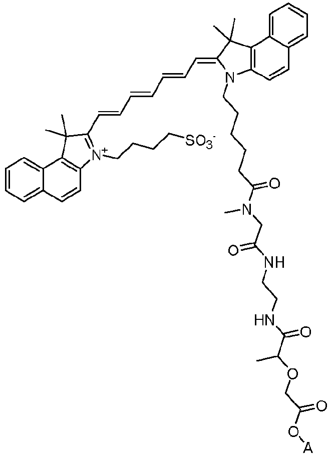 | 163 | 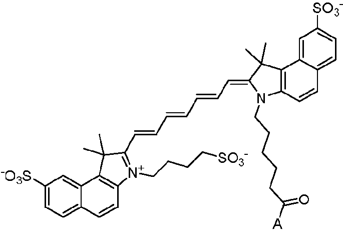 |
| 134 | 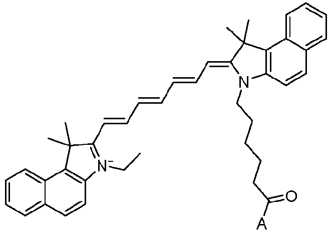 | 164 | 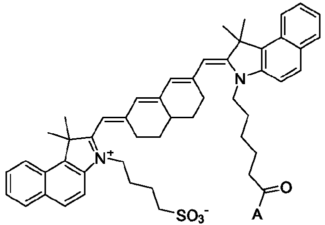 |
| 135 | 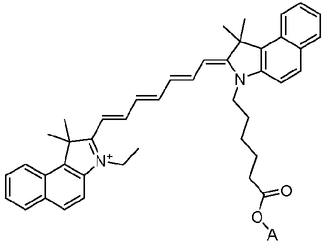 | 165 | 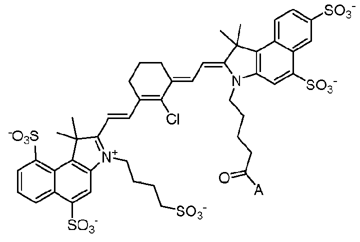 |
| 136 | 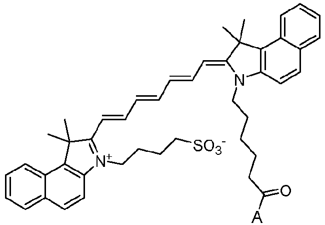 | 166 | 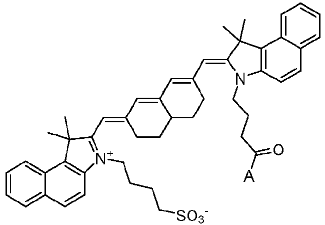 |
| 137 | 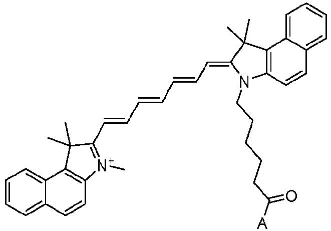 | 167 | 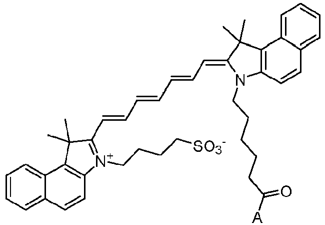 |
| 138 | 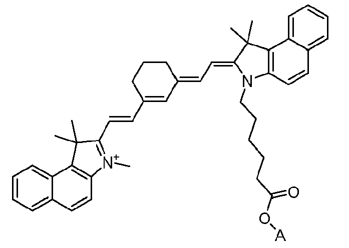 | 168 | 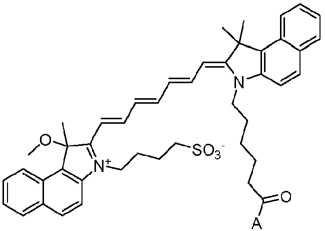 |
| 139 | 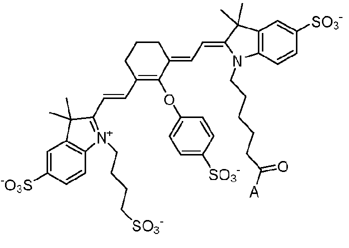 | 169 | 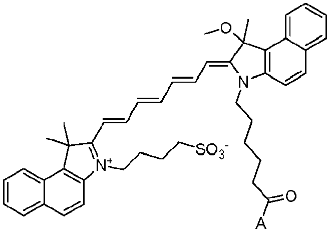 |
| 140 | 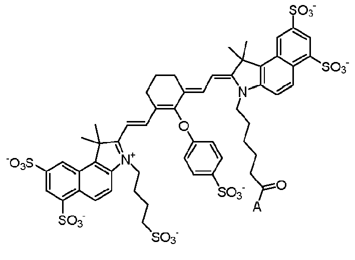 | 170 | 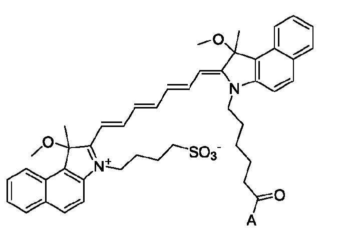 |
| 141 | 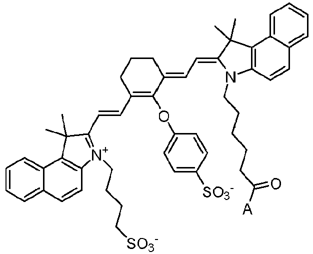 | 171 | 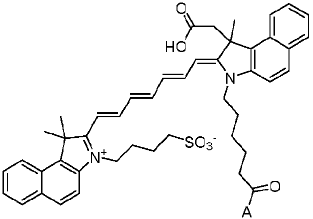 |
| 142 | 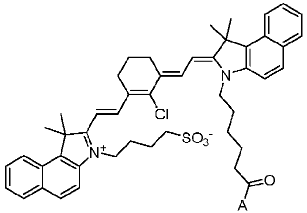 | 172 | 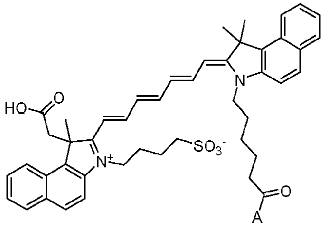 |
| 143 | 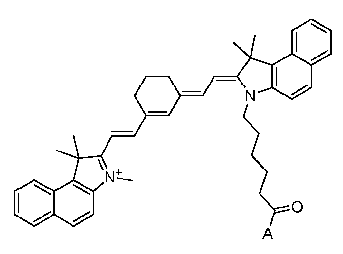 | 173 | 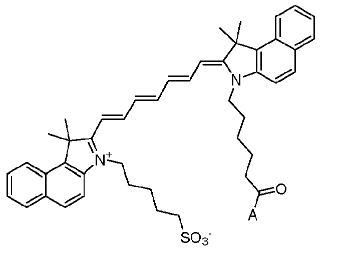 |
| 144 | 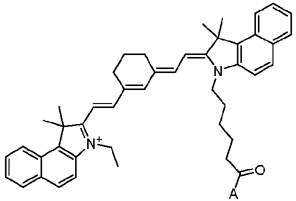 | 174 | 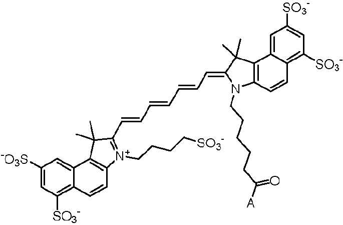 |
| 145 | 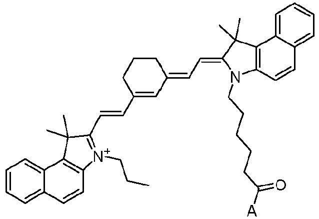 | 175 | 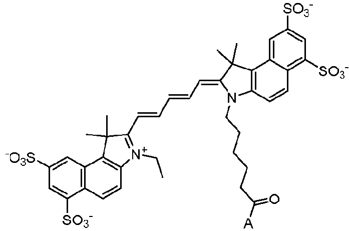 |
| 146 | 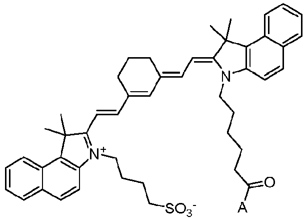 | 176 | 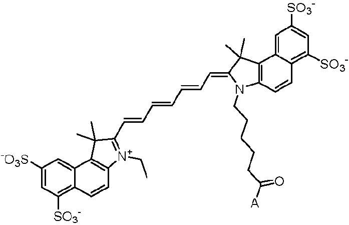 |
| 147 | 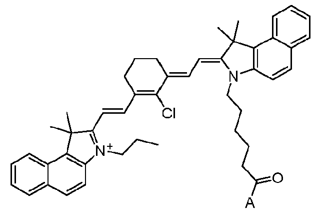 | 177 | 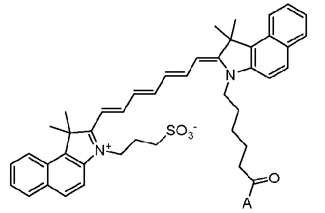 |
| 148 | 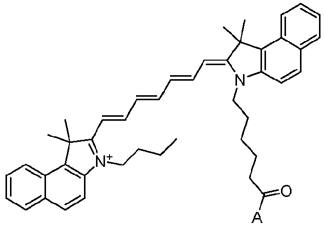 | 178 | 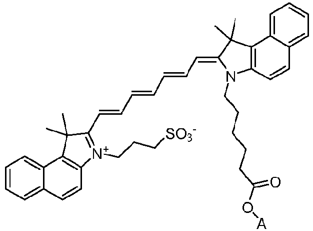 |
| 149 | 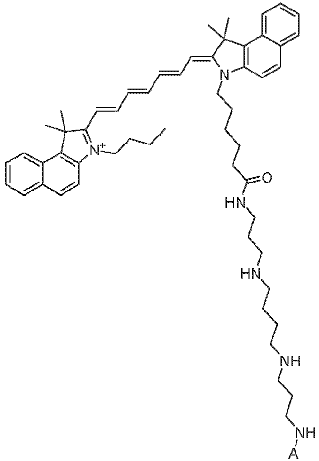 | 179 | 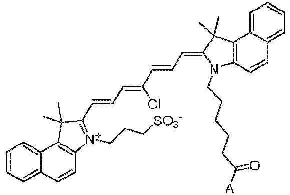 |
| 150 | 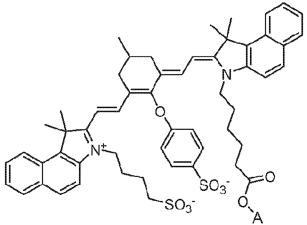 | 180 | 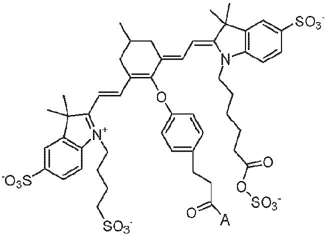 |

**Table 5. Compounds.**

| A = MCMPCFTTDHQMARACDDCCGGRGRG**K**CYGPQCLCR (SEQ ID NO: 8) (attached at K-27) | | | |
|---|---|---|---|
| **No.** | **Structure** | **No.** | **Strucure** |
| 181 | 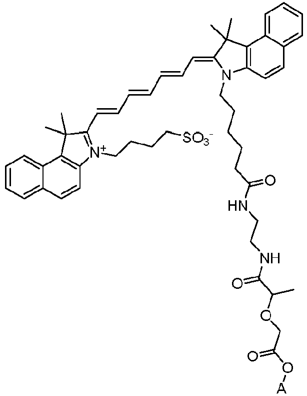 | 211 | 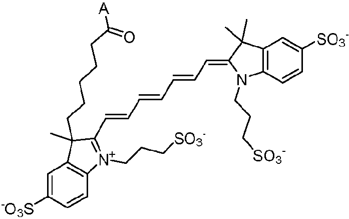 |
| 182 | 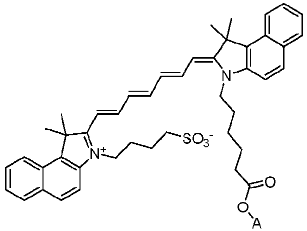 | 212 | 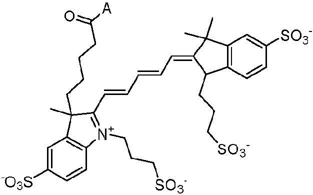 |
| 183 | 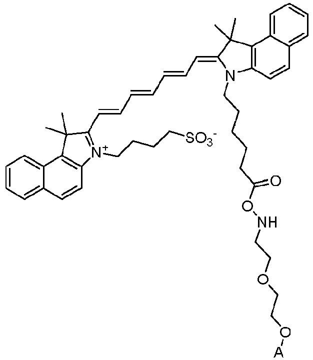 | 213 | 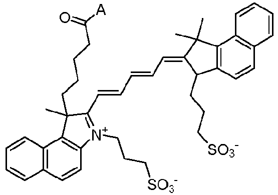 |
| 184 | 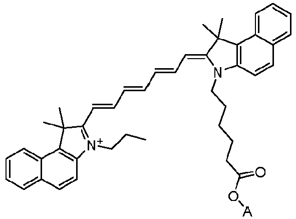 | 214 | 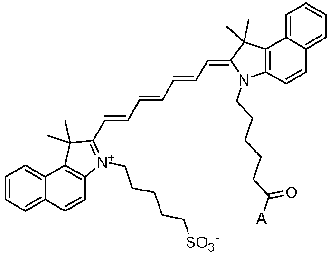 |
| 185 | 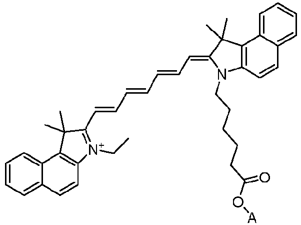 | 215 | 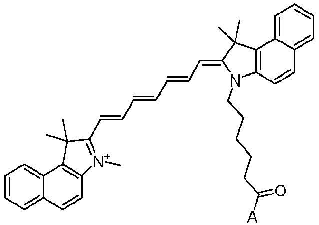 |
| 186 | 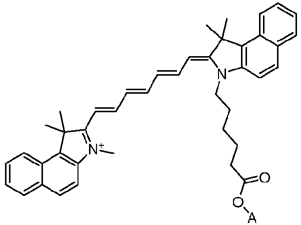 | 216 | 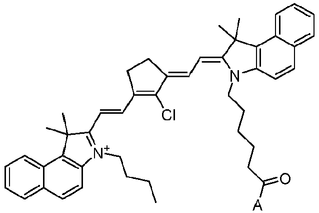 |
| 187 | 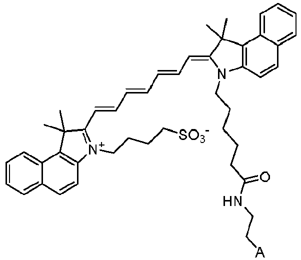 | 217 | 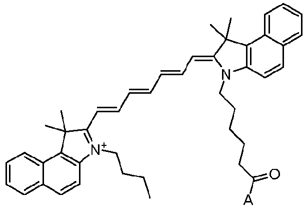 |
| 188 | 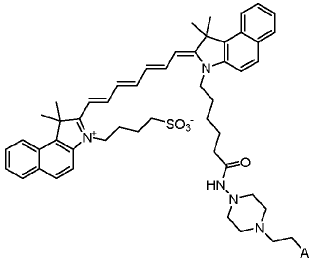 | 218 | 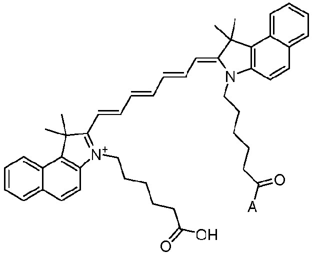 |
| 189 | 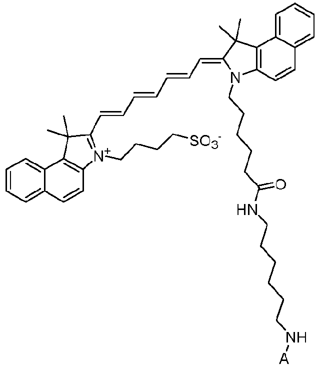 | 219 | 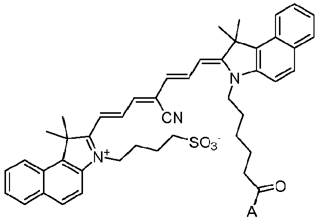 |
| 190 | 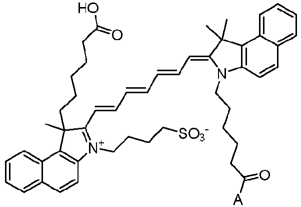 | 220 | 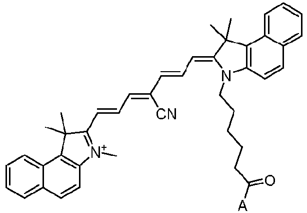 |
| 191 | 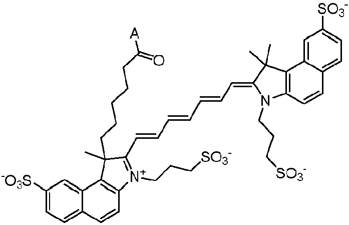 | 221 | 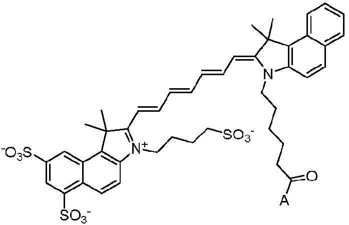 |
| 192 | 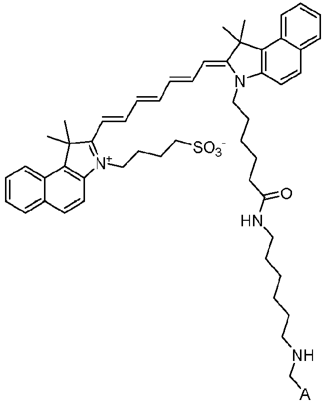 | 222 | 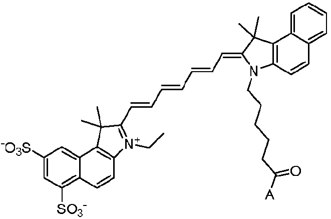 |
| 193 | 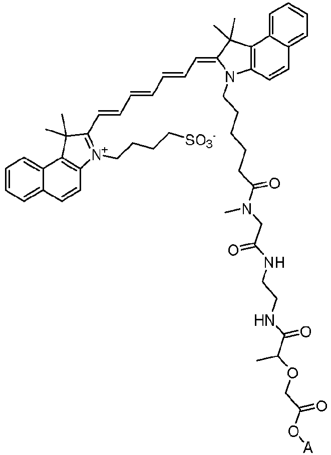 | 223 | 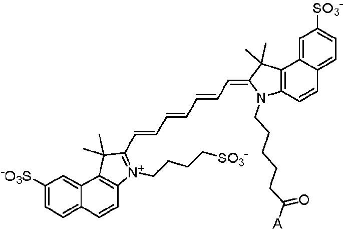 |
| 194 | 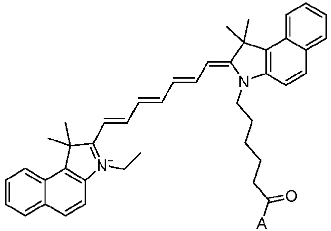 | 224 | 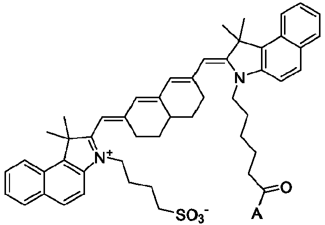 |
| 195 | 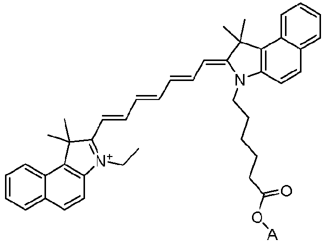 | 225 | 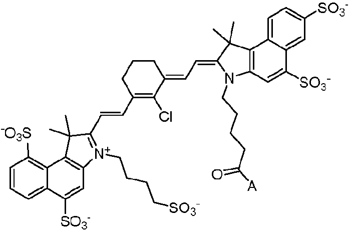 |
| 196 | 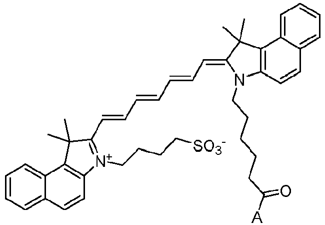 | 226 | 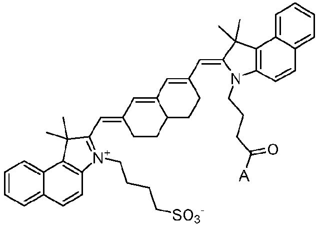 |
| 197 | 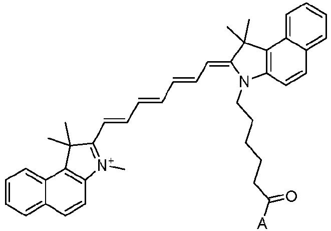 | 227 | 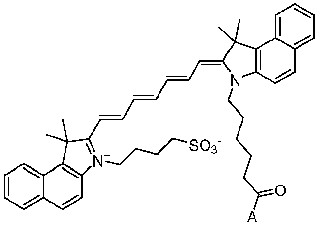 |
| 198 | 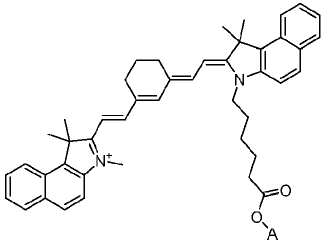 | 228 | 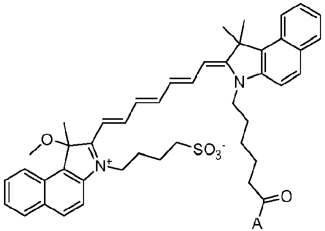 |
| 199 | 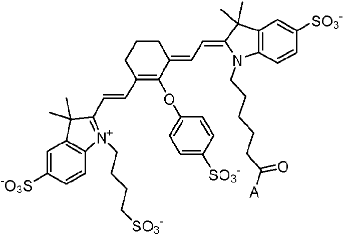 | 229 | 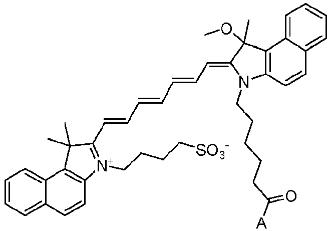 |
| 200 | 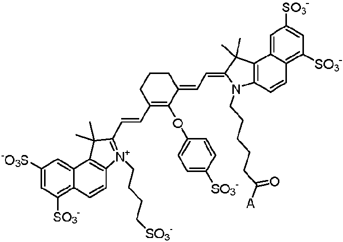 | 230 | 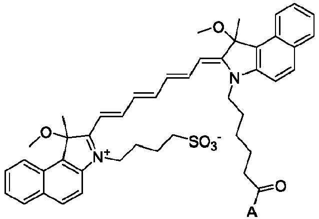 |
| 201 | 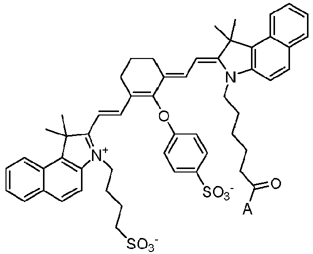 | 231 | 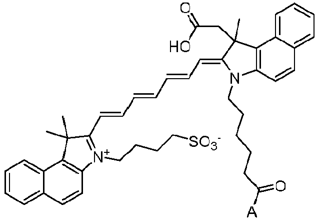 |
| 202 | 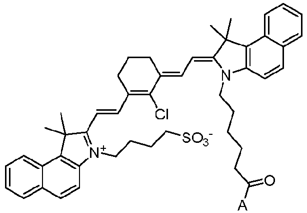 | 232 | 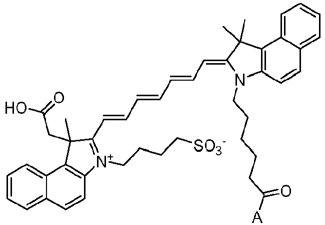 |
| 203 | 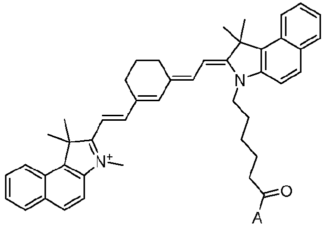 | 233 | 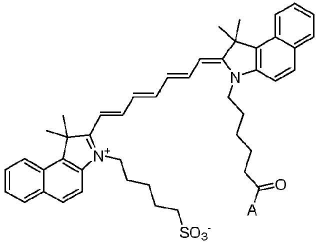 |
| 204 | 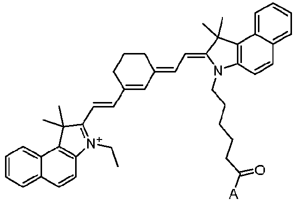 | 234 | 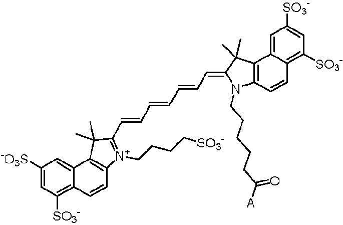 |
| 205 | 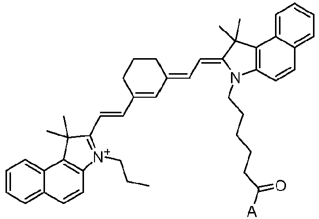 | 235 | 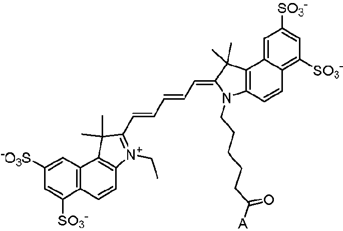 |
| 206 | 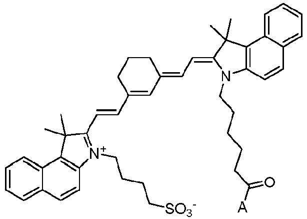 | 236 | 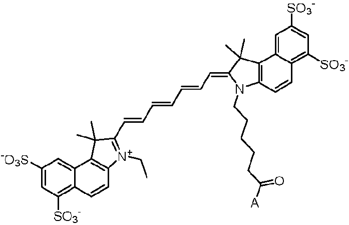 |
| 207 | 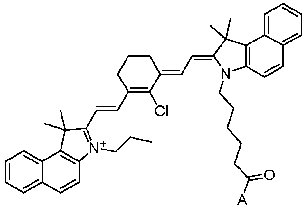 | 237 | 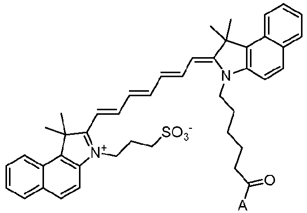 |
| 208 | 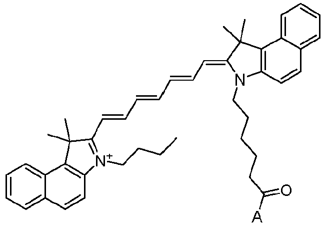 | 238 | 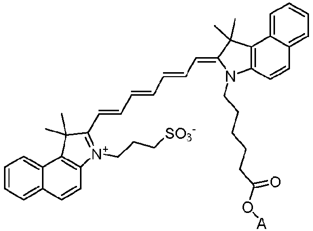 |
| 209 | 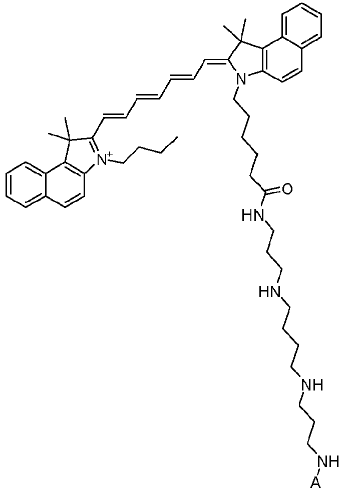 | 239 | 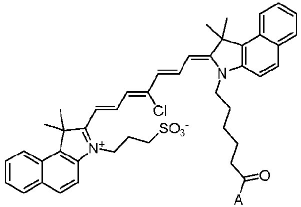 |
| 210 | 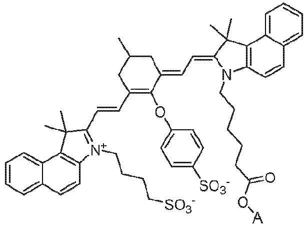 | 240 | 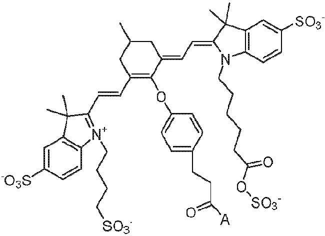 |

**Table 6. Compounds.**

| A = MCMPCFTTDHQMARACDDCCGG**K**GRGACYGPQCLCR (SEQ ID NO: 11) (attached at K-23) | | | |
|---|---|---|---|
| **No.** | **Structure** | **No.** | **Strucure** |
| 241 | 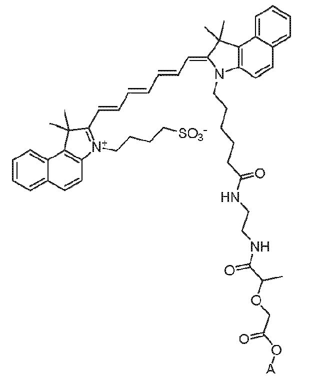 | 271 | 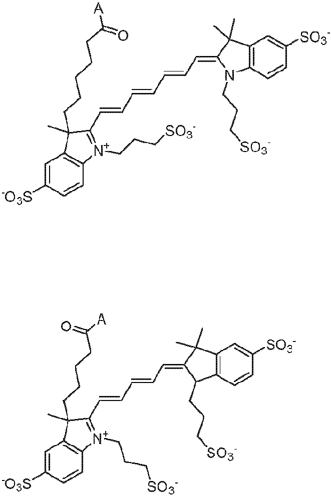 |
| 242 | 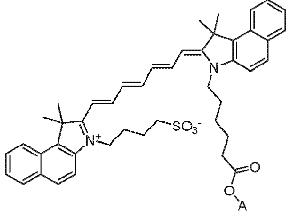 | 272 | 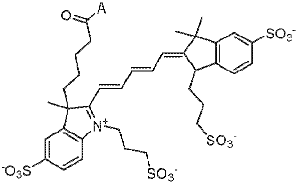 |
| 243 | 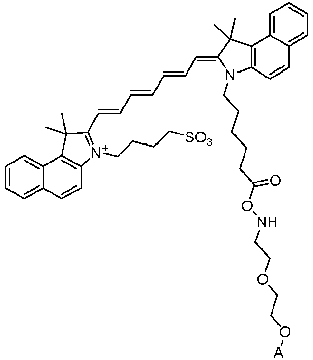 | 273 | 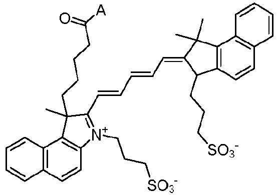 |
| 244 | 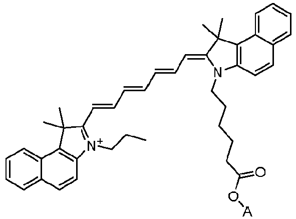 | 274 | 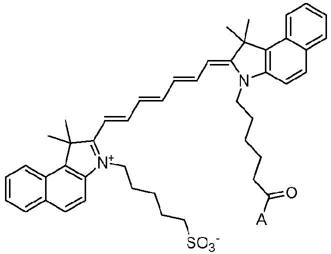 |
| 245 | 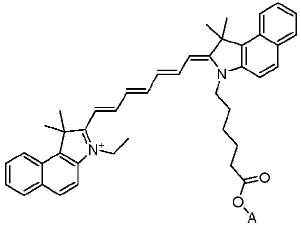 | 275 | 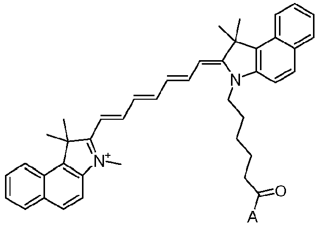 |
| 246 | 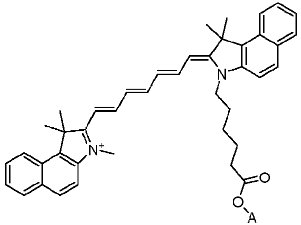 | 276 | 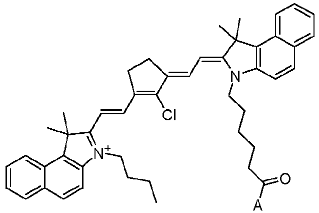 |
| 247 | 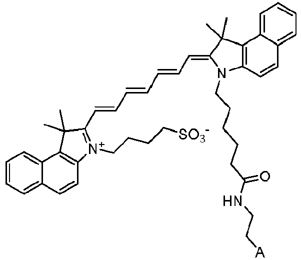 | 277 | 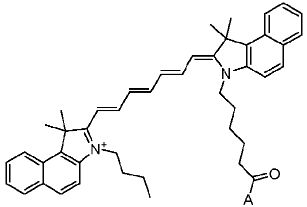 |
| 248 | 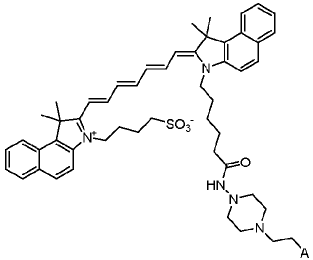 | 278 | 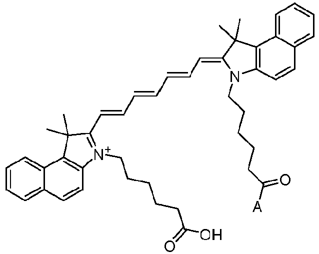 |
| 249 | 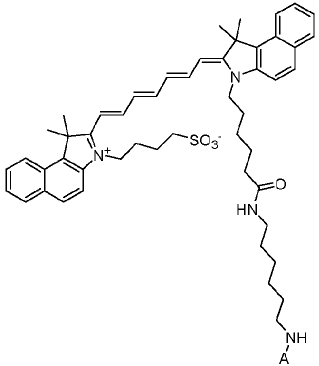 | 279 | 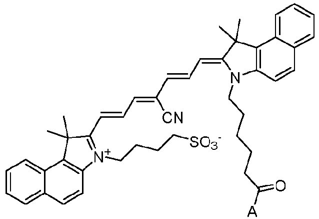 |
| 250 | 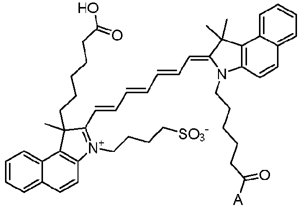 | 280 | 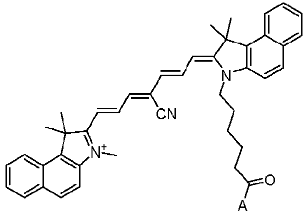 |
| 251 | 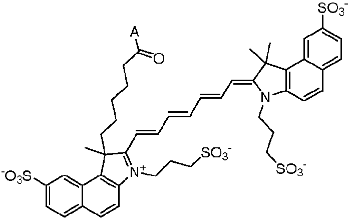 | 281 | 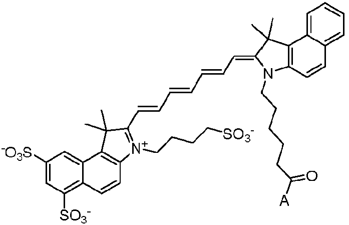 |
| 252 | 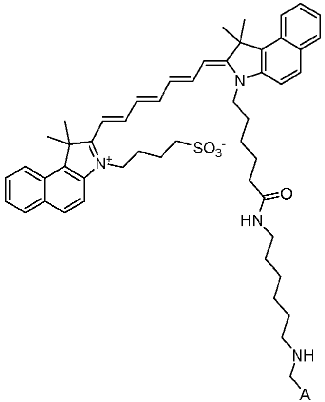 | 282 | 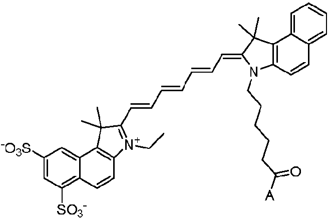 |
| 253 | 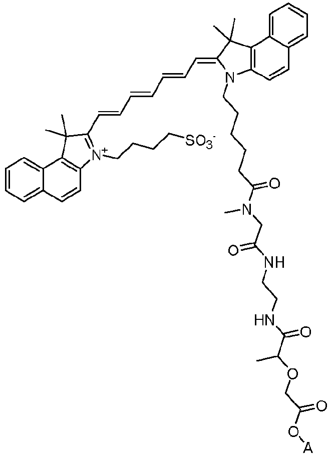 | 283 | 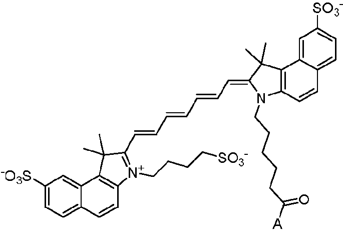 |
| 254 | 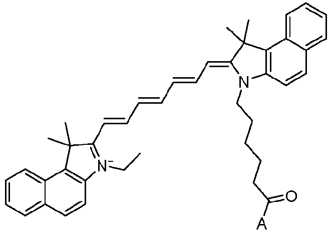 | 284 | 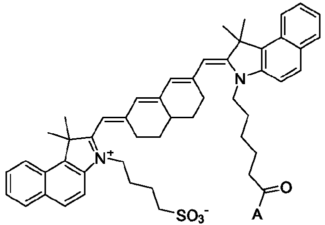 |
| 255 | 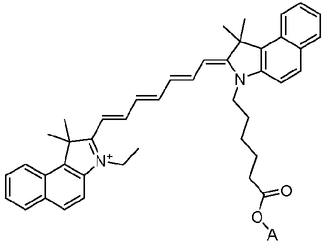 | 285 | 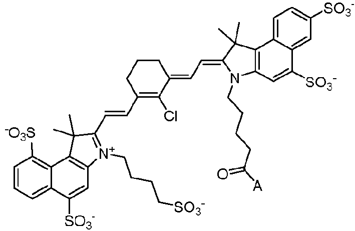 |
| 256 | 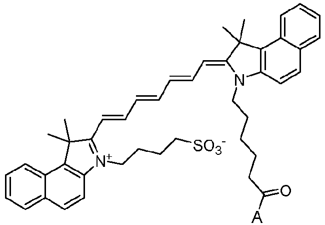 | 286 | 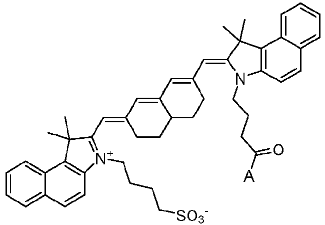 |
| 257 | 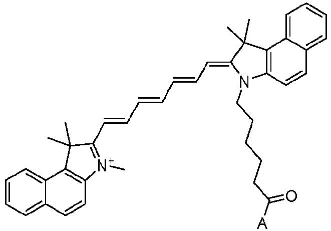 | 287 | 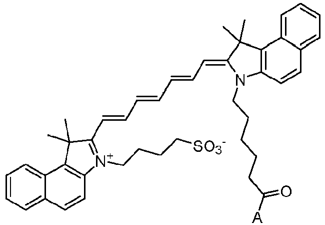 |
| 258 | 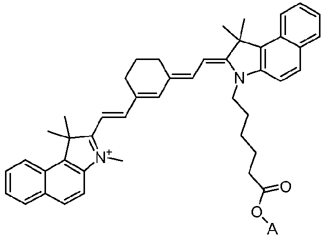 | 288 | 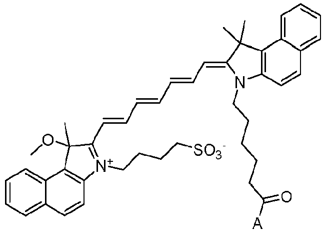 |
| 259 | 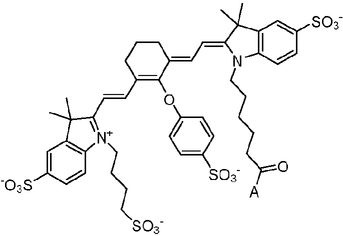 | 289 | 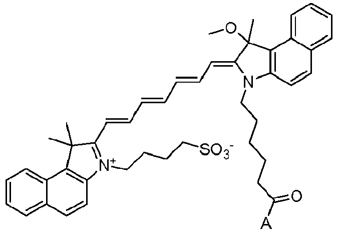 |
| 260 | 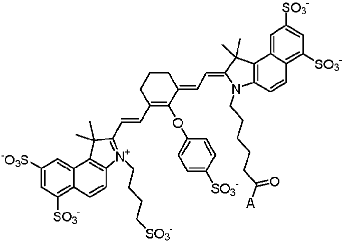 | 290 | 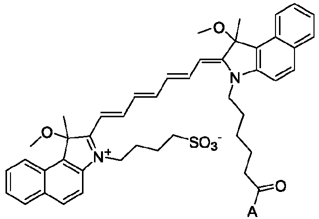 |
| 261 | 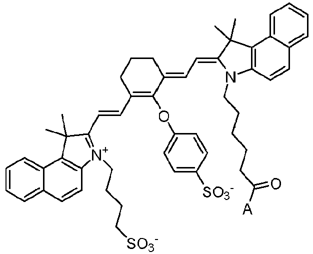 | 291 | 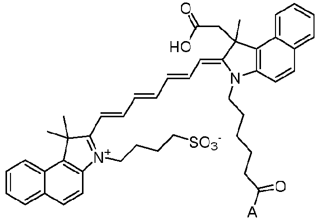 |
| 262 | 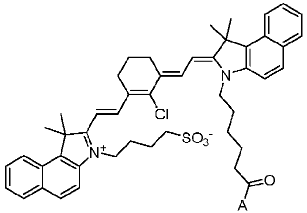 | 292 | 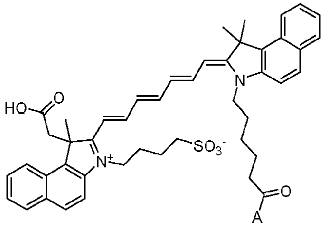 |
| 263 | 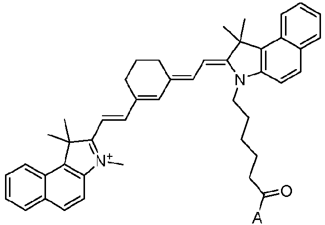 | 293 | 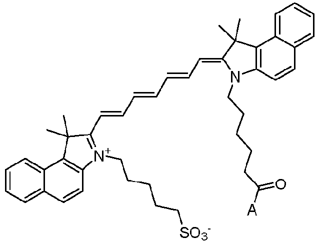 |
| 264 | 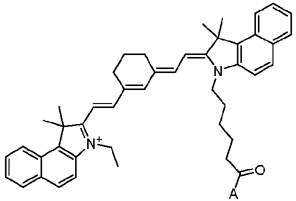 | 294 | 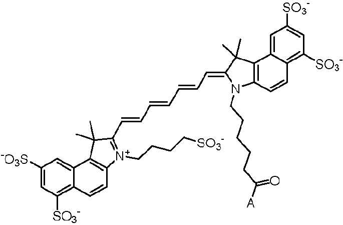 |
| 265 | 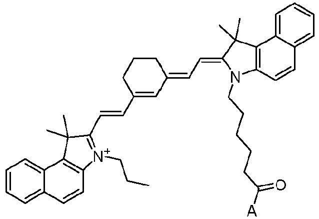 | 295 | 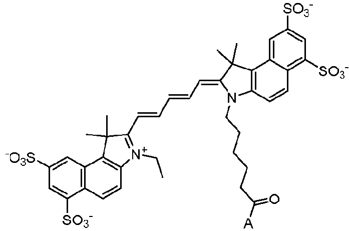 |
| 266 | 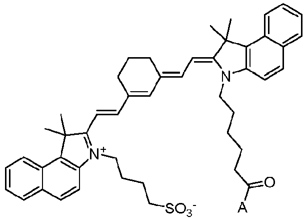 | 296 | 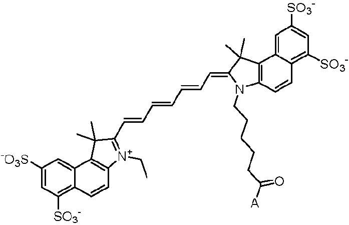 |
| 267 | 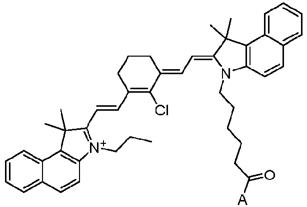 | 297 | 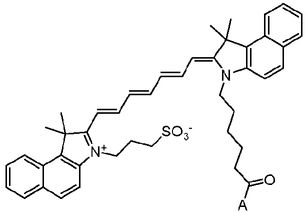 |
| 268 | 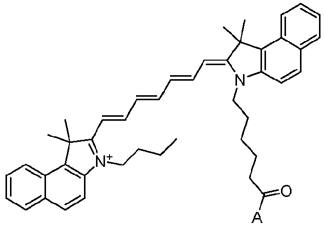 | 298 | 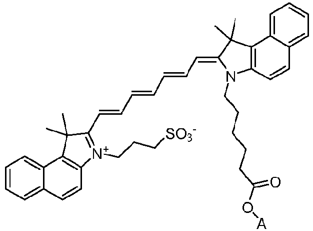 |
| 269 | 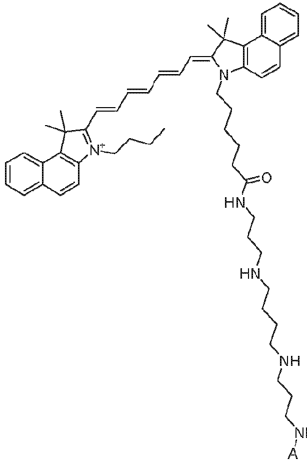 | 299 | 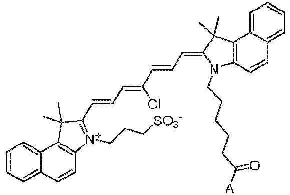 |
| 270 | 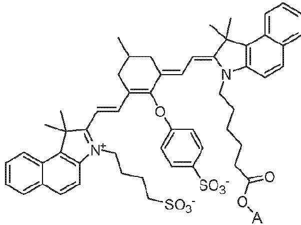 | 300 | 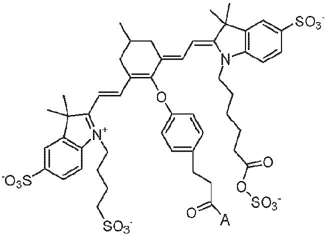 |

**Table 7. Compounds.**

| A = MCMPCFTTDHQMARRCDDCCGG**K**GRGACYGPQCLCR (SEQ ID NO: 12) (attached at K-23) | | | |
|---|---|---|---|
| **No.** | **Structure** | **No.** | **Strucure** |
| 301 | 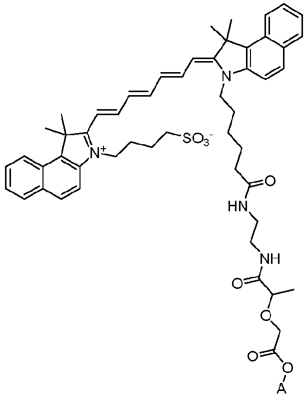 | 331 | 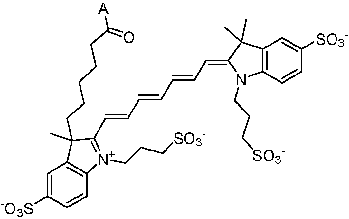 |
| 302 | 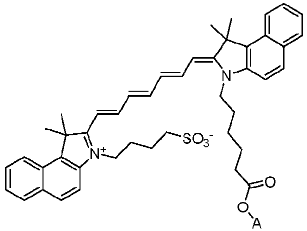 | 332 | 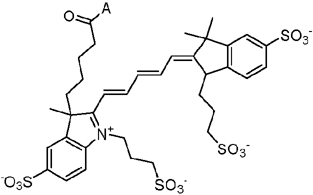 |
| 303 | 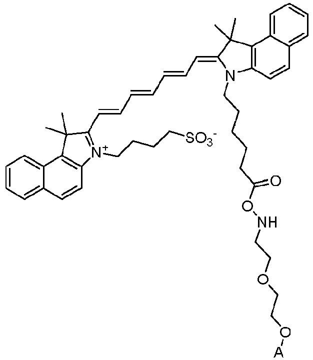 | 333 | 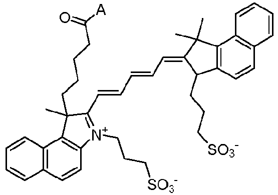 |
| 304 | 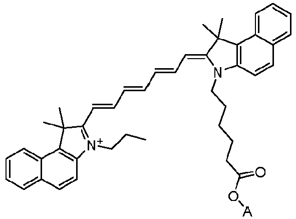 | 334 | 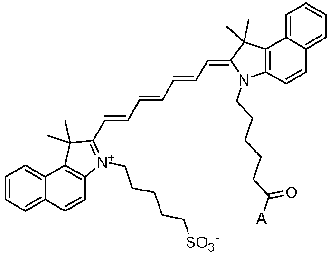 |
| 305 | 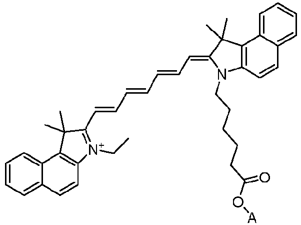 | 335 | 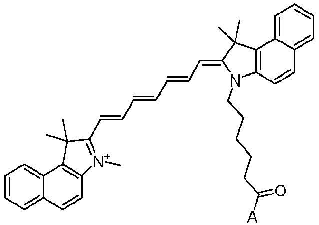 |
| 306 | 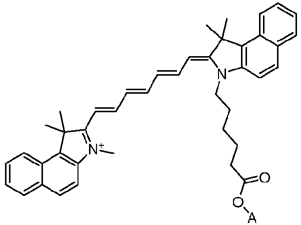 | 336 | 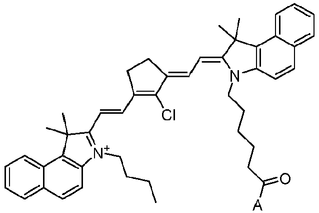 |
| 307 | 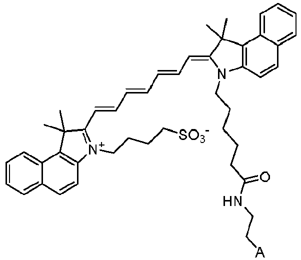 | 337 | 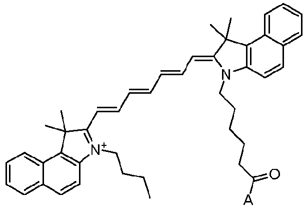 |
| 308 | 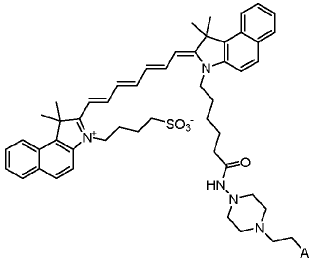 | 338 | 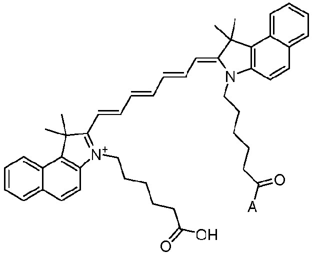 |
| 309 | 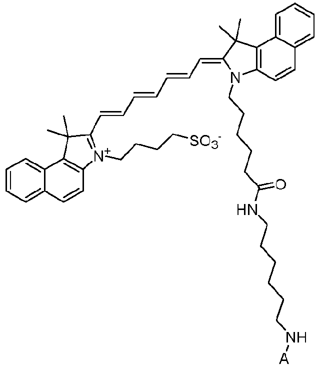 | 339 | 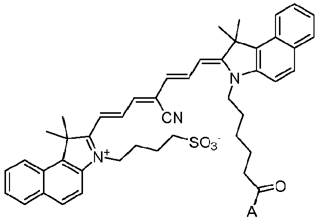 |
| 310 | 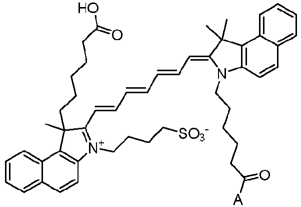 | 340 | 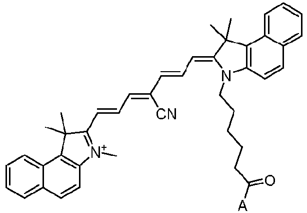 |
| 311 | 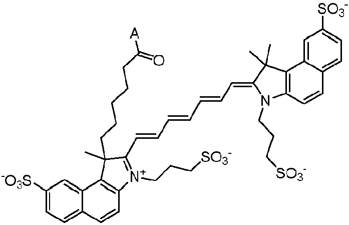 | 341 | 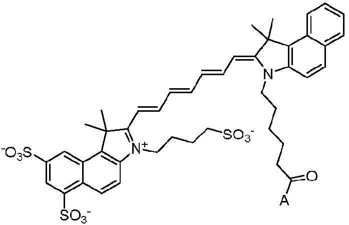 |
| 312 | 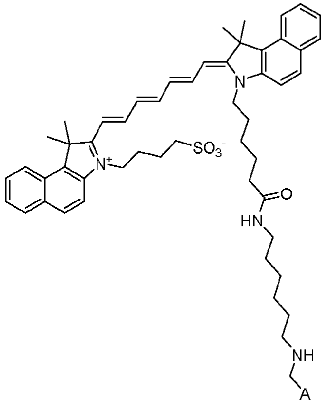 | 342 | 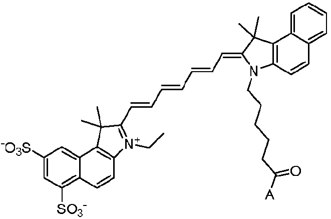 |
| 313 | 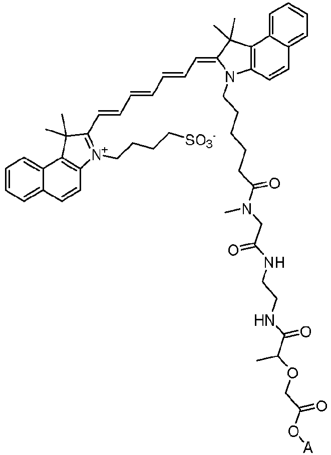 | 343 | 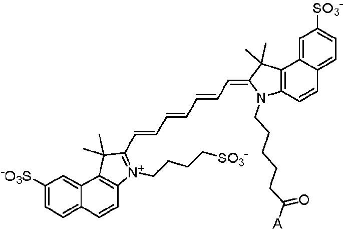 |
| 314 | 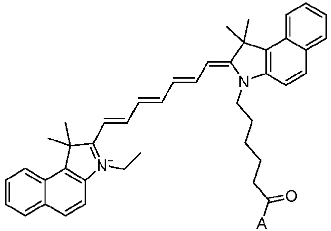 | 344 | 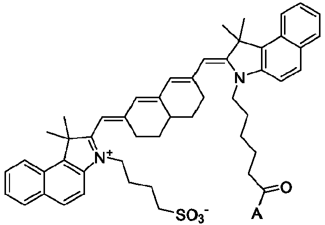 |
| 315 | 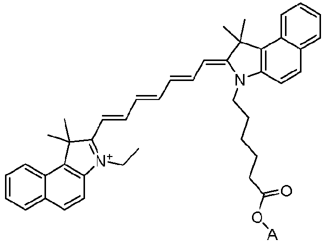 | 345 | 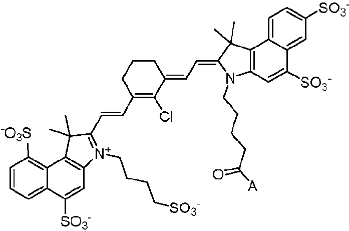 |
| 316 | 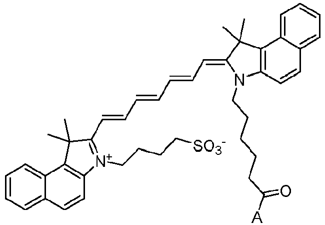 | 346 | 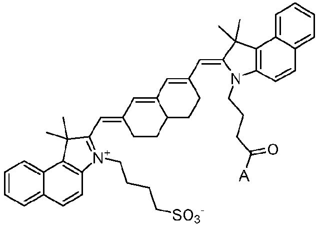 |
| 317 | 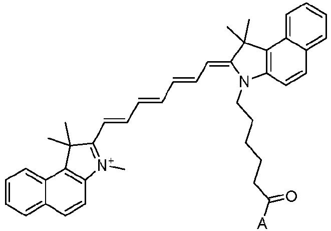 | 347 | 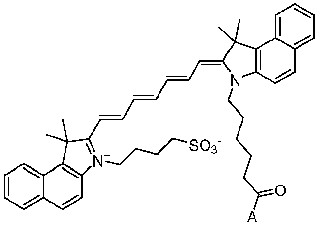 |
| 318 | 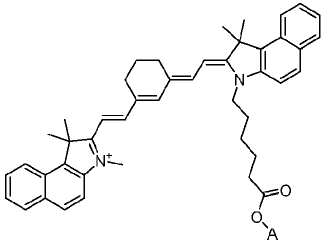 | 348 | 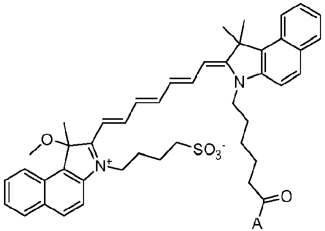 |
| 319 | 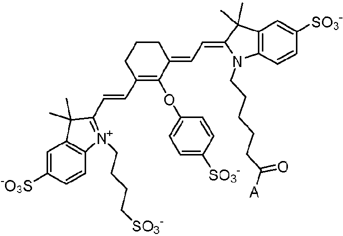 | 349 | 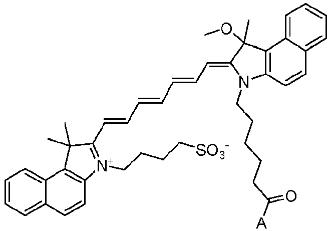 |
| 320 | 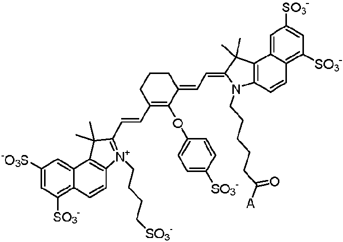 | 350 | 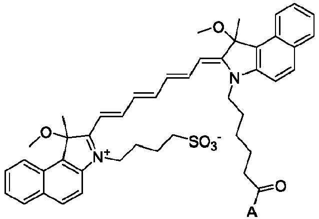 |
| 321 | 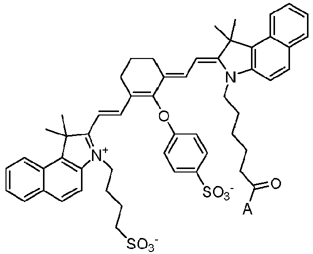 | 351 | 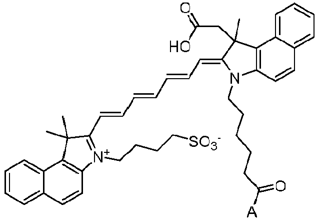 |
| 322 | 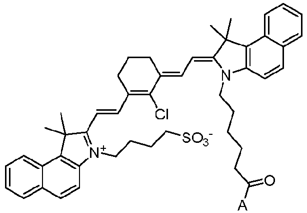 | 352 | 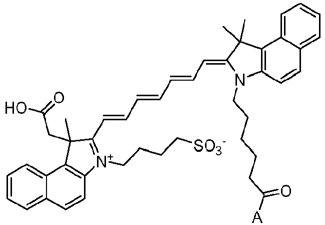 |
| 323 | 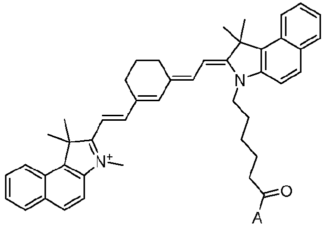 | 353 | 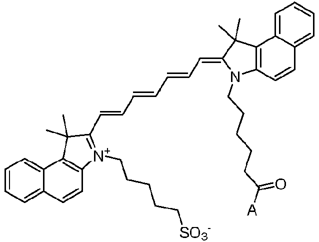 |
| 324 | 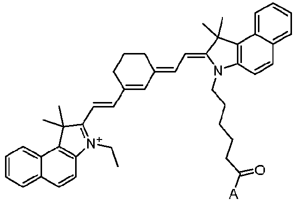 | 354 | 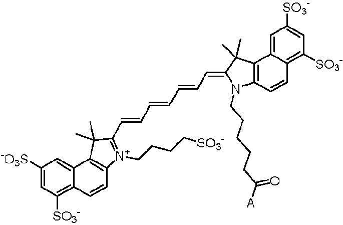 |
| 325 | 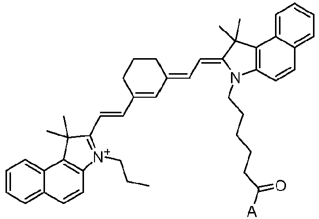 | 355 | 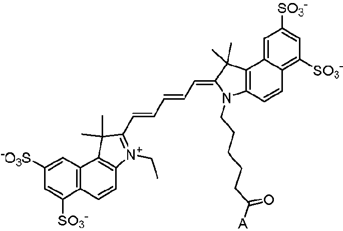 |
| 326 | 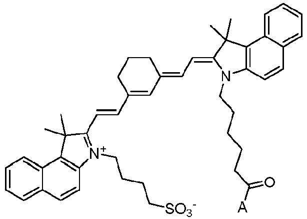 | 356 | 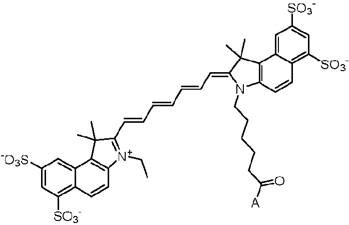 |
| 327 | 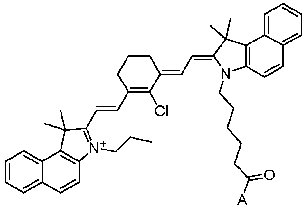 | 357 | 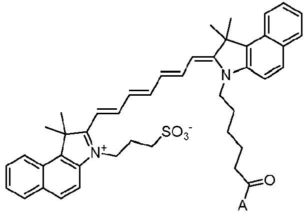 |
| 328 | 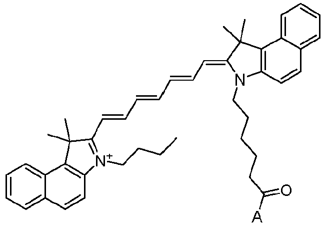 | 358 | 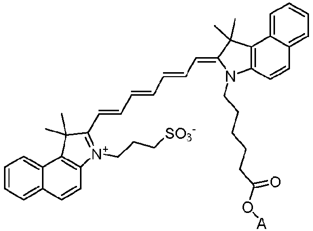 |
| 329 | 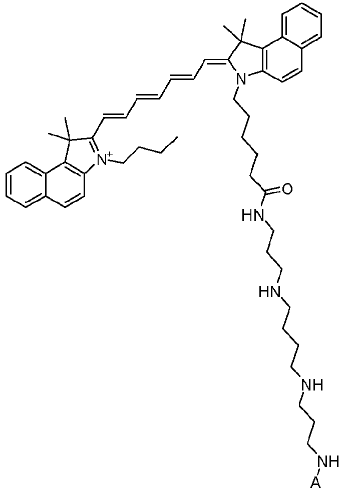 | 359 | 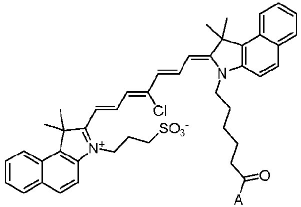 |
| 330 | 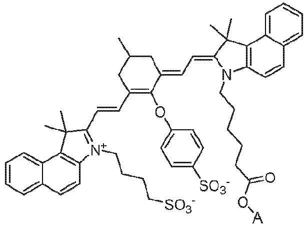 | 360 | 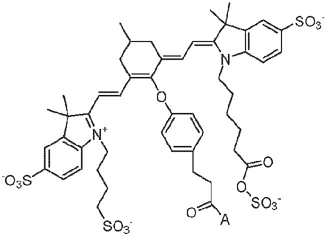 |

**, Table 8. Compounds.**

| A = MCMPCFTTDHQMAR**K**CDDCCGGAGRGACYGPQCLCR (SEQ ID NO: 13) (attached at K-15) | | | |
|---|---|---|---|
| **No.** | **Structure** | **No.** | **Strucure** |
| 361 | 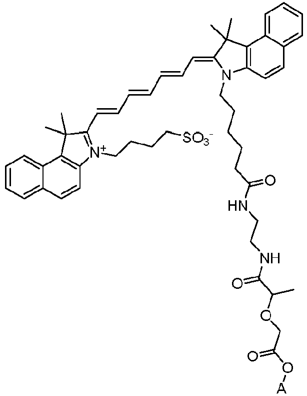 | 391 | 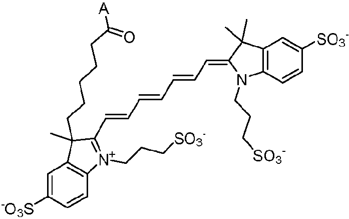 |
| 362 | 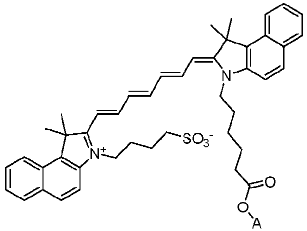 | 392 | 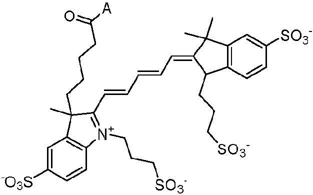 |
| 363 | 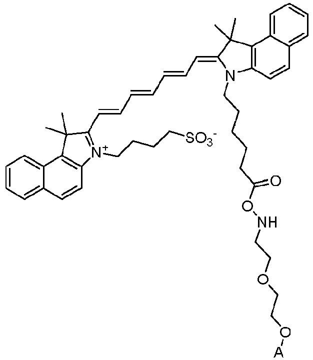 | 393 | 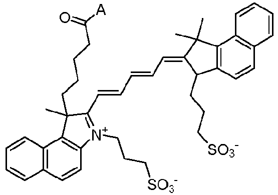 |
| 364 | 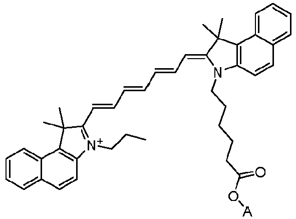 | 394 | 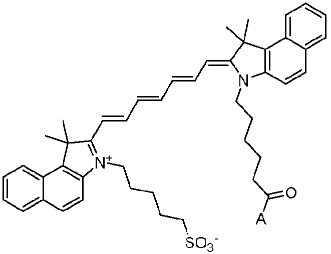 |
| 365 | 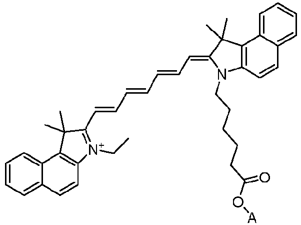 | 395 | 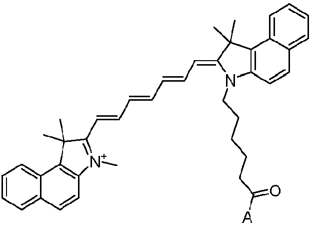 |
| 366 | 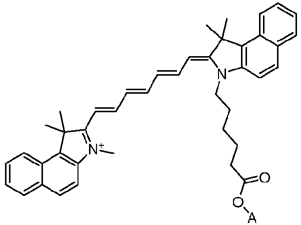 | 396 | 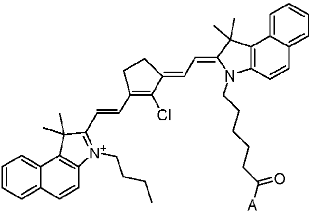 |
| 367 | 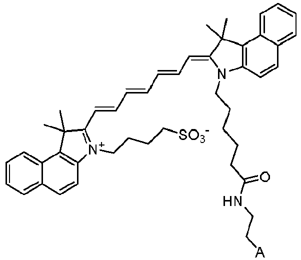 | 397 | 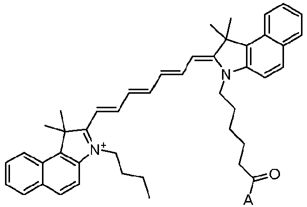 |
| 368 | 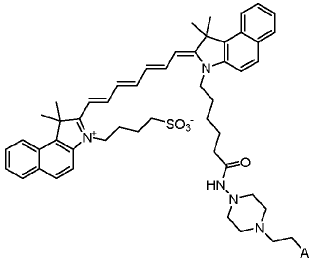 | 398 | 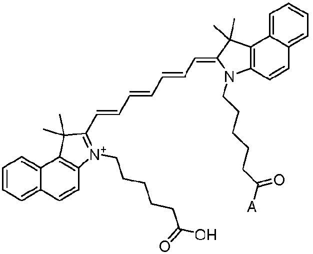 |
| 369 | 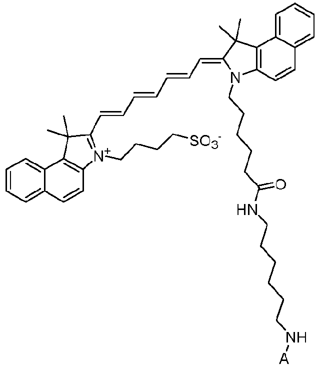 | 399 | 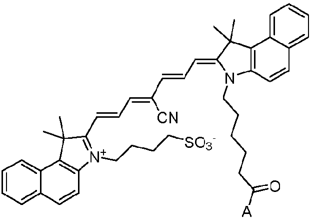 |
| 370 | 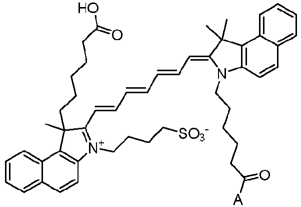 | 400 | 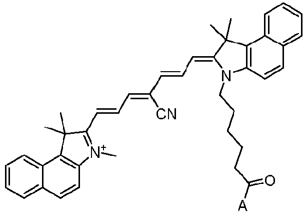 |
| 371 | 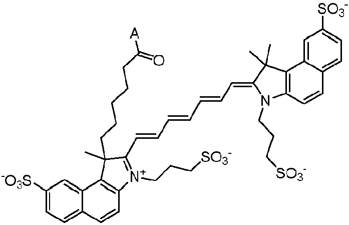 | 401 | 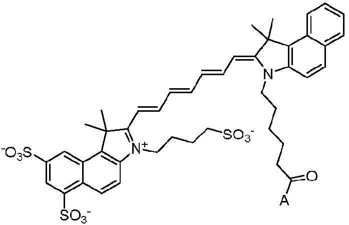 |
| 372 | 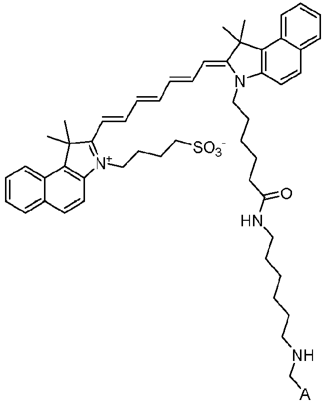 | 402 | 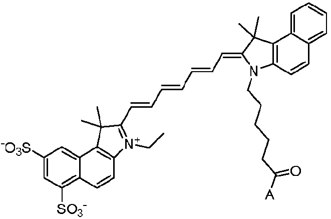 |
| 373 | 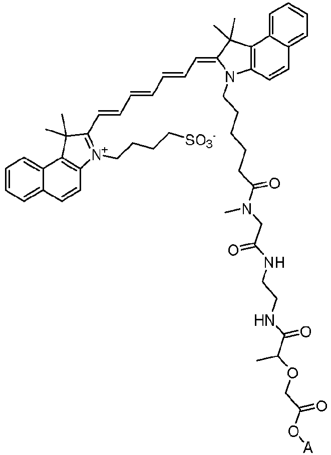 | 403 | 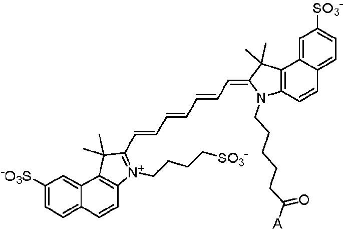 |
| 374 | 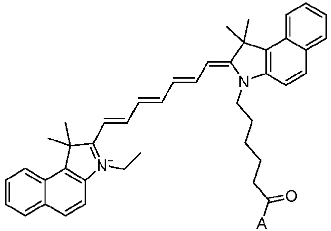 | 404 | 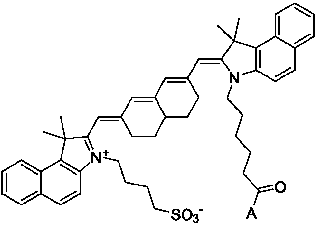 |
| 375 | 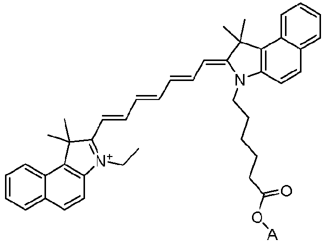 | 405 | 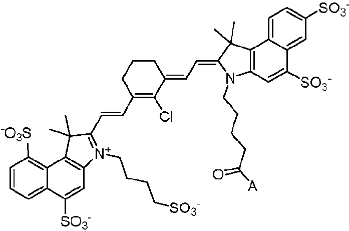 |
| 376 | 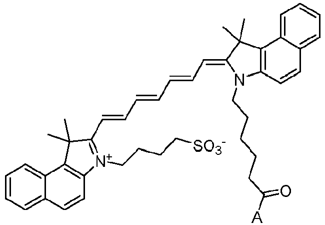 | 406 | 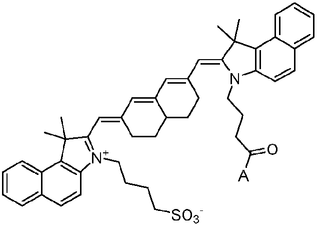 |
| 377 | 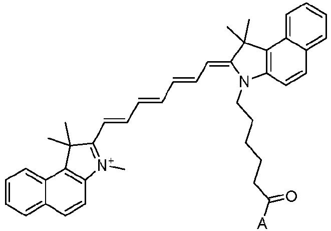 | 407 | 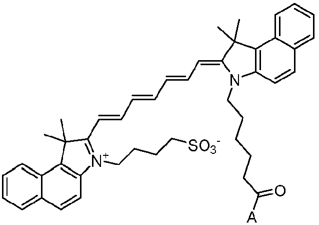 |
| 378 | 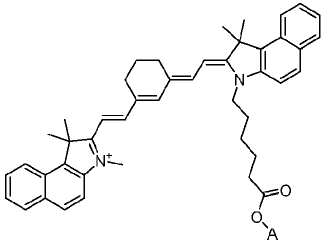 | 408 | 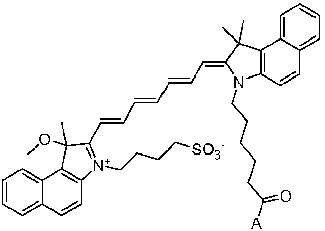 |
| 379 | 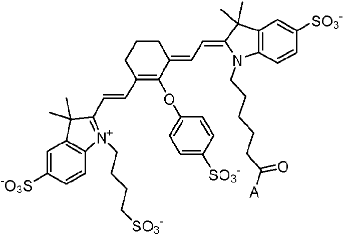 | 409 | 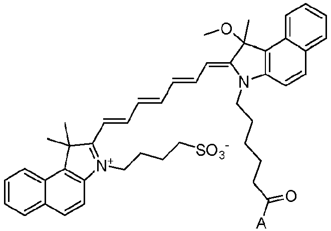 |
| 380 | 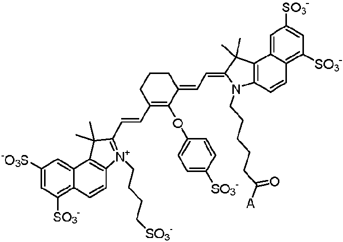 | 410 | 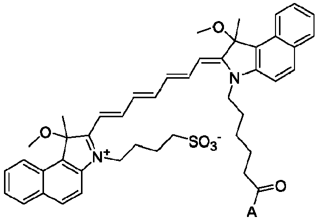 |
| 381 | 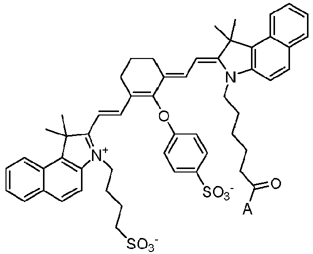 | 711 | 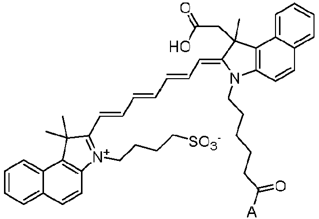 |
| 382 | 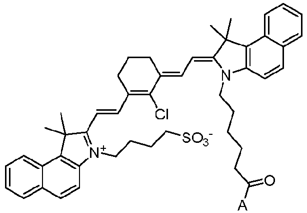 | 712 | 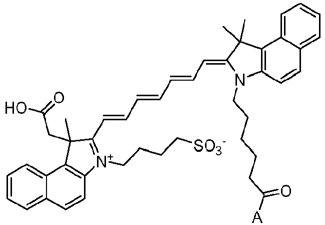 |
| 383 | 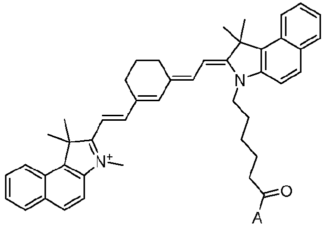 | 713 | 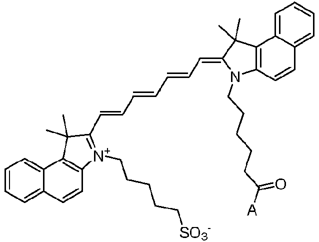 |
| 384 | 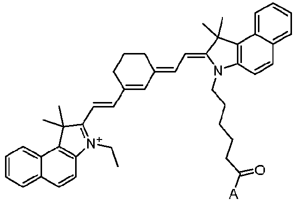 | 714 | 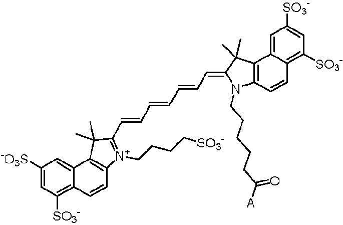 |
| 385 | 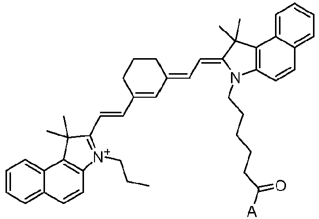 | 715 | 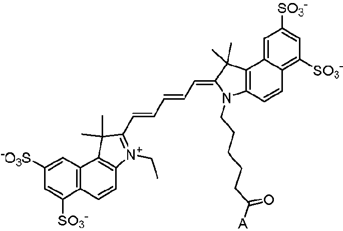 |
| 386 | 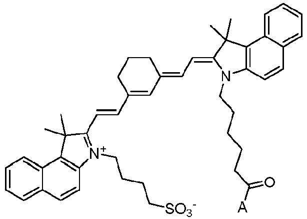 | 716 | 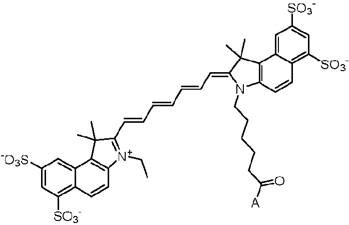 |
| 387 | 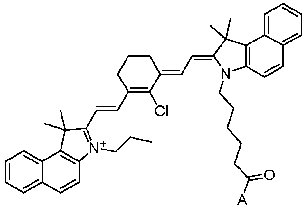 | 717 | 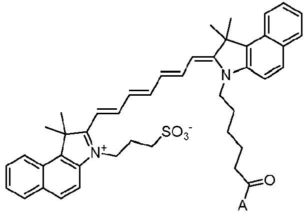 |
| 388 | 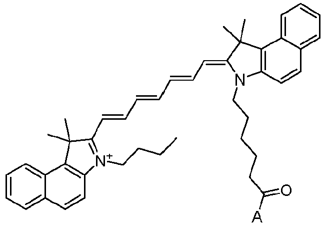 | 718 | 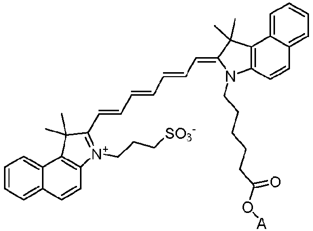 |
| 389 | 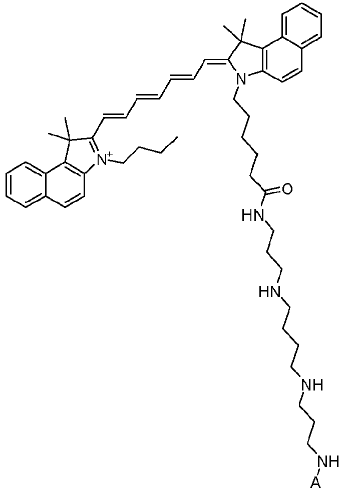 | 719 | 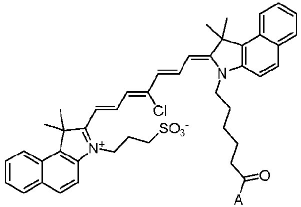 |
| 390 | 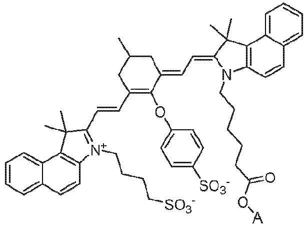 | 720 | 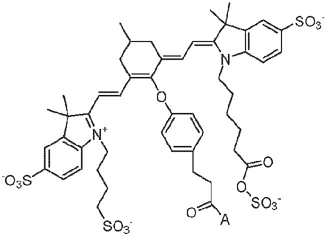 |

**Table 9. Compounds.**

| A = MCMPCFTTDHQMAR**K**CDDCCGGRGRGACYGPQCLCR (SEQ ID NO: 16) (attached at K-15) | | | |
|---|---|---|---|
| **No.** | **Structure** | **No.** | **Strucure** |
| 411 | 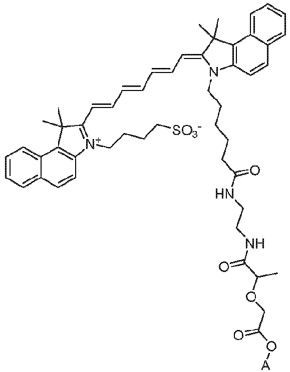 | 441 | 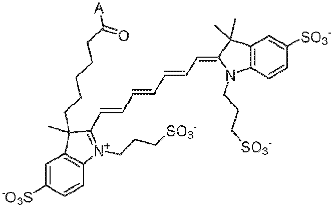 |
| 412 | 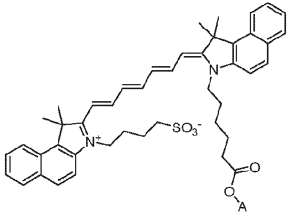 | 442 | 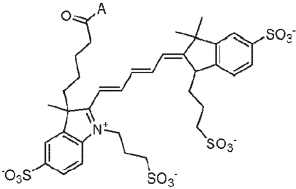 |
| 413 | 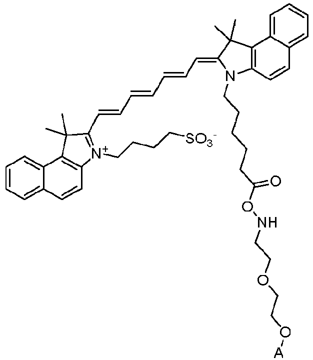 | 443 | 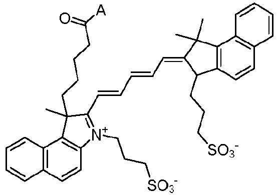 |
| 414 | 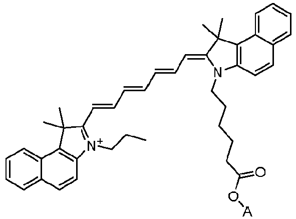 | 444 | 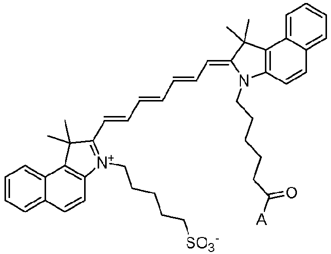 |
| 415 | 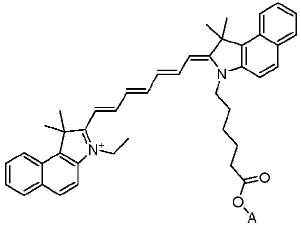 | 445 | 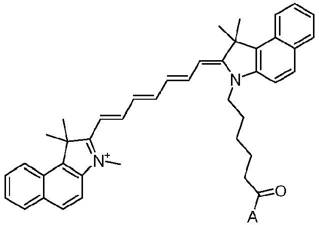 |
| 416 | 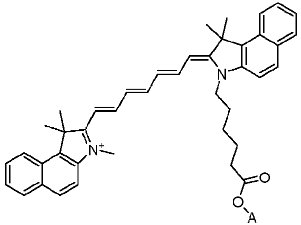 | 446 | 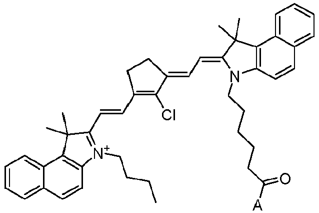 |
| 417 | 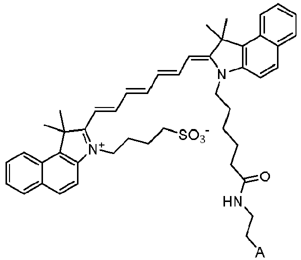 | 447 | 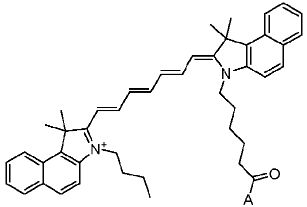 |
| 418 | 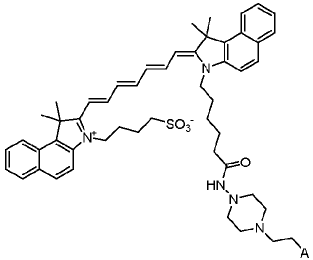 | 448 | 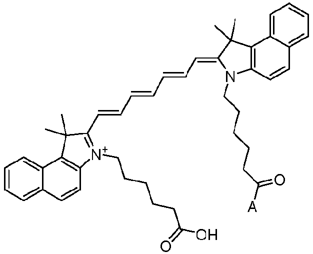 |
| 419 | 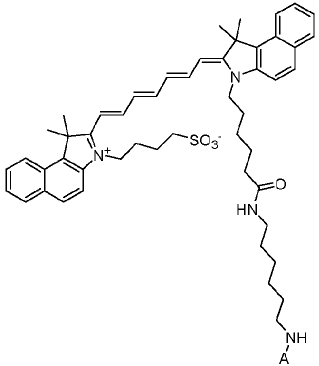 | 449 | 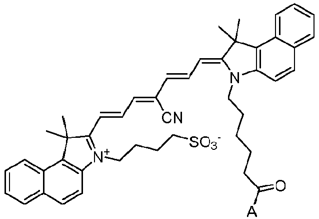 |
| 420 | 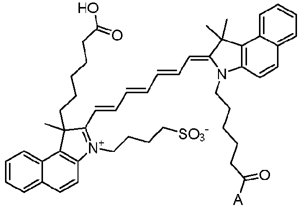 | 450 | 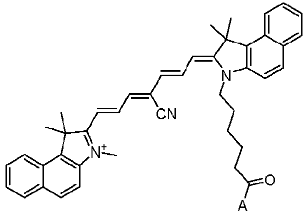 |
| 421 | 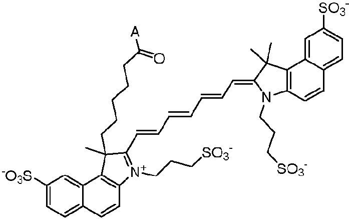 | 451 | 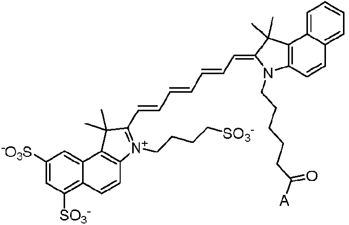 |
| 422 | 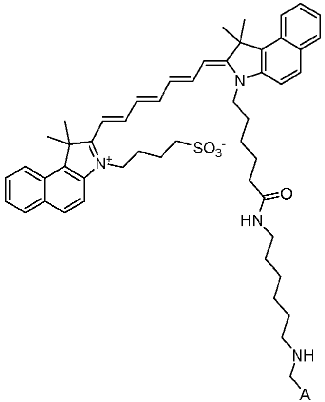 | 452 | 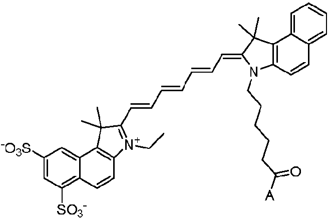 |
| 423 | 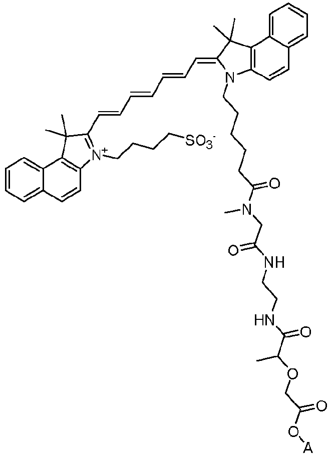 | 453 | 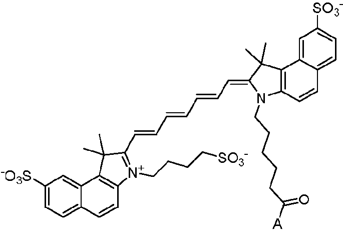 |
| 424 | 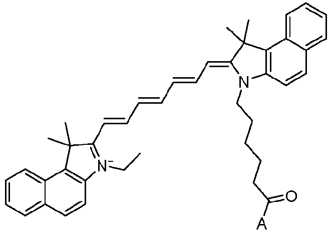 | 454 | 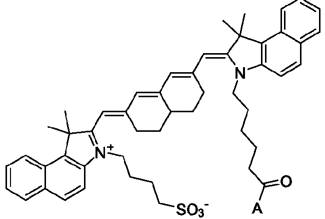 |
| 425 | 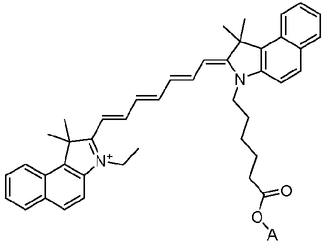 | 455 | 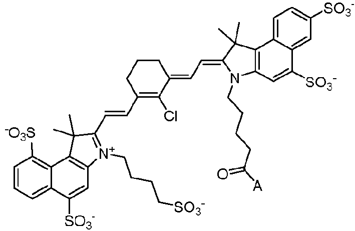 |
| 426 | 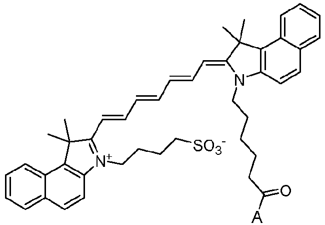 | 456 | 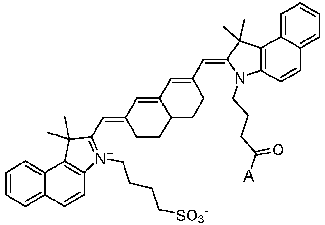 |
| 427 | 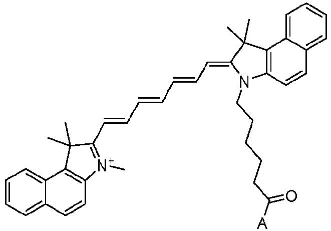 | 457 | 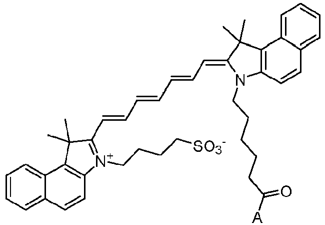 |
| 428 | 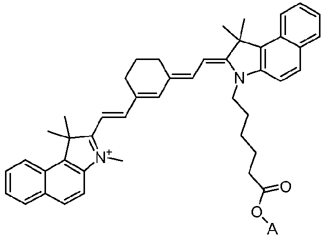 | 458 | 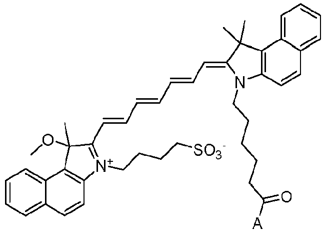 |
| 429 | 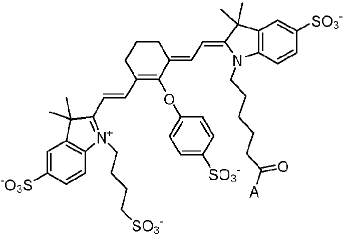 | 459 | 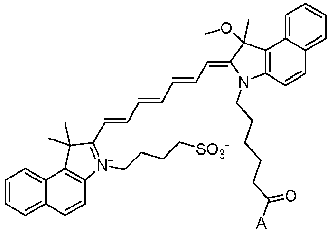 |
| 430 | 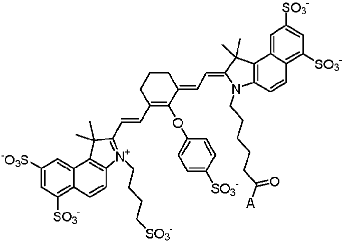 | 460 | 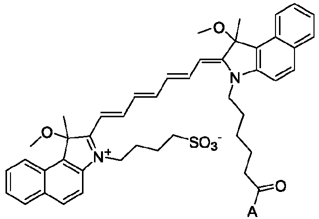 |
| 431 | 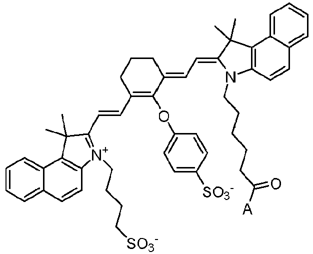 | 461 | 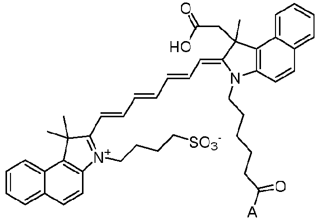 |
| 432 | 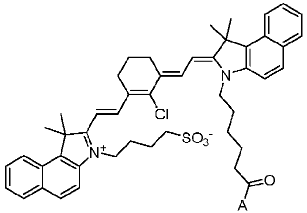 | 462 | 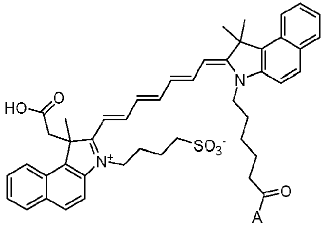 |
| 433 | 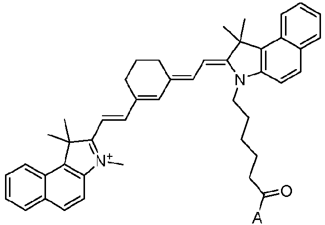 | 463 | 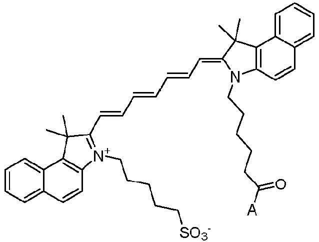 |
| 434 | 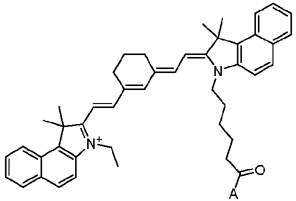 | 464 | 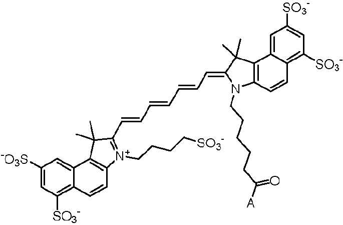 |
| 435 | 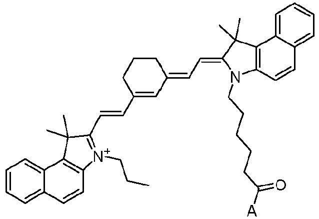 | 465 | 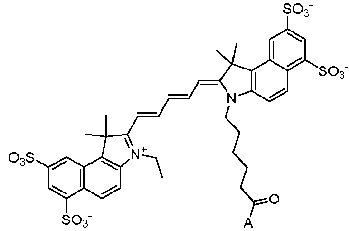 |
| 436 | 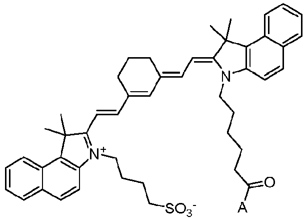 | 466 | 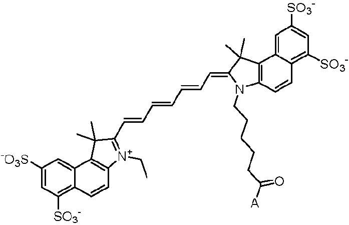 |
| 437 | 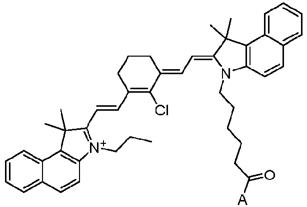 | 467 | 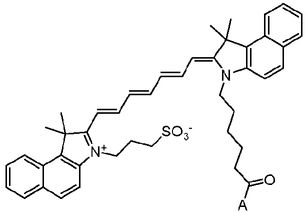 |
| 438 | 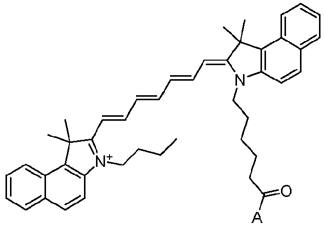 | 468 | 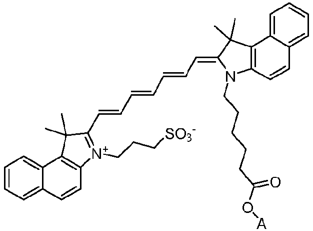 |
| 439 | 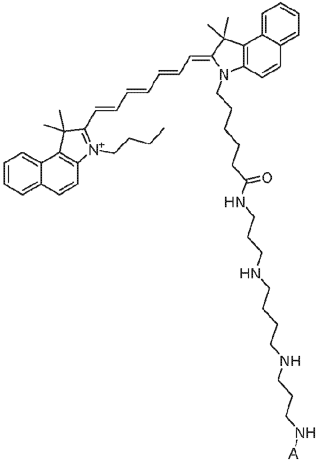 | 469 | 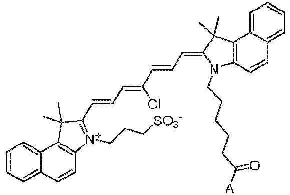 |
| 440 | 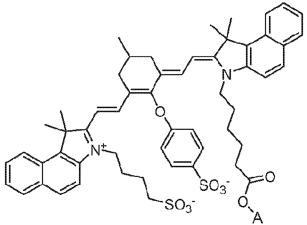 | 470 | 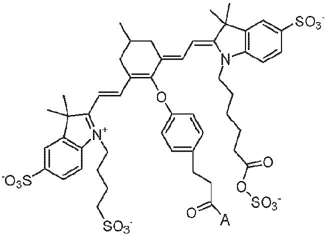 |

**Table 10. Compounds.**

| A = MCNPCFTTDHQMARACDDCCGG**K**GRGRCYGPQCLCR (SEQ ID NO: 20) (attached at K-23) | | | |
|---|---|---|---|
| **No.** | **Structure** | **No.** | **Strucure** |
| 471 | 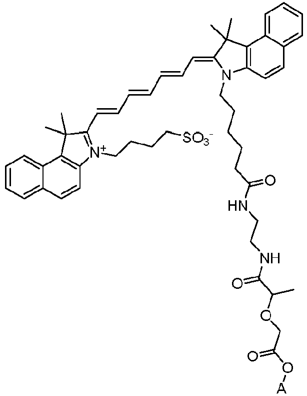 | 501 | 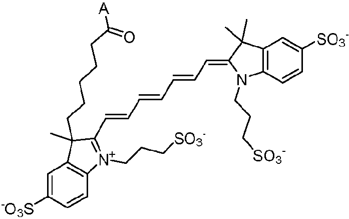 |
| 472 | 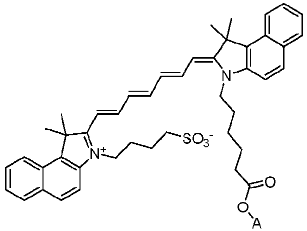 | 502 | 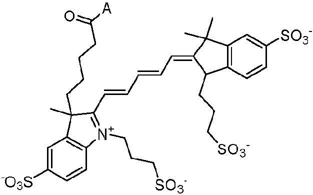 |
| 473 | 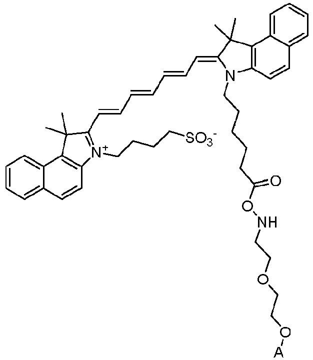 | 503 | 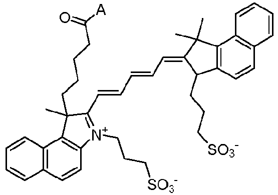 |
| 474 | 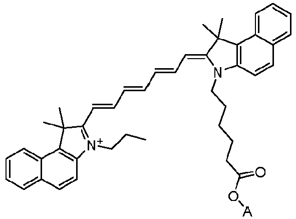 | 504 | 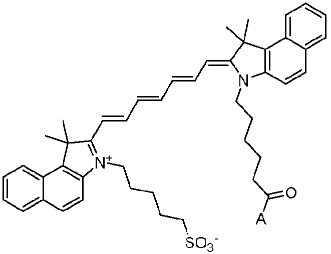 |
| 475 | 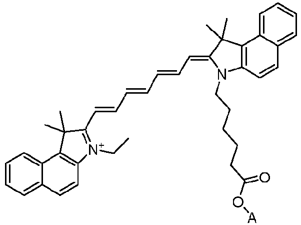 | 505 | 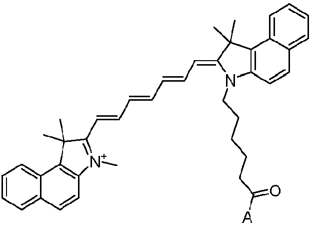 |
| 476 | 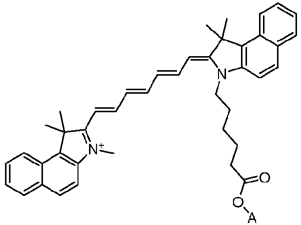 | 506 | 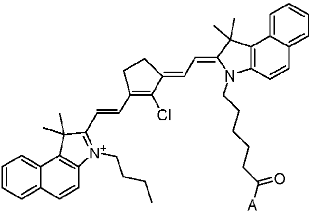 |
| 477 | 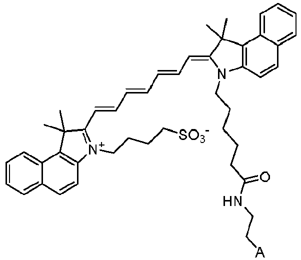 | 507 | 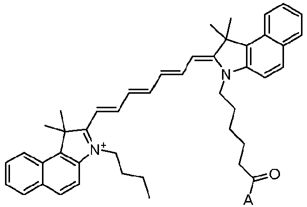 |
| 478 | 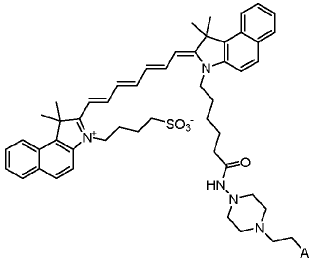 | 508 | 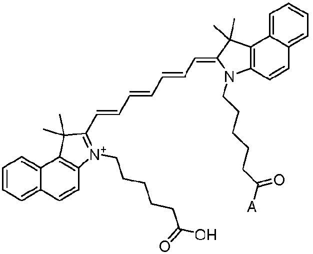 |
| 479 | 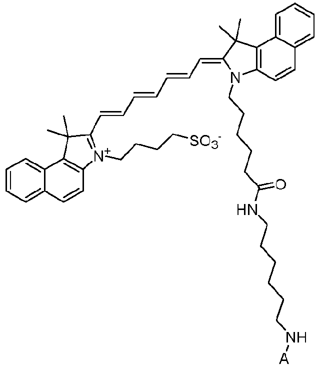 | 509 | 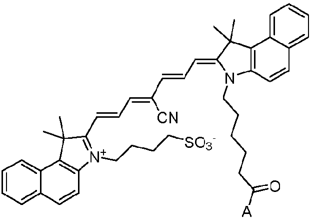 |
| 480 | 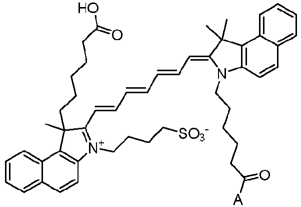 | 510 | 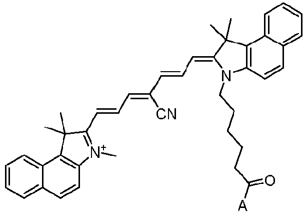 |
| 481 | 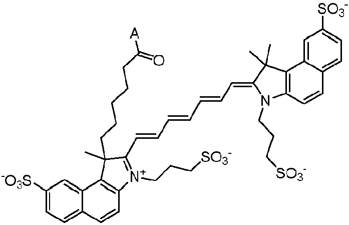 | 511 | 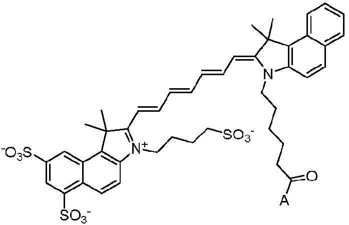 |
| 482 | 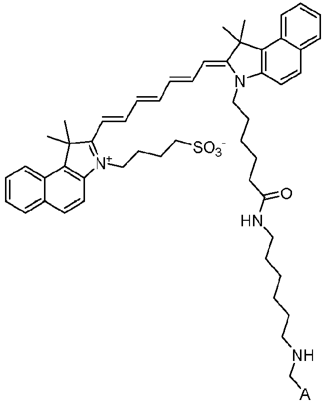 | 512 | 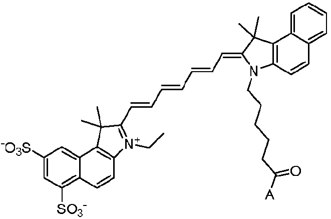 |
| 483 | 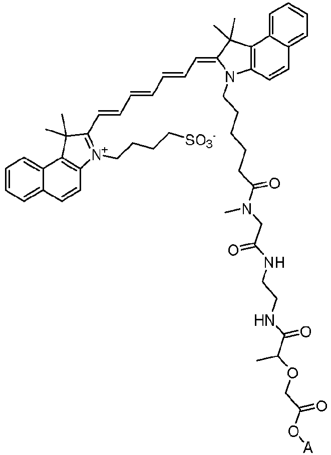 | 513 | 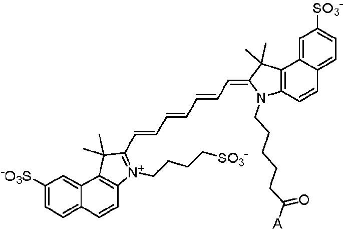 |
| 484 | 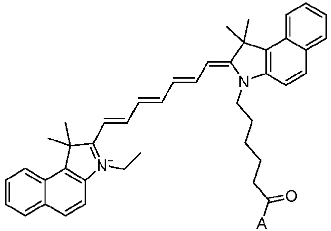 | 514 | 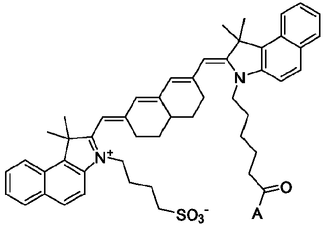 |
| 485 | 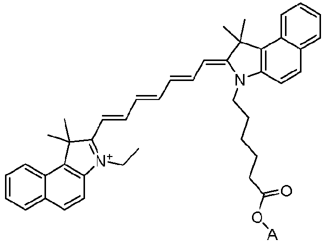 | 515 | 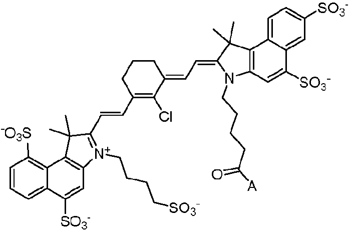 |
| 486 | 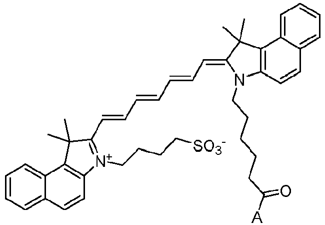 | 516 | 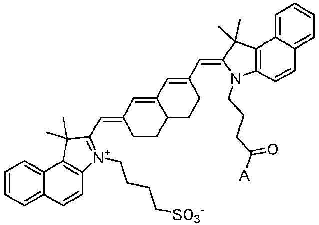 |
| 487 | 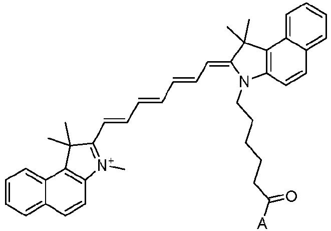 | 517 | 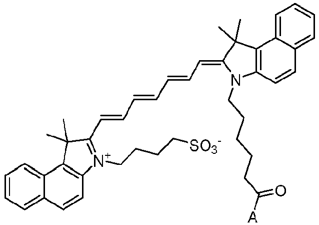 |
| 488 | 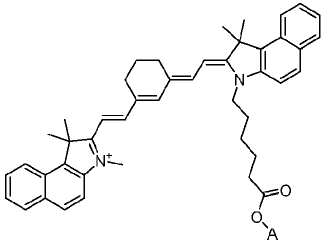 | 518 | 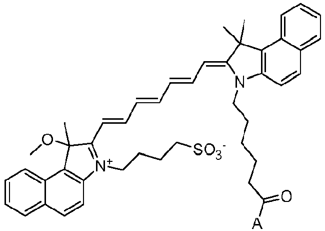 |
| 489 | 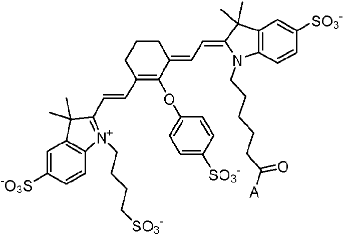 | 519 | 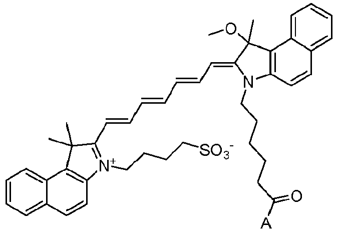 |
| 490 | 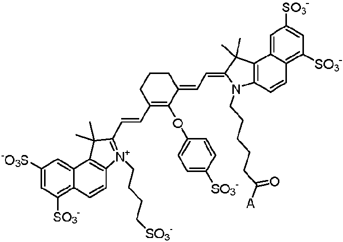 | 520 | 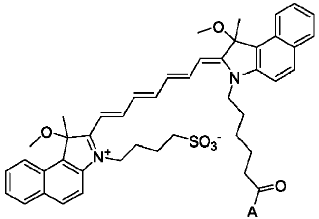 |
| 491 | 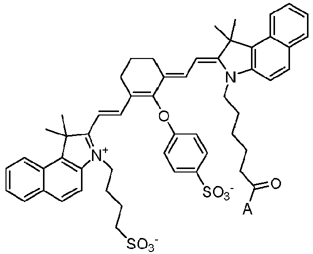 | 521 | 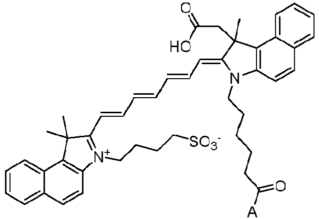 |
| 492 | 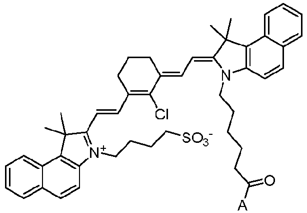 | 522 | 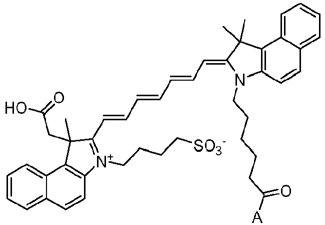 |
| 493 | 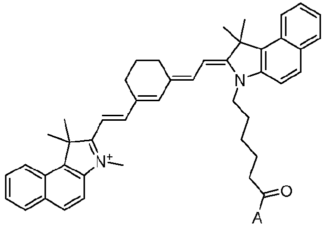 | 523 | 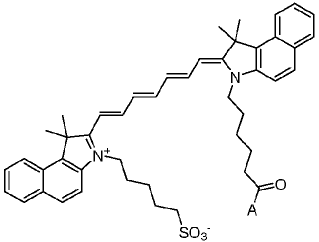 |
| 494 | 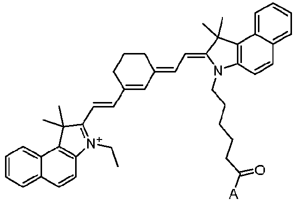 | 524 | 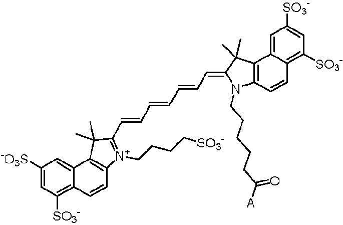 |
| 495 | 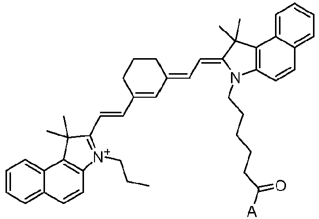 | 525 | 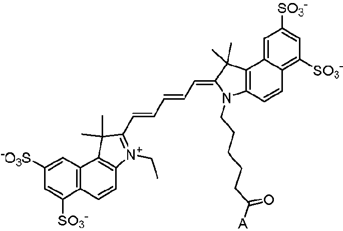 |
| 496 | 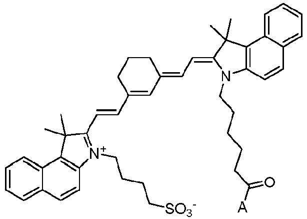 | 526 | 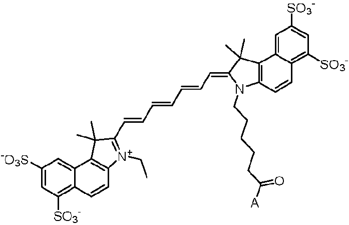 |
| 497 | 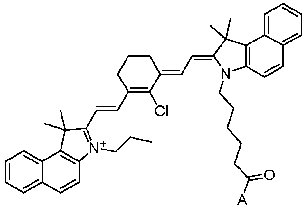 | 527 | 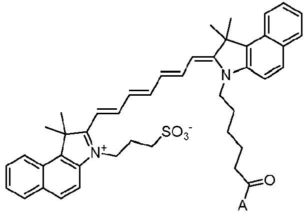 |
| 498 | 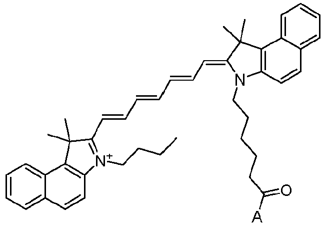 | 528 | 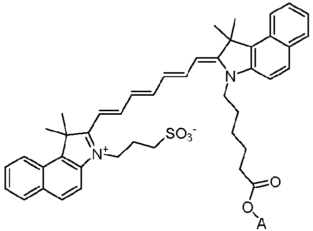 |
| 499 | 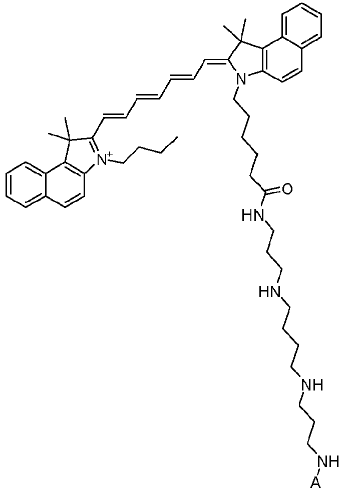 | 529 | 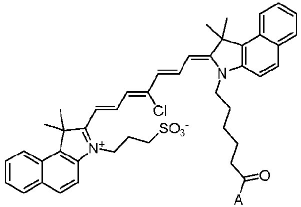 |

| A = MCMPCFTTDHQMARACDDCCGG**K**GRGRCYGPQCLCR (SEQ ID NO: 20) (attached at K-23) | | | |
|---|---|---|---|
| **No.** | **Structure** | **No.** | **Strucure** |
| 500 | 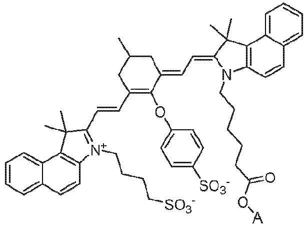 | 530 | 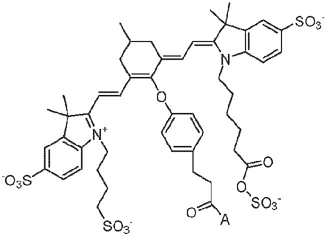 |

**Table 11. Compounds.**

| A = MCMPCFTTDHQMARRCDDCCGG**K**GRGRCYGPQCL (SEQ ID NO: 21) (attached at K-23) | | | |
|---|---|---|---|
| **No.** | **Structure** | **No.** | **Strucure** |
| 531 | 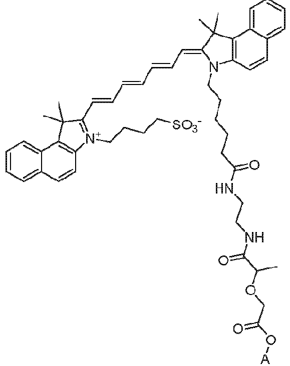 | 561 | 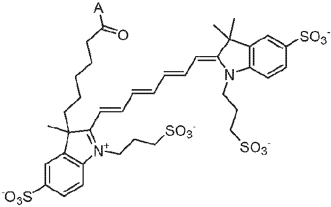 |
| 532 | 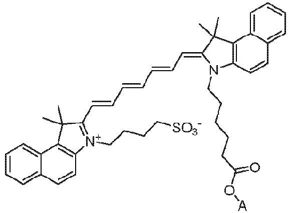 | 562 | 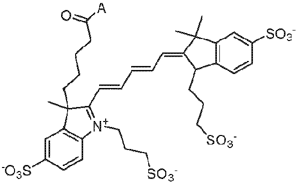 |
| 533 | 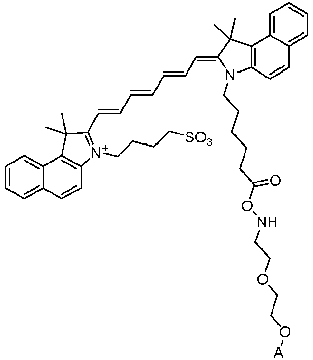 | 563 | 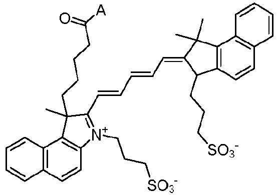 |
| 534 | 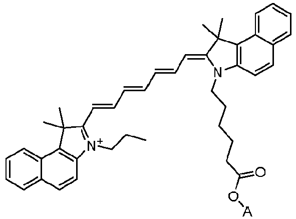 | 564 | 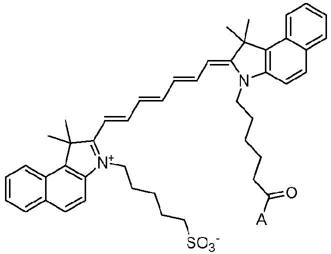 |
| 535 | 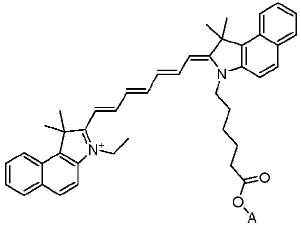 | 565 | 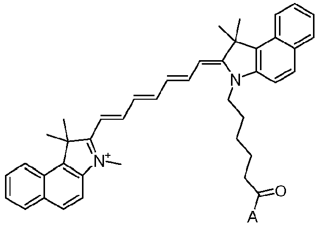 |
| 536 | 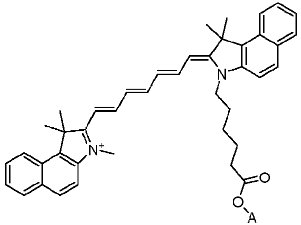 | 566 | 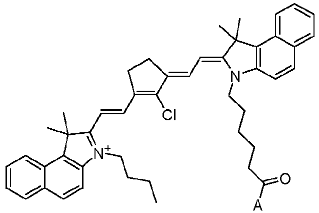 |
| 537 | 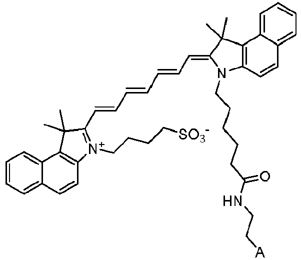 | 567 | 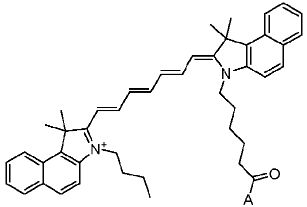 |
| 538 | 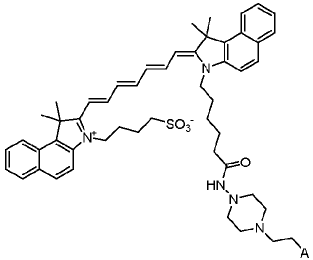 | 568 | 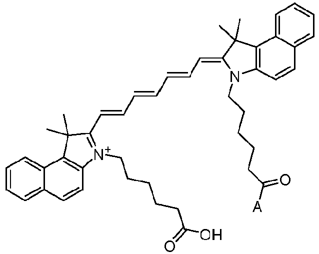 |
| 539 | 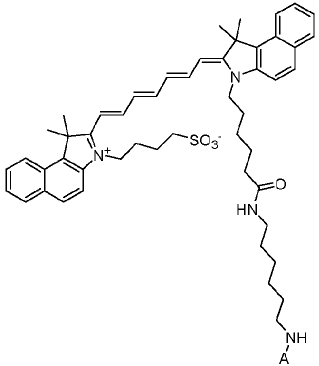 | 569 | 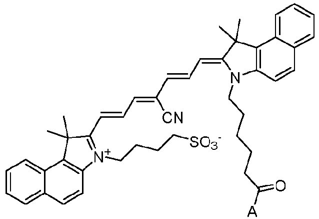 |
| 540 | 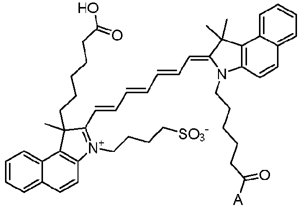 | 570 | 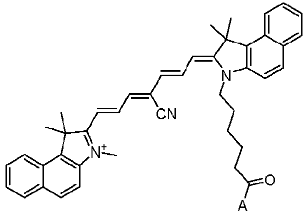 |
| 541 | 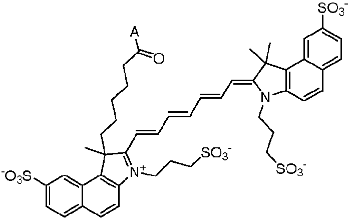 | 571 | 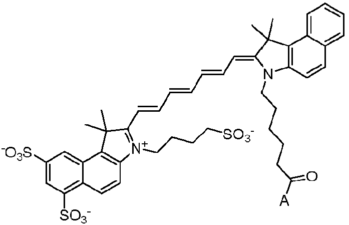 |
| 542 | 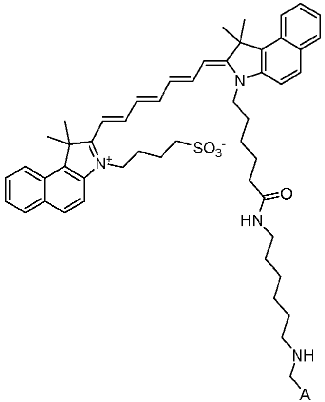 | 572 | 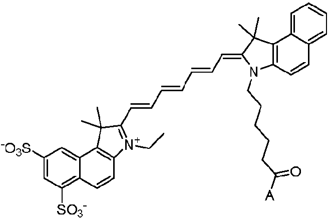 |
| 543 | 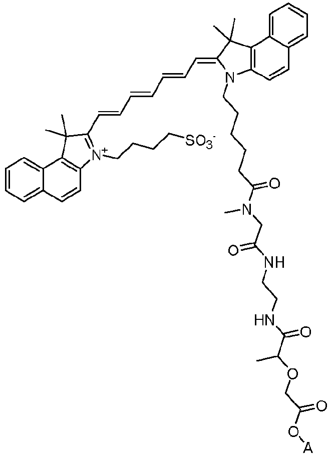 | 573 | 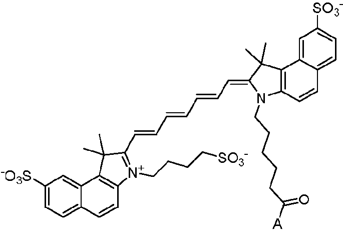 |
| 544 | 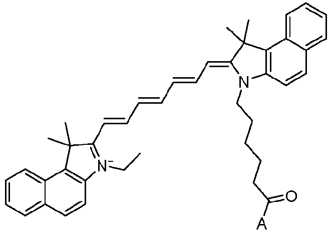 | 574 | 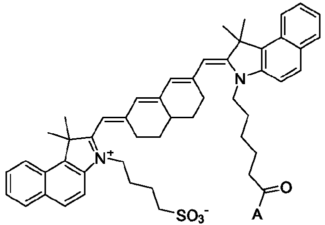 |
| 545 | 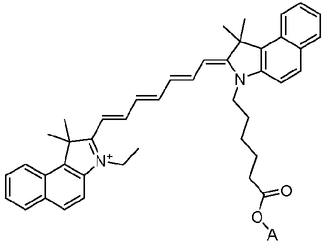 | 575 | 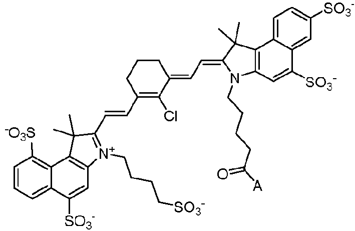 |
| 546 | 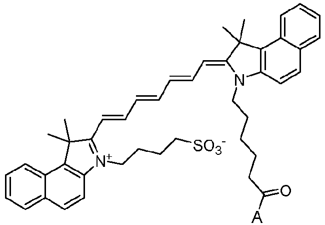 | 576 | 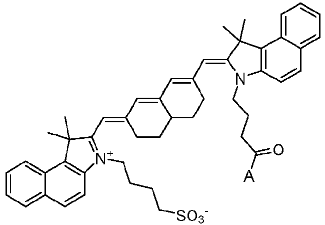 |
| 547 | 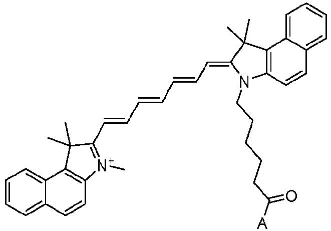 | 577 | 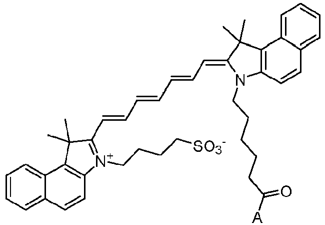 |
| 548 | 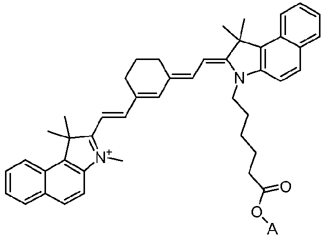 | 578 | 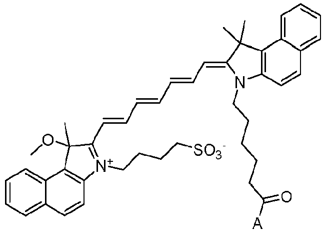 |
| 549 | 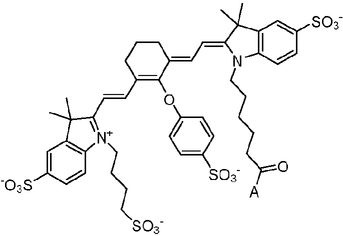 | 579 | 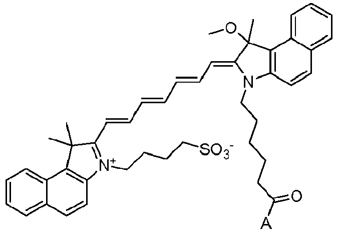 |
| 550 | 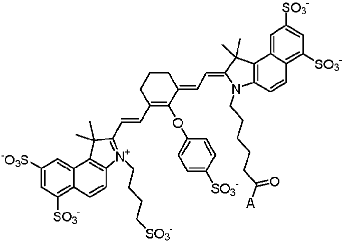 | 580 | 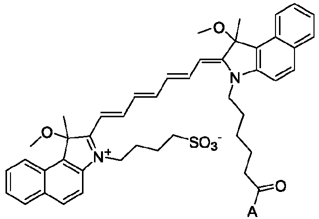 |
| 551 | 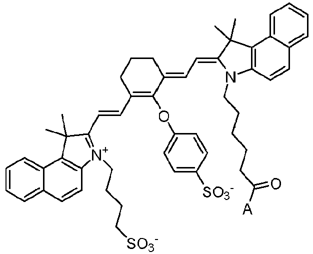 | 581 | 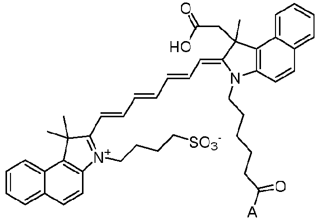 |
| 552 | 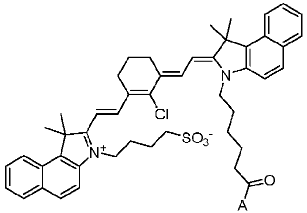 | 582 | 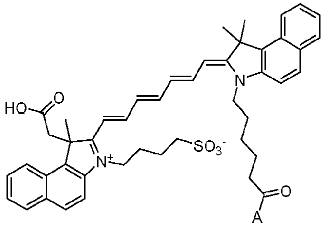 |
| 553 | 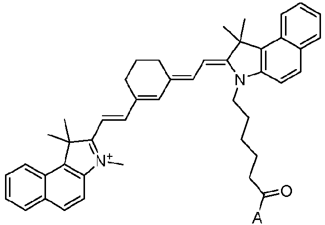 | 583 | 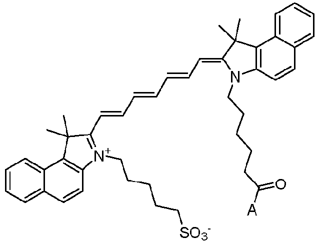 |
| 554 | 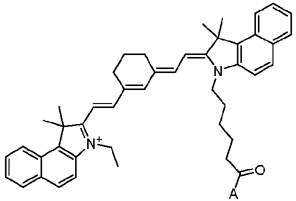 | 584 | 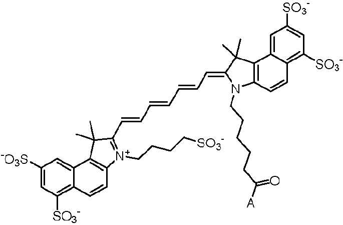 |
| 555 | 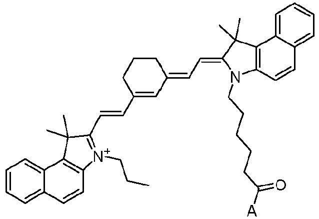 | 585 | 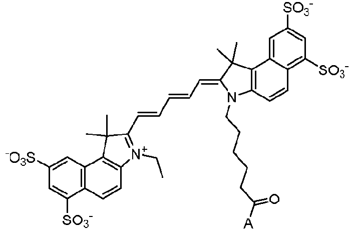 |
| 556 | 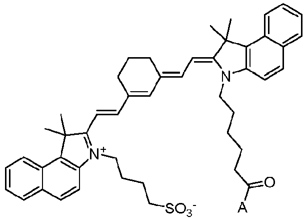 | 586 | 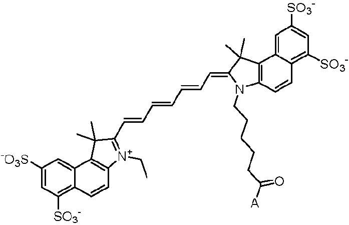 |
| 557 | 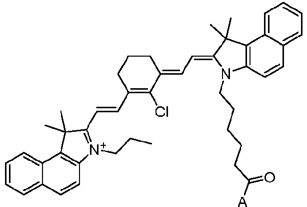 | 587 | 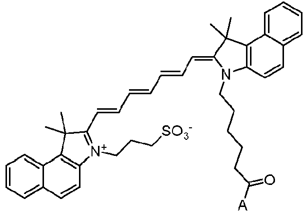 |
| 558 | 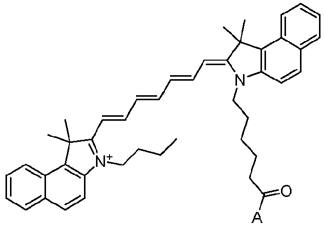 | 588 | 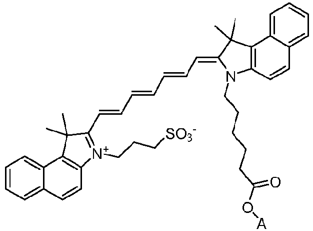 |
| 559 | 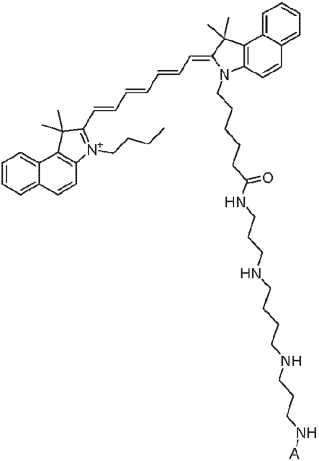 | 589 | 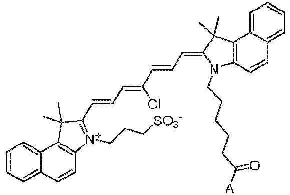 |
| 560 | 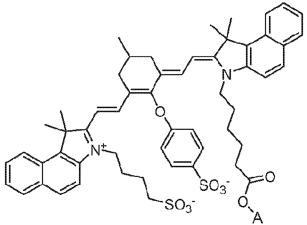 | 590 | 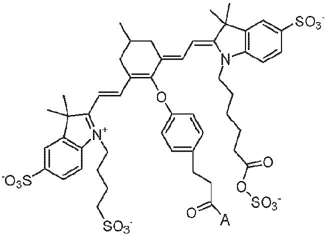 |

**Table 12. Compounds.**

| A = MCMPCFTTDHQMAR**K**CDDCCGGAGRGRCYGPQCLC (SEQ ID NO: 22) (attached at K-15) | | | |
|---|---|---|---|
| **No.** | **Structure** | **No.** | **Strucure** |
| 591 | 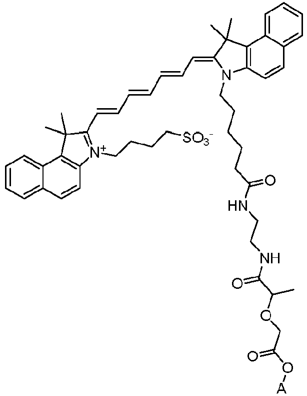 | 621 | 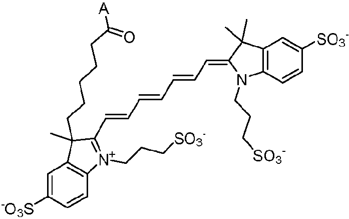 |
| 592 | 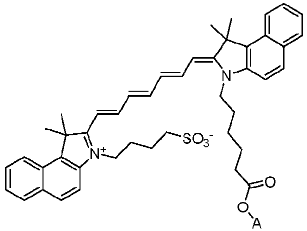 | 622 | 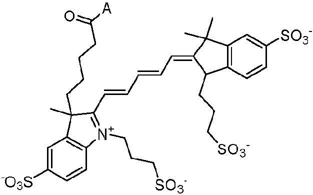 |
| 593 | 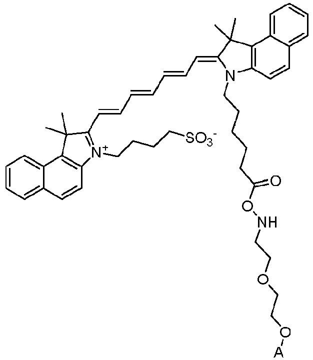 | 623 | 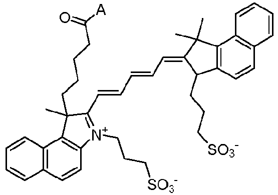 |
| 594 | 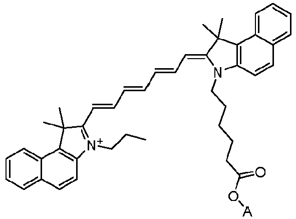 | 624 | 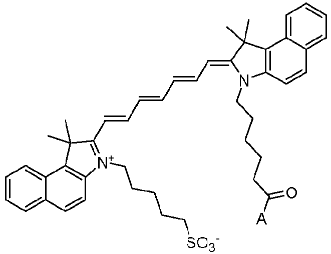 |
| 595 | 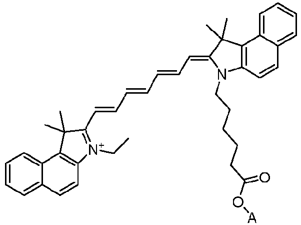 | 625 | 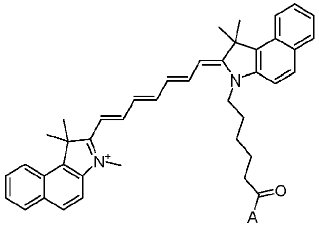 |
| 596 | 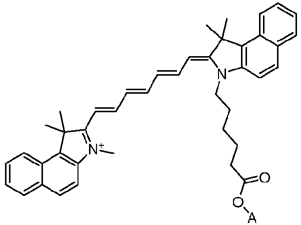 | 626 | 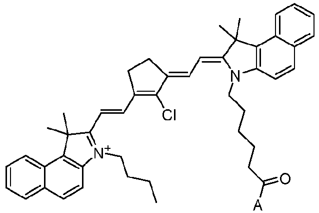 |
| 597 | 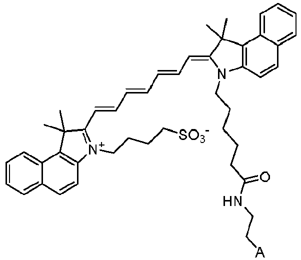 | 627 | 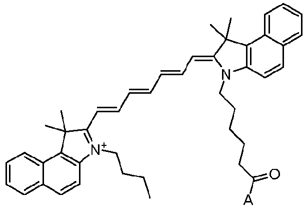 |
| 598 | 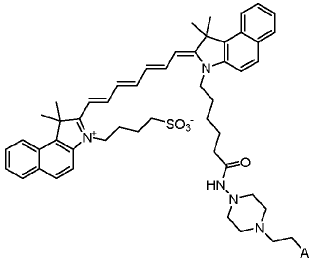 | 628 | 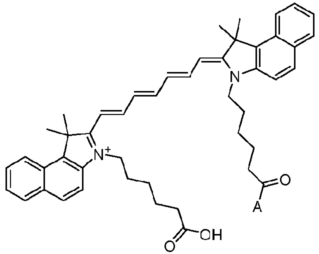 |
| 599 | 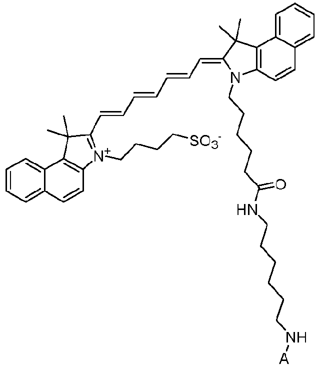 | 629 | 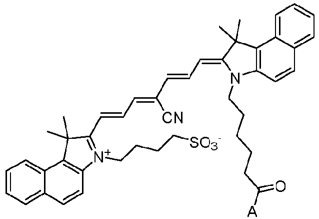 |
| 600 | 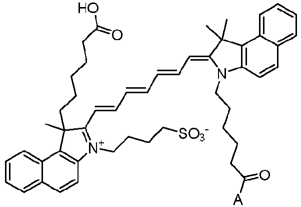 | 630 | 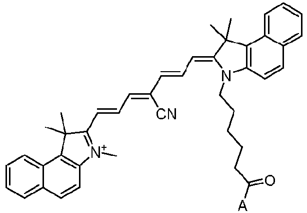 |
| 601 | 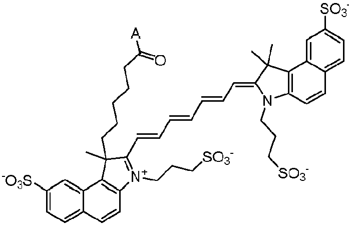 | 631 | 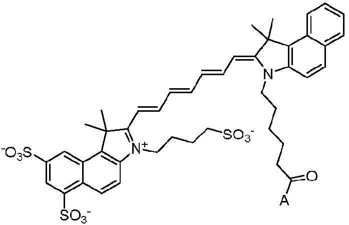 |
| 602 | 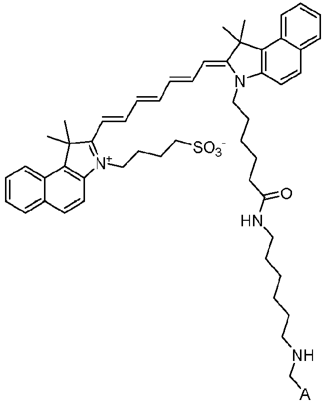 | 632 | 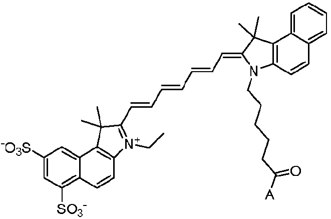 |
| 603 | 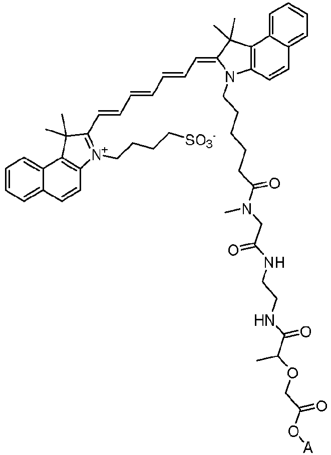 | 633 | 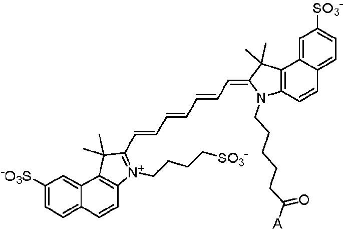 |
| 604 | 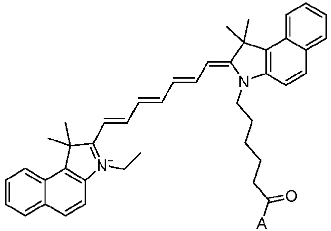 | 634 | 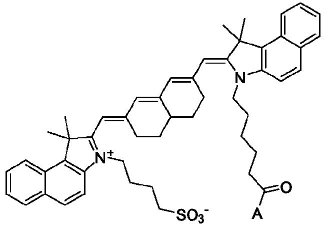 |
| 605 | 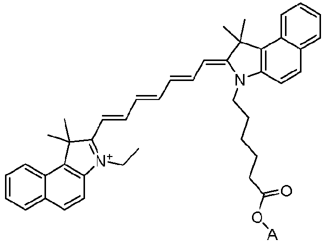 | 635 | 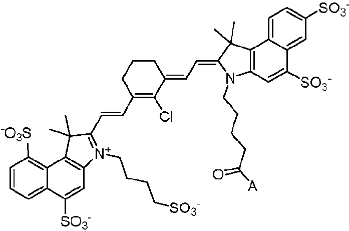 |
| 606 | 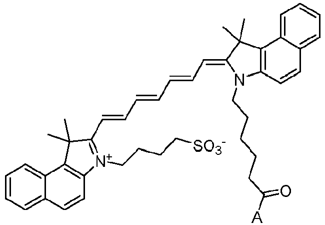 | 636 | 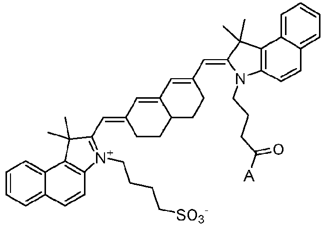 |
| 607 | 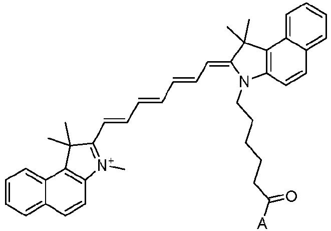 | 637 | 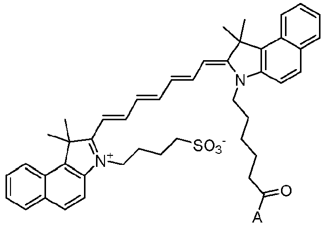 |
| 608 | 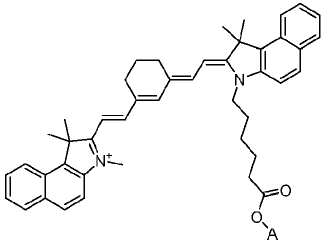 | 638 | 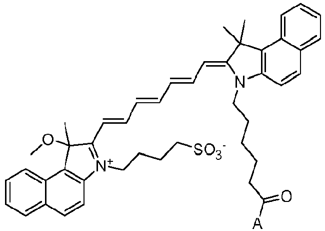 |
| 609 | 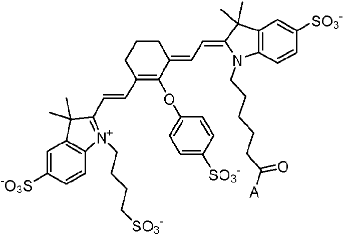 | 639 | 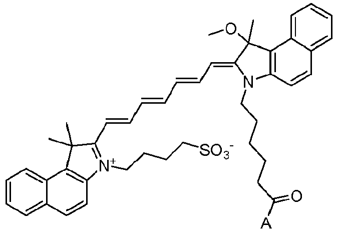 |
| 610 | 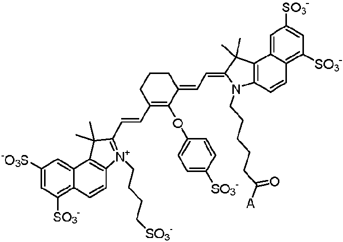 | 640 | 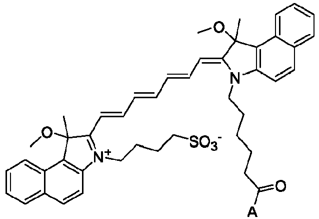 |
| 611 | 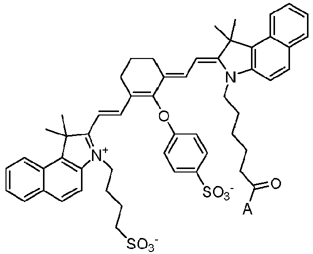 | 641 | 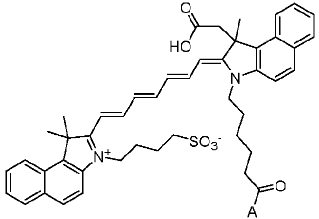 |
| 612 | 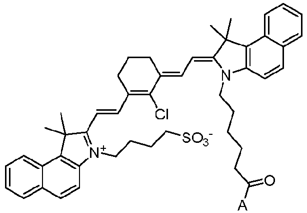 | 642 | 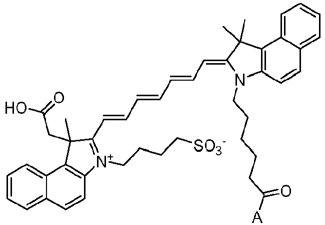 |
| 613 | 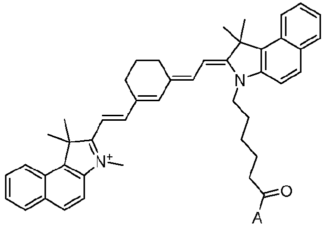 | 643 | 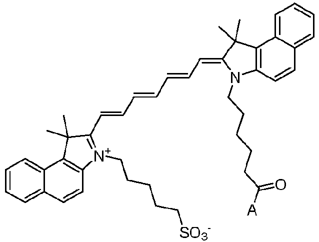 |
| 614 | 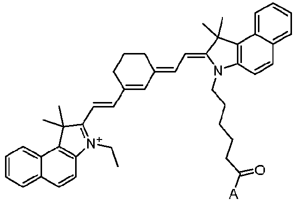 | 644 | 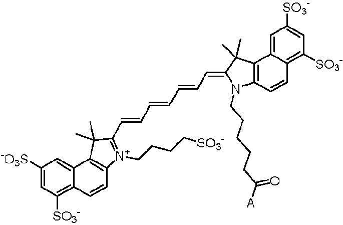 |
| 615 | 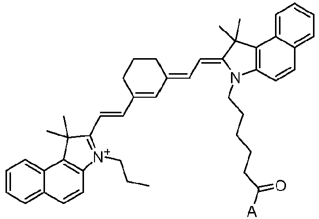 | 645 | 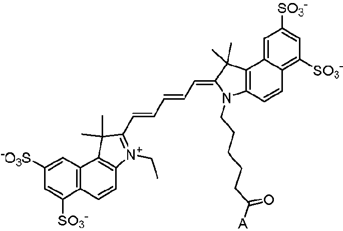 |
| 616 | 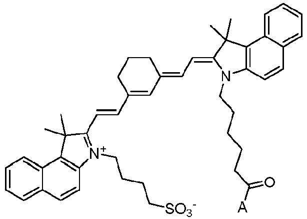 | 646 | 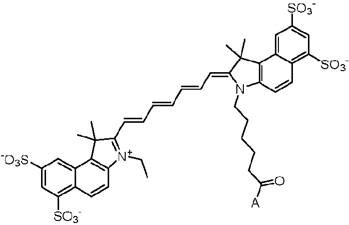 |
| 617 | 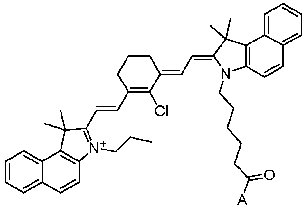 | 647 | 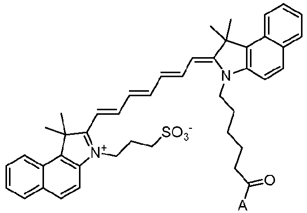 |
| 618 | 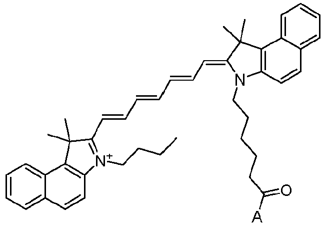 | 648 | 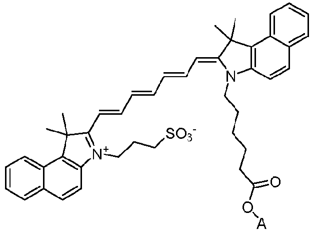 |
| 619 | 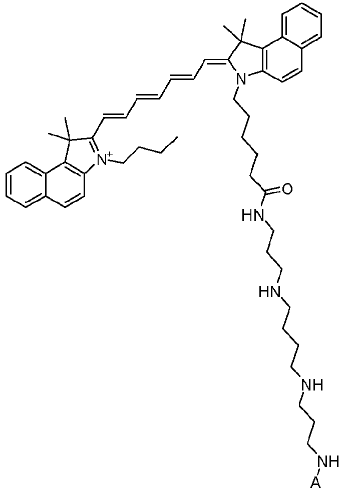 | 649 | 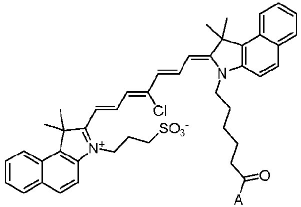 |
| 620 | 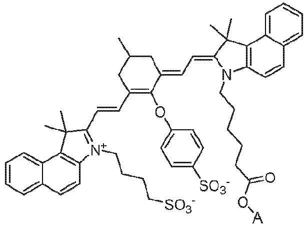 | 650 | 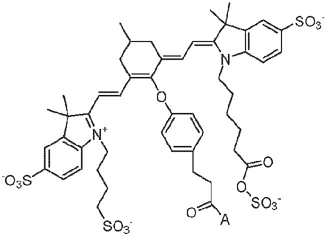 |

**Table 13. Compounds.**

| A = MCMPCFTTDHQMAR**K**CDDCCGGRGRGRCYGPQCLCR (SEQ ID NO: 25) (attached at K-15) | | | |
|---|---|---|---|
| **No.** | **Structure** | **No.** | **Strucure** |
| 651 | 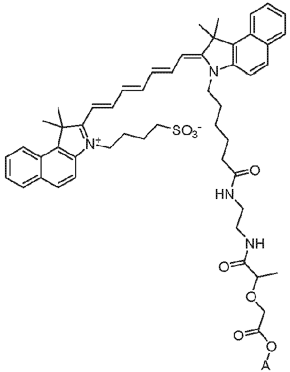 | 681 | 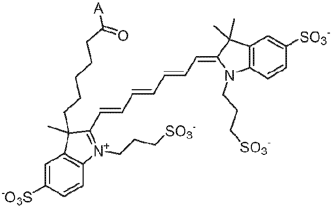 |
| 652 | 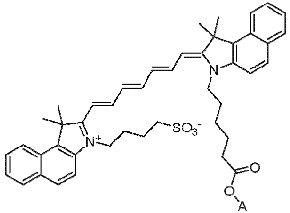 | 682 | 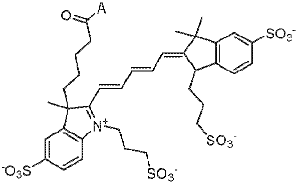 |
| 653 | 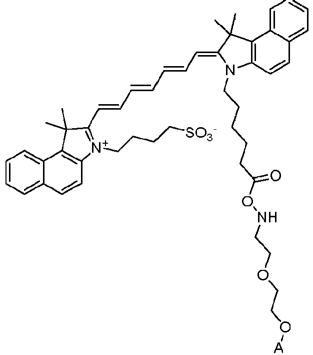 | 683 | 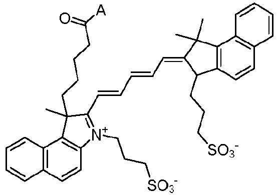 |
| 654 | 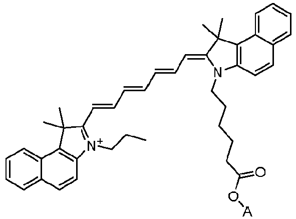 | 684 | 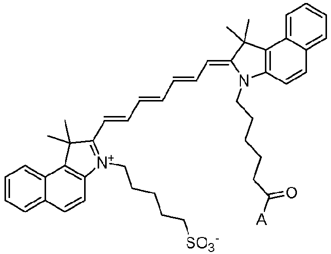 |
| 655 | 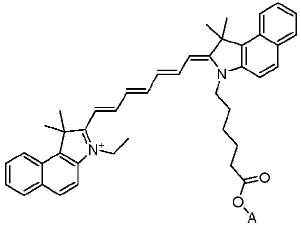 | 685 | 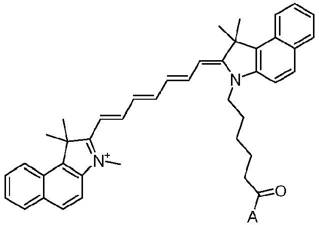 |
| 656 | 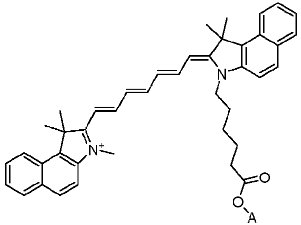 | 686 | 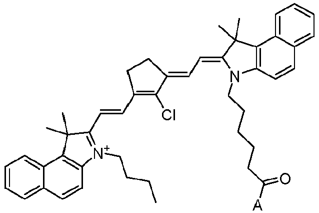 |
| 657 | 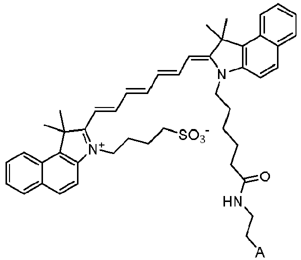 | 687 | 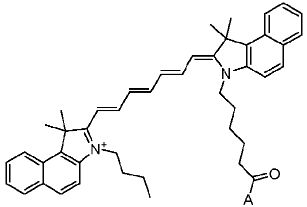 |
| 658 | 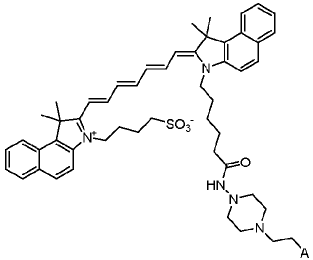 | 688 | 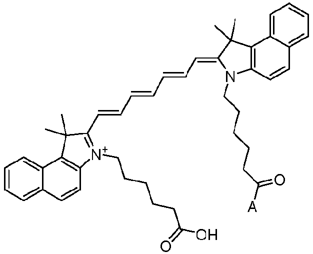 |
| 659 | 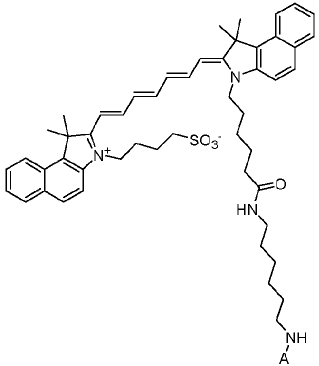 | 689 | 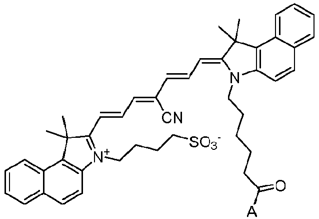 |
| 660 | 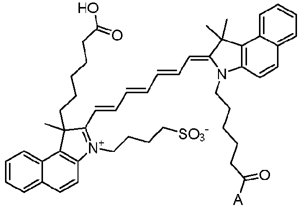 | 690 | 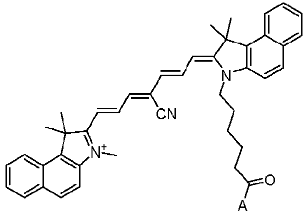 |
| 661 | 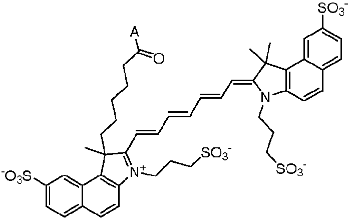 | 691 | 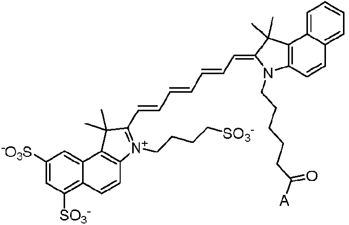 |
| 662 | 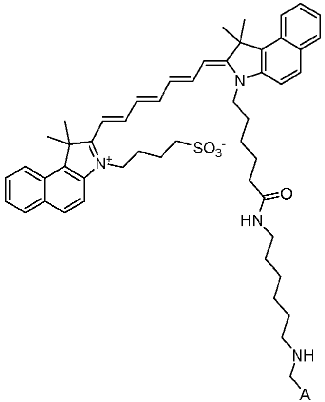 | 692 | 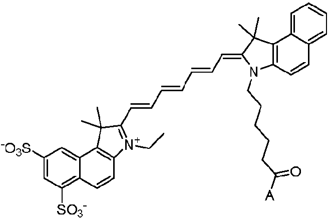 |
| 663 | 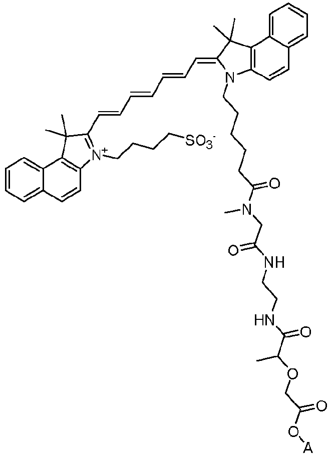 | 693 | 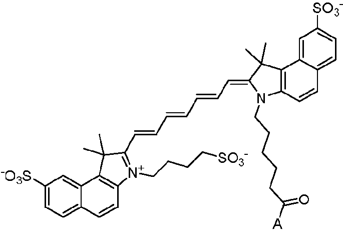 |
| 664 | 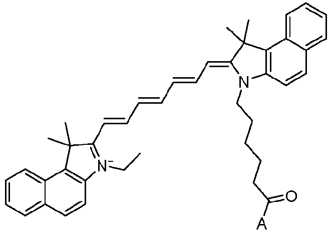 | 694 | 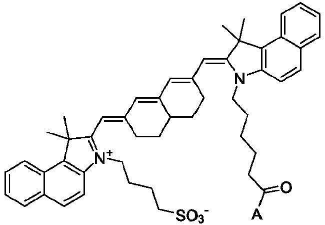 |
| 665 | 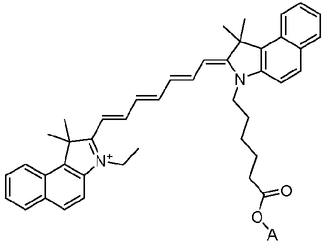 | 695 | 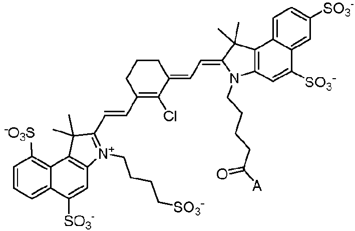 |
| 666 | 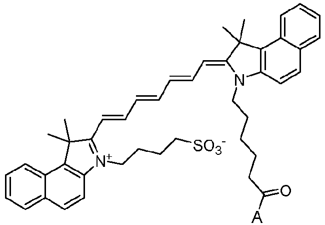 | 696 | 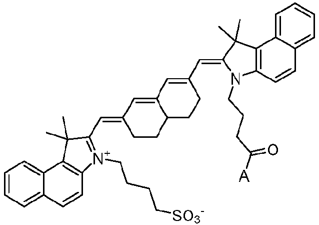 |
| 667 | 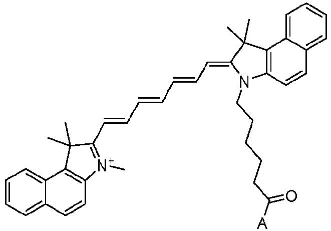 | 697 | 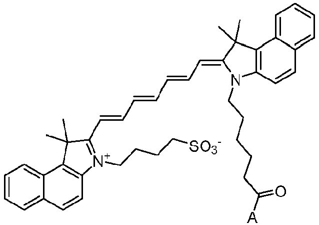 |
| 668 | 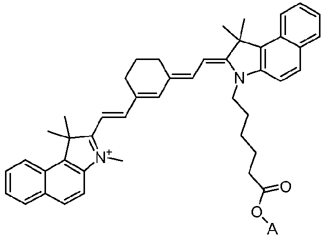 | 698 | 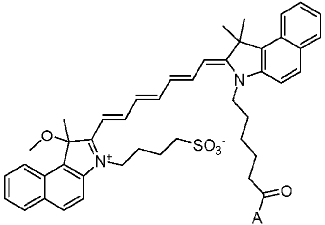 |
| 669 | 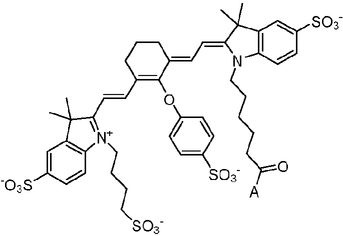 | 699 | 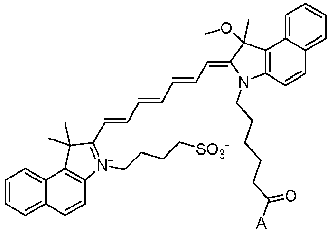 |
| 670 | 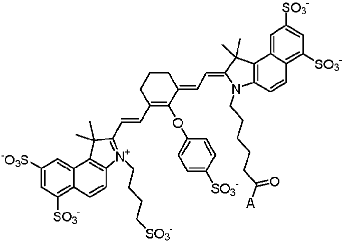 | 700 | 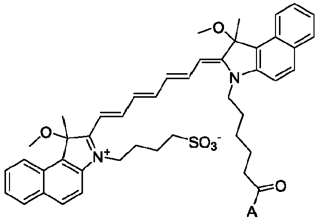 |
| 671 | 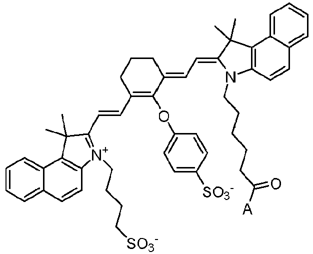 | 701 | 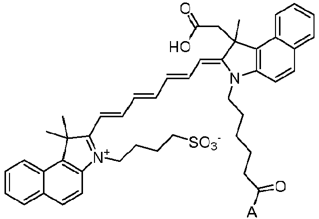 |
| 672 | 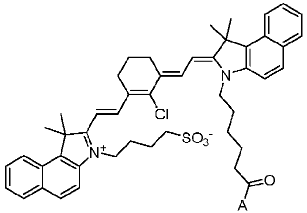 | 702 | 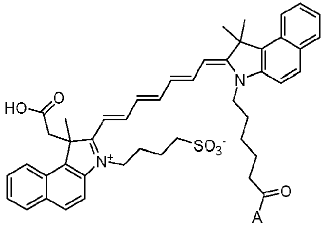 |
| 673 | 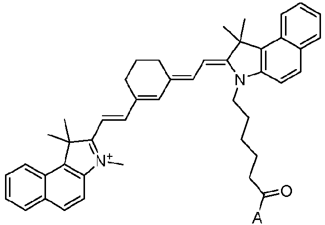 | 703 | 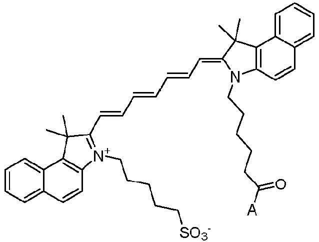 |
| 674 | 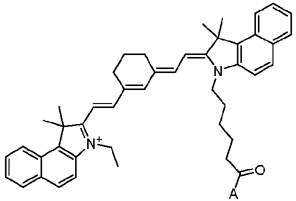 | 704 | 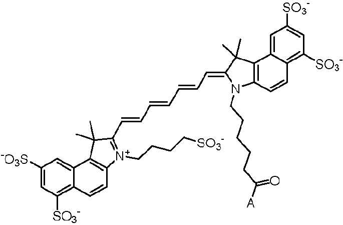 |
| 675 | 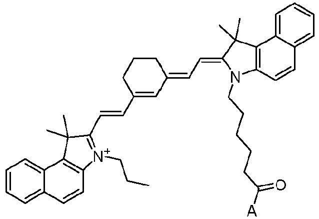 | 705 | 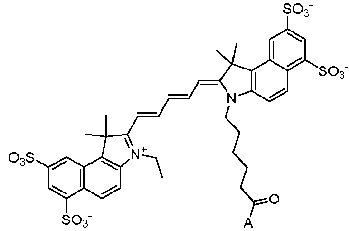 |
| 676 | 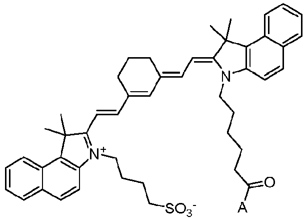 | 706 | 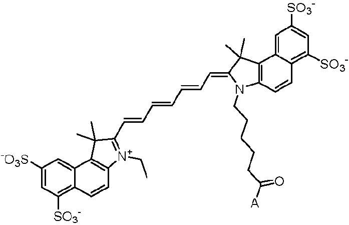 |
| 677 | 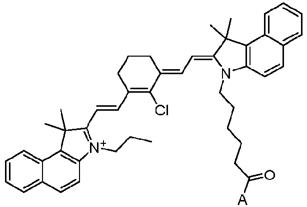 | 707 | 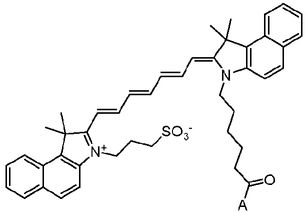 |
| 678 | 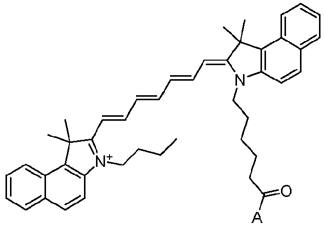 | 708 | 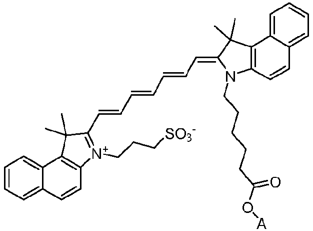 |
| 679 | 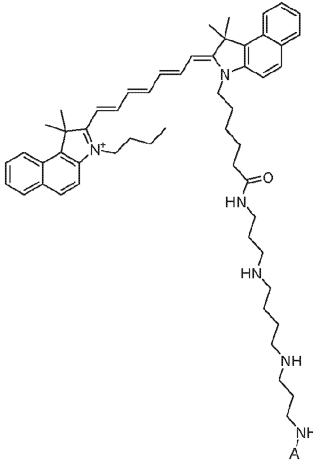 | 709 | 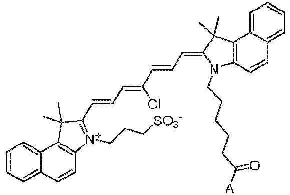 |
| 680 | 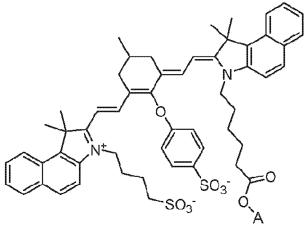 | 710 | 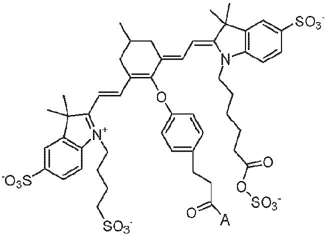 |

In certain aspects, the presently described peptides are conjugated to moieties, such as detectable labels (e.g., dyes or radiolabels) that are detected (e.g., visualized) in a subject. In some aspects, the chlorotoxin and/or chlorotoxin variants is conjugated to detectable labels to enable tracking of the bio-distribution of a conjugated peptide. The fluorescent moiety is covalently coupled to the chlorotoxin to allow for the visualization of the conjugate by fluorescence imaging, either directly or through a linker as described herein and known to one of ordinary skill in the art.

In some aspects, the fluorescent label has emission characteristics that are desired for a particular application. For example, the fluorescent label is a fluorescent dye that has a emission wavelength maximum between a range of 500 nm to 1100 nm, between a range of 600 nm to 1000 nm, between a range of 600 to 800 nm, between a range of 650 nm to 850 nm, or between a range of 700 nm to 800 nm. For another example, the fluorescent label is a fluorescent dye that has a emission wavelength maximum between a range of about 500 nm to about 1100 nm, between a range of about 600 nm to about 1000 nm, between a range of about 600 to about 800 nm, between a range of about 650 nm to about 850 nm, or between a range of about 700 nm to about 800 nm. One of ordinary skill in the art will appreciate the various dyes that are used as detectable labels and that have the emission characteristics above.

Some other examplary dyes include near-infrared dyes, such as, but not limited to, DyLight-680, DyLight-750, VivoTag-750, DyLight-800, IRDye-800, VivoTag-680, Cy5.5, or indocyanine green (ICG). In some aspects, near infrared dyes often include cyanine dyes. Additional non-limiting examples of fluorescent dyes for use as a conjugating molecule in the present disclosure include acradine orange or yellow, Alexa Fluors and any derivative thereof, 7-actinomycin D, 8-anilinonaphthalene-1-sulfonic acid, ATTO dye and any derivative thereof, auramine-rhodamine stain and any derivative thereof, bensantrhone, bimane, 9-10-bis(phenylethynyl)anthracene, 5,12 - bis(phenylethynyl)naththacene, bisbenzimide, brainbow, calcein, carbodyfluorescein and any derivative thereof, 1-chloro-9,10-bis(phenylethynyl)anthracene and any derivative thereof, DAPI, DiOC6, DyLight Fluors and any derivative thereof, epicocconone, ethidium bromide, FlAsH-EDT2, Fluo dye and any derivative thereof, FluoProbe and any derivative thereof, Fluorescein and any derivative thereof, Fura and any derivative thereof, GelGreen and any derivative thereof, GelRed and any derivative thereof, fluorescent proteins and any derivative thereof, m isoform proteins and any derivative thereof such as for example mCherry, hetamethine dye and any derivative thereof, hoeschst stain, iminocoumarin, indian yellow, indo-1 and any derivative thereof, laurdan, lucifer yellow and any derivative thereof, luciferin and any derivative thereof, luciferase and any derivative thereof, mercocyanine and any derivative thereof, nile dyes and any derivative thereof, perylene, phloxine, phyco dye and any derivative thereof, propium iodide, pyranine, rhodamine and any derivative thereof, ribogreen, RoGFP, rubrene, stilbene and any derivative thereof, sulforhodamine and any derivative thereof, SYBR and any derivative thereof, synapto-pHluorin, tetraphenyl butadiene, tetrasodium tris, Texas Red, Titan Yellow, TSQ, umbelliferone, violanthrone, yellow fluroescent protein and YOYO-1. Other Suitable fluorescent dyes include, but are not limited to, fluorescein and fluorescein dyes (e.g., fluorescein isothiocyanine or FITC, naphthofluorescein, 4',5'-dichloro-2',7' -dimethoxyfluorescein, 6-carboxyfluorescein or FAM, etc.), carbocyanine, merocyanine, styryl dyes, oxonol dyes, phycoerythrin, erythrosin, eosin, rhodamine dyes (e.g., carboxytetramethyl-rhodamine or TAMRA, carboxyrhodamine 6G, carboxy-X-rhodamine (ROX), lissamine rhodamine B, rhodamine 6G, rhodamine Green, rhodamine Red, tetramethylrhodamine (TMR), etc.), coumarin and coumarin dyes (e.g., methoxycoumarin, dialkylaminocoumarin, hydroxycoumarin, aminomethylcoumarin (AMCA), etc.), Oregon Green Dyes (e.g., Oregon Green 488, Oregon Green 500, Oregon Green 514., etc.), Texas Red, Texas Red-X, SPECTRUM RED, SPECTRUM GREEN, cyanine dyes (e.g., CY-3, Cy-5, CY-3.5, CY-5.5, etc.), ALEXA FLUOR dyes (e.g., ALEXA FLUOR 350, ALEXA FLUOR 488, ALEXA FLUOR 532, ALEXA FLUOR 546, ALEXA FLUOR 568, ALEXA FLUOR 594, ALEXA FLUOR 633, ALEXA FLUOR 660, ALEXA FLUOR 680, etc.), BODIPY dyes (e.g., BODIPY FL, BODIPY R6G, BODIPY TMR, BODIPY TR, BODIPY 530/550, BODIPY 558/568, BODIPY 564/570, BODIPY 576/589, BODIPY 581/591, BODIPY 630/650, BODIPY 650/665, etc.), IRDyes (e.g., IRD40, IRD 700, IRD 800, etc.), and the like. In some aspects, conjugates of the present disclosure comprise other dyes, including but not limited to those provided below in Table 14.

**Table 14. Exemplary fluorescent reporter molecules with peak absorbance (Abs.) and emission (Em.) wavelengths specifiec (in nanometers).**

| **Dye** | **Peak Abs.** | **Peak Em.** | | **Dye** | **Peak Abs.** | **Peak Em.** |
|---|---|---|---|---|---|---|
| Methoxycoumarin | 360 | 410 | | BFP | 382 | 448 |
| Fluospheres Blue | 356 | 412 | | 7-hydroxy-4-methylcoumarin | 360 | 449 |
| Cascade Blue | 377 | 420 | | | | |
| PBFI | 360 | 420 | | SpectrumBlue | 405 | 449 |
| DyeLight 405 | 400 | 420 | | DiFMU (pH 9.0) | 357 | 450 |
| Cascade Blue | 400 | 420 | | sgBFP (Super Glow BFP) | 387 | 450 |
| Alexa Fluor 405 | 401 | 421 | | SpectrumBlue | 400 | 450 |
| Alexa Fluor 405 | 401 | 421 | | Cell Trace Calcein Violet | 401 | 451 |
| LysoTracker Blue | 373 | 422 | | DAPI | 345 | 455 |
| LysoSensor Blue | 374 | 424 | | NucBlue Fixed Cell Stain | 345 | 455 |
| AMCA | 345 | 425 | | Pacific Blue | 405 | 455 |
| True Blue | 365 | 425 | | Pacific Blue | 410 | 455 |
| 7-amino-4-methylcoumarin (AMC) | 351 | 430 | | PO-PRO-1 | 435 | 455 |
| | | | | PO-PRO-1 | 435 | 455 |
| Phorwite AR | 360 | 430 | | POPO-1 | 434 | 456 |
| DyLight 350 | 353 | 432 | | POPO-1 | 434 | 456 |
| Uvitex SFC | 365 | 435 | | TagBFP | 402 | 457 |
| 4-methylumbelliferone | 360 | 440 | | Marina Blue | 365 | 460 |
| CellTrace Calcein Blue | 373 | 440 | | SITS | 365 | 460 |
| Calcofluor White | 350 | 440 | | Thioflavin TCN | 350 | 460 |
| Fast Blue | 360 | 440 | | Monochlorobimane(mBCI) | 380 | 461 |
| LysoSensor Yellow/Blue (p H 8.0) | 329 | 440 | | Quinine Sulfate | 349 | 461 |
| | | | | Acridine | 362 | 462 |
| LysoSensor Yellow/Blue (p H 8.0) | 329 | 440 | | CellLights CFP | 434 | 477 |
| | | | | ECFP | 434 | 477 |
| LysoSensor Yellow/Blue (p H 8.0) | 329 | 440 | | CFP | 434 | 477 |
| | | | | 1,8-ANS | 372 | 480 |
| LysoSensor Yellow/Blue (p H 8.0) | 329 | 440 | | SYTOX Blue | 444 | 480 |
| | | | | SYTOX Blue | 444 | 480 |
| Alexa Fluor 350 | 346 | 442 | | Hoechst 33342 | 347 | 483 |
| AMCAX | 353 | 442 | | NucBlue Live Cell Stain | 347 | 483 |
| LIVE/DEAD Fixable Blue D ead Cell Stain | 344 | 442 | | Thiolyte | 378 | 483 |
| | | | | SYTO 45 | 452 | 484 |
| Y66H | 360 | 442 | | SYTO 45 | 452 | 484 |
| ABQ | 344 | 445 | | SYTO 45 | 452 | 484 |
| BFP | 382 | 448 | | SYTO 45 | 452 | 484 |
| SYTO 45 | 452 | 484 | | YO-PRO-1 | 491 | 507 |
| Hoechst 33258 | 345 | 487 | | GFP | 488 | 507 |
| AmCyan | 548 | 489 | | YO-PRO-1 | 491 | 507 |
| Auramine O | 445 | 500 | | Premo FUCCI Cell Cycle Sensor (S/G2/M phases) | 474 | 509 |
| SYTO 9 | 482 | 500 | | | | |
| SYTO 9 | 482 | 500 | | sgGFP (Super Glow GFP) | 474 | 509 |
| SYTO 9 | 482 | 500 | | wtGFP (wild type GFP, non-UV excitation) | 475 | 509 |
| SYTO 9 | 482 | 500 | | | | |
| SYTO 9 | 482 | 500 | | YOYO-1 | 491 | 509 |
| DiO | 484 | 501 | | YOYO-1 | 491 | 509 |
| DiO | 484 | 501 | | YOYO-1 | 491 | 509 |
| DiO | 484 | 501 | | YOYO-1 | 491 | 509 |
| LysoSensor Green | 448 | 503 | | YOYO-1 | 491 | 509 |
| LysoSensor Green | 448 | 503 | | HPTS (Solvent Green 7) | 455 | 510 |
| LysoSensor Green | 448 | 503 | | Nitrobenzoxadiazole | 465 | 510 |
| LysoSensor Green | 448 | 503 | | S65L | 484 | 510 |
| LysoSensor Green | 448 | 503 | | LysoTracker Green | 504 | 511 |
| SYTO 13 | 487 | 505 | | S65T | 488 | 511 |
| LysoSensor Green (pH 5) | 442 | 505 | | LysoTracker Green | 504 | 511 |
| SYTO 13 | 487 | 505 | | LysoTracker Green | 504 | 511 |
| SYTO 13 | 487 | 505 | | MitoTracker Green FM | 490 | 512 |
| SYTO 13 | 487 | 505 | | MitoTracker Green FM | 490 | 512 |
| SYTO 13 | 487 | 505 | | MitoTracker Green FM | 490 | 512 |
| DiO (Vybrant DiO) | 489 | 506 | | MitoTracker Green FM | 490 | 512 |
| HCS LipidTox Green | 498 | 506 | | FluoSpheres Yellow-Green | 501 | 513 |
| LIVE/DEAD Fixable Green | 498 | 506 | | Evans Blue | 460 | 515 |
| LIVE/DEAD Fixable Green | 498 | 506 | | Evans Blue | 460 | 515 |
| ATTO 465 | 453 | 507 | | rsGFP (red shifted GFP, S65 T) | 498 | 516 |
| CellLights GFP | 488 | 507 | | | | |
| CellEvent Caspase-3/7 Green | 488 | 507 | | Cell Tracker Violet BMQC | 415 | 516 |
| Diversa Green-FP | 484 | 507 | | HCS CellMask Green | 493 | 516 |
| GFP (EGFP) | 488 | 507 | | Cell Tracker Violet BMQC | 415 | 516 |
| S65C | 479 | 507 | | Cell Tracker Violet BMQC | 415 | 516 |
| YO-PRO-1 | 491 | 507 | | Cell Tracker Violet BMQC | 415 | 516 |
| GFP | 488 | 507 | | Cell Tracker Violet BMQC | 415 | 516 |
| YO-PRO-1 | 491 | 507 | | HCS CellMask Green | 493 | 516 |
| GFP | 488 | 507 | | | | |
| 5-carboxyfluorescein(5-FAM) | 492 | 518 | | Rhodol Green | 496 | 523 |
| | | | | SYTOX Green | 504 | 523 |
| ActinGreen (Alexa Fluor 488 phalloidin) | 496 | 518 | | Rhodamine Green | 497 | 523 |
| | | | | Rhodamine Green | 497 | 523 |
| Alexa Fluor 488 | 496 | 518 | | Rhodamine Green | 497 | 523 |
| Click-iT EdU Alexa Fluor 488 | 496 | 518 | | Neurotrace 500/525 Green | 497 | 524 |
| | | | | Oregon Green 488 | 498 | 524 |
| DyLight+C110 488 | 493 | 518 | | SYBR Safe | 507 | 524 |
| Fluoro-Emerald | 494 | 518 | | NeuroTrace 500/525 Nissl st ain | 497 | 524 |
| Aiexa Fluor 488 | 496 | 518 | | | | |
| Carboxyfluorescein (5-FAM) | 492 | 518 | | Oregon Green 488 | 498 | 524 |
| Alexa Fluor 488 | 496 | 518 | | NeuroTrace 500/525 Nissl st ain | 497 | 524 |
| Carboxyfluorescein (5-FAM) | 492 | 518 | | | | |
| CellRox Green | 485 | 520 | | Oregon Green 488 | 498 | 524 |
| FITC (Fluorescein) | 492 | 520 | | NeuroTrace 500/525 Nissl st ain | 497 | 524 |
| Fluor-X | 494 | 520 | | | | |
| Rhodamine 110 | 496 | 520 | | NeuroTrace 500/525 Nissl st ain | 497 | 524 |
| SYTO 16 | 490 | 520 | | | | |
| FITC | 492 | 520 | | Oregon Green 488 | 498 | 524 |
| Rhodamine 110 | 496 | 520 | | Dansyl | 335 | 525 |
| SYTO 16 | 490 | 520 | | Fluoro-Jade B | 480 | 525 |
| FITC | 492 | 520 | | Qdot 525 | UV | 525 |
| Rhodamine 110 | 496 | 520 | | SYTO 11 | 506 | 525 |
| SYTO 16 | 490 | 520 | | Qdot 525 | UV | 525 |
| SYTO 16 | 490 | 520 | | Qdot 525 | UV | 525 |
| FITC | 492 | 520 | | Acridine Orange + DNA | 500 | 526 |
| Rhodamine 110 | 496 | 520 | | LIVE/DEAD Fixable Green | 498 | 526 |
| SYTO 16 | 490 | 520 | | Surf Green EX | 469 | 526 |
| SYBR Green I | 497 | 521 | | Acridine Orange + DNA | 500 | 526 |
| SYBR Green I | 497 | 521 | | Acridine Orange + DNA | 500 | 526 |
| SYBR Green I | 497 | 521 | | Acridine Orange + DNA | 500 | 526 |
| SYBR Green I | 497 | 521 | | Acridine Orange (+DNA) | 500 | 526 |
| SYBR Green I | 497 | 521 | | Thiol Tracker Violet | 405 | 526 |
| Quant-iT PicoGreen | 502 | 522 | | ThiolTracker Violet | 405 | 526 |
| Spectru mgreen | 498 | 522 | | ThiolTracker Violet | 405 | 526 |
| NucGreen Dead Cell Stain | 504 | 523 | | Thiol Tracker Violet | 405 | 526 |
| Rhodamine Green | 497 | 523 | | Acridine Orange (+DNA) | 500 | 526 |
| ThiolTracker Violet | 405 | 526 | | NBD-X | 467 | 538 |
| SYTO RNASelect | 503 | 527 | | SYBR Gold | 495 | 539 |
| EYFP | 514 | 527 | | SYBR Gold | 495 | 539 |
| SYTO RNASelect | 503 | 527 | | SYBR Gold | 495 | 539 |
| SYTO RNASelect | 503 | 527 | | SYBR Gold | 495 | 539 |
| SYTO RNASelect | 503 | 527 | | SYBR Gold | 495 | 539 |
| SYTO RNASelect | 503 | 527 | | Alexa Fluor 430 | 432 | 540 |
| Rhodamine 123 | 507 | 529 | | Auramine | 460 | 540 |
| YFP | 512 | 529 | | Aurophosphine | 470 | 540 |
| F2N12S | 405 | 530, 585 | | BCECF | 499 | 540 |
| | | | | BODIPY 492/515 | 490 | 540 |
| F2N12S | 405 | 530, 585 | | BODIPY 505/515 | 502 | 540 |
| | | | | BODIPY FL | 502 | 540 |
| F2N12S | 405 | 530, 585 | | BTC | 464 | 540 |
| | | | | Calcein | 494 | 540 |
| F2N12S | 405 | 530, 585 | | Calcium Green-1 | 506 | 540 |
| | | | | Catskill Green 540 | 482 | 540 |
| F2N12S | 405 | 530, 585 | | CellTracker Green | 490 | 540 |
| | | | | CFDA | 494 | 540 |
| F2N12S | 405 | 530, 585 | | CFP | 434 | 540 |
| | | | | Cy2 | 492 | 540 |
| F2N12S | 405 | 530, 585 | | CyQUANT Direct (CyQUANT GR) | 500 | 540 |
| Magnesium Green | 506 | 530 | | DAF-FM | 493 | 540 |
| NBD Amine | 450 | 530 | | Emerald Green | 490 | 540 |
| TO-PRO-1 | 515 | 530 | | Fluo-3 | 506 | 540 |
| TOTO-1 | 513 | 531 | | Fluo-4 | 494 | 540 |
| Oregon Green 514 | 512 | 532 | | H2DCFDA (H2-DCF,DCFR) | 504 | 540 |
| Sodium Green | 506 | 532 | | | | |
| Vybrant DyeCycle Green | 505 | 532 | | Alexa Fluor 430 | 434 | 540 |
| pHrodo Green | 509 | 533 | | Alexa Fluor 430 | 432 | 540 |
| NBD-X | 467 | 538 | | BCECF (pH 5.2) | 499 | 540 |
| NBD-X | 467 | 538 | | Calcein | 494 | 540 |
| NBD-X | 467 | 538 | | CellTracker Green CMFDA | 490 | 540 |
| NBD-X | 467 | 538 | | CFP | 434 | 540 |
| NBD-X | 467 | 538 | | Cy2 | 492 | 540 |
| NBD-X | 467 | 538 | | CyQUANT Direct | 500 | 540 |
| DAF-FM | 493 | 540 | | Lucifer yellow | 428 | 544 |
| Fluo-4 | 494 | 540 | | Lucifer Yellow | 428 | 544 |
| Alexa Fluor 430 | 432 | 540 | | Lucifer yellow | 428 | 544 |
| BCECF (pH 5.2) | 499 | 540 | | Eosin | 524 | 545 |
| Calcein | 494 | 540 | | JOJO-1 | 529 | 545 |
| CellTracker Green CMFDA | 490 | 540 | | Qdot 545 | UV | 545 |
| CFP | 434 | 540 | | Qdot 545 | UV | 545 |
| Cy2 | 492 | 540 | | Auramine O | 460 | 550 |
| CyQUANT Direct | 500 | 540 | | Pacific Orange | 440 | 551 |
| Alexa Fluor 430 | 432 | 540 | | Pacific Orange | 440 | 551 |
| BCECF (pH 5.2) | 499 | 540 | | Pacific Orange | 440 | 551 |
| CFP | 434 | 540 | | Pacific Orange | 440 | 551 |
| Cy2 | 492 | 540 | | Pacific Orange | 440 | 551 |
| Alexa Fluor 430 | 432 | 540 | | Pacific Orange | 440 | 551 |
| BCECF (pH 5.2) | 499 | 540 | | mBanana | 540 | 553 |
| Alexa Fluor 430 | 432 | 540 | | ER-Tracker Blue-White DPX | 371 | 554 |
| BCECF (pH 5.2) | 499 | 540 | | | | |
| Alexa Fluor 430 | 432 | 540 | | Alexa Fluor 532 | 532 | 554 |
| BCECF (pH 5.2) | 499 | 540 | | FocalCheck Double Orange | 540 | 555 |
| Calcein | 494 | 540 | | HEX | 533 | 558 |
| CellTracker Green CMFDA | 490 | 540 | | Fluospheres Orange | 539 | 560 |
| CFP | 434 | 540 | | mHoneydew | 478 | 561 |
| Cy2 | 492 | 540 | | Vybrant DyeCycle Orange | 518 | 562 |
| CyQUANT Direct | 500 | 540 | | ActinRed 555 (rhodamin pphalloidin) | 540 | 565 |
| DAF-FM | 493 | 540 | | | | |
| Fluo-4 | 494 | 540 | | Alexa Fluor 555 | 555 | 565 |
| TET | 520 | 541 | | CellRox Orange | 545 | 565 |
| TET | 521 | 542 | | Qdot 565 | UV | 565 |
| Lucifer Yellow | 423 | 543 | | Qdot 565 | UV | 565 |
| Qdot 545 | UV | 543 | | DiI (CellTracker Dil) | 551 | 568 |
| Lucifer Yellow | 423 | 543 | | mOrange | 548 | 568 |
| Lucifer Yellow | 423 | 543 | | OFP | 546 | 568 |
| Lucifer Yellow | 423 | 543 | | Bodipy TMR | 544 | 569 |
| Lucifer Yellow | 423 | 543 | | Cy3 | 552 | 570 |
| Lucifer Yellow | 423 | 543 | | PO-PRO-3 | 539 | 570 |
| Lucifer Yellow | 423 | 543 | | SYTOX Orange | 567 | 570 |
| Lucifer Yellow | 423 | 543 | | CellMask Orange | 556 | 571 |
| Alexa Fluor 546 | 561 | 572 | | RFP | 552 | 585 |
| POPO-3 | 532 | 573 | | Qdot 585 | UV | 585 |
| TurboRFP | 553 | 574 | | Qdot 585 | UV | 585 |
| Calcium Orange | 549 | 575 | | DsRed Monomer | 556 | 586 |
| CellTracker Orange | 547 | 575 | | pHrodo Red | 559 | 586 |
| LIVE/DEAD Fixable Yellow | 405 | 575 | | Carboxy SNARF-1 | 548 | 587 |
| LIVE/DEAD Fixable Yellow | 405 | 575 | | pHrodo Red | 559 | 587 |
| LIVE/DEAD Fixable Yellow | 405 | 575 | | SpectrumOrange | 559 | 588 |
| LIVE/DEAD Fixable Yellow | 405 | 575 | | DsRed2 | 563 | 588 |
| LIVE/DEAD Fixable Yellow | 405 | 575 | | DiA | 456 | 590 |
| LIVE/DEAD Fixable Yellow | 405 | 575 | | DiA | 456 | 590 |
| DyLight 594 | 562 | 576 | | DiA | 456 | 590 |
| MitoTracker Orange CMTMRos(MitoTracker Orange CM-H2TMRos) | 551 | 576 | | DiA | 456 | 590 |
| | | | | DiA | 456 | 590 |
| | | | | DiA | 456 | 590 |
| Phycoerythrin (PE, R-phycoerythrin) | 567 | 576 | | DiA | 456 | 590 |
| | | | | DiA | 456 | 590 |
| Rhod-2 | 551 | 576 | | rhodamine Red-X | 572 | 591 |
| Rhodamine Phalloidin | 557 | 576 | | CellTrace calcein red-orange | 575 | 592 |
| X-Rhod-1 | 570 | 576 | | LysoTracker Red | 573 | 592 |
| DsRed-Express | 557 | 579 | | Sulforhodamine 101 | 578 | 593 |
| Rhodamine Red | 560 | 580 | | sulforhodamine 101 | 577 | 593 |
| TAMRA | 565 | 580 | | ROX (6-ROX) | 568 | 595 |
| Tetramethylrhodamine (TRI TC) | 555 | 580 | | 2-dodecylresorufin | 582 | 595 |
| | | | | Cy3.5 | 579 | 597 |
| dTomato | 554 | 581 | | Cy 3.5 | 581 | 597 |
| DsRed2 | 563 | 582 | | MitoTracker Red CMXRos | 578 | 597 |
| Amplex Ultra Red | 567 | 582 | | BOBO-3 | 570 | 602 |
| Amplex Red | 571 | 583 | | Ethidium Bromide | 521 | 602 |
| Amplex UltraRed | 568 | 583 | | X-rhod-1 | 579 | 602 |
| Amplex Red | 570 | 583 | | BOBO-1 | 570 | 602 |
| Premo FUCCI Cell Cycle Sensor (G1 phase) | 555 | 584 | | BOBO-1 | 570 | 602 |
| | | | | BOBO-1 | 570 | 602 |
| TagRFP | 555 | 584 | | 5-ROX | 577 | 603 |
| CellLights RFP | 552 | 585 | | Alexa Fluor 568 | 578 | 603 |
| mTangerine | 568 | 585 | | Qdot 605 | UV | 605 |
| Resorufm | 570 | 585 | | Qdot 605 | UV | 605 |
| BOBO-3 | 571 | 606 | | FM 143 | 510 | 626 |
| Calcium Crimson | 589 | 608 | | FM 143 | 510 | 626 |
| Fluospheres Red microspher es | 577 | 608 | | FM 143 | 510 | 626 |
| | | | | FM 143 | 510 | 626 |
| ReAsH (TC-ReAsH) | 593 | 608 | | FM 143 | 510 | 626 |
| CellTracker Red | 585 | 612 | | FM 143 | 510 | 626 |
| LIVE/DEAD Fixable Red | 593 | 613 | | YO-PRO-3 | 612 | 628 |
| CellTracker Red CMTPX | 584 | 613 | | Alexa Fluor 610 | 610 | 629 |
| LIVE/DEAD Fixable Red Dead Cell stain | 595 | 613 | | Magic Red | 570 | 630 |
| | | | | CTC Formazan | 450 | 630 |
| DiA (FAST DiA) | 491 | 613 | | CTC Formazan | 450 | 630 |
| DiA | 491 | 613 | | YOYO-3 | 612 | 631 |
| HCS CellMask Red stain | 587 | 614 | | Katushka (Turbo FP635) | 588 | 635 |
| HCS LipidTox Red | 582 | 615 | | mKate | 588 | 635 |
| HCS LipidTOX Red | 582 | 615 | | SYTO 17 | 620 | 635 |
| mCherry | 587 | 615 | | Di-8 ANEPPS | 468 | 635 |
| Texas Red | 592 | 615 | | Di-8 ANEPPS | 468 | 635 |
| Ethidium Homodimer-1 (EthD-1) | 530 | 618 | | Di-8-ANEPPS | 465 | 635 |
| | | | | Di-8-ANEPPS | 465 | 635 |
| Propidium Iodide (PI) | 530 | 618 | | Di-8-ANEPPS | 465 | 635 |
| Alexa Fluor 594 | 590 | 618 | | Di-8-ANEPPS | 465 | 635 |
| Click-iT Alexa Fluor 594 | 590 | 618 | | Di-8-ANEPPS | 465 | 635 |
| DyLight 594 | 593 | 618 | | Di-8-ANEPPS | 465 | 635 |
| SYPRO Ruby | 450 | 618 | | Di-8-ANEPPS | 465 | 635 |
| SYPRO Ruby | 450 | 618 | | Nile Red | 551 | 636 |
| SYPRO Ruby | 450 | 618 | | Nile red (triglyceride) | 552 | 636 |
| SYPRO Ruby | 450 | 618 | | Nile red (triglyceride) | 552 | 636 |
| SYPRO Ruby | 450 | 618 | | Nile red (triglyceride) | 552 | 636 |
| SYPRO Ruby | 450 | 618 | | Fura Red (high Ca2+) | 436 | 637 |
| Bodipy TR-X | 588 | 621 | | Nile Red phospholipid | 551 | 638 |
| CellTrace BODIPY TR meth yl esther | 597 | 625 | | SYTO 17 | 619 | 638 |
| | | | | Bodipy 630/650-X | 625 | 641 |
| mRaspberry | 598 | 625 | | BODIPY 630/650X | 626 | 641 |
| Qdot 625 | UV | 625 | | 7-AAD | 549 | 644 |
| Qdot 625 | UV | 625 | | HCS NuclearMask Red | 624 | 644 |
| FM 1-43 | 510 | 626 | | HCS NuclearMask Red | 622 | 644 |
| FM 1-43 | 510 | 626 | | SYTO 59 | 621 | 644 |
| SYTO 59 | 622 | 645 | | LIVE/DEAD Fixable Far Red | 650 | 665 |
| Fluospheres Crimson micros pheres | 620 | 646 | | | | |
| | | | | Cy5 | 648 | 666 |
| FluoSpheres crimson micros pheres | 621 | 646 | | Lysotracker Deep Red | 647 | 668 |
| | | | | Alexa Fluor 647 | 650 | 670 |
| SYTOX AADvanced dead c ell stain | 546 | 647 | | Click-iT Alexa Fluor 647 | 650 | 670 |
| | | | | DiD (Vybrant DiD) | 645 | 670 |
| Alexa Fluor 635 | 634 | 647 | | HCS CellMask Deep Red sta in | 649 | 670 |
| HcRed | 594 | 649 | | | | |
| mPlum | 590 | 649 | | ATTO 647 | 644 | 670 |
| SYTO 61 | 619 | 649 | | Fura Red (-Ca2+) | 473 | 670 |
| Alexa Fluor 633 | 631 | 650 | | Fura Red (-Ca2+) | 473 | 670 |
| Acridine Orange + RNA | 460 | 650 | | Fura Red (-Ca2+) | 473 | 670 |
| Acridine Orange + RNA | 460 | 650 | | Fura Red (-Ca2+) | 473 | 670 |
| Acridine Orange (+RNA) | 460 | 650 | | Fura Red (-Ca2+) | 473 | 670 |
| Acridine Orange (+RNA) | 460 | 650 | | DyLight 649 | 654 | 673 |
| HCS LipidTOX Deep Red | 634 | 652 | | Carboxynaphthofluorescein | 600 | 674 |
| Fura Red (+Ca2+) | 436 | 655 | | PerCP | 488 | 675 |
| Fura Red (+Ca2+) | 436 | 655 | | CellMask Deep Red plasma membrane stain | 658 | 676 |
| Fura Red (+Ca2+) | 436 | 655 | | | | |
| Fura Red (+Ca2+) | 436 | 655 | | DRAQ5 | 650 | 680 |
| Qdot 655 | UV | 655 | | SYTO 60 | 649 | 681 |
| Fura Red (+Ca2+) | 436 | 655 | | SYTO 62 | 650 | 681 |
| Fura Red (+Ca2+) | 436 | 655 | | SYTO 60 | 650 | 681 |
| Qdot 655 | UV | 655 | | FluoSpheres dark red micros pheres | 657 | 683 |
| FxCycle Far Red | 641 | 657 | | | | |
| TO-PRO-3 | 642 | 657 | | ATTO 655 | 663 | 683 |
| DDAO | 648 | 658 | | FluoSpheres Dark Red fluorescent microspheres | 656 | 683 |
| DyLight 633 | 638 | 658 | | | | |
| SYTOX Red | 640 | 658 | | NucRed Live 647 | 638 | 686 |
| ATTO 635 | 635 | 658 | | Vybrant DyeCycle Ruby | 638 | 686 |
| APC (Allophycocyanin) | 651 | 660 | | HCS NuclearMask Deep Red | 635 | 687 |
| MitoTracker Deep Red FM | 641 | 661 | | Cy5.5 | 672 | 690 |
| NucRed Dead 647 | 642 | 661 | | Alexa Fluor 660 | 663 | 691 |
| TOTO-3 | 642 | 661 | | Alexa Fluor 660 | 663 | 691 |
| BODIPY 650/665 | 647 | 665 | | Cy5.5 | 678 | 696 |
| CellRox Deep Red | 640 | 665 | | DY-675 | 675 | 699 |
| IRDye 700 Phosphoramidite | 691 | 699 | | Alexa Fluor 700 | 696 | 719 |
| ATTO 680 | 680 | 700 | | ATTO 700 | 699 | 719 |
| Alexa Fluor 680 | 679 | 702 | | FM 4-64 | 558 | 734 |
| HiLyte Fluor 680 | 688 | 702 | | FM 4-64 | 558 | 734 |
| Qdot 705 Nanocrystals | 300 | 702 | | FM 4-64 | 558 | 734 |
| Alexa Fluor 680 | 679 | 704 | | FM 4-64 | 558 | 734 |
| DyLight 680 | 676 | 705 | | Cy7 | 745 | 766 |
| Qdot 705 | UV | 705 | | LIVE/DEAD Fixable near-IR | 750 | 775 |
| Qdot 705 | UV | 705 | | | | |
| Quasa 705 | 688 | 706 | | CellVue NIR780 | 743 | 776 |
| IRDye 680 NHS Ester | 683 | 710 | | DyLight 750 | 752 | 778 |
| RH 795 | 530 | 712 | | IRDye 800CW | 774 | 789 |
| RH 795 | 530 | 712 | | XenoLight CF770 | 770 | 797 |
| RH 795 | 530 | 712 | | Qdot 800 | UV | 800 |
| RH 795 | 530 | 712 | | Qdot 800 | UV | 800 |
| RH 795 | 530 | 712 | | Indocyanine Green | 768 | 807 |

In some other aspects, the conjugate compounds include a chemiluminescent compound, colloidal metal, luminescent compound, enzyme, radioisotope, or paramagnetic labels.

In certain aspects, the conjugates of the present disclosure are conjugated to radioactive isotopes instead of or in addition to other types of detectable agents. Certain isotopes sutable for use in the present compounds include, but not limited to, iodine-131, iodine-125, bismuth-212, bismuth-213, lutetium-177, rhenium-186, rhenium-188, yttrium-90, astatine-211, phosphorus-32 and/or samarium-153. In some aspects, the conjugates of the present disclosure contain one or more atoms having an atomic mass or mass number different from the atomic mass or mass number usually found in nature, including but not limited to hydrogen, carbon, fluorine, phosphorous, copper, gallium, yttrium, technetium, indium, iodine, rhenium, thallium, bismuth, astatine, samarium, and lutetium (for example, 3H , ³H, ¹³C, ¹⁴C, ¹⁸F, ³²P, ³⁵S, ⁶⁴Cu, ⁶⁷Ga, ⁹⁰Y, ^{99M}Tc, ¹¹¹In, ¹²⁵I, ¹²³I, ¹³¹I, ¹³⁵I, ¹⁸⁶Re, ¹⁸⁷Re, ²⁰¹Tl, ²¹²Bi, ²¹¹At, ¹⁵³Sm and/or ¹⁷⁷Lu). In other aspects, the conjugates of the present disclosure are labeled with a paramagnetic metal ion that is a good contrast enhancer in Magnetic Resonance Imaging (MRI). Examples of such paramagnetic metal ions include, but are not limited to, gadolinium III (Gd3+), chromium 111 (Cr3+), dysprosium III (Dy3+), iron 111 (Fe3+), manganese II (Mn2+), and ytterbium III (Yb3+). In certain embodiments, the labeling moieties comprises gadolinium III (Gd3+)

In some aspects, the conjugates of the present disclosure are conjugated to biotin. In addition of extension of half-life, biotin also acts as an affinity handle for retrieval of the peptides from tissues or other locations. In one aspect, the conjugates are conjugated, e.g., to a biotinidase resistant biotin with a PEG linker (e.g., NHS-dPEG4-Biotinidase resistant biotin). In some aspects, fluorescent biotin conjugates that can act both as a detectable label and an affinity handle are used. Non-limiting examples of commercially available fluorescent biotin conjugates include Atto 425-Biotin, Atto 488-Biotin, Atto 520-Biotin, Atto-550 Biotin, Atto 565-Biotin, Atto 590-Biotin, Atto 610-Biotin, Atto 620-Biotin, Atto 655-Biotin, Atto 680-Biotin, Atto 700-Biotin, Atto 725-Biotin, Atto 740-Biotin, fluorescein biotin, biotin-4-fluorescein, biotin-(5-fluorescein) conjugate, and biotin-B-phycoerythrin, alexa fluor 488 biocytin, alexa flour 546, alexa fluor 549, lucifer yellow cadaverine biotin-X, Lucifer yellow biocytin, Oregon green 488 biocytin, biotin-rhodamine and tetramethylrhodamine biocytin.

**Linkers.** In some aspects, the peptides of the present disclosure are directly conjugated to a detectable label, such as a dye, fluorescent moiety or the like such that no additional amino acids, carbohydrates, nucleic acids, polymers, organic chains, or the like are added to the chlorotoxin or chlorotoxin variant and/or the dye, fluorescent moiety or the like to comprise the chlorotoxin conjugates described herein. In some other aspects, a linker is used to conjugate the chlorotoxin or chlorotoxin variant is not directly conjugated to a dye, fluorescent moiety or the like such that additional amino acids, carbohydrates, nucleic acids or the like are added to the chlorotoxin or chlorotoxin variant and/or the dye, fluorescent moiety or the like to comprise the chlorotoxin conjugates described herein. A "linker" as used herein refers to at least one compound comprising two functional groups that are capable of reacting specifically with other moieties to form covalent or non-covalent linkages. Such moieties include, but are not limited to, the side groups on natural or non-natural amino acids or peptides which contain such natural or non-natural amino acids. By way of example, a linker has a functional group reactive with a group on a first peptide, and another functional group which is reactive with a group on a second peptide, whereby forming a conjugate that includes the first peptide, the linker and the second peptide. Many procedures and linker molecules for attachment of various compounds to peptides are known. See, e.g., European Patent Application No. 188,256; U.S. Pat. Nos. 4,671,958, 4,659,839, 4,414,148, 4,699,784; 4,680,338; and 4,569,789.

The term "linkage," as used herein refers to a bond or a chemical moiety formed from a chemical reaction between the functional group of a linker and another molecule. Such bonds include, but are not limited to, covalent linkages and non-covalent bonds, while such chemical moieties include, but are not limited to, esters, carbonates, imines phosphate esters, hydrazones, acetals, orthoesters, peptide linkages, and oligonucleotide linkages. Hydrolytically stable linkages means that the linkages are substantially stable in water and do not react with water at neutral pH values, including but not limited to, under physiological conditions for an extended period of time, perhaps even indefinitely. Hydrolytically unstable or degradable linkages mean that the linkages are degradable in water or in aqueous solutions, including for example, blood. Enzymatically unstable or degradable linkages mean that the linkage is often degraded by one or more enzymes. By way of example, PEG and related polymers include degradable linkages in the polymer backbone or in the linker group between the polymer backbone and one or more of the terminal functional groups of the polymer molecule. Such degradable linkages include, but are not limited to, ester linkages formed by the reaction of PEG carboxylic acids or activated PEG carboxylic acids with alcohol groups on a biologically active agent, wherein such ester groups generally hydrolyze under physiological conditions to release the biologically active agent. Other hydrolytically degradable linkages include but are not limited to carbonate linkages; imine linkages resulted from reaction of an amine and an aldehyde; phosphate ester linkages formed by reacting an alcohol with a phosphate group; hydrazone linkages which are reaction product of a hydrazide and an aldehyde; acetal linkages that are the reaction product of an aldehyde and an alcohol; orthoester linkages that are the reaction product of a formate and an alcohol; peptide linkages formed by an amine group, including but not limited to, at an end of a polymer such as PEG, and a carboxyl group of a peptide; and oligonucleotide linkages formed by a phosphoramidite group, including but not limited to, at the end of a polymer, and a 5' hydroxyl group of an oligonucleotide.

The conjugates for use in the method described herein is conjugated by using any art-recognized method forming a complex including covalent, ionic, or hydrogen bonding of the ligand to the imaging agent, either directly or indirectly via a linking group such as a linker. The conjugate is typically formed by covalent bonding of the ligand to the imaging agent through the formation of amide, ester or imino bonds between acid, aldehyde, hydroxy, amino, or hydrazo groups on the respective components of the complex or, for example, by the formation of disulfide bonds.

In addition, structural modifications of a linker portion of the conjugates are contemplated herein. For example, a number of amino acid substitutions are often made to the linker portion of the conjugate, including but not limited to naturally occurring amino acids, as well as those available from conventional synthetic methods. In one aspect, beta, gamma, and longer chain amino acids are used in place of one or more alpha amino acids. In another aspect, the stereochemistry of the chiral centers found in such molecules is selected to form various mixture of optical purity of the entire molecule, or only of a subset of the chiral centers present. In another aspect, the length of the peptide chain included in the linker is shortened or lengthened, either by changing the number of amino acids included therein, or by including more or fewer beta, gamma, or longer chain amino acids. In another aspect, the selection of amino acid side chains in the peptide portion is made to increase or decrease the relative hydrophilicity of the linker portion specifically or of the overall molecule generally.

Similarly, the length and shape of other chemical fragments of the linkers described herein is often modified. In some aspects, the linker includes an alkylene chain. The alkylene chain often varies in length, or includes branched groups, or includes a cyclic portion, which are in line or spiro relative to the allylene chain. In another aspect, where the linker includes a beta thiol releasable fragment, it is appreciated that other intervening groups connecting the thiol end to the hydroxy or carbonate end are used in place of the ethylene bridge, such as but not limited to optionally substituted benzyl groups, where the hydroxy end is connected at the benzyl carbon and the thiol end is connected through the ortho or para phenyl position, and vice versa.

### Formulations of Chlorotoxin Conjugates

In various aspects, the present disclosure provides compositions comprising the above-described compounds and a pharmaceutically acceptable carrier. In some aspects, the composition is formulated for parenteral administration. In further aspects, the composition is formulated for intravenous administration, intramuscular administration, subcutaneous administration, or a combination thereof.

Certain methods described herein comprise administering to the subject an intravenous pharmaceutical composition comprising a chlorotoxin conjugate, for example, as described herein. Intravenous pharmaceutical compositions of chlorotoxin conjugates include any formulation suitable for administration to a subject via any intravenous method, including a bolus, an infusion which occurs over time or any other intravenous method known in the art. In some aspects, the rate of infusion is such that the dose is administered over a period of less than five minutes, more than five minutes but less than 15 minutes or greater than 15 minutes. In other aspects, the rate of infusion is such that the dose is administered over a period of less than 5 minutes. In other aspects, the rate of infusion is such that the dose is administered over a period of greater than 5 minutes and less than 15 minutes. In some other aspects, the rate of infusion is such that the dose is administered over a period of greater than 15 minutes.

"Product" or "dosage form" as used herein refers to any solid, semi-solid, lyophilized, aqueous, liquid or frozen formulation or preparation used for administration. Upon administration, the rate of release of an active moiety from a product is often greatly influenced by the excipients and/or product characteristics which make up the product itself. For example, an enteric coat on a tablet is designed to separate that tablet's contents from the stomach contents to prevent, for example, degradation of the stomach which often induces gastrointestinal discomfort or injury. According to the currently accepted conventional understanding, systemic exposure of the active moiety will be relatively insensitive to the small formulation changes.

As used herein "pharmaceutically acceptable" or "pharmacologically acceptable" includes molecular entities and compositions that do not produce an adverse, allergic or other untoward reaction when administered to a subject, as appropriate. "Pharmaceutically acceptable carrier" includes any and all solvents, dispersion media, coatings, antibacterial and antifungal agents, isotonic and absorption delaying agents and the like. The use of such media and agents for pharmaceutical active substances is well known in the art. Except insofar as any conventional media or agent is incompatible with the active ingredient, its use in the therapeutic compositions is contemplated. Supplementary active ingredients are often also incorporated into the compositions.

In various aspects, the present compositions comprise a concentration of the compound as an active pharmaceutical ingredient having a concentration of from 1 mg/mL to 40 mg/mL. In further aspects, the concentration of the compound is from 1 mg/mL to 20 mg/mL. In still other aspects, the concentration of the compound is from 4 mg/mL to 10 mg/mL. In additional aspects, the concentration of the compound is from 5 mg/mL to 8 mg/mL. In yet further aspects, concentration of the compound is from 5 mg/mL to 6 mg/mL.

In some aspects, pharmaceutically acceptable carrier comprises tris, D-mannitol, and a pH of essentially 6.8. In other aspects, the compositions consist essentially of tris, D-mannitol, and a pH of 6.8.

In some aspects, pharmaceutically acceptable carrier comprises histidine and mannitol. In some aspects, pharmaceutically acceptable carrier comprises histidine and mannitol with polysorbate 20. In some aspects, pharmaceutically acceptable carrier comprises L-histidine, D-mannitol, L-methionine, and a pH of essentially 6.8. In additional aspects, the pharmaceutically acceptable carrier consists essentially of L-histidine, D-mannitol, L-methionine, and a pH of 6.8.

In some aspects, the pharmaceutically acceptable carrier comprises L-histidine, D-mannitol, polysorbate 20, and a pH of essentially 6.8. In some aspects, the pharmaceutically acceptable carrier comprises L-histidine, D-mannitol, and a pH of essentially 6.8. In some aspects, the pharmaceutically acceptable carrier comprises L-histidine, D-mannitol, polysorbate 20, trehalose, and a pH of essentially 6.8.

A pharmaceutical composition comprising a chlorotoxin conjugate is formulated according to known methods to prepare pharmaceutically useful compositions, for example, as found in "Excipient Selection in Parenteral Formulation Development" Pramanick et.al., Pharma Times, Vol. 45., No. 3, March 2013.

In some aspects, the chlorotoxin conjugate is combined with a pharmaceutically acceptable carrier. A composition is said to be a pharmaceutically acceptable carrier if its administration is tolerated by a recipient patient. Sterile phosphate-buffered saline is one example of a pharmaceutically acceptable carrier. Other suitable carriers are well-known to those in the art. See, for example, Gennaro (ed.), Remington's Pharmaceutical Sciences, 19th Edition (Mack Publishing Company 1995).

Formulations for administration of chlorotoxin conjugates are typically provided but are not limited to as liquid, solid or semi-solid products or dosage forms, exemplified by tablets, capsules, pellets, a powder or a lyophilized product. In some aspects, the chlorotoxin conjugate is formulated to comprise no additional materials except for a pharmaceutical carrier. In some other aspects, the chlorotoxin conjugate is formulated such that it comprises a core "matrix material" which encapsulates, binds to, coats or is adjacent to the chlorotoxin conjugate. In some other aspects, the chlorotoxin conjugate and matrix material further comprises a protective coatings. Various formulations are well-known to those in the art. See, for example, Gennaro (ed.), Remington's Pharmaceutical Sciences, 19th Edition (Mack Publishing Company 1995).

Suitable excipients for use with chlorotoxin conjugates are often included in formulations for intravenous use, for example, an injection. Injections are sterile, pyrogenfree solutions or dispersions (emulsions or suspensions) of one or more active ingredients in a suitable vehicle or carrier. Injections that are dispersions should remain sufficiently stable so that, after shaking, a homogeneous dose is withdrawn. More specifically, formulations which include chlorotoxin conjugates and one or more but not limited to suitable excipients, exemplified by matrix materials, binders, lubricants, glidants or disintegrants which aid in modulating the PK profile of administered chlorotoxin conjugates are preferred. In some aspects, compositions comprising chlorotoxin conjugates in combination with one or more suitable excipients and one or more specific product characteristics (such as dissolution or water content) which result in improved pharmacokinetic profiles of chlorotoxin conjugates *in vivo.* Thus, the *in vivo* performance of chlorotoxin conjugates dosage forms/products included herein is based upon the composition of the excipients added during manufacturing and/or the final product characteristics generated through specific processing parameters and methods. Other excipients are well-known to those in the art. See, for example, Gennaro (ed.), Remington's Pharmaceutical Sciences, 19th Edition (Mack Publishing Company 1995).

Suitable carriers for intravenous administration include for example but are not limited to physiological saline or phosphate buffered saline (PBS), Tris, and solutions containing solubilizing agents, such as glucose, polyethylene glycol, polypropylene glycol, additional agents such as histidine, dextrose, mannitol and mixtures thereof. In some aspects, carriers for intravenous administration include a mixture of histidine and dextrose, Tris and dextrose or Tris and mannitol. Other carriers are well-known to those in the art. See, for example, Gennaro (ed.), Remington's Pharmaceutical Sciences, 19th Edition (Mack Publishing Company 1995).

The formulation often includes an aqueous vehicle. Aqueous vehicles include, by way of example and without limitation, sodium chloride solution, Ringers solution, isotonic dextrose solution, sterile water solution, dextrose and lactated Ringers solution. Nonaqueous vehicles include, by way of example and without limitation, fixed oils of vegetable origin, cottonseed oil, corn oil, sesame oil and peanut oil, benzyl benzoate, castor oil, N,N-dimethylacetamide, ethanol, dehydrated ethanol, glycerin, glycerol, N-methyl-2-pyrrolidone, polyethylene glycol and any derivative thereof, propylene glycol, safflower oil and soybean oil. Other vehicles are well-known to those in the art. See, for example, Gennaro (ed.), Remington's Pharmaceutical Sciences, 19th Edition (Mack Publishing Company 1995).

In some aspects, the composition the pharmaceutically acceptable carrier comprises an osmolyte. In some aspects, the osmolyte comprises a sugar, a sugar alcohol, or a combination thereof.

In certain aspects, the composition comprises a sugar alcohol selected from sorbitol, inositol, mannitol, xylitol and glycerol, or a combination thereof. In further aspects, the sugar alcohol comprises mannitol. In certain aspects, the composition comprises from 2% to 20% (wt/vol %) mannitol. In some aspects, the composition comprises from 2% to 10% (wt/vol %) mannitol. In further aspects, the composition comprises essentially 5% (wt/vol %) mannitol.

In other aspects, the composition comprises a sugar. In certain aspects, the sugar is selected from trehalose, lactose, sucrose, glucose, galactose, maltose, mannose, fructose, dextrose, or a combination thereof. In additional aspects, the sugar is selected from trehalose, sucrose, or a combination thereof. In some aspects, the composition comprises from 1% to 40% (wt/vol %) of trehalose, sucrose, or a combination of trehalose and sucrose. In other aspects, the composition comprises from 1% to 20% (wt/vol %) of trehalose, sucrose, or a combination of trehalose and sucrose. In additional aspects, the composition comprises 2% (wt/vol %) of trehalose, sucrose, or a combination of trehalose and sucrose.

In certain aspects, the composition further comprises an osmolyte selected from glycine, carnitine, ethanolamine, their phosphates, mono sugars, or a combination thereof.

In some aspects, the present compositions are isotonic. In other aspects, the compositions are essentially isotonic.

In certain aspects, the ionic strength of the composition is less than 50 mM. In other aspects, the ionic strength of the composition is less than 10 mM.

Antimicrobial agents in bacteriostatic or fungistatic concentrations are typically added to preparations packaged in multiple dose containers which include by way of example and without limitation, phenols or cresols, mercurials, benzyl alcohol, chlorobutanol, methyl and propyl p-hydroxybenzoic acid esters, thimerosal, benzalkonium chloride and benzethonium chloride. Other antimicrobial agents are well-known to those in the art. See, for example, Gennaro (ed.), Remington's Pharmaceutical Sciences, 19th Edition (Mack Publishing Company 1995).

Buffers include by way of example and without limitation, acetate, ammonium sulfate, ammonium hydroxide, arginine, aspartic acid, benzene sulfonic acid, benzoate sodium, benzoate acid, carbonate, sodium carbonate, carbon dioxide, citrate, diethanolamine, glucono delta lactone, glycine, glycine HCl, histidine, histidine HCl, hydrochloric acid, hydrobromic acid, lysine maleic acid, meglumine, methanesulfonic acid, monoethanolamine, phosphate, sodium phosphate, citrate, succinate sodium, sulfuric acid, tartarate sodium, trmethamine, sodium citrate, hydroxide, sodium hydroxide, Tris base, Tris base -65, Tris acetate, Tris HCl, and Tris HCl-65.

In various aspects, the pharmaceutically acceptable carrier comprises a buffer. In some aspects, the buffer is selected from tris, HEPES, histidine, ethylene diamine, or a combination thereof. In other aspects, the buffer is selected from tris, histidine, or a combination thereof. In further aspects, the buffer comprises histidine, which is optionally L-histidine. In additional aspects, the composition comprises at least 100 mM histidine. In further aspects, the composition comprises at least 50 mM histidine. In some aspects, the composition comprises at least 20 mM histidine. In additional aspects, the composition comprises 10 to 100 mM histidine. In other aspects, the composition comprises 10 to 20 mM histidine.

Antioxidants include by way of example and without limitation, sodium bisulfate, acetone sodium bisulfate, argon, ascorbyl palmitate, ascorbate sodium, ascorbate acid, butylated hydroxy anisole, butylated hydroxy toluene, cysteine, cystenate HCl, dithionite sodium, gentistic acid, gentistic acid ethanoloamine, glutamate monosodium, glutathione, formaldehyde solfoxylate sodium, metabisulfite potassium, metabisulfite sodium, methionine, monothioglycerol, nitrogen, propyl gallate, sulfite sodium, tocopherol alpha, alpha tocopherol hydrogen succinate and thioglycolyate sodium.

In some aspects, the compositions comprise an antioxidant, a free radical scavenger, a quencher, an antioxidant synergist or a combination thereof.

In some aspects, the antioxidant is selected from methionine, butylated hydroxytoluene, butylated hydroxyanisole, propyl gallate, or a combination thereof. In other aspects, the antioxidant comprises methionine. In further aspects, the antioxidant is L-methionine. In certain aspects, the compositions comprise at least 20 mM methionine. In other aspects, aspects, the compositions comprise at least 10 mM methionine.

Suspending, emulsifying and/or dispersing agents include by way of example and without limitation, sodium carboxymethylcelluose, hydroxypropyl methylcellulose, Polysorbate 80 (TWEEN^{®} 80) and polyvinylpyrrolidone.

In various aspects, the compositions comprise a surfactant. In certain aspects, the surfactant is selected from polysorbate 20, polysorbate 80, a pluronic, polyoxyethylene sorbitan mono-oleate, polyethylene mono-laureate, N-actylglucoside, or a combination thereof. In certain aspects, the surfactant is polysorbate 20. In further aspects, the compositions comprise from 0.0001% to 0.1% (wt/vol %) polysorbate 20. In additional aspects, the compositions comprise cyclodextrin. In further aspects, the cyclodextrin comprises (2-hydroxypropyl)-β-cyclodextrin.

A sequestering or chelating agent of metal ions include by way of example and without limitation, calcium disodium EDTA, disodium EDTA, sodium EDTA, calcium versetaminde sodium, calteridol and DPTA. In some aspects, the present compositions comprise a metal chelator. In certain aspects, the metal chelator is selected from EDTA, deferoxamine mesylate, EGTA, fumaric acid, and malic acid, salts thereof, or combinations thereof. In further aspects, the metal chelator comprises EDTA or salts thereof. In certain aspects, the compositions have an EDTA concentration of about 0.1 mg/ml to about 1.0 mg/ml.

Other isotonic agents, buffers, antioxidants, anesthetics, suspending and dispersing agents, emulsifying agents and chelating agents are well-known to those in the art. See, for example, Gennaro (ed.), Remington's Pharmaceutical Sciences, 19th Edition (Mack Publishing Company 1995).

Pharmaceutical carriers also include, by way of example and without limitation, ethyl alcohol, polyethylene glycol and propylene glycol for water miscible vehicles and sodium hydroxide, hydrochloric acid, citric acid or lactic acid. Other pharmaceutical carriers are well-known to those in the art. See, for example, Gennaro (ed.), Remington's Pharmaceutical Sciences, 19th Edition (Mack Publishing Company 1995).

The chlorotoxin conjugates described herein are often formulated using a variety of parameters including by way of example and without limitation, pH, molarity, % weight/volume, % volume/volume and the like. Other factors considered in the formulation of, stability of, storage of, shipping of chlorotoxin conjugates include by way of example and without limitation, the gas environment, container material, container color, cap material, cap color, presence of additional aspects, such as antioxidants, stabilizers, photoprotective compounds, protectants, sugars, ion chelators, ion donors or the like. Any factor which serves as any one of the above factors known to one of ordinary skill in the art is often used with the chlorotoxin conjugates described herein but not limited as such.

The preparation of pharmaceutical or pharmacological compositions are known to those of skill in the art in light of the present disclosure. General techniques for formulation and administration are found in "Remington: The Science and Practice of Pharmacy, Twentieth Edition," Lippincott Williams & Wilkins, Philadelphia, Pa. Tablets, capsules, pills, powders, granules, dragees, gels, slurries, ointments, solutions suppositories, injections, inhalants and aerosols are examples of such formulations.

The chlorotoxin conjugates are often stored at various temperatures, including by way of example and without limitation, freezing, for example at about -20°C, about -70°C, about -100 °C, about -120 °C, about -150 °C, about -200 °C or more than about -200 °C, cold storage, for example at about 10 °C, about 5 °C, about 4 °C, about 2 °C, about 0°C, about -2°C or more than about -5°C, or any other suitable temperature such that the composition remains stable.

In some aspects, compositions comprising the compounds described herein are stored as lyophilized solids. In some aspects, the present disclosure provides methods for producing the lyophilized composition, the method comprising providing the composition; and lyophilizing the composition, thereby producing the lyophilized composition.

Using lyophilization, it is possible to store the compounds in a manner that maintains physiological or otherwise optimal pH, isotonicity and stability. Such materials include pH buffers, preservatives, tonicity adjusting agents, anti-oxidants, other polymers (e.g., viscosity adjusting agents or extenders) and excipients to stabilize the labile protein against the stresses of drying and storage of the dried product. Specific illustrative examples of such additives include phosphate, citrate, or borate buffers; thimerosal; sorbic acid; methyl or propyl paraben, and chlorobutanol preservatives; sodium chloride: polyvinyl alcohol, polyvinyl pyrrolidone; mannitol, dextrose, dextran, lactose, sucrose, ethylene diamine tetraacetic acid, and the like. Suitable formulations, known in the art, (Remington's Pharmaceutical Sciences (latest edition), Mack Publishing Company, Easton, Pa.; Arakawa et al. (1990), supra; Carpenter et al. (1991), supra; and Pikal (1990), supra).

In certain aspects, the pharmaceutically acceptable carrier comprises a reconstitution stabilizer. In other aspects, the reconstitution stabilizer comprises a water-soluble polymer. In additional aspets, the water-soluble polymer is selected from a polaxamer, a polyol, a polyethylene glycol, a polyvinylalcohol, a hydroxyethyl starch, dextran, polyvinylpyrrolidene poly(acrylic acid), or a combination thereof.

The term "reconstitution stabilizer" means any excipient which is capable of preventing aggregation of a reconstituted protein in an aqueous medium. Excipients possessing the necessary characteristics for the present invention are well-known in the art and generally function by the mechanisms of charge replusion, steric hindrance, hydrophobic binding or specific high-affinity binding to the dried protein. Exemplary excipients include various osmolytes, various salts, water soluble synthetic and natural polymers, surfactants, sulfated polysaccharides, carrier proteins, buffers and the like (Manning et al. (1989), Pharmaceutical Research, 6:903-918; and Paborji, et al. (1994), Pharmaceutical Research, 11:764-771).

The present compounds and an effective amount of the reconstitution stabilizer are admixed under conditions effective to reduce aggregation of present compounds upon reconstitution with the reconstitution medium (e.g., a solvent and optionally other components such as antibacterials). The reconstitution stabilizer may be admixed with the compouns at a suitable time before, during or after reconstitution; preferably the reconstitution stabilizer will be pre-dissolved in the reconstitution medium. The compound is reconstituted at a temperature which is above the freezing point of the reconstitution medium, but which will not degrade the compound and which will not be deleterious to the reconstitution stabilizer; preferably the temperature will be between about 2 °C to 50 °C. The time taken to mix the reconstitution stabilizer and the dried compound should be for a sufficient period to prepare a suitable admixture; preferably mixing will be for between about 1 to 30 minutes. Generally, the reconstituted formulation is used soon after reconstitution.

In certain aspects, the present compositions are reconstituted from a lyophilized form. In other aspects, the present disclosure provides methods for producing the reconstituted composition, the method comprising providing a lyophilized composition; and reconstituting the composition with a solution to produce a reconstituted composition. In various aspects, the reconstituting solution comprises water. In some aspects, the reconstituting solution is selected from sterile water, physiological saline solution, glucose solution or other aqueous solvents (e.g., alcohols such as ethyl, n-propyl or isopropyl, butyl alcohol), or a combination thereof, which are capable of dissolving the dried composition and compatible with the selected administration route and which does not negatively interfere with the compound and the reconstitution stabilizers employed.

### Storage Vessels

In some aspects, the chlorotoxin conjugates are placed into containers following formulation. Often the containers include by way of example and without limitation, glass, for example amber glass or colorless glass. The containers often include by way of example and without limitation, a plastic, a rubber, a metal, a biodegradable material or the like known to one of skill in the art and are any color, colorless, opaque or clear. In some aspects, the chlorotoxin conjugates are placed into containers which are sealed following formulation. Often the seal is a cap, for example, the cap is a plastic, a rubber, a metal, a biodegradable material, a combination of or the like known to one of skill in the art and are any color, colorless, opaque or clear, sometimes a film. In some aspects, a gas is included in the container, often to enhance stability of the chlorotoxin conjugate or prevent oxygen from contacting the chlorotoxin conjugate. For example, gas includes by way of example and without limitation, an inert gas, such as nitrogen or argon, occasionally a noble gas and is used at any suitable concentration.

In some aspects the compounds of the present disclosure are stored in a vessel comprising glass, particularly a Type I glass, which has been subjected to a washing or extraction treatment which reduces the level of extractable trivalent and divalent metal ions present in/on the surface of the glass. Such treatments include steeping in (extraction with) hot (preferably at least 90 °C) water or another aqueous medium, e.g. ammonium sulfate solution, or treatment with sulfur dioxide.

In some aspects, the compounds of the present disclosure are stored in a vessel comprising USP Type 1 borosilicate glass vial with a 13 mm chlorobutyl based stopper with flourotech coating on plug and B2 coating on the top and an aluminum over seal with flip top cap.

In certain aspects, the compounds of the present disclosure are stored in a foil-lined chamber. In some aspects, the chamber comprises a dry inert atmosphere, preferably nitrogen, and a desiccant or oxygen absorber.

In various aspects, the present disclosure provides a kit comprising vessel configured to contain a fluid; any of the compounds and compositions described herein; and an elastomeric closure affixed to the vessel.

In some aspects, the kit further comprises a light shield. In further aspects, the light shield is a physical barrier configured to block at least a portion of the light incident on the vessel from the composition. In still further aspects, the physical barrier comprises an opaque or semi-opaque material.

In some aspects, the vessel is a glass vial. In further aspects, the glass vial comprises clear or amber glass. In some aspects, the glass vial is an untreated glass container. In certain aspects, the glass vial comprises USP Type I, Type II, Type III, or Type IV glass. In some aspects, the inner portion of the vessel further comprises a silica (SiO₂) coating or silicone coating. In some aspects, the untreated glass container is selected from an ampoule, vial, ready-to-use syringe, or carpoule.

In some aspects, the elastomeric closure is a halobutyl rubber closure. In further aspects, the halobutyl rubber closure is selected from a chlorobutyl rubber closure or a bromobutyl rubber closure. In some aspects, elastomeric closure is coated with Fluorotec, B2, or a combination thereof.

In some aspects, the kit further comprises an opaque secondary package surrounding the vessel. In certain aspects, the opaque secondary package comprises an opaque box, an opaque aluminum foil pouch, or a combination thereof. In further aspects, the opaque secondary package is configured to block at least 90% of the light incident on the package exterior from the composition. In still further aspects, the opaque secondary package is configured to block at least 95% of the light incident on the package exterior from the composition. In still further aspects, the opaque secondary package is configured to block at least 99% of the light incident on the package exterior from the composition. In still further aspects, the opaque secondary package is configured to block at least 99.9% of the light incident on the package exterior from the composition.

In some aspects, the vessel comprises a reduced-oxygen environment in contact with the composition. In certain aspects, the vessel comprises an inert gas in contact with the composition.In some aspects, the composition is sparged with an inert gas, thereby producing the reduced-oxygen environment in the vessel. In certain aspects, the inert gas comprises nitrogen or argon.

### Dosages and Toxicity of Compounds

The product or dosage form characteristics which result from the processing methods and/or parameters for generating formulations such as powders, lyophilized compositions, and the like include, but are not limited to, density, water content, friability, disintegration, dissolution profile(s), shape, size, weight, uniformity and composition of the particles. These product characteristics are often modulated in a number of ways and affect the final *in vitro* and/or *in vivo* performance of the formulations. Product or dosage form characteristics are often a consequence of excipient selection, excipient composition, manufacturing methods applied or a combination of any of these. The combination of excipients as well as product characteristics (including processing methods or processing parameters) of the final dosage form will ultimately determine the pharmacokinetic profile of the active ingredient *in vivo.* The administered chlorotoxin conjugate formulations described herein are often processed or manufactured under specific conditions such as, for example, mixing methods (including sieve size, rpm, and milling), drying time, conditions, environmental parameters (e.g., temperature and humidity) and combinations thereof) which themselves modulate the pharmacokinetic profile of chlorotoxin compositions *in vivo* (i.e., increase the average Cₘₐₓ or AUC). In order to quantitatively compare one formulation to another, it is customary to measure several of these product or dosage form characteristics. This is also necessary when attempting to duplicate multiple batches.

Dissolution and drug release from formulations depends on many factors including the solubility and concentration of the active ingredient, the nature and composition of the excipients, content uniformity, water content, product shape and size, porosity, disintegration time and other factors. The release of a drug or active ingredient from a final dosage form *in vitro* is typically characterized by its dissolution profile under standardized conditions (using United States Pharmacopeia (USP) or similar accepted methods for reference) and at the appropriate pH, often a neutral pH. The dissolution profile shows the amount of drug released over time into the test media under specified conditions. Standard conditions make use of buffers at an appropriate pH in order to best mimic the pH of a subject's blood.

Typically a therapeutically effective dosage is formulated to contain a dose of at least about 0.1 mg up to about 1.5 mg or more, such as more than 1.5 mg of chlorotoxin conjugate. In some aspects, the effective dosage is formulated to contain a dose of at least about 0.01 mg, about 0.02 mg, about 0.03 mg, about 0.5 mg, about 0.07 mg, about 0.1 mg, about 0.2 mg, about 0.3 mg, about 0.35 mg, about 0.375 mg, about 0.4 mg, about 0.5 mg, about 0.6 mg, about 0.7 mg, about 0.75 mg, about 0.8 mg, about 0.9 mg, about 1 mg, about 1.3 mg, about 1.4 mg, about 1.5 mg, about 1.8 mg, about 1.9 mg, about 2 mg, about 2.4 mg, about 3 mg, about 5 mg, about 6 mg, about 7 mg, about 12 mg, about 18 mg or about 60 mg more of chlorotoxin conjugate. In an exemplary aspect, the dose is 0.1 mg for a mouse, 1 mg for a dog, 0.3 mg for a rat, 0.6 mg for a monkey and 3 mg for a human. The amount of chlorotoxin conjugate administered to a subject is often the total about amount listed herein. In some aspects, the amount of chlorotoxin conjugate administered to a subject is often the about per milligram, gram or kilogram of subject weight for each amount listed herein. In other aspects, the amount of chlorotoxin conjugate administered to a subject is often the about per milliliter or liter of fluid volume for each amount listed herein. In yet other aspects, the amount of chlorotoxin conjugate administered to a subject is often the about per square millimeter, square centimeter or square meter of subject surface body area or subject body area for each amount listed herein.

As used herein a "dosage regimen" refers to the protocol used to administer an intravenous pharmaceutical formulation comprising chlorotoxin conjugate to a subject. In some aspects, the dosage regimen comprises a dose amount and dosing interval. In some aspects, the dosage regimen further comprises a dosing duration. As used herein "dosing duration" refers to the period of time over which a dose is administered.

In some aspects, the dose of chlorotoxin conjugate is administered to a subject using either a fixed or a scaling dosing scheme. For example, a fixed dosing scheme includes administration of a bolus or continuous dose of chlorotoxin conjugate to a subject via an intravenous administration route wherein the fixed dose is, for example and without limitation, about 3 mg to about 6 mg and does not account or adjust for a subject's age, weight, height, body mass index, metabolism, or the like. For example, a scaling dosing scheme includes administration of a bolus or continuous dose of chlorotoxin conjugate to a subject via an intravenous administration route wherein the scaled dose is, for example and without limitation, about 3 mg to about 6 mg and accounts or adjusts for a subject's age, weight, height, body mass index, metabolism, or the like. In some aspects, the fixed dose and/or the scaled dose are determined for one subject based upon the dose administered to a different subject wherein the subjects are or are not the same species, for example a mouse and a human or a rat and a human, or a dog and a human or a monkey and a human or a non-human primate and a human. Often in a fixed dose, the same dose or about the same dose is administered to all subjects, for example a mouse and a human or a rat and a human, or a dog and a human or a monkey and a human or a non-human primate and a human. In some aspects, the scaled dose to be administered to a subject is determined from the dose administered to a different subject wherein the subjects are or are not the same species, for example a mouse and a human or a rat and a human, or a dog and a human or a monkey and a human or a non-human primate and a human. The scaled dose is therefore increased from the dose administered to the mouse, rat, dog, monkey or non-human primate to the dose administered to the human based upon the difference between the mouse, rat, dog, monkey or non-human primate and the human on factors such as subject age, weight, height, metabolism, size or the like. In a preferred aspect, the dose is scaled from a rat to a human.

The compounds and compositions described herein are administered to a subject before surgery, during surgery and/or the excised tissue from the subject is contacted with compositions of the chlorotoxin conjugates. In some aspects, compositions of chlorotoxin conjugates are intravenously administered to a subject about 1 hour, about 2 hours, about 3 hours, about 4 hours, about 5 hours, about 6 hours, about 9 hours, about 12 hours, about 24 hours, about 36 hours, about 48 hours or about 72 hours before surgery. In some aspects, compositions of chlorotoxin conjugates are intravenously administered to a subject between 0 and 1 hours, between 1 and 2 hours, between 2 and 3 hours, between 3 and 4 hours, between 4 and 5 hours, between 5 and 6 hours, between 6 and 9 hours, between 9 and 12 hours, between 12 and 24 hours, between 24 and 36 hours, between 36 and 48 hours or between 48 and 72 hours (inclusive) before surgery. Tissue or fluid samples are often isolated from a subject prior to administration of a chlorotoxin conjugate, sometimes as a baseline reference. Samples are also isolated from a subject after administration of the compounds of the present disclosure, often less than about 1 minute after, about 2 minutes after, about 3 minutes after, about 4 minutes after, about 5 minutes after, about 6 minutes after, about 7 minutes after, about 8 minutes after, about 9 minutes after, about 10 minutes after, about 11 minutes after, about 12 minutes after, about 13 minutes after, about 14 minutes after, about 15 minutes after, about 20 minutes after, about 30 minutes after, about 40 minutes after, about 50 minutes after, about 60 minutes after, about 1 hour after, about 2 hours after, about 3 hours after, about 4 hours after, about 5 hours after, about 6 hours after, about 12 hours after, about 18 hours after, about 24 hours after, about 36 hours after, about 48 hours after, about 72 hours after, about 96 hours after, about 5 days after, about 7 days after, about 10 days after, about 14 days after, about 21 days after, about 4 weeks after, about 6 weeks after, about 8 weeks after, about 12 weeks after, about 16 weeks after, about 20 weeks after or more than 20 weeks after.

### Pharmacokinetics

The methods and compositions described herein relate to pharmacokinetics of intravenous administration of chlorotoxin conjugates to a subject. Pharmacokinetics are often described using models, for example, compartmental or noncompartmental models. Compartmental models include but are not limited to monocompartmental model, the two compartmental model, the multicompartmental model or the like. Models are often divided into different compartments and described by the corresponding scheme. For example, one scheme is the absorption, distribution, metabolism and excretion (ADME) scheme. For another example, another scheme is the liberation, absorption, distribution, metabolism and excretion (LADME) scheme. In some aspects, metabolism and excretion are grouped into one compartment referred to as the elimination compartment. For example, liberation includes liberation of the active portion of the composition from the delivery system, absorption includes absorption of the active portion of the composition by the subject, distribution includes distribution of the composition through the blood plasma and to different tissues, metabolism, which includes metabolism or inactivation of the composition and finally excretion, which includes excretion or elimination of the composition or the products of metabolism of the composition. Often, compositions administered intravenously to a subject are subject to multiphasic absorption including but not limited to aspects of tissue distribution and metabolism/excretion. As such, the decrease in plasma concentration of the composition is often biphasic, including, for example an alpha phase and a beta phase, occasionally a gamma, delta or other phase is observed. In some aspects, the bioavailability of the compositions described herein is absolute bioavailability, often 1 or 100% given intravenous administration.

Pharmacokinetics includes determining at least one parameter associated with intravenous administration of chlorotoxin conjugates to a subject. In some aspects, parameters include at least the dose (D), dosing interval (τ), area under curve (AUC), maximum concentration (Cₘₐₓ), minimum concentration reached before a subsequent dose is administered (Cₘᵢₙ), minimum time (Tₘᵢₙ), maximum time to reach Cₘₐₓ (Tₘₐₓ), volume of distribution (V_{d}), back-extrapolated concentration at time 0 (C₀), steady state concentration (Cₛₛ), elimination rate constant (kₑ), infusion rate (kᵢₙ), clearance (CL), bioavailability (f), fluctuation (%PTF) and elimination half-life (T_{1/2}). In another aspect, the Cₘₐₓ, C₀. and AUC increase in a dose-dependent manner.

The compounds described herein have values for at least one of the pharmacokinetic parameters listed herein and known to those of ordinary skill in the art. Often, the values for the pharmacokinetic parameters are recorded, observed, measured, processed, analyzed or the like as data. The pharmacokinetics parameters are any parameters suitable for describing the plasma or serum profiles of chlorotoxin conjugates described herein. For example, the pharmacokinetics profile are often obtained at a time after dosing of, for example, about zero minutes, about 1 minute, about 2 minutes, about 3 minutes, about 4 minutes, about 5 minutes, about 6 minutes, about 7 minutes, about 8 minutes, about 9 minutes, about 10 minutes, about 11 minutes, about 12 minutes, about 13 minutes, about 14 minutes, about 15 minutes, about 16 minutes, about 17 minutes, about 18 minutes, about 19 minutes, about 20 minutes, about 21 minutes, about 22 minutes, about 23 minutes, about 24 minutes, about 25 minutes, about 26 minutes, about 27 minutes, about 28 minutes, about 29 minutes, about 30 minutes, about 31 minutes, about 32 minutes, about 33 minutes, about 34 minutes, about 35 minutes, about 36 minutes, about 37 minutes, about 38 minutes, about 39 minutes, about 40 minutes, about 41 minutes, about 42 minutes, about 43 minutes, about 44 minutes, about 45 minutes, about 46 minutes, about 47 minutes, about 48 minutes, about 49 minutes, about 50 minutes, about 51 minutes, about 52 minutes, about 53 minutes, about 54 minutes, about 55 minutes, about 56 minutes, about 57 minutes, about 58 minutes, about 59 minutes, about 60 minutes, about zero hours, about 0.5 hours, about 1 hour, about 1.5 hours, about 2 hours, about 2.5 hours, about 3 hours, about 3.5 hours, about 4 hours, about 4.5 hours, about 5 hours, about 5.5 hours, about 6 hours, about 6.5 hours, about 7 hours, about 7.5 hours, about 8 hours, about 8.5 hours, about 9 hours, about 9.5 hours, about 10 hours, about 10.5 hours, about 11 hours, about 11.5 hours, about 12 hours, about 12.5 hours, about 13 hours, about 13.5 hours, about 14 hours, about 14.5 hours, about 15 hours, about 15.5 hours, about 16 hours, about 16.5 hours, about 17 hours, about 17.5 hours, about 18 hours, about 18.5 hours, about 19 hours, about 19.5 hours, about 20 hours, about 20.5 hours, about 21 hours, about 21.5 hours, about 22 hours, about 22.5 hours, about 23 hours, about 23.5 hours, or about 24 hours.

The pharmacokinetics parameters are any parameters suitable for describing the plasma or serum profiles of chlorotoxin conjugates described herein. In some aspects, the dose (D) includes by way of example but is not limited to, about 0.01 mg, about 0.02 mg, about 0.03 mg, about 0.5 mg, about 0.07 mg, about 0.1 mg, about 0.2 mg, about 0.3 mg, about 0.35 mg, about 0.375 mg, about 0.4 mg, about 0.5 mg, about 0.6 mg, about 0.7 mg, about 0.75 mg, about 0.8 mg, about 0.9 mg, about 1 mg, about 1.3 mg, about 1.4 mg, about 1.5 mg, about 1.8 mg, about 1.9 mg, about 2 mg, about 2.4 mg, about 3 mg, about 5 mg, about 6 mg, about 7 mg, about 12 mg, about 18 mg or about 60 mg more of chlorotoxin conjugate. In some aspects, the dosing interval (τ) includes by way of example but is not limited to, about 12 hours, about 24 hours, about 36 hours, about 48 hours or about 72 hours before surgery.

The pharmacokinetics parameters are any parameters suitable for describing the plasma or serum profiles of chlorotoxin conjugates described herein. In some aspects, the area under curve (AUC) includes by way of example but is not limited to, not less than about 50 hr*ng/mL, not less than about 75 hr*ng/mL, not less than about 100 hr*ng/mL, not less than about 125 hr*ng/mL, not less than about 150 hr*ng/mL, not less than about 175 hr*ng/mL, not less than about 200 hr*ng/mL, not less than about 250 hr*ng/mL, not less than about 300 hr*ng/mL, not less than about 350 hr*ng/mL, not less than about 400 hr*ng/mL, not less than about 500 hr*ng/mL, not less than about 600 hr*ng/mL, not less than about 700 hr*ng/mL, not less than about 800 hr*ng/mL, not less than about 900 hr*ng/mL, not less than about 1000 hr*ng/mL, not less than about 2000 hr*ng/mL, not less than about 3000 hr*ng/mL, not less than about 4000 hr*ng/mL, not less than about 5000 hr*ng/mL, not less than about 6000 hr*ng/mL, not less than about 7000 hr*ng/mL, not less than about 8000 hr*ng/mL, not less than about 9000 hr*ng/mL, not less than about 10000 hr*ng/mL, not less than about 11000 hr*ng/mL, not less than about 12000 hr*ng/mL, not less than about 13000 hr*ng/mL, not less than about 14000 hr*ng/mL, not less than about 15000 hr*ng/mL, not less than about 16000 hr*ng/mL, not less than about 17000 hr*ng/mL, not less than about 18000 hr*ng/mL, not less than about 19000 hr*ng/mL, not less than about 20000 hr*ng/mL, not less than about 21000 hr*ng/mL, not less than about 22000 hr*ng/mL, not less than about 23000 hr*ng/mL, not less than about 24000 hr*ng/mL, not less than about 25000 hr*ng/mL, not less than about 26000 hr*ng/mL, not less than about 27000 hr*ng/mL, not less than about 28000 hr*ng/mL, not less than about 29000 hr*ng/mL, not less than about 30000 hr*ng/mL, not less than about 31000 hr*ng/mL, not less than about 32000 hr*ng/mL, not less than about 33000 hr*ng/mL, not less than about 34000 hr*ng/mL, not less than about 35000 hr*ng/mL, not less than about 40000 hr*ng/mL not less than about 45000 hr*ng/mL not less than about 50000 hr*ng/mL not less than about 55000 hr*ng/mL not less than about 60000 hr*ng/mL not less than about 65000 hr*ng/mL not less than about 70000 hr*ng/mL not less than about 75000 hr*ng/mL not less than about 80000 hr*ng/mL not less than about 85000 hr*ng/mL not less than about 90000 hr*ng/mL not less than about 95000 hr*ng/mL not less than about 100000 hr*ng/mL not less than about 125000 hr*ng/mL not less than about 150000 hr*ng/mL not less than about 175000 hr*ng/mL not less than about 200000 hr*ng/mL not less than about 250000 hr*ng/mL not less than about 300000 hr*ng/mL not less than about 350000 hr*ng/mL not less than about 400000 hr*ng/mL not less than about 450000 hr*ng/mL not less than about 500000 hr*ng/mL not less than about 550000 hr*ng/mL not less than about 600000 hr*ng/mL not less than about 650000 hr*ng/mL not less than about 700000 hr*ng/mL not less than about 750000 hr*ng/mL not less than about 800000 hr*ng/mL not less than about 850000 hr*ng/mL not less than about 900000 hr*ng/mL not less than about 950000 hr*ng/mL not less than about 1000000 hr*ng/mL not less than about 1100000 hr*ng/mL not less than about 1200000 hr*ng/mL not less than about 1300000 hr*ng/mL not less than about 1400000 hr*ng/mL not less than about 1500000 hr*ng/mL not less than about 1600000 hr*ng/mL not less than about 1700000 hr*ng/mL not less than about 1800000 hr*ng/mL not less than about 1900000 hr*ng/mL not less than about 2000000 hr*ng/mL or any other AUC appropriate for describing a pharmacokinetic profile of a chlorotoxin conjugate described herein.

The AUC of a chlorotoxin described herein by way of example can be, but is not limited to, about 1,000 hr*ng/mL to about 1,250 hr*ng/mL; about 1,250 hr*ng/mL to about 1,500 hr*ng/mL; about 1,500 hr*ng/mL to about 1,750 hr*ng/mL; about 1,750 hr*ng/mL to about 2,000 hr*ng/mL; about 2,000 hr*ng/mL to about 2,500 hr*ng/mL; about 2,500 hr*ng/mL to about 3,000 hr*ng/mL; about 3,000 hr*ng/mL to about 3,500 hr*ng/mL; about 3,500 hr*ng/mL to about 4,000 hr*ng/mL; about 4,000 hr*ng/mL to about 4,500 hr*ng/mL; about 4,500 hr*ng/mL to about 5,000 hr*ng/mL; about 5,000 hr*ng/mL to about 5,500 hr*ng/mL; about 5,500 hr*ng/mL to about 6,000 hr*ng/mL; about 6,000 hr*ng/mL to about 6,500 hr*ng/mL; about 6,500 hr*ng/mL to about 7,000 hr*ng/mL; about 7,000 hr*ng/mL to about 7,500 hr*ng/mL; about 7,500 hr*ng/mL to about 8,000 hr*ng/mL; about 8,000 hr*ng/mL to about 8,500 hr*ng/mL; about 8,500 hr*ng/mL to about 9,000 hr*ng/mL; about 9,000 hr*ng/mL to about 9,500 hr*ng/mL; about 9,500 hr*ng/mL to about 10,000 hr*ng/mL; about 10,000 hr*ng/mL to about 20,000 hr*ng/mL; about 20,000 hr*ng/mL to about 30,000 hr*ng/mL; about 30,000 hr*ng/mL to about 40,000 hr*ng/mL; about 40,000 hr*ng/mL to about 50,000 hr*ng/mL; about 50,000 hr*ng/mL to about 60,000 hr*ng/mL; about 60,000 hr*ng/mL to about 70,000 hr*ng/mL; about 70,000 hr*ng/mL to about 80,000 hr*ng/mL; about 80,000 hr*ng/mL to about 90,000 hr*ng/mL; about 90,000 hr*ng/mL to about 100,000 hr*ng/mL; about 100,000 hr*ng/mL to about 150,000 hr*ng/mL; about 150,000 hr*ng/mL to about 200,000 hr*ng/mL; about 200,000 hr*ng/mL to about 250,000 hr*ng/mL; about 250,000 hr*ng/mL to about 300,000 hr*ng/mL; about 300,000 hr*ng/mL to about 350,000 hr*ng/mL; about 350,000 hr*ng/mL to about 400,000 hr*ng/mL; about 400,000 hr*ng/mL to about 450,000 hr*ng/mL; about 450,000 hr*ng/mL to about 500,000 hr*ng/mL; about 500,000 hr*ng/mL to about 550,000 hr*ng/mL; about 550,000 hr*ng/mL to about 600,000 hr*ng/mL; about 600,000 hr*ng/mL to about 650,000 hr*ng/mL; about 650,000 hr*ng/mL to about 700,000 hr*ng/mL; about 700,000 hr*ng/mL to about 750,000 hr*ng/mL; about 750,000 hr*ng/mL to about 800,000 hr*ng/mL; about 800,000 hr*ng/mL to about 850,000 hr*ng/mL; about 850,000 hr*ng/mL to about 900,000 hr*ng/mL; about 900,000 hr*ng/mL to about 950,000 hr*ng/mL; about 950,000 hr*ng/mL to about 1,000,000 hr*ng/mL; about 1,000,000 hr*ng/mL to about 1,100,000 hr*ng/mL; about 1,100,000 hr*ng/mL to about 1,200,000 hr*ng/mL; about 1,200,000 hr*ng/mL to about 1,300,000 hr*ng/mL; about 1,300,000 hr*ng/mL to about 1,400,000 hr*ng/mL; about 1,40,000 hr*ng/mL to about 1,500,000 hr*ng/mL; or about 1,50,000 hr*ng/mL to about 2,000,000 hr*ng/mL.

The pharmacokinetic parameters is any parameters suitable for describing a chlorotoxin conjugate described herein. The Cₘₐₓ includes by way of example but is not limited to not less than about 1 ng/mL; not less than about 5 ng/mL; not less than about 10 ng/mL; not less than about 15 ng/mL; not less than about 20 ng/mL; not less than about 25 ng/mL; not less than about 50 ng/mL; not less than about 75 ng/mL; not less than about 100 ng/mL; not less than about 200 ng/mL; not less than about 300 ng/mL; not less than about 400 ng/mL; not less than about 500 ng/mL; not less than about 600 ng/mL; not less than about 700 ng/mL; not less than about 800 ng/mL; not less than about 900 ng/mL; not less than about 1000 ng/mL; not less than about 1250 ng/mL; not less than about 1500 ng/mL; not less than about 1750 ng/mL; not less than about 2000 ng/mL; not less than about 2100 ng/mL; not less than about 2200 ng/mL; not less than about 2300 ng/mL; not less than about 2400 ng/mL; not less than about 2500 ng/mL; not less than about 2600 ng/mL; not less than about 2700 ng/mL; not less than about 2800 ng/mL; not less than about 2900 ng/mL; not less than about 3000 ng/mL; not less than about 3100 ng/mL; not less than about 32000 ng/mL; not less than about 3300 ng/mL; not less than about 3400 ng/mL; not less than about 3500 ng/mL; not less than about 3600 ng/mL; not less than about 3700 ng/mL; not less than about 3800 ng/mL; not less than about 3900 ng/mL; not less than about 4000 ng/mL; not less than about 4500 ng/mL; not less than about 5000 ng/mL; not less than about 5500 ng/mL; not less than about 6000 ng/mL; not less than about 6500 ng/mL; not less than about 2700 ng/mL; not less than about 7500 ng/mL; not less than about 8000 ng/mL; not less than about 8500 ng/mL; not less than about 9000 ng/mL; not less than about 9500 ng/mL; not less than about 10000 ng/mL; not less than about 11000 ng/mL; not less than about 12000 ng/mL; not less than about 13000 ng/mL; not less than about 14000ng/mL; not less than about 15000 ng/mL; not less than about 16000 ng/mL; not less than about 17000 ng/mL; not less than about 18000 ng/mL; not less than about 19000 ng/mL; not less than about 20000 ng/mL; not less than about 25000 ng/mL; not less than about 30000 ng/mL; not less than about 35000 ng/mL; not less than about 40000 ng/mL; not less than about 45000 ng/mL; not less than about 50000 ng/mL; not less than about 55000 ng/mL; not less than about 60000 ng/mL; not less than about 65000ng/mL; not less than about 70000 ng/mL; not less than about 750000 ng/mL; not less than about 80000 ng/mL; not less than about 85000 ng/mL; not less than about 90000 ng/mL; not less than about 95000 ng/mL; not less than about 100000 ng/mL; or any other Cₘₐₓ appropriate for describing a pharmacokinetic profile of a chlorotoxin conjugate described herein. The Cₘₐₓ is, for example, about 1 ng/mL to about 100,000 ng/mL; about 1 ng/mL to about 95,00 ng/mL; about 1 ng/mL to about 90,000 ng/mL; about 1 ng/mL to about 8500 ng/mL; about 1 ng/mL to about 80000 ng/mL; about 1 ng/mL to about 7500 ng/mL; about 1 ng/mL to about 70,000 ng/mL; about 1 ng/mL to about 65 00 ng/mL; about 1 ng/mL to about 60,000 ng/mL; about 1 ng/mL to about 55000 ng/mL; about 1 ng/mL to about 50000 ng/mL; about 1 ng/mL to about 40000 ng/mL; about 1 ng/mL to about 30000 ng/mL; about 1 ng/mL to about 20000 ng/mL; about 1 ng/mL to about 10000 ng/mL; about 1 ng/mL to about 5000 ng/mL; about 1 ng/mL to about 1000 ng/mL; about 1 ng/mL to about 500 ng/mL; about 1 ng/mL to about 500 ng/mL; about 1 ng/mL to about 1000 ng/mL; about 1 ng/mL to about 5000 ng/mL; about 10000 ng/mL to about 5,000 ng/mL; about 10 ng/mL to about 7,000 ng/mL; about 10 ng/mL to about 10,000 ng/mL; about 10 ng/mL to about 10,500 ng/mL; about 10 ng/mL to about 100,000 ng/mL; about 10 ng/mL to about 90000 ng/mL; about 10 ng/mL to about 80000 ng/mL; about 10 ng/mL to about 70000 ng/mL; about 10 ng/mL to about 60000 ng/mL; about 10 ng/mL to about 50000 ng/mL; about 10 ng/mL to about 40000 ng/mL; about 10 ng/mL to about 30000 ng/mL; about 10 ng/mL to about 20000 ng/mL; about 10 ng/mL to about 10000 ng/mL; about 10 ng/mL to about 5000 ng/mL; about 25000 ng/mL to about 50000 ng/mL; about 250 ng/mL to about 10000 ng/mL; about 500 ng/mL to about 50000 ng/mL; about 50 ng/mL to about 10000 ng/mL; about 100 ng/mL to about 50000 ng/mL; about 100 ng/mL to about 40000 ng/mL; about 100 ng/mL to about 30000 ng/mL; or about 100 ng/mL to about 20000 ng/mL.

The plasma concentration of a chlorotoxin conjugate described herein includes by way of example but is not limited to, not less than about 1 ng/mL, not less than about 2 ng/mL, not less than about 3 ng/mL, not less than about 4 ng/mL, not less than about 5 ng/mL, not less than about 6 ng/mL, not less than about 7 ng/mL, not less than about 8 ng/mL, not less than about 9 ng/mL, not less than about 10 ng/mL, not less than about 11 ng/mL, not less than about 12 ng/mL, not less than about 13 ng/mL, not less than about 14 ng/mL, not less than about 15 ng/mL, not less than about 16 ng/mL, not less than about 17 ng/mL, not less than about 18 ng/mL, not less than about 19 ng/mL, not less than about 20 ng/mL, not less than about 21 ng/mL, not less than about 22 ng/mL, not less than about 23 ng/mL, not less than about 24 ng/mL, not less than about 25 ng/mL, not less than about 26 ng/mL, not less than about 27 ng/mL, not less than about 28 ng/mL, not less than about 29 ng/mL, not less than about 30 ng/mL, not less than about 31 ng/mL, not less than about 32 ng/mL, not less than about 33 ng/mL, not less than about 34 ng/mL, not less than about 35 ng/mL, not less than about 36 ng/mL, not less than about 37 ng/mL, not less than about 38 ng/mL, not less than about 39 ng/mL, not less than about 40 ng/mL, not less than about 41 ng/mL, not less than about 42 ng/mL, not less than about 43 ng/mL, not less than about 44 ng/mL, not less than about 45 ng/mL, not less than about 46 ng/mL, not less than about 47 ng/mL, not less than about 48 ng/mL, not less than about 49 ng/mL, not less than about 50 ng/mL, not less than about 51 ng/mL, not less than about 52 ng/mL, not less than about 53 ng/mL, not less than about 54 ng/mL, not less than about 55 ng/mL, not less than about 56 ng/mL, not less than about 57 ng/mL, not less than about 58 ng/mL, not less than about 59 ng/mL, not less than about 60 ng/mL, not less than about 61 ng/mL, not less than about 62 ng/mL, not less than about 63 ng/mL, not less than about 64 ng/mL, not less than about 65 ng/mL, not less than about 66 ng/mL, not less than about 67 ng/mL, not less than about 68 ng/mL, not less than about 69 ng/mL, not less than about 70 ng/mL, not less than about 71 ng/mL, not less than about 72 ng/mL, not less than about 73 ng/mL, not less than about 74 ng/mL, not less than about 75 ng/mL, not less than about 76 ng/mL, not less than about 77 ng/mL, not less than about 78 ng/mL, not less than about 79 ng/mL, not less than about 80 ng/mL, not less than about 81 ng/mL, not less than about 82 ng/mL, not less than about 83 ng/mL, not less than about 84 ng/mL, not less than about 85 ng/mL, not less than about 86 ng/mL, not less than about 87 ng/mL, not less than about 88 ng/mL, not less than about 89 ng/mL, not less than about 90 ng/mL, not less than about 91 ng/mL, not less than about 92 ng/mL, not less than about 93 ng/mL, not less than about 94 ng/mL, not less than about 95 ng/mL, not less than about 96 ng/mL, not less than about 97 ng/mL, not less than about 98 ng/mL, not less than about 99 ng/mL, not less than about 100 ng/mL, not less than about 105 ng/mL, not less than about 110 ng/mL, not less than about 115 ng/mL, not less than about 120 ng/mL, not less than about 125 ng/mL, not less than about 130 ng/mL, not less than about 135 ng/mL, not less than about 140 ng/mL, not less than about 145 ng/mL, not less than about 150 ng/mL, not less than about 155 ng/mL, not less than about 160 ng/mL, not less than about 165 ng/mL, not less than about 170 ng/mL, not less than about 175 ng/mL, not less than about 180 ng/mL, not less than about 185 ng/mL, not less than about 190 ng/mL, not less than about 195 ng/mL, not less than about 200 ng/mL, not less than about 205 ng/mL, not less than about 210 ng/mL, not less than about 215 ng/mL, not less than about 220 ng/mL, not less than about 225 ng/mL, not less than about 230 ng/mL, not less than about 235 ng/mL, not less than about 240 ng/mL, not less than about 245 ng/mL, not less than about 250 ng/mL, or any other plasma concentration of a chlorotoxin conjugate described herein.

The plasma concentration includes by way of example but is not limited to, about 1 ng/mL to about 2ng/mL; about 1 ng/mL to about 5 ng/mL; about 5 ng/mL to about 10 ng/mL; about 10 ng/mL to about 25 ng/mL; about 25 ng/mL to about 50 ng/mL; about 50 ng/mL to about 75 ng/mL; about 75 ng/mL to about 100 ng/mL; about 100 ng/mL to about 150 ng/mL; about 100 ng/mL to about 200 ng/mL about 150 ng/mL to about 200 ng/mL; about 200 ng/mL to about 250 ng/mL; about 250 ng/mL to about 300 ng/mL; about 300 ng/mL to about 350 ng/mL; about 350 ng/mL to about 400 ng/mL; about 400 ng/mL to about 450 ng/mL; about 450 ng/mL to about 500 ng/mL; about 500 ng/mL to about 600 ng/mL; about 600 ng/mL to about 700 ng/mL; about 700 ng/mL to about 800 ng/mL; about 800 ng/mL to about 900 ng/mL; about 900 ng/mL to about 1,000 ng/mL; about 1,000 ng/mL to about 1,100 ng/mL; about 1,100 ng/mL to about 1,200 ng/mL; about 1,200 ng/mL to about 1,300 ng/mL; about 1,300 ng/mL to about 1,400 ng/mL; about 1,400 ng/mL to about 1,500 ng/mL; about 1,500 ng/mL to about 1,600 ng/mL; about 1,600 ng/mL to about 1,700 ng/mL; about 1,700 ng/mL to about 1,800 ng/mL; about 1,800 ng/mL to about 1,900 ng/mL; about 1,900 ng/mL to about 2,000 ng/mL; about 2,000 ng/mL to about 3,000 ng/mL; about 3,000 ng/mL to about 4,000 ng/mL; about 4,000 ng/mL to about 5,000 ng/mL; about 5,000 ng/mL to about 6,000 ng/mL; about 6,000 ng/mL to about 7,000 ng/mL; about 7,000 ng/mL to about 8,000 ng/mL; about 8,000 ng/mL to about 9,000 ng/mL; or about 9,000 ng/mL to about 10,000 ng/mL.

The Tₘₐₓ of a chlorotoxin conjugate described herein includes by way of example but is not limited to, not greater than about 0.5 minutes, not greater than about 1 minutes, not greater than about 1.5 minutes, not greater than about 2 minutes, not greater than about 2.5 minutes, not greater than about 3 minutes, not greater than about 3.5 minutes, not greater than about 4 minutes, not greater than about 4.5 minutes, not greater than about 5 minutes, or any other Tₘₐₓ appropriate for describing a pharmacokinetic profile of a chlorotoxin conjugate described herein. The Tₘₐₓ further includes by way of example but is not limited to about 0.1 minutes to about 24 minutes; about 0.1 minutes to about 0.5 minutes; about 0.5 minutes to about 1 minute; about 1 minute to about 1.5 minutes; about 1.5 minutes to about 2 minute; about 2 minutes to about 2.5 minutes; about 2.5 minutes to about 3 minutes; about 3 minutes to about 3.5 minutes; about 3.5 minutes to about 4 minutes; about 4 minutes to about 4.5 minutes; about 4.5 minutes to about 5 minutes; about 5 minutes to about 5.5 minutes; about 5.5 minutes to about 6 minutes; about 6 minutes to about 6.5 minutes; about 6.5 minutes to about 7 minutes; about 7 minutes to about 7.5 minutes; about 7.5 minutes to about 8 minutes; about 8 minutes to about 8.5 minutes; about 8.5 minutes to about 9 minutes; about 9 minutes to about 9.5 minutes; about 9.5 minutes to about 10 minutes; about 10 minutes to about 10.5 minutes; about 10.5 minutes to about 11 minutes; about 11 minutes to about 11.5 minutes; about 11.5 minutes to about 12 minutes; about 12 minutes to about 12.5 minutes; about 12.5 minutes to about 13 minutes; about 13 minutes to about 13.5 minutes; about 13.5 minutes to about 14 minutes; about 14 minutes to about 14.5 minutes; about 14.5 minutes to about 15 minutes; about 15 minutes to about 15.5 minutes; about 15.5 minutes to about 16 minutes; about 16 minutes to about 16.5 minutes; about 16.5 minutes to about 17 minutes; about 17 minutes to about 17.5 minutes; about 17.5 minutes to about 18 minutes; about 18 minutes to about 18.5 minutes; about 18.5 minutes to about 19 minutes; about 19 minutes to about 19.5 minutes; about 19.5 minutes to about 20 minutes; about 20 minutes to about 20.5 minutes; about 20.5 minutes to about 21 minutes; about 21 minutes to about 21.5 minutes; about 21.5 minutes to about 22 minutes; about 22 minutes to about 22.5 minutes; about 22.5 minutes to about 23 minutes; about 23 minutes to about 23.5 minutes; about 23.5 minutes to about 24 minutes; about 24 minutes to about 25 minutes; about 25 minutes to about 25.5 minutes; about 25.5 minutes to about 26 minutes; about 26 minutes to about 26.5 minutes; about 26.5 minutes to about 27 minutes; about 27 minutes to about 28 minutes; about 28 minutes to about 28.5 minutes; about 28.5 minutes to about 29 minutes; about 29 minutes to about 29.5 minutes; about 29.5 minutes to about 30 minutes; about 30 minutes to about 31 minutes; about 31 minutes to about 31.5 minutes; about 31.5 minutes to about 32 minutes; about 32 minutes to about 32.5 minutes; about 32.5 minutes to about 33 minutes; about 33 minutes to about 34 minutes; about 34 minutes to about 35 minutes; about 35 minutes to about 36 minutes; about 36 minutes to about 37 minutes; about 37 minutes to about 38 minutes; about 38 minutes to about 39 minutes; about 39 minutes to about 40 minutes; about 40 minutes to about 41 minutes; about 41 minutes to about 42 minutes; about 42 minutes to about 43 minutes; about 43 minutes to about 44 minutes; about 45 minutes to about 46 minutes; about 46 minutes to about 47 minutes; about 47 minutes to about 48 minutes; about 48 minutes to about 49 minutes; about 49 minutes to about 50 minutes; about 50 minutes to about 51 minutes; about 51 minutes to about 52 minutes; about 52 minutes to about 53 minutes; about 53 minutes to about 55 minutes; about 55 minutes to about 56 minutes; about 56 minutes to about 57 minutes; about 57 minutes to about 58 minutes; about 58 minutes to about 59 minutes; about 59 minutes to about 60 minutes; or any other Tₘₐₓ of a chlorotoxin conjugate described herein of a chlorotoxin conjugate described herein.

The Tₘₐₓ of a chlorotoxin conjugate described herein includes by way of example but is not limited to, not greater than about 0.5 hours, not greater than about 1 hours, not greater than about 1.5 hours, not greater than about 2 hours, not greater than about 2.5 hours, not greater than about 3 hours, not greater than about 3.5 hours, not greater than about 4 hours, not greater than about 4.5 hours, not greater than about 5 hours, or any other Tₘₐₓ appropriate for describing a pharmacokinetic profile of a chlorotoxin conjugate described herein. The Tₘₐₓ further includes by way of example but is not limited to about 0.1 hours to about 24 hours; about 0.1 hours to about 0.5 hours; about 0.5 hours to about 1 hour; about 1 hour to about 1.5 hours; about 1.5 hours to about 2 hour; about 2 hours to about 2.5 hours; about 2.5 hours to about 3 hours; about 3 hours to about 3.5 hours; about 3.5 hours to about 4 hours; about 4 hours to about 4.5 hours; about 4.5 hours to about 5 hours; about 5 hours to about 5.5 hours; about 5.5 hours to about 6 hours; about 6 hours to about 6.5 hours; about 6.5 hours to about 7 hours; about 7 hours to about 7.5 hours; about 7.5 hours to about 8 hours; about 8 hours to about 8.5 hours; about 8.5 hours to about 9 hours; about 9 hours to about 9.5 hours; about 9.5 hours to about 10 hours; about 10 hours to about 10.5 hours; about 10.5 hours to about 11 hours; about 11 hours to about 11.5 hours; about 11.5 hours to about 12 hours; about 12 hours to about 12.5 hours; about 12.5 hours to about 13 hours; about 13 hours to about 13.5 hours; about 13.5 hours to about 14 hours; about 14 hours to about 14.5 hours; about 14.5 hours to about 15 hours; about 15 hours to about 15.5 hours; about 15.5 hours to about 16 hours; about 16 hours to about 16.5 hours; about 16.5 hours to about 17 hours; about 17 hours to about 17.5 hours; about 17.5 hours to about 18 hours; about 18 hours to about 18.5 hours; about 18.5 hours to about 19 hours; about 19 hours to about 19.5 hours; about 19.5 hours to about 20 hours; about 20 hours to about 20.5 hours; about 20.5 hours to about 21 hours; about 21 hours to about 21.5 hours; about 21.5 hours to about 22 hours; about 22 hours to about 22.5 hours; about 22.5 hours to about 23 hours; about 23 hours to about 23.5 hours; about 23.5 hours to about 24 hours; about 24 hours to about 25 hours; about 25 hours to about 25.5 hours; about 25.5 hours to about 26 hours; about 26 hours to about 26.5 hours; about 26.5 hours to about 27 hours; about 27 hours to about 28 hours; about 28 hours to about 28.5 hours; about 28.5 hours to about 29 hours; about 29 hours to about 29.5 hours; about 29.5 hours to about 30 hours; about 30 hours to about 31 hours; about 31 hours to about 31.5 hours; about 31.5 hours to about 32 hours; about 32 hours to about 32.5 hours; about 32.5 hours to about 33 hours; about 33 hours to about 34 hours; about 34 hours to about 35 hours; about 35 hours to about 36 hours; about 36 hours to about 37 hours; about 37 hours to about 38 hours; about 38 hours to about 39 hours; about 39 hours to about 40 hours; about 40 hours to about 41 hours; about 41 hours to about 42 hours; about 42 hours to about 43 hours; about 43 hours to about 44 hours; about 45 hours to about 46 hours; about 46 hours to about 47 hours; about 47 hours to about 48 hours; about 48 hours to about 49 hours; about 49 hours to about 50 hours; about 50 hours to about 51 hours; about 51 hours to about 52 hours; about 52 hours to about 53 hours; about 53 hours to about 55 hours; about 55 hours to about 56 hours; about 56 hours to about 57 hours; about 57 hours to about 58 hours; about 58 hours to about 59 hours; about 59 hours to about 60 hours; about 60 hours to about 61 hours; about 61 hours to about 62 hours; about 62 hours to about 63 hours; about 63 hours to about 64 hours; about 64 hours to about 66 hours; about 66 hours to about 67 hours; about 67 hours to about 68 hours; about 68 hours to about 69 hours; about 69 hours to about 70 hours; about 70 hours to about 71 hours; about 71 hours to about 72 hours; about 72 hours to about 73 hours; about 73 hours to about 74 hours; about 774hours to about 75 hours; about 75 hours to about 77 hours; about 77 hours to about 78 hours; about 78 hours to about 79 hours; about79 hours to about 80 hours; about 80 hours to about 81 hours; about 81 hours to about 82 hours; about 82 hours to about 83 hours; about 83 hours to about 84 hours; about 84 hours to about 85 hours; about 85 hours to about 87 hours; about 87 hours to about 88 hours; about 88 hours to about 89 hours; about 89 hours to about 90 hours; about 90 hours to about 91 hours; about 91 hours to about 92 hours; about 92 hours to about 93 hours; about 93 hours to about 94 hours; about 94 hours to about 95 hours; about 95 hours to about 97 hours; about 97 hours to about 99 hours; about 99 hours to about 100 hours; or any other Tₘₐₓ of a chlorotoxin conjugate described herein of a chlorotoxin conjugate described herein.

In some aspects, the chlorotoxin conjugates distribute into the subject tissues. For example, distribution into the tissues is often rapid compared to the elimination phase. In some aspects, the chlorotoxin conjugates are eliminated from the subject tissues. For example, elimination from the subject tissues is often slow compared to the distribution phase. Often the kidney is important in the clearance and elimination of the chlorotoxin conjugates, often contributing to the elimination phase.

The pharmacokinetics parameters are any parameters suitable for describing the plasma profiles of chlorotoxin conjugates described herein and are often associated with a curve. As described elsewhere herein, dose is either scaled or fixed, said scaled dose useful for scaling the dose from one subject to another wherein the subjects are the same species, different species, same sex or different sex. The phases of the curve are often representative of data obtained from at least one subject, sometimes more than one subject, and the phases of the curve and/or data of the curve is often scaled in a manner similar to the manner in which doses are scaled.

In some aspects, the curve is plotted on a graph, often a graph with an x-axis and a y-axis referred to for example as an x-y plot, a scatter plot or the like. Each axis of the graph has units, the y-axis often having units of time, for example in hours, and x-axis often having units of concentration, for example as ng/mL, of a chlorotoxin conjugate described herein present in a subject sample as described herein and are representative of a single measurement, a mean, an average, or any other suitable mathematical calculation performed on a set of data. When a suitable mathematical calculation is performed, a statistic is also calculated, for example, a standard error, standard error of the mean, standard deviation, standard deviation of the mean, or any other suitable statistic useful for the described disclosure.

In some aspects, the curve has phases, for example, distribution phase, metabolism phase and elimination phase. In some aspects, the distribution phase begins at time of about 0 hours and extends until a time of about 0.01 hours, about 0.02 hours, about 0.03 hours, about 0.04 hours, about 0.05 hours, about 0.06 hours, about 0.07 hours, about 0.08 hours, about 0.09 hours, about 0.11 hours, about 0.12 hours, about 0.13 hours, about 0.14 hours, about 0.15 hours, about 0.16 hours, about 0.17 hours, about 0.18 hours, about 0.19 hours, about 0.20 hours, 0.21 hours, about 0.22 hours, about 0.23 hours, about 0.24 hours, about 0.25 hours, about 0.26 hours, about 0.27 hours, about 0.28 hours, about 0.29 hours, about 0.30 hours, about 0.31 hours, about 0.32 hours, about 0.33 hours, about 0.34 hours, about 0.35 hours, about 0.36 hours, about 0.37 hours, about 0.38 hours, about 0.39 hours, about 0.40 hours, about 0.41 hours, about 0.42 hours, about 0.43 hours, about 0.44 hours, about 0.45 hours, about 0.46 hours, about 0.47 hours, about 0.48 hours, about 0.49 hours, about 0.50 hours, about 0.51 hours, about 0.52 hours, about 0.53 hours, about 0.54 hours, about 0.55 hours, about 0.56 hours, about 0.57 hours, about 0.58 hours, about 0.59 hours, about 0.60 hours, about 0.61 hours, about 0.62 hours, about 0.63 hours, about 0.64 hours, about 0.65 hours, about 0.66 hours, about 0.67 hours, about 0.68 hours, about 0.69 hours, about 0.70 hours, about 0.71 hours, about 0.72 hours, about 0.73 hours, about 0.74 hours, about 0.75 hours, about 0.76 hours, about 0.77 hours, about 0.78 hours, about 0.79 hours, about 0.80 hours, about 0.81 hours, about 0.82 hours, about 0.83 hours, about 0.84 hours, about 0.85 hours, about 0.86 hours, about 0.87 hours, about 0.88 hours, about 0.89 hours, about 0.90 hours, about 0.91 hours, about 0.92 hours, about 0.93 hours, about 0.94 hours, about 0.95 hours, about 0.96 hours, about 0.97 hours, about 0.98 hours, about 0.99 hours, about 1.00 hours, about 1.01 hours, about 1.02 hours, about 1.03 hours, about 1.04 hours, about 1.05 hours, about 1.06 hours, about 1.07 hours, about 1.08 hours, about 1.09 hours, about 1.11 hours, about 1.12 hours, about 1.13 hours, about 1.14 hours, about 1.15 hours, about 1.16 hours, about 1.17 hours, about 1.18 hours, about 1.19 hours, about 1.20 hours, 1.21 hours, about 1.22 hours, about 1.23 hours, about 1.24 hours, about 1.25 hours, about 1.26 hours, about 1.27 hours, about 1.28 hours, about 1.29 hours, about 1.30 hours, about 1.31 hours, about 1.32 hours, about 1.33 hours, about 1.34 hours, about 1.35 hours, about 1.36 hours, about 1.37 hours, about 1.38 hours, about 1.39 hours, about 1.40 hours, about 1.41 hours, about 1.42 hours, about 1.43 hours, about 1.44 hours, about 1.45 hours, about 1.46 hours, about 1.47 hours, about 1.48 hours, about 1.49 hours, about 1.50 hours, about 1.51 hours, about 1.52 hours, about 1.53 hours, about 1.54 hours, about 1.55 hours, about 1.56 hours, about 1.57 hours, about 1.58 hours, about 1.59 hours, about 1.60 hours, about 1.61 hours, about 1.62 hours, about 1.63 hours, about 1.64 hours, about 1.65 hours, about 1.66 hours, about 1.67 hours, about 1.68 hours, about 1.69 hours, about 1.70 hours, about 1.71 hours, about 1.72 hours, about 1.73 hours, about 1.74 hours, about 1.75 hours, about 1.76 hours, about 1.77 hours, about 1.78 hours, about 1.79 hours, about 1.80 hours, about 1.81 hours, about 1.82 hours, about 1.83 hours, about 1.84 hours, about 1.85 hours, about 1.86 hours, about 1.87 hours, about 1.88 hours, about 1.89 hours, about 1.90 hours, about 1.91 hours, about 1.92 hours, about 1.93 hours, about 1.94 hours, about 1.95 hours, about 1.96 hours, about 1.97 hours, about 1.98 hours, about 1.99 hours, about 2.00 hours, about 2.20 hours, about 2.40 hours, about 2.60 hours, about 2.80 hours, about 3.00 hours, about 4.20 hours, about 4.40 hours, about 4.60 hours, about 4.80 hours, about 5.00 hours, about 5.20 hours, about 5.40 hours, about 5.60 hours, about 5.80 hours, about 6.00 hours, about 6.20 hours, about 6.40 hours, about 6.60 hours, about 6.80 hours, about 7.00 hours, about 7.20 hours, about 7.40 hours, about 7.60 hours, about 7.80 hours, about 8.00 hours, about 8.20 hours, about 8.40 hours, about 8.60 hours, about 8.80 hours, about 9.00 hours, about 9.20 hours, about 9.40 hours, about 9.60 hours, about 9.80 hours, about 10.00 hours or more than about 10.00 hours.

In some aspects, the metabolism phase begins at time of about 0.5 hours and extends until a time of about about 0.50 hours, about 0.51 hours, about 0.52 hours, about 0.53 hours, about 0.54 hours, about 0.55 hours, about 0.56 hours, about 0.57 hours, about 0.58 hours, about 0.59 hours, about 0.60 hours, about 0.61 hours, about 0.62 hours, about 0.63 hours, about 0.64 hours, about 0.65 hours, about 0.66 hours, about 0.67 hours, about 0.68 hours, about 0.69 hours, about 0.70 hours, about 0.71 hours, about 0.72 hours, about 0.73 hours, about 0.74 hours, about 0.75 hours, about 0.76 hours, about 0.77 hours, about 0.78 hours, about 0.79 hours, about 0.80 hours, about 0.81 hours, about 0.82 hours, about 0.83 hours, about 0.84 hours, about 0.85 hours, about 0.86 hours, about 0.87 hours, about 0.88 hours, about 0.89 hours, about 0.90 hours, about 0.91 hours, about 0.92 hours, about 0.93 hours, about 0.94 hours, about 0.95 hours, about 0.96 hours, about 0.97 hours, about 0.98 hours, about 0.99 hours, about 1.00 hours, about 1.01 hours, about 1.02 hours, about 1.03 hours, about 1.04 hours, about 1.05 hours, about 1.06 hours, about 1.07 hours, about 1.08 hours, about 1.09 hours, about 1.11 hours, about 1.12 hours, about 1.13 hours, about 1.14 hours, about 1.15 hours, about 1.16 hours, about 1.17 hours, about 1.18 hours, about 1.19 hours, about 1.20 hours, 1.21 hours, about 1.22 hours, about 1.23 hours, about 1.24 hours, about 1.25 hours, about 1.26 hours, about 1.27 hours, about 1.28 hours, about 1.29 hours, about 1.30 hours, about 1.31 hours, about 1.32 hours, about 1.33 hours, about 1.34 hours, about 1.35 hours, about 1.36 hours, about 1.37 hours, about 1.38 hours, about 1.39 hours, about 1.40 hours, about 1.41 hours, about 1.42 hours, about 1.43 hours, about 1.44 hours, about 1.45 hours, about 1.46 hours, about 1.47 hours, about 1.48 hours, about 1.49 hours, about 1.50 hours, about 1.51 hours, about 1.52 hours, about 1.53 hours, about 1.54 hours, about 1.55 hours, about 1.56 hours, about 1.57 hours, about 1.58 hours, about 1.59 hours, about 1.60 hours, about 1.61 hours, about 1.62 hours, about 1.63 hours, about 1.64 hours, about 1.65 hours, about 1.66 hours, about 1.67 hours, about 1.68 hours, about 1.69 hours, about 1.70 hours, about 1.71 hours, about 1.72 hours, about 1.73 hours, about 1.74 hours, about 1.75 hours, about 1.76 hours, about 1.77 hours, about 1.78 hours, about 1.79 hours, about 1.80 hours, about 1.81 hours, about 1.82 hours, about 1.83 hours, about 1.84 hours, about 1.85 hours, about 1.86 hours, about 1.87 hours, about 1.88 hours, about 1.89 hours, about 1.90 hours, about 1.91 hours, about 1.92 hours, about 1.93 hours, about 1.94 hours, about 1.95 hours, about 1.96 hours, about 1.97 hours, about 1.98 hours, about 1.99 hours, about 2.00 hours, about 2.20 hours, about 2.40 hours, about 2.60 hours, about 2.80 hours, about 3.00 hours, about 4.20 hours, about 4.40 hours, about 4.60 hours, about 4.80 hours, about 5.00 hours, about 5.20 hours, about 5.40 hours, about 5.60 hours, about 5.80 hours, about 6.00 hours, about 6.20 hours, about 6.40 hours, about 6.60 hours, about 6.80 hours, about 7.00 hours, about 7.20 hours, about 7.40 hours, about 7.60 hours, about 7.80 hours, about 8.00 hours, about 8.20 hours, about 8.40 hours, about 8.60 hours, about 8.80 hours, about 9.00 hours, about 9.20 hours, about 9.40 hours, about 9.60 hours, about 9.80 hours, about 10.00 hours, about 10.20 hours, about 10.40 hours, about 10.60 hours, about 10.80 hours, about 12.00 hours, about 12.20 hours, about 12.40 hours, about 12.60 hours, about 12.80 hours, about 14.00 hours, about 14.20 hours, about 14.40 hours, about 14.60 hours, about 14.80 hours, about 16.00 hours, about 16.20 hours, about 16.40 hours, about 16.60 hours, about 16.80 hours, about 18.00 hours, about 18.20 hours, about 18.40 hours, about 18.60 hours, about 18.80 hours, about 20.00 hours, about 20.20 hours, about 20.40 hours, about 20.60 hours, about 20.80 hours, about 22.00 hours, about 22.20 hours, about 22.40 hours, about 22.60 hours, about 22.80 hours, about 24.00 hours, about 24.20 hours, about 24.40 hours, about 24.60 hours, about 24.80 hours, about 26.00 hours, about 26.20 hours, about 26.40 hours, about 26.60 hours, about 26.80 hours, about 28.00 hours, about 28.20 hours, about 28.40 hours, about 28.60 hours, about 28.80 hours, about 30 hours or more than about 30.00 hours.

In some aspects, the elimination phase begins at time of about 2 hours and extends until a time of about 2.00 hours, about 2.20 hours, about 2.40 hours, about 2.60 hours, about 2.80 hours, about 3.00 hours, about 4.20 hours, about 4.40 hours, about 4.60 hours, about 4.80 hours, about 5.00 hours, about 5.20 hours, about 5.40 hours, about 5.60 hours, about 5.80 hours, about 6.00 hours, about 6.20 hours, about 6.40 hours, about 6.60 hours, about 6.80 hours, about 7.00 hours, about 7.20 hours, about 7.40 hours, about 7.60 hours, about 7.80 hours, about 8.00 hours, about 8.20 hours, about 8.40 hours, about 8.60 hours, about 8.80 hours, about 9.00 hours, about 9.20 hours, about 9.40 hours, about 9.60 hours, about 9.80 hours, about 10.00 hours, about 10.20 hours, about 10.40 hours, about 10.60 hours, about 10.80 hours, about 12.00 hours, about 12.20 hours, about 12.40 hours, about 12.60 hours, about 12.80 hours, about 14.00 hours, about 14.20 hours, about 14.40 hours, about 14.60 hours, about 14.80 hours, about 16.00 hours, about 16.20 hours, about 16.40 hours, about 16.60 hours, about 16.80 hours, about 18.00 hours, about 18.20 hours, about 18.40 hours, about 18.60 hours, about 18.80 hours, about 20.00 hours, about 20.20 hours, about 20.40 hours, about 20.60 hours, about 20.80 hours, about 22.00 hours, about 22.20 hours, about 22.40 hours, about 22.60 hours, about 22.80 hours, about 24.00 hours, about 24.20 hours, about 24.40 hours, about 24.60 hours, about 24.80 hours, about 26.00 hours, about 26.20 hours, about 26.40 hours, about 26.60 hours, about 26.80 hours, about 28.00 hours, about 28.20 hours, about 28.40 hours, about 28.60 hours, about 28.80 hours, about 30.00 hours, about 30.20 hours, about 30.40 hours, about 30.60 hours, about 30.80 hours, about 32.00 hours, about 32.20 hours, about 32.40 hours, about 32.60 hours, about 32.80 hours, about 34.00 hours, about 34.20 hours, about 34.40 hours, about 34.60 hours, about 34.80 hours, about 36.00 hours, about 36.20 hours, about 36.40 hours, about 36.60 hours, about 36.80 hours, about 38.00 hours, about 38.20 hours, about 38.40 hours, about 38.60 hours, about 38.80 hours, about 40.00 hours, about 40.20 hours, about 40.40 hours, about 40.60 hours, about 40.80 hours, about 42.00 hours, about 42.20 hours, about 42.40 hours, about 42.60 hours, about 42.80 hours, about 44.00 hours, about 44.20 hours, about 44.40 hours, about 44.60 hours, about 44.80 hours, about 46.00 hours, about 46.20 hours, about 46.40 hours, about 46.60 hours, about 46.80 hours, about 48.00 hours, about 48.20 hours, about 48.40 hours, about 48.60 hours, about 48.80 hours, about 50.00 hours, about 50.20 hours, about 50.40 hours, about 50.60 hours, about 50.80 hours, about 52.00 hours, about 52.20 hours, about 52.40 hours, about 52.60 hours, about 52.80 hours, about 54.00 hours, about 54.20 hours, about 54.40 hours, about 54.60 hours, about 54.80 hours, about 56.00 hours, about 56.20 hours, about 56.40 hours, about 56.60 hours, about 56.80 hours, about 58.00 hours, about 58.20 hours, about 58.40 hours, about 58.60 hours, about 58.80 hours, about 60.00 hours, about 60.20 hours, about 60.40 hours, about 60.60 hours, about 60.80 hours, about 62.00 hours, about 62.20 hours, about 62.40 hours, about 62.60 hours, about 62.80 hours, about 64.00 hours, about 64.20 hours, about 64.40 hours, about 64.60 hours, about 64.80 hours, about 66.00 hours, about 66.20 hours, about 66.40 hours, about 66.60 hours, about 66.80 hours, about 68.00 hours, about 68.20 hours, about 68.40 hours, about 68.60 hours, about 68.80 hours, about 70.00 hours, about 70.20 hours, about 70.40 hours, about 70.60 hours, about 70.80 hours, about 72.00 hours, about 72.20 hours, about 72.40 hours, about 72.60 hours, about 72.80 hours, about 74.00 hours, about 74.20 hours, about 74.40 hours, about 74.60 hours, about 74.80 hours, about 76.00 hours, about 76.20 hours, about 76.40 hours, about 76.60 hours, about 76.80 hours, about 78.00 hours, about 78.20 hours, about 78.40 hours, about 78.60 hours, about 78.80 hours, about 80.00 hours, about 80.20 hours, about 80.40 hours, about 80.60 hours, about 80.80 hours, about 82.00 hours, about 82.20 hours, about 82.40 hours, about 82.60 hours, about 82.80 hours, about 84.00 hours, about 84.20 hours, about 84.40 hours, about 84.60 hours, about 84.80 hours, about 86.00 hours, about 86.20 hours, about 86.40 hours, about 86.60 hours, about 86.80 hours, about 88.00 hours, about 88.20 hours, about 88.40 hours, about 88.60 hours, about 88.80 hours, about 90.00 hours or about more than 90.00 hours.

In some aspects, a single fixed bolus dose intravenous chlorotoxin conjugate often results in mean serum concentrations measurable up to about 12 hours post-dose, about 24 hours post-dose, up to about 36 hours post-dose, up to about 48 hours post-dose or more than about 48 hours post-dose. Often, for subjects such as rats, Cₘₐₓ and C₀ parameters increase in about a dose-proportional manner. In some aspects, the AUC₀₋ₜ parameter, for subjects such as rats, is about dose-proportional at less than about a 1 mg dose levels, and increases in a greater than dose-proportional manner at greater than about 1 mg dose levels. Often there is no effect of gender on any PK parameters for subjects such as rats. In some aspects, PK parameters are predictive in rats of a human subject.

In some aspects, a single fixed bolus dose intravenous chlorotoxin conjugate often results in mean serum concentrations measurable up to about 12 hours post-dose, about 24 hours post-dose, up to about 36 hours post-dose, up to about 48 hours post-dose or more than about 48 hours post-dose. Often, for subjects such as rats, Cₘₐₓ and C₀ parameters increase in about a dose-proportional manner. In some aspects, the AUC₀₋ₜ parameter, for subjects such as monkeys, is greater than dose-proportional manner at greater than about 1 mg dose levels for example such that chlorotoxin conjugates exhibit reduced clearance at higher doses in monkeys. Often there is an effect of gender on PK parameters for subjects such as monkeys, for example, the C₀ and AUC are about 5 to about 30% higher in females relative to males.

As used herein, two pharmacokinetic profiles are "about equivalent" if they are defined by at least one parameter that is about equivalent between the two profiles. Non-limiting examples of such parameters include the area under plasma concentration over time curve (AUC) and the maximal plasma concentration reached following administration of a dose (Cₘₐₓ).

In some aspects two pharmacokinetic parameters are about equivalent if the lower value is greater than 70%, greater than 75%, greater than 80%, greater than 85%, greater than 90%, greater than 95%, greater than 96%, greater than 97%, greater than 98%, or greater than 99% of the higher value.

The pharmacokinetic profiles of two dosage regimens are compared by determining the average pharmacokinetic profile in a population of subjects receiving the first dosage regimen, determining the average pharmacokinetic profile in a population of subjects receiving the second dosage regimen, and then comparing those two population dosage regimens. In some aspects, a population of subjects is one subject. In other aspects, a population of subjects is more than one subject, for example, two subjects, three subjects, four subjects, five subjects, six subjects, seven subjects, eight subjects, nine subjects, ten subjects, 11 subjects, 12 subjects, 13 subjects, 14 subjects, 15 subjects, 20 subjects, 25 subjects, 30 subjects, 35 subjects, 40 subjects, 45 subjects, 50 subjects, or more than 50 subjects.

In various aspects, the present disclosure provides a claimed compound for use in a method of administering to a human subject, the method comprising: intravenously administering to the human subject a dose of from 1 mg to 30 mg of the compound; and producing in the human subject an average maximum compound blood plasma concentration (average Cₘₐₓ) of at least from 110 ng/mL to 240 ng/mL per each 1 mg dosage of the compound administered.

In various aspects, the present disclosure provides a claimed compound for use in a method of detecting a cancer cell in a human subject, the method comprising: intravenously administering to the human subject a dose of from 1 mg to 30 mg of the compound; producing in the human subject an average maximum compound blood plasma concentration (average Cₘₐₓ) of at least from 110 ng/mL to 240 ng/mL per each 1 mg dosage of the compound administered; and detecting the presence or absence of the detectable label in the human subject, wherein the presence of the detectable label indicates the presence of the cancer cell.

In various aspects, the present disclosure provides a claimed compound for use in a method of diagnosing cancer in a human subject, the method comprising: intravenously administering to the human subject a dose of from 1 mg to 30 mg of the compound; producing in the human subject an average maximum compound blood plasma concentration (average Cₘₐₓ) of at least from 110 ng/mL to 240 ng/mL per each 1 mg dosage of the compound administered; and detecting the presence of absence of the detectable label in the human subject, wherein the presence of the detectable label indicates a diagnosis of cancer.

In various aspects, the present disclosure provides a claimed compound for use in a method of treating cancer in a human subject, the method comprising: intravenously administering to the human subject a dose of from 1 mg to 30 mg of the compound conjugated to a therapeutic agent; producing in the human subject an average maximum compound blood plasma concentration (average Cₘₐₓ) of at least from 110 ng/mL to 240 ng/mL per each 1 mg dosage of the compound administered; and reducing or improving a symptom or condition associated with cancer in the human subject. In some aspects, the human subject is in need thereof. In some aspects, the methods comprise administering a therapeutically effectie dose of the compound to the human subject.

In various aspects, the present disclosure provides a claimed compound for use in a method of administering to a human subject, the method comprising: administering to the human subject a dose of from 1 mg to 30 mg of the compound; and producing in the human subject pharmacokinetic profile of FIG. 27.

In some aspects, the average time (average Tₘₐₓ) at which the average Cₘₐₓ is reached is at 5 ± 4 minutes following administration of the compound. In some aspects, the average time (average T₇₅) at which the average compound blood plasma concentration reaches 75% of the average Cₘₐₓ (average C₇₅) is reached is at 8 ± 5 minutes following administration of the compound. In some aspects, the average time (average T₅₀) at which the average compound blood plasma concentration reaches 50% of the average Cₘₐₓ (average C₅₀) is reached is at 20 ± 8 minutes following administration of the compound. In some aspects, the average time (average T₂₅) at which the average compound blood plasma concentration reaches 25% of the average Cₘₐₓ (average C₂₅) is reached is at 30 ± 12 minutes following administration of the compound.

In some aspects, the methods further comprise producing in the human subject an average chlorotoxin polypeptide plasma area under the curve (average AUC) of from 50 hr*ng/mL to 120 hr*ng/mL per each 1 mg dosage of chlorotoxin polypeptide administered.

In some aspects, the methods further comprise producing in the human subject an average chlorotoxin polypeptide plasma area under the curve (average AUC) of from 60 hr*ng/mL to 110 hr*ng/mL per each 1 mg dosage of chlorotoxin polypeptide administered.

In some aspects, 75% of the average AUC occurs within 40 ± 15 minutes after administering the compound. In some aspects, 50% of the average AUC occurs within 21 ± 8 minutes after administering the compound. In some aspects, 25% of the average AUC occurs within 9 ± 5 minutes after administering the compound.

In various aspects, the present disclosure provides a claimed compound for use in a method for detecting a cancer cell in a subject, the method comprising administering the compound of the present disclosure; and detecting the presence or absence of the compound in the subject, wherein the presence of the compound indicates the presence of a cancer cell.

In some aspects, the method further comprises administering the compound as a part of a composition.

In some aspects, the cancer is selected from glioma, astrocytoma, medulloblastoma, choroids plexus carcinoma, ependymoma, brain tumor, neuroblastoma, adenocarcinoma, basal cell carcinoma, squamous cell carcinoma, head and neck cancer, lung cancer, breast cancer, intestinal cancer, pancreatic cancer, liver cancer, kidney cancer, sarcoma, osteosarcoma, rhabdomyosarcoma, Ewing's sarcoma, carcinoma, melanoma, ovarian cancer, cervical cancer, lymphoma, thyroid cancer, anal cancer, colo-rectal cancer, endometrial cancer, germ cell tumor, laryngeal cancer, multiple myeloma, prostate cancer, retinoblastoma, gastric cancer, testicular cancer, or Wilm's tumor. In some aspects, the cancer is selected from glioma, medulloblastoma, sarcoma, breast cancer, lung cancer, prostate cancer, or intestinal cancer. In some aspects, the cancer cell expresses a site to which native chlorotoxin binds.

In some aspects, the method comprises detecting the compound by fluorescence imaging.

In some aspects, the method further comprises differentiating a focus of a cancer that expresses a site to which native chlorotoxin binds from non-neoplastic tissue.

In some aspects, the method further comprises surgically removing from the subject a cancer cell that is detected.

In some aspects, the method further comprises determining the location of a cancer cell in the subject before surgically removing the cancer cell from the subject, during surgical removal of the cancer cell from the subject, after removing the cancer cell from the subject, or a combination thereof.

In various aspects, the present disclosure provides a claimed compound for use in a method of administering the compound to a subject, the method comprising administering a therapeutically effectie amount of the compound to the subject.

In some aspects, the subject is in need thereof.

In some aspects, a therapeutically effectie amount is a dosage sufficient for the detection of a cancer cell in the subject. In some aspects, the dosage is from 0.1 mg to 100 mg. In some aspects, dosage is from 1 mg to 30 mg. In some aspects, the dosage is from 3 mg to 30 mg.

In various aspects, the present disclosure provides a claimed compound for use in a method of treating a subject in need thereof, the method comprising administering to the subject the compound further comprising a therapeutic agent in an amount sufficient to treat cancer in the subject. In certain aspects, the therapeutic agent is a cytotoxic agent.

In some aspects, the cancer is selected from glioma, astrocytoma, medulloblastoma, choroids plexus carcinoma, ependymoma, brain tumor, neuroblastoma, head and neck cancer, lung cancer, breast cancer, intestinal cancer, pancreatic cancer, liver cancer, kidney cancer, sarcoma, osteosarcoma, rhabdomyosarcoma, Ewing's sarcoma, carcinoma, melanoma, ovarian cancer, cervical cancer, lymphoma, thyroid cancer, anal cancer, colo-rectal cancer, endometrial cancer, germ cell tumor, laryngeal cancer, multiple myeloma, prostate cancer, retinoblastoma, gastric cancer, testicular cancer, or Wilm's tumor. In some aspects, the cancer cell is selected from glioma, medulloblastoma, sarcoma, prostate cancer, or intestinal cancer. In certain aspects, the cancer cell expresses a site to which native chlorotoxin binds. In further aspects, the binding is selective.

In some aspects, the compound is administered parenterally. In other aspects, the compound is administered intravenously. In still other aspects, the compound is administered subcutaneously.

### Methods for Analysis to Generate Pharmacokinetic Profiles

In some aspects, samples are analyzed to obtain parameters useful to determine a pharmacokinetic profile. Often the samples are diluted, for example, using a buffer or pharmaceutically acceptable carrier as defined herein.

Pharmacokinetic standard curves are often generated using a chlorotoxin conjugate, serum and a pharmaceutical carrier as described herein. The proportion of each chlorotoxin conjugate, concentrated source of sample (for example serum, urine, etc.) and pharmaceutical carrier often differs, for example, the concentration of compound of the present disclosure is often between about10 µg/mL and about 4 ng/mL. Often the standard curve is used to calculate the concentration of the compound in the sample.

In some aspects, pharmacokinetic parameters, or pharmacokinetic data are analyzed using standard pharmacokinetic data analysis methods, including concentration of chlorotoxin conjugates versus time. For example, a software program, such as Phoenix WinNonlin 6.3 is used to analyze pharmacokinetics data. In some aspects, the pharmacokinetic data analysis uses standard noncompartmental methods of intravenous bolus, intravenous infusion, or extravascular input as appropriate. In other aspects, the pharmacokinetic data analysis uses nonstandard noncompartmental methods of intravenous bolus, intravenous infusion, or extravascular input as appropriate. Often, the data are analyzed by the mean serum concentration versus time. The data are also analyzed by individual subject followed by group summary statistics.

Pharmacokinetic profiles of the compositions described herein are often obtained using at least one, sometimes more than one bioanalytical method. In some aspects, bioanalytical methods include the addition of chemicals to a sample containing a composition of which the pharmacokinetic profile is desired. Addition of the chemical to the sample often comprises performing a chemical technique to measure the concentration of a composition or a metabolite thereof in a sample or, sometimes, in a biological matrix. For example, microscale thermophoresis, mass spectrometry often including liquid chromatorgraph and a triple quadropole mass spectrometer, tandem mass spectrometry, high sensitivity mass spectrometry for microdosing studeies and the like are often performed.

The disclosure further describes a compound or composition as claimed for use in a method of administration to a subject, often methods include intravenous administration of a chlorotoxin conjugate composition to a subject. In some aspects, the method of administering a chlorotoxin polypeptide to a subject comprises intravenously administering a dose of from 0.8 to 25 mg of the chlorotoxin polypeptide to the subject; producing in the subject an average chlorotoxin polypeptide plasma area under the curve (average AUC) of from 50 to 120 per each 1 mg dosage of chlorotoxin polypeptide administered; and producing in the subject an average maximum chlorotoxin peptide blood plasma concentration (average Cₘₐₓ) of at least from 110 to 240 per each 1 mg dosage of chlorotoxin peptide administered. In some aspects, the chlorotoxin polypeptide is conjugated to a fluorescent agent.

The disclosure further describes a composition as claimed for use in a method of treating and/or detecting cancer with the composition following administration to a subject or contacting tumor tissue isolated from a subject, often the methods include intravenous administration of a chlorotoxin conjugate composition to a subject, *in vivo* contact of a tumor tissue or *ex vivo* contact of a tumor tissue from a subject. In some aspects, the method treating and/or detecting cancer with chlorotoxin conjugate compositions following administration to a subject or contacting tumor tissue isolated from a subject, often the methods include intravenous administration of a chlorotoxin conjugate composition to a subject, *in vivo* contact of a tumor tissue or *ex vivo* contact of a tumor tissue from a subject comprises intravenously administering a dose of from 0.8 to 25 mg of the chlorotoxin polypeptide to the subject; producing in the subject an average chlorotoxin polypeptide plasma area under the curve (average AUC) of from 50 to 120 per each 1 mg dosage of chlorotoxin polypeptide administered; and producing in the subject an average maximum chlorotoxin peptide blood plasma concentration (average Cₘₐₓ) of at least from 110 to 240 per each 1 mg dosage of chlorotoxin peptide administered. In some aspects, the chlorotoxin polypeptide is conjugated to a fluorescent agent.

The disclosure further describes compositions of chlorotoxin conjugates as claimed, often the chlorotoxin conjugate composition comprises a physiologically effective amount of the chlorotoxin conjugate, wherein intravenous administration of the composition to a subject produces in the subject: an average chlorotoxin polypeptide plasma area under the curve (average AUC) of from 50 to 120 per each 1 mg dosage of chlorotoxin polypeptide administered; and an average maximum chlorotoxin peptide blood plasma concentration (average Cₘₐₓ) of at least from 110 to 240 per each 1 mg dosage of chlorotoxin peptide administered. In some aspects, the chlorotoxin polypeptide is conjugated to a fluorescent agent.

### Activity of Chlorotoxin Conjugates

The present disclosure provides, but is not limited to, methods for intraoperative imaging and resection of tumors with chlorotoxin conjugates detectable by fluorescence imaging that allows for intraoperative visualization of cancerous tissues, compositions that include the chlorotoxin conjugate, and methods for using the chlorotoxin conjugate. The chlorotoxin is a targeting agent that directs the conjugate to a tissue of interest. In one aspect, the chlorotoxin conjugate of the disclosure includes one or more labeling agents. In a further aspect, the labeling agent comprises a fluorescent moiety (e.g., red or near infrared emitting fluorescent moieties) covalently coupled to the chlorotoxin. In another aspect, the labeling agent comprises a radionuclide.

The chlorotoxin conjugates described herein are often used for detection and treatment of, for example imaging, resection of, diagnosis of and treatment of tumors. In some aspects, tumors amenable to detection with a chlorotoxin conjugate of the present disclosure include, but are not limited to: adenocarcinoma, fibrosarcoma, hemangiosarcoma, mastocytoma, squamous cell carcinoma, chondrosarcoma, adenosquamous carcinoma, hemangiopericytoma, follicular carcinoma, meningioma, mucosal squamous cell cancer, glioma, sarcomas, such as soft-tissue sarcomas or the like. Soft-tissue sarcomas amenable to detection with a chlorotoxin conjugate of the present disclosure include, but are not limited to: fat tissue tumors, liposarcomas, muscle tissue tumors including smooth muscle sarcomas and leiomyosarcomas, skeletal muscle sarcomas, rhabdomyosarcomas, peripheral nerve tumors, fibrous tissue tumors, myxofibrosarcomas, fibromatosis, joint tissue tumors, tumors of blood vessels and lymph vessels, angiosarcomas, tumors of peripheral nerves such as malignant peripheral nerve sheath tumors, malignant schwannomas, neurofibrosarcomas, fibrosarcomas, synovial sarcomas, malignant fibrous histiocytoma (MFH) hemangiosarcomas, lymphangiosarcomas, gastrointestinal stromal tumors, alveolar soft part sarcoma, dermatofibrosarcoma protuberans (DFSP), desmoplastic small round cell tumour, epithelioid sarcoma, extra skeletal myxoid chondrosarcoma, and giant cell fibroblastoma (GCF).

The chlorotoxin conjugates described herein can be used for detection and treatment of tumors present in any organ and in any anatomical location, including but not limited to, breast, lung, brain, colon, rectum, prostate, head, neck, stomach, anus, and/or vaginal tissues, for example. Tumors of any grade or stage known to one of skill in the art, including low-grade tumors, are often detected by the chlorotoxin conjugates described herein. In some aspects, tumor detection includes imaging, resection, diagnostics and treatment.

In certain aspects, the present compounds are capable of passing across the blood brain barrier. Passing across the blood brain barrier is advantageous when detecting or treating a cancer cell in the brain, such as for example, a glioma cell or a brain tumor.

In some aspects, the dose of chlorotoxin conjugate is administered such that a threshold amount of chlorotoxin conjugate is achieved in the subject. For example, the threshold amount often depends upon the patient's age, weight, height, sex, general medical condition and previous medical history. For another example, the threshold amount does not depend upon the patient's age, weight, height, sex, general medical condition and previous medical history.

Other dosage forms is devised by those skilled in the art, as shown, for example, by Ansel and Popovich, Pharmaceutical Dosage Forms and Drug Delivery Systems, 5th Edition (Lea & Febiger 1990), Gennaro (ed.), Remington's Pharmaceutical Sciences, 19th Edition (Mack Publishing Company 1995), and by Ranade and Hollinger, Drug Delivery Systems (CRC Press 1996).

As an illustration, pharmaceutical compositions are often supplied as a kit comprising a container that comprises a chlorotoxin conjugate. Chlorotoxin conjugates are often provided in the form of an injectable solution for single or multiple doses, or as a sterile powder that will be reconstituted before injection. Alternatively, such a kit often includes a dry-powder disperser, liquid aerosol generator, or nebulizer for administration of a therapeutic conjugate. Such a kit further comprises written information on indications and usage of the pharmaceutical composition.

### Methods of Tumor Prevention, Detection and Treatment

Subjects include, but are not limited to humans, non-human primates, monkeys, cows, dogs, rabbits, pigs, guinea pigs, rats, mice and zebrafish.

A sample includes any sample isolated from a subject, for example but not limited to, blood, serum, plasma, circulating cells, urine, saliva, and/or tissue removed from the body such as in a biopsy. Samples are often prepared using methods known to those of ordinary skill in the art, for example, blood samples are collected and incubated at room temperature for about 0.5 hours up to 2 hours prior to centrifugation, serum removal and storage at at least about 20 °C, but more often -70 °C.

Additional tests are often performed using samples from subjects, including complete blood counts, serum chemistry profiles and urinalysis.

The present disclosure provides a compound as claimed for use in methods for treating a disease or condition treatable by administering chlorotoxin. In one embodiment, the method includes administering an effective amount of a modified chlorotoxin peptide of the invention to a subject in need thereof.

The term "effective amount," as used herein, refers to a sufficient amount of an agent or a compound being administered which will relieve to some extent one or more of the symptoms of the disease or condition being treated. The result can be reduction and/or alleviation of the signs, symptoms, or causes of a disease, or any other desired alteration of a biological system. Compositions containing such agents or compounds can be administered for prophylactic, enhancing, and/or therapeutic treatments. An appropriate "effective" amount in any individual case may be determined using techniques, such as a dose escalation study.

In one embodiment, the invention provides a compound as claimed for use in a method for treating a cancer that expresses chlorotoxin binding sites in a patient, comprising administering to a patient in need thereof an effective amount of a chlorotoxin variant of the invention.

In one embodiment, the invention provides a compound as claimed for use in a method for treating a cancer that expresses chlorotoxin binding sites, comprising administering to a patient in need thereof an effective amount of a pharmaceutical composition comprising a chlorotoxin variant of the invention and a pharmaceutically acceptable carrier.

In one embodiment, the invention provides a compound as claimed for use in a method for treating a tumor expressing chlorotoxin binding sites, comprising administering to a patient in need thereof an effective amount of a chlorotoxin variant of the invention.

In one embodiment, the invention provides a compound as claimed for use in a method for inhibiting invasive activity of cells that express chlorotoxin binding sites, comprising administering an effective amount of a chlorotoxin variant to cells that express chlorotoxin binding sites

The methods of treatment of the invention are applicable to human and animal subjects in need of such treatment.

Virtually every type of malignant cancer expressing chlorotoxin binding sites can be treated by the chlorotoxin variants and conjugates of the invention. These malignant cancers include gliomas, astrocytomas, medulloblastomas, choroid plexus carcinomas, ependymomas, meningioma, glioblastoma, ganglioma, pheochromocytoma, and metastatic brain tumors, other brain tumors, neuroblastoma, head and neck cancer, non-small cell lung cancer, small cell lung cancer, breast cancer, intestinal cancer, pancreatic cancer, colon cancer, liver cancer, kidney cancer, skin cancer, sarcomas (over 30 types), osteosarcoma, rhabdomyosarcoma, Ewing's sarcoma, carcinomas, melanomas, ovarian cancer, cervical cancer, lymphoma, thyroid cancer, anal cancer, colo-rectal cancer, endometrial cancer, germ cell tumors, laryngeal cancer, multiple myeloma, prostate cancer, retinoblastoma, gastric cancer, testicular cancer, and Wilm's tumor.

In certain aspects, the chlorotoxin conjugate is administered to an individual having or suspected of having a tumor, such that the conjugate binds specifically to the tumor. Such methods are useful in reducing the likelihood that the individual will develop a tumor, that one or more tumors in the individual will increase in size, that one or more tumors in the individual will metastasize, and/or that the cancer will progress by some other measure. As used herein, the term "metastasis" refers to the spread of tumor cells from one organ or tissue to another location, and also refers to tumor tissue that forms in a new location as a result of metastasis.

In some aspects, the chlorotoxin conjugate is useful for the treatment and/or diagnosis of neuroectodermal tumors such as gliomas, medulloblastomas, neuroblastomas, pheochromocytomas, melanomas, peripheral primitive neuroectodermal tumors, small cell carcinoma of the lung, Ewing's sarcoma, and metastatic tumors in the brain. In some aspects, the chlorotoxin conjugate is useful for the treatment and/or diagnosis of brain tumors, including but not limited to, glioma, including glioblastoma multiforme, anaplastic astrocytomas, low grade gliomas, pliocytic astrocytomas, oligodendrogliomas, gangliomas, meningiomas, and ependymomas.

In other aspects, the compounds of the present disclosure are used to detect and/or treat soft-tissue sarcomas. Soft-tissue sarcomas are a group of malignant tumors that form in fat, muscles, nerves, joints, and blood vessels. In 2012, it was estimated that approximately 11,280 Americans would be diagnosed with soft-tissue sarcomas and approximately 3,900 would be expected to die from soft-tissue sarcomas. Soft tissue and bone sarcoma incidence rates have increased slightly over the past 30 years; however, soft-tissue sarcoma is more deadly, possibly because the lack of specific symptoms at early disease stages may lead to delays in diagnosis.

Moreover, certain inherited disorders and past treatment with radiation therapy can increase the risk of soft-tissue sarcoma. No modifiable risk factors for sarcoma have been identified. Standard treatments for soft-tissue sarcoma include surgery, chemotherapy, and radiation therapy.

Since symptoms of soft-tissue sarcomas often do not appear until the disease is advanced, only about 50% of soft-tissue sarcomas are found in the early stages, before they have spread.

The present invention provides such methods of detection, imaging, visualization, analysis and treatment for these and other uses that should be apparent to those skilled in the art from the teachings herein.

The present invention is based in part upon the identification by the inventors that soft-tissue sarcomas have a high level of uptake of the conjugate compared to other tumors or normal tissues and are particularly well-suited for detection by means of administering a chlorotoxin conjugated to a labeling agent for detection, visualization, imaging, or analysis. Such visualization can be during or related to surgical (intraoperative) resection or during or related to initial identification of the sarcoma or during or related to monitoring of the sarcoma relevant to treatment.

Real-time intraoperative visualization of solid tumors enables more complete resection while sparing surrounding normal tissue. Improvement in intraoperative tumor visualization would be of benefit for any resectable solid tumor, as it would enable surgeons to better determine the extent of local invasion as well as the presence of metastatic spread in nearby lymph nodes and fatty tissue. This kind of information would be helpful in making surgical decisions, for example, decisions regarding which patients would respond well to limb-sparing approaches in the treatment of sarcomas.

For brain tumors this is of paramount importance, since removal of additional tissue can unnecessarily increase cognitive and functional impairment, yet being too conservative in the amount of resection may leave tumor tissue behind.

Surgeons who specialize in human breast cancer surgery have indicated that precise margins are less important in this indication, and the surgical approach is generally a wide excision with 0.2 - 1 cm margins on all sides. It is difficult for surgeons to obtain wide margins using only white light and preoperative imaging information. In 20-50% of breast cancer surgeries, failure to obtain clean margins leads to second surgeries.

The present invention shows that soft-tissue sarcomas are particularly identifiable when bound by a chlorotoxin conjugate, which can be effectively used to detect these sarcomas, especially during or related to surgery and intraoperative resection. For example, the chlorotoxin conjugate can be used alone or on combination with other detection agents, to detect, image, visualize, or analyze the tumor in advance of, during, or following anti-tumor treatments, which can include surgery and surgical resection, chemotherapy, radiation therapy, and immunotherapy. In addition, the chlorotoxin conjugate can be used alone or with other detection agents for follow-up monitoring post treatment as well as for general monitoring for full-body screening.

Low-grade tumors generally tend to be slow growing, slower to spread, and often have better prognosis than higher-grade tumors, making them more curable with surgical resection than high-grade tumors, which may need more systemic treatment. The inventors show that use of a chlorotoxin conjugated to a labeling agent can be particularly effective in detection, imaging, visualization, or analysis of low-grade tumors, such as meningiomas, allowing their complete resection before they metastasize or spread.

Intraoperative resection of tumor types may vary depending on the anatomic location and type of tumor. For example, when a tumor is located in brain tissue, the surgeon is likely to require perfect or near perfect specificity between a tumor imaging or detection agent and the tumor tissue so that only diseased tissue is resected. On the other hand, when the tumor is located in a tissue where wider margins are generally resected, such as breast or mammary cancer or colon cancer, or in cancers where the tumor is likely to spread locally, such as squamous cell carcinoma, it would be advantageous for a surgeon to be able to use a tumor imaging or detection agent to identify peritumoral tissue that is likely to become tumor tissue.

Soft-tissue sarcomas can develop from soft tissues like fat, muscle, nerves, fibrous tissues, blood vessels, or deep skin tissues. They can be found in any part of the body. Most of them develop in the arms or legs. They can also be found in the trunk, head and neck area, internal organs, and the area in back of the abdominal cavity (known as the retroperitoneum).

Soft-tissue sarcomas that may be amenable to detection with a chlorotoxin conjugate of the present invention include, but are not limited to: fat tissue tumors, muscle tissue tumors, skeletal muscle sarcomas, rhabdomyosarcomas, peripheral nerve tumors, fibrous tissue tumors, myxofibrosarcomas, fibromatosis, joint tissue tumors, tumors of blood vessels and lymph vessels, angiosarcomas, gastrointestinal stromal tumors, alveolar soft part sarcoma, dermatofibrosarcoma protuberans (DFSP), desmoplastic small round cell tumour, epithelioid sarcoma, extra skeletal myxoid chondrosarcoma, and giant cell fibroblastoma (GCF).

Sarcomas that start in the body's fat cells are called liposarcomas. They can grow anywhere in the body and most commonly affect people aged 50-65 years. Some grow very slowly, taking many years to develop, whereas others grow more quickly.

Muscle tissue sarcomas include smooth muscle sarcomas and skeletal muscle sarcomas. Smooth muscle forms the walls of internal organs such as the stomach, intestine, womb (uterus), and blood vessels. The muscle causes these organs to contract, which happens without our control. Smooth muscle is also called involuntary muscle. Sarcomas that develop in smooth muscle are called leiomyosarcomas. They are one of the more common types of sarcoma and can occur anywhere in the body, especially in the back of the abdominal area (retroperitoneum). Leiomyosarcomas are less often found in the deep, soft tissues of the legs or arms. They tend to occur in adults, particularly in the elderly. Skeletal muscles are the active muscles in our arms and legs or other parts of the body that we control. They are voluntary muscles and sometimes called striated muscles because the cells look stripy when examined under a microscope.

Sarcomas that grow in the voluntary muscles of the body are called rhabdomyosarcomas. They are found mostly in the head and neck, but also in organs such as the bladder, vagina and the arms or legs. Rhabdomyosarcomas are more commonly diagnosed in children than in adults.

Peripheral nerve tumors can be found in the peripheral nervous system, which consists of all the nerves that run throughout the body. Sarcomas of the peripheral nerves develop in the cells that cover the nerves. They're known as malignant peripheral nerve sheath tumors (MPNST) and can occur anywhere in the body. There are different types of MPNSTs, including malignant schwannomas and neurofibrosarcomas. They most commonly occur in people who have a rare genetic disorder called neurofibromatosis (von Recklinghausen's disease).

Fibrous tissue tumors occur in tissues that join muscles to bones. This tissue is made up of cells called fibrocytes. A sarcoma of the fibrous tissue is called a fibrosarcoma. They are most commonly found on the arms, legs or trunk, but can occur deeper in the body. They can occur at any age but are more commonly seen in people aged 20-60 years. Most people first notice them as a painless, firm lump.

Soft-tissue sarcomas that develop very close to the body's joints are known as synovial sarcomas. They commonly develop near, but not inside, joints such as the knee or elbow, but they can occur in any part of the body. They usually appear as hard lumps and are more common in children and young adults.

Blood and lymph vessel tumors include sarcomas that start from the cells that make up the walls of blood or lymph vessels and are called angiosarcomas. Haemangiosarcomas develop from blood vessels and lymphangiosarcomas develop from the lymph vessels.

Angiosarcomas are sarcomas that sometimes occur in a part of the body that has been treated with radiotherapy many years before.

Gastrointestinal stromal tumors (GIST) are soft-tissue sarcomas that develop in nerve cells in the walls of the digestive system.

The inventors have also identified that low-grade tumors can be detected with a chlorotoxin conjugated to a labeling agent. This is particularly useful since low-grade tumors have a better prognosis if they can be fully resected.

In addition, the invention is based in part on the identification by the inventors of an optimal dose for tumor imaging in dogs of at least about 0.8 mg/m². One skilled in the art will recognize that dosage for the chlorotoxin conjugate will be determined based on the amount of conjugate administered and the amount of time after administration after which the imaging is performed. In some aspects the optimal dose for tumor imaging is in the range of about 0.85 mg/m² to about 1.2 mg/m², or in the range of about 0.9 to about 1.1 mg/m². At doses up to 0.9 mg/m², signal in gross tumor samples increases as a function of dose. At doses above 0.9 mg/m², the correlation between signal and dose is lost, suggesting that in this model system the signal in tumor is maximal above this dose. However, it is recognized that in other cases more conjugate can be administered with acceptable imaging. Thus, in some aspects, the amount of chlorotoxin conjugate that can be administered can be in the range of about 1.3 mg/m² to about 2.5 mg/m², or in the range of about 2.6 mg/m² to about 3.5 mg/m², or in the range of about 3.6 mg/m² to about 4.5 mg/m², or in the range of about 4.6 mg/m² to about 5.5 mg/m², or upwards of 5.5 mg/m².

### Imaging Methods

In a further aspect of the invention, methods of using the chlorotoxin conjugates are provided. In one embodiment, the invention provides a method for imaging a tissue imagable by chlorotoxin. In the method, a tissue imagable by chlorotoxin is contacted with a chlorotoxin conjugate. In one embodiment, the imaging method is a fluorescence imaging method. Representative methods for making and using fluorescent chlorotoxin conjugates are described in U.S. Patent Application Publication No. 20080279780 Al, Fluorescent Chlorotoxin Conjugate and Method for Intra-Operative Visualization of Cancer, and in U.S. Patent Application Publication No. 20130195760, Chlorotoxin Variants, Conjugates, And Methods For Their Use.

In many cases, chlorotoxin conjugates can be administered to human and animal subjects, such as with a pharmaceutically acceptable carrier. In some aspects, the composition includes a pharmacologically effective amount of a modified chlorotoxin conjugate. An effective amount can be routinely determined by established procedures. An effective amount is an amount sufficient to occupy chlorotoxin binding sites in cancer cells, but low enough to minimize non-specific binding to non-neoplastic tissues. An effective amount optimizes signal-to-noise ratio for intra-operative imaging.

The disclosure provides methods for detecting a tissue using the chlorotoxin conjugates. The chlorotoxin conjugates of the invention target and are bound by chlorotoxin binding sites. It will be appreciated that chlorotoxin binding sites may take two forms: sites that bind chlorotoxin and sites that bind the chlorotoxin conjugates of the invention. It will be appreciated that chlorotoxin binding sites may be distinct from chlorotoxin conjugate binding sites.

In some aspects, a method for differentiating foci of cancers that express chlorotoxin binding sites from non-neoplastic tissue is provided. The method includes contacting a tissue of interest with a chlorotoxin conjugate having affinity and specificity for cells that express chlorotoxin binding sites, wherein the chlorotoxin conjugate comprises one or more red or near infrared emitting fluorescent moieties covalently coupled to a chlorotoxin, and measuring the level of binding of the chlorotoxin conjugate, wherein an elevated level of binding, relative to normal tissue, is indicative that the tissue is neoplastic.

In some aspects, a method for detecting cancers that express chlorotoxin binding sites is provided. The method includes the steps of contacting a tissue of interest with a chlorotoxin conjugate having affinity and specificity for cells that express chlorotoxin binding sites, wherein the chlorotoxin conjugate comprises one or more red or near infrared emitting fluorescent moieties covalently coupled to a chlorotoxin, and measuring the level of binding of the chlorotoxin conjugate, wherein an elevated level of binding, relative to normal tissue, is indicative that the tissue is neoplastic.

In some aspects, a compound as claimed for use in a method for determining the location of cancer cells that express chlorotoxin binding sites in a patient intra-operatively is provided. The method includes the steps of administering a pharmaceutical composition to a patient, wherein the pharmaceutical composition comprises a pharmaceutically acceptable carrier and an amount of a chlorotoxin conjugate sufficient to image cancer cells that express chlorotoxin binding sites *in vivo,* wherein the chlorotoxin conjugate comprises one or more red or near infrared emitting fluorescent moieties covalently coupled to a chlorotoxin, measuring the level of binding of the chlorotoxin conjugate by fluorescence imaging to determine the location of cancer cells that express chlorotoxin binding sites, wherein an elevated level of binding, relative to normal tissue, is indicative of the presence of cancer cells that express chlorotoxin binding sites; and surgically removing from the patient at least some cells that express chlorotoxin binding sites located by fluorescence imaging.

Imaging methods for detection of cancer foci disclosed herein are applicable to mouse and other animal models of cancer as well as to veterinary practice.

The present invention provides a compound as claimed for use in methods for intraoperative imaging and resection of tumors with chlorotoxin conjugates detectable by fluorescence imaging that allows for intraoperative visualization of cancerous tissues, compositions that include the chlorotoxin conjugate, and methods for using the chlorotoxin conjugate. The chlorotoxin is a targeting agent that directs the conjugate to a tissue of interest. In one embodiment, the chlorotoxin conjugate of the invention includes one or more labeling agents. In a further embodiment, the labeling agent comprises a fluorescent moiety (e.g., red or near infrared emitting fluorescent moieties) covalently coupled to the chlorotoxin. In another embodiment, the labeling agent comprises a radionuclide.

As used herein, the term "red or near infrared emitting fluorescent moiety" refers to a fluorescent moiety having a fluorescence emission maximum greater than about 600 nm.

In certain embodiments of the chlorotoxin conjugate, the fluorescent moieties are derived from fluorescent compounds characterized by emission wavelength maxima greater than about 600 nm to avoid autofluorescence, emission that travels through millimeters to one centimeter of tissue/blood/fluids, emission that is not absorbed by hemoglobin, other blood components, or proteins in human or animal tissue. In some aspects, the emission wavelength maximum is greater than 600 nm, greater than 650 nm, greater than 700 nm, greater than 750 nm, greater than 800 nm, greater than 850 nm, greater than 900 nm, or greater than 950 nm.

The fluorescent moiety is covalently coupled to the chlorotoxin to allow for the visualization of the conjugate by fluorescence imaging. The fluorescent moiety is derived from a fluorescent compound. Suitable fluorescent compounds are those that can be covalently coupled to a chlorotoxin without substantially adversely affecting the targeting and binding function of the chlorotoxin conjugate. Similarly, suitable fluorescent compounds retain their fluorescent properties after conjugation to the chlorotoxin.

Generally, the dosage of administered chlorotoxin conjugates may vary depending upon such factors as the patient's age, weight, height, sex, general medical condition and previous medical history. Typically, it is desirable to provide the recipient with a dosage of chlorotoxin conjugated to a chemotherapeutic, an anti-cancer agent, or an anti-cancer drug that is effective to achieve inhibition, shrinkage, killing, minimization, or prevention of metastasis. In many cases, it is desirable to provide the recipient with a dosage of a chlorotoxin conjugate that is in the range of from about 3 mg to about 6 mg, although a lower or higher dosage also may be administered as circumstances dictate.

Administration of a chlorotoxin conjugate to a subject can be topical, inhalant, intravenous, intraarterial, intraperitoneal, intramuscular, subcutaneous, intrapleural, intrathecal, by perfusion through a regional catheter, or by direct intralesional injection. When administering conjugates by injection, the administration may be by continuous infusion or by single or multiple boluses.

Additional routes of administration include oral, mucosal-membrane, pulmonary, and transcutaneous. Oral delivery is suitable for polyester microspheres, zein microspheres, proteinoid microspheres, polycyanoacrylate microspheres, and lipid-based systems (see, for example, DiBase and Morrel, "Oral Delivery of Microencapsulated Proteins," in Protein Delivery: Physical Systems, Sanders and Hendren (eds.), pages 255-288 (Plenum Press 1997)). The feasibility of an intranasal delivery is exemplified by such a mode of insulin administration (see, for example, Hinchcliffe and Ilium, Adv. Drug Deliv. Rev. 35:199 (1999)). Dry or liquid particles comprising a chlorotoxin conjugate can be prepared and inhaled with the aid of dry-powder dispersers, liquid aerosol generators, or nebulizers (e.g., Pettit and Gombotz, TIBTECH 16:343 (1998); Patton et al., Adv. Drug Deliv. Rev. 35:235 (1999)). This approach is illustrated by the AERX diabetes management system, which is a hand-held electronic inhaler that delivers aerosolized insulin into the lungs. Transdermal delivery using electroporation provides another means to administer a chlorotoxin conjugate.

A pharmaceutical composition comprising a chlorotoxin conjugate can be formulated according to known methods to prepare pharmaceutically useful compositions, whereby the conjugate is combined with a pharmaceutically acceptable carrier. A composition is said to be a "pharmaceutically acceptable carrier" if its administration can be tolerated by a recipient patient. Sterile phosphate-buffered saline is one example of a pharmaceutically acceptable carrier. Other suitable carriers are well-known to those in the art. See, for example, Gennaro (ed.), Remington's Pharmaceutical Sciences, 19th Edition (Mack Publishing Company 1995).

A pharmaceutical composition comprising a chlorotoxin conjugate can be furnished in liquid form, in an aerosol, or in solid form. Liquid forms, are illustrated by injectable solutions, aerosols, droplets, topological solutions and oral suspensions. Exemplary solid forms include capsules, tablets, and controlled-release forms. The latter form is illustrated by miniosmotic pumps and implants (Bremer et al., Pharm. Biotechnol. 10:239 (1997); Ranade, "Implants in Drug Delivery," in Drug Delivery Systems, Ranade and Hollinger (eds.), pages 95-123 (CRC Press 1995); Bremer et al., "Protein Delivery with Infusion Pumps," in Protein Delivery: Physical Systems, Sanders and Hendren (eds.), pages 239-254 (Plenum Press 1997); Yewey et al., "Delivery of Proteins from a Controlled Release Injectable Implant," in Protein Delivery Physical Systems, Sanders and Hendren (eds.), pages 93-117 (Plenum Press 1997)). Other solid forms include creams, pastes, other topological applications, and the like.

Other dosage forms can be devised by those skilled in the art, as shown, for example, by Ansel and Popovich, Pharmaceutical Dosage Forms and Drug Delivery Systems, 5.sup.th Edition (Lea & Febiger 1990), Gennaro (ed.), Remington's Pharmaceutical Sciences, 19.sup.th Edition (Mack Publishing Company 1995), and by Ranade and Hollinger, Drug Delivery Systems (CRC Press 1996).

As an illustration, pharmaceutical compositions may be supplied as a kit comprising a container that comprises a chlorotoxin conjugate. Therapeutic conjugates can be provided in the form of an injectable solution for single or multiple doses, or as a sterile powder that will be reconstituted before injection. Alternatively, such a kit can include a dry-powder disperser, liquid aerosol generator, or nebulizer for administration of a therapeutic conjugate. Such a kit may further comprise written information on indications and usage of the pharmaceutical composition.

The present disclosure is not to be limited in scope by the specific embodiments described herein. Indeed, various modifications of the invention in addition to those described herein will become apparent to those skilled in the art from the foregoing description and accompanying figures.

All features discussed in connection with any aspect or embodiment herein can be readily adapted for use in other aspects and embodiments herein. The use of different terms or reference numerals for similar features in different embodiments does not necessarily imply differences other than those expressly set forth. Accordingly, the present invention is intended to be described solely by reference to the appended claims, and not limited to the embodiments disclosed herein.

Unless otherwise specified, the presently described methods and processes can be performed in any order. For example, a method describing steps (a), (b), and (c) can be performed with step (a) first, followed by step (b), and then step (c). Or, the method can be performed in a different order such as, for example, with step (b) first followed by step (c) and then step (a). Furthermore, those steps can be performed simultaneously or separately unless otherwise specified with particularity.

The particulars shown herein are by way of example and for purposes of illustrative discussion of the preferred embodiments of the present invention only and are presented in the cause of providing what is believed to be the most useful and readily understood description of the principles and conceptual aspects of various embodiments of the invention. In this regard, no attempt is made to show structural details of the invention in more detail than is necessary for the fundamental understanding of the invention, the description taken with the drawings and/or examples making apparent to those skilled in the art how the several forms of the invention may be embodied in practice.

Where a range of values is provided, it is understood that each intervening value, to the tenth of the unit of the lower limit unless the context clearly dictates otherwise, between the upper and lower limit of that range, and any other stated or intervening value in that stated range, is encompassed within the disclosure provided herein. The upper and lower limits of these smaller ranges may independently be included in the smaller ranges, and are also encompassed within the invention, subject to any specifically excluded limit in the stated range. Where the stated range includes one or both of the limits, ranges excluding either or both of those included limits are also included in the disclosure provided herein.

All features discussed in connection with an aspect or embodiment herein can be readily adapted for use in other aspects and embodiments herein. The use of different terms or reference numerals for similar features in different embodiments does not necessarily imply differences other than those expressly set forth. Accordingly, the present invention is intended to be described solely by reference to the appended claims, and not limited to the embodiments disclosed herein.

It is intended that the following claims define the scope of the invention and that methods and structures within the scope of these claims be covered thereby.

### EXAMPLES

The invention is further illustrated by the following non-limiting examples.

### EXAMPLE 1

### Stability of Compound 16 with Ammonium Acetate Salt

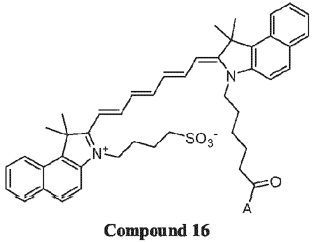

A = MCMPCFTTDHQMARRCDDCCGGRGRGKCYGPQCLCR (K-27 is point of attachment)

This example demonstrates the production and stability of Compound 16 under various pH and temperature storage conditions over time.

The peptide portion of Compound 16 is a targeting peptide (modified chlorotoxin) conjugated to the fluorescent dye. The targeting peptide binds selectively to cancerous cells and the dye portion facilitates detection via imaging. The peptide is a 36 amino acid modified chlorotoxin (having a sequence of H-Met-Cys-Met-Pro-Cys-Phe-Thr-Thr-Asp-His-Gln-Met-Ala-Arg-Arg-Cys-Asp-Asp-Cys-Cys-Gly-Gly-Arg-Gly-Arg-Gly-Lys-Cys-Tyr-Gly-Pro-Gln-Cys-Leu-Cys-Arg-OH) wherein two of three lysine amino acids in native chlorotoxin are substituted with arginine (K15R and K23R) to facilitate the subsequent conjugation with a fluorophore to the single remaining lysine (K27) residue resulting in a mono-labeled fluorescent active pharmaceutical ingredient.

**Methods and Results:** Product solutions were stored at ≤ 8 °C during manufacturing. All analysis utilized the in-process HPLC method PR to determine the stability of the product in the different buffers.

Dye Conjugation: 500 mg of the peptide portion of Compound 16:(TFA salt), (net peptide content 76.7, net peptide 384 mg, 0.095mmol) were dissolved in a solution of sodium bicarbonate. DMSO and 140 mg of ICG-Sulfo-ATT dissolved in dried DMSO (net dye 126 mg 0.152mmol, 1.6 eq. based on a product activity of 90%) were added, resulting in a final reaction volume of approximately 200mL. The reaction was followed by RP-HPLC and considered completed after 3 hours, with ~ 3.4% un-reacted Compound 16 remaining.

Ammonium Bicarbonate Purification: The reaction solution was filtered and then diluted with 400mL of water. The solution was loaded on an RP-HPLC column equilibrated with 0.1M ammonium bicarbonate, and the product recovered by applying a linear gradient of acetonitrile.

The product eluted as a single peak. Fractions were collected and analyzed by analytical HPLC. During analysis the fractions were stored at ≤ 8°C protected from light.

The purity, estimated concentration, and estimated amount of product in each fraction are reported in Table 15. The estimation was based on the peak area observed during release analysis of a solution of the product at a concentration of 1 mg/mL in water.

**Table 15: Ammonium Bicarbonate Purification Part I**

| **Fraction Number** | **Purity (%)** | **Concentration ( mg/mL)** | **Amount ( mg)** |
|---|---|---|---|
| 6-1-3 | 5.48 | 0.0025 | 0.1 |
| 6-1-4 | 73.36 | 0.0970 | 4.8 |
| 6-1-5 | 98.67 | 1.2638 | 63.2 |
| 6-1-6 | 98.96 | 3.0056 | 150.3 |
| 6-1-7 | 95.59 | 2.0464 | 102.3 |
| 6-1-8 | 92.16 | 1.0622 | 53.1 |
| 6-1-9 | 81.87 | 0.3959 | 19.8 |
| 6-1-10 | 66.56 | 0.0703 | 3.5 |
| Total product recovery in fractions 3 to 10 | | | 303 |
| Product recovery in main pool (fractions 5 to 8) | | | 282 |

The main pool (fractions 6-1-5 to 6-1-8, purity 97%) was combined and transferred to the salt exchange step. Samples of the main pool were analyzed for a period of 5 day of storage protected from light at ≤ 8°C. The material was stable under these conditions.

Ammonium Acetate Salt Exchange: The primary purification main pool solution (~ 200mL) was diluted with 100mL of water and loaded on an RP-HPLC column equilibrated with 0.1M ammonium bicarbonate. Following loading of the sample, the column was equilibrated with 4 bed volumes of 0.1M ammonium acetate adjusted to pH 7.6 with ammonium hydroxide. Finally the column was equilibrated with 0.01M ammonium acetate pH 7.6 and the product was recovered by applying a linear gradient of 0.01M ammonium acetate pH 7.6 in 75% Acetonitrile.

The product eluted as a single peak at approximately 39% acetonitrile concentration. The fractions were collected and analyzed by analytical HPLC. During analysis, the fractions were stored at < 8°C protected from light.

The purity, estimated concentration, and estimated amount of product in each fraction are reported in Table 16. The estimation was based on the peak area observed during release analysis of a solution of the product at a concentration of 1 mg/mL in water.

**Table 16: Ammonium Bicarbonate Purification Part II**

| **Fraction Number** | **Purity (%)** | **Concentration (mg/mL)** | **Amount ( mg)** |
|---|---|---|---|
| 7-1-1 | 79.1 | 0.020084 | 1.0 |
| 7-1-2 | 98.6 | 1.115285 | 55.8 |
| 7-1-3 | 99.0 | 1.804414 | 90.2 |
| 7-1-4 | 98.1 | 1.198464 | 59.9 |
| 7-1-5 | 94.8 | 0.425017 | 21.3 |
| 7-1-6 | 84.2 | 0.101113 | 5.1 |
| 7-1-7 | 65.4 | 0.037211 | 1.9 |
| 7-1-8 | 55.5 | 0.021173 | 1.1 |
| Total product recovery in fractions 3 to 10 | | | 236 |
| Product recovery in main pool (fractions 5 to 8) | | | 232 |

The main pool (fractions 7-1-2 to 7-1-6, purity 97.2%) were combined and transferred to lyophilization. Samples of the main pool were analyzed for a period of 5 day of storage protected from light at ≤ 8°C. The material was stable under these conditions. Dilution with a volume equal to approximately half the main pool volume provided a stable solution without the presence of a precipitate. This dilution was utilized at the preparative scale.

Lyophilization: The main pool at stage 7 was diluted with 100mL of water and lyophilized over a period of 4 days using a bottle lyophilizer. There was no problem with solubility after dilution as had been observed with the ammonium bicarbonate main pool; a sample was diluted to 15% acetonitrile without any observed precipitation. During lyophilization, the product formed a stable self-supporting cake.

Reconstitution: The product was readily soluble in water at 1, 5 and 10 mg/mL. Water was selected as the reconstitution solution. Additionally, an LC-MS analysis was performed on the final material.

A sample of the main pool of Stage 6 (ammonium bicarbonate purification) was stored without dilution at ≤ 8°C for 5 days protected from light. The sample was analyzed daily. Results are reported in Table 17. The results indicate a low level of instability.

**Table 17: Stage 6 Main Pool Stability**

| **Time point (hour)** | **Purity (%)** |
|---|---|
| 0 | 96.6 |
| 26 | 96.4 |
| 48 | 96.4 |
| 72 | 96.3 |
| 105 | 95.7 |
| 191 | 95.4 |

A sample of the main pool of Stage 7 (ammonium acetate purification) was stored without dilution at ≤ 8°C for 5 days protected from light. The sample was analyzed daily. Results are reported in Table 18. The results indicate a low level of instability.

**Table 18: Stage 7 Main Pool Stability**

| **Time point (hour)** | **Purity (%)** |
|---|---|
| Zero | 97.8 |
| 26 | 97.6 |
| 48 | 97.4 |
| 79 | 97.1 |
| 91 | 96.8 |
| 610 | 96.4 |

An optimized and scalable conjugation procedure for production of Compound 16 for GMP manufacturing was developed. Two different dye reagents were evaluated: ICG-Sulfo-NHS ester and ICG-Sulfo-ATT (both resulting in Compound 16). Design of Experiments (DOE) studies was prepared.

After conjugation of Compound 16, the crude peptide conjugate was purified by RP-HPLC with a TFA and acetonitrile gradient elution. Fractions were characterized for purity with a RP-HPLC assay, pooled and desalted into acetate by HPLC prior to lyophilization to bulk conjugate product.

Compound 16 was stable for up to 14 days at pH 7.5 and 8.5 when stored in the dark at 4°C in 10mM Tris, 5% Dextrose and is sensitive to low pH and temperatures above 4°C.

### EXAMPLE 2

### Evaluation of the Stability of Compound 16 in Various Buffers and at Various pHs

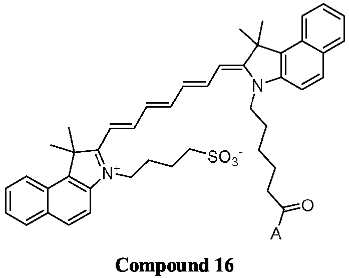

A = MCMPCFTTDHQMARRCDDCCGGRGRGKCYGPQCLCR (K-27 is point of attachment)

This example shows the stability of Compound 16 with respect to pH and provides an evaluation of the use of alternative buffers and various excipients and configurations.

This example further shows that various classes of excipients protect Compound 16 from thermal, photo, oxidative and freeze/thaw stress.

In some cases, formulations were stored in microcentrifuge tubes with an air atmosphere and assayed after 2d and 5d at 40°C in the dark, after 3d at room remperature (rt) in the dark (dk) or in ambient light (lt), and after 3X freeze thaw (F/T) between -20°C and room temperature in the dark. Formulations were evaluated by visual examination, centrifugation, RP-HPLC, concentration by A₇₈₆, pH, and SDS-PAGE.

In some cases, the timepoints were 4, 7, and 14 days at room temperature with evaluation by visual examination, centrifugation, RP-HPLC, concentration by A₇₈₆, pH, and SDS-PAGE. Where extra material remained, additional stressing conditions were evaluated for further information. The formulations were contained in microcentrifugation tubes in an air environment.

Methods: The following stock solutions were prepared and passed through a 0.2 µm filter (Table 19). Dialysis buffers were prepared immediately before use by combining a stock from Table 19 with sparged water (sparged by bubbling with argon for 15 min - 3h).

**Table 19: Stock Buffer Solutions**

| **Buffer** | **Molarity** | **pH** |
|---|---|---|
| **Tris** | 0.5 | 7.0, 7.5, 8.0, 8.5 |
| **His** | 0.25 | 5.5, 6.5, 7.0 |
| **maleic acid** | 0.5 | 7.0 |
| **HEPES** | 0.5 | 7.0 |
| **EDA** | 0.5 | 7.0 |
| **acetic** | 0.5 | 4.5 |

Compound 16 was prepared at 3 mg/mL in water (see concentration by A₇₈₆ below). Slide-a-lyzer cassettes were pre-soaked in water, then the Compound 16 was dispensed into the cassettes (~11 mL for formulation 1, ~5 mL for formulations 2-6, ~2.7 mL for formulations 7-12). The cassettes were placed into beakers containing 550 mL of the corresponding formulation buffer. Samples were formulated by dialyzing with stirring in the dark at room temperature (on a multiposition stir plate, covered with a box covered with aluminum foil). Dialysis buffer was changed after 1 h, again after 1.25 h, and allowed to continue overnight.

For some formulations, the formulation was placed in a fresh tube and combined with additional sterile 0.25M or 0.5M buffer stock to yield a new formulation with 30 mM concentration of buffer. All formulations were then dispensed into 1.5mL microcentrifuge tubes, 0.5mL per tube.

The designed stability timepoints were 4, 7, and 14 days at room temperature. Where extra material remained, additional testing was performed. One tube of each formulation was assayed immediately ("t0"). For additional information, remaining material from these t0 tubes was then frozen at -20°C, then thawed at 5 °C, and assayed for pH and concentration ("1x F/T"). Other tubes were incubated in the dark at room temperature (approximately 22-23°C). A tube was removed and assayed at 4, 7, and 14d room temperature (rt) ("4d rt", "7d rt", "14d rt"). For additional information, after the 7d room temperature sample was assayed, the remaining material was placed at room temperature in the light for one day and then assayed again ("7+1d light"). These tubes were placed on their sides on the benchtop. Stability samples were assayed by visual inspection, centrifugation, RP-HPLC, A₇₈₆, pH, and, in some cases, SDS-PAGE.

Samples were examined visually for clarity, color, and visible particulates. They were then mixed, withdrawn into glass Pasteur pipets, and examined again. Samples were centrifuged ~10,000 rpm for ~2 min. Samples were then examined visually for any visible pellet.

Samples were analyzed by RP-HPLC using the Zorbax_10 method.

**Table 20: Tested Formulations**

| # | **Composition** | **pH** |
|---|---|---|
| 1,(1B) | 10, (30) mM Tris | 7.0 |
| 2,(2B) | 10, (30) mM His | 7.0 |
| 3 | 10 mM maleic acid | 7.0 |
| 4, (4B) | 10, (30) mM EDA | 7.0 |
| 6, (6B) | 10, (30) mM HEPES | 7.0 |
| 7 | 10 mM acetic | 4.5 |
| 8 | 10 mM His | 5.5 |
| 9 | 10 mM His | 6.5 |
| 10 | 10 mM Tris | 7.5 |
| 11 | 10 mM Tris | 8.0 |
| 12 | 10 mM Tris | 8.5 |

100µl of sample was placed in an HPLC vial insert, placed in an amber HPLC vial, and held at 2-8 °C before the 2 µl injection. The formulation buffer was injected twice at the beginning of each run, and a matched formulation buffer blank was run each time before injection of a sample in a new buffer.

The spectrophotometer was blanked with matching formulation buffer. Concentration was calculated using the extinction coefficient. The pH was measured using a calibrated micro pH electrode.

Samples were prepared per the manufacturer's recommendations and then heated at 70 °C for 10 min. 2 µg in 10 µL was loaded onto the SDS-PAGE gels and then run at 200V for approximately 35 min. After electrophoresis, the gels were washed in water for 5 min at room temperature, 3 times. Then they were stained at room temperature for 1.5 h, destained in water overnight, and then destained again with water and imaged on the same day.

Results. Formulations were produced by dialyzing a stock of Compound 16 at 3 mg/mL in water into various buffers. The formulations were then dispensed into microcentrifuge tubes, in air, and stored in the dark at room temperature for up to 14 days. The results from visual examination of the formulations are given in Table 21.

**Table 21: Visual Inspection. C = clear; G = emerald green; N = essentially no visible particles; F = very few particles or fibers visible; nt = not tested.**

| **Formulation** | | | **Visual** | | | | |
|---|---|---|---|---|---|---|---|
| | | | **Days at rt** | | | | |
| **#** | **Composition** | **pH** | **0** | **4** | **7** | **14** | **7+1d light** |
| 1 | 10 mM tris | 7.0 | CGN | CGN | CGN | CGN | CGN |
| 2 | 10 mM His | 7.0 | CGN | CGN | CGF | CGN | CGF |
| 2B | 30 mM His | 7.0 | CGF | CGF | CGF | CGN | nt |
| 3 | 10 mM maleic acid | 7.0 | insoluble | nt | nt | nt | nt |
| 4 | 10 mM EDA | 7.0 | CGN | CGF | CGN | CGF | CGN |
| 6 | 10 mM HEPES | 7.0 | CGF | CGN | CGN | CGN | CGN |
| 6B | 30 mM HEPES | 7.0 | CGN | CGF | CGF | CGN | Nt |
| 7 | 10 mM acetic | 4.5 | CGN | CGF | CGN | CGN | CGN |
| 8 | 10 mM His | 5.5 | CGN | CGN | CGN | CGN | CGN |
| 9 | 10 mM His | 6.5 | CGF | CGN | CGN | CGN | CGN |
| 10 | 10 mM tris | 7.5 | CGF | CGF | CGN | CGF | nt |
| 11 | 10 mM tris | 8.0 | CGN | CGN | CGN | CGN | CGF |
| 12 | 10 mM tris | 8.5 | CGF | CGF | CGF | CGF | CGF |

The results after centrifugation are provided in Table 22. The pH measurements are given in Table 23.

**Table 22: Pellet Analysis after Centrifugation. N = no pellet; P = pellet.**

| **Formulation** | | | **Centrifugation** | | |
|---|---|---|---|---|---|
| | | | **Days at rt** | | |
| # | **Composition** | **pH** | **7** | **14** | **7+1d light** |
| 1 | 10 mM tris | 7.0 | N | N | N |
| 2 | 10 mM His | 7.0 | N | N | N |
| 4 | 10 mM EDA | 7.0 | N | N | N |
| 6 | 10 mM HEPES | 7.0 | N | N | N |
| 6B | 30 MM HEPES | 7.0 | N | N | nt |
| 7 | 10 mM acetic | 4.5 | N | N | N |
| 8 | 10mM His | 5.5 | N | N | N |
| 9 | 10mM His | 6.5 | N | N | N |
| 10 | 10mM tris | 7.5 | P | P | nt |
| 11 | 10mM tris | 8.0 | P | P | P |
| 12 | 10mM tris | 8.5 | P | P | P |

**Table 23: pH stability analysis.**

| **Formulation** | | | **pH** | | | | |
|---|---|---|---|---|---|---|---|
| | | | **Days at rt** | | | **F/T** | |
| **#** | **Composition** | **pH** | **4** | **7** | **14** | **1X F/T** | **Buffer alone** |
| 1 | 10 mM tris | 7.0 | 7.0 | 6.8 | 6.8 | 6.9 | 7.0 |
| 2 | 10 mM His | 7.0 | 6.9 | 6.8 | 6.9 | 6.8 | 6.9 |
| 2B | 30 mM His | 7.0 | 6.9 | 6.8 | 6.9 | 6.8 | nt |
| 4 | 10 mM EDA | 7.0 | 6.6 | 6.6 | 6.6 | 6.6 | 6.7 |
| 6 | 10 mM HEPES | 7.0 | 7.0 | 6.9 | 7.0 | 7.0 | 6.9 |
| 6B | 30 MM HEPES | 7.0 | 7.0 | 6.9 | 6.9 | 6.9 | nt |
| 7 | 10 mM acetic | 4.5 | 4.6 | 4.6 | 4.6 | 4.6 | 4.5 |
| 8 | 10mM His | 5.5 | 5.4 | 5.3 | 5.4 | 5.3 | 5.4 |
| 9 | 10mM His | 6.5 | 6.4 | 6.4 | 6.4 | 6.4 | 6.4 |
| 10 | 10mM tris | 7.5 | 7.4 | 7.3 | 7.4 | 7.4 | 7.4 |
| 11 | 10mM tris | 8.0 | 7.9 | 7.8 | 7.9 | 7.8 | 7.9 |
| 12 | 10mM tris | 8.5 | 8.3 | 8.3 | 8.3 | 8.3 | 8.4 |

The % main peak by RP-H PLC is given in Table 24. Stability drops off rapidly at pH ≤6.5.

**Table 24: % Main Peak by RP-HPLC; Δ light v dark = (7+1d light) - (7d dark).**

| **Formulation** | | | **% Main peak** | | | | |
|---|---|---|---|---|---|---|---|
| | | | **Days at rt** | | | | |
| # | **Composition** | **pH** | **0** | **4** | **7** | **14** | |
| 1 | 10 mM tris | 7.0 | 97.9% | 96.8% | 95.5% | 93.5% | |
| 2 | 10 mM His | 7.0 | 98.0% | 96.1% | 94.8% | 90.3% | |
| 2B | 30 mM His | 7.0 | 97.7% | 96.2% | 94.7% | 91.9% | |
| 4 | 10 mM EDA | 7.0 | 98.3% | 97.1% | 96.2% | 94.6% | |
| 6 | 10 mM HEPES | 7.0 | 97.0% | 96.6% | 94.2% | 91.0% | |
| 6B | 30 mM HEPES | 7.0 | 97.6% | 96.2% | 94.9% | 92.6% | |
| 7 | 10 mM acetic | 4.5 | 92.6% | 64.8% | 47.4% | 21.6% | |
| 8 | 10mM His | 5.5 | 97.1% | 90.9% | 87.1% | 75.8% | |
| 9 | 10mM His | 6.5 | 97.8% | 95.7% | 94.2% | 90.9% | |
| 10 | 10mM tris | 7.5 | 97.7% | 96.7% | 96.0% | 94.0% | |
| 11 | 10mM tris | 8.0 | 97.7% | 97.2% | 96.6% | 93.4% | |
| 12 | 10mM tris | 8.5 | 97.9% | 95.5% | 93.9% | 91.7% | |

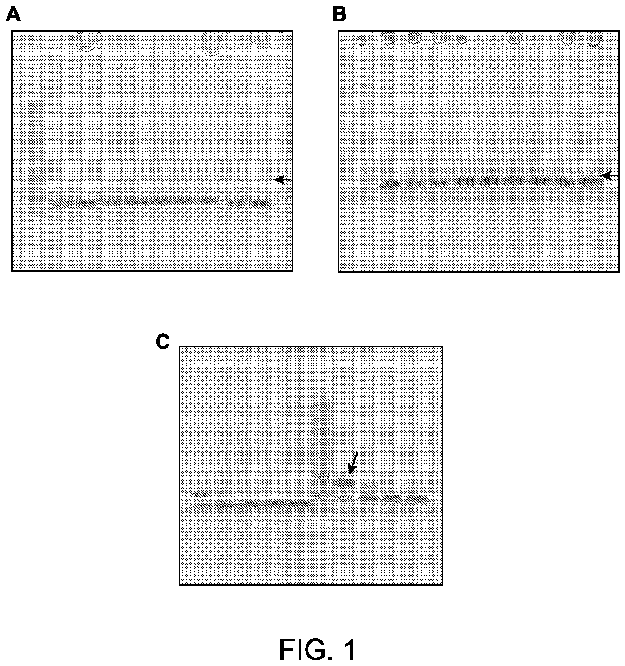

FIG. 1 shows SDS-PAGE analysis of the formulations after 14 days at room temperature. FIGS. 1A and 1B show SDS-PAGE analysis of, from left to right, (molecular weight marker) MWM, 1, 2, 2B, 4, 6, 6B, 9, 10 and reference. FIG. 1A was performed with a reducing agent and FIG. 1B was performed without a reducing agent. FIG. 1C shows SDS-PAGE analysis of, from left to right, 7, 8, 11, 12, reference and MWM (no reducing agent). (Reference = Pilot Lot, Sublot #2. MWM: 188k, 98k, 62k, 49k, 38k, 28k, 17k, 14k, 6k, 3k. Arrow points to a higher molecular weight species.

Compound 16 was formulated by additional means. Stocks were prepared and the pH of some stocks was adjusted to near 6.8 to avoid pH shift upon addition to formulations (Table 25). All stocks were 0.2µm filtered except BHT, BHA, propyl gallate, and polysorbates. Polysorbate and Nal stocks were stored with an argon blanket. His, Met, Nal, polysorbate, BHT, BHA, and propyl gallate stocks were stored in the dark.

**Table 25: Stock Buffer Solutions**

| **Component** | **mM** | **pH** |
|---|---|---|
| Tris | 500 | 7.5 |
| His | 250 | 7.5 |
| NaCl | 4000 | |
| Met | 200 | ~6.8 |
| EDTA | 200 | ~6.8 |
| Glv | 200 | ~6.8 |
| **Component** | **w/v%** | **Solvent** |
| Mannitol | 10% | H₂O |
| Sucrose | 20% | H₂O |
| Trehalose*2H2O | 22% | H₂O |
| BHT | 2.50% | 10% H2O in EtOH |
| BHA | 2.50% | 50% EtOH/ H₂O |
| Propvl gallate | 2.50% | 25% EtOH in H₂O |
| PS80 | 10% | H₂O |
| PS20 | 10% | H₂O |
| Nal | 20% | H₂O |
| HPCD | 40% | H₂O |
| Captisol | 40% | H₂O |

Compound 16 was prepared at 10 mg/mL in argon-sparged water. The Compound 16 stock was combined with buffer stock, osmolyte stock, and water to make parent stocks A-E containing Compound 16 (Table 26). The pH of each soluble parent stock was adjusted (from starting values of 6.6-6.7) to 6.8 with 0.1N NaOH.

**Table 26: Parent Stock Formulations**

| **Parent Stocks** | | | **Parent Stock composition** | | | **mLs:** | | | |
|---|---|---|---|---|---|---|---|---|---|
| | Total mL | **Buffer** | mM Buffer | Osmolyte | [Osmolytel | Buffer stock | Osmolyte stock | Cmpd 16 stock (10 mg/mL) | H2O |
| A | 4.50 | **Tris** | 11.7 | mannitol | 5.8% | 0.105 | 2.625 | 1.575 | 0.195 |
| B | 24.15 | **His** | 11.7 | mannitol | 5.8% | 1.127 | 14.088 | 8.453 | 0.483 |
| C | 2.4 | **His** | 11.7 | sucrose | 11.1% | 0.112 | 1.330 | 0.840 | 0.118 |
| D | 2.4 | **His** | 11.7 | trehalose | 12.3% | 0.112 | 1.336 | 0.840 | 0.112 |
| E | 0.1 | **His** | 11.7 | NaCL (mM) | 163.3 | 0.005 | 0.004 | 0.035 | 0.056 |

The final formulations were then created by combining the parent stocks with other excipient stocks and water. The formulations were passed through 0.2pm sterile syringe filters. 0.4mL of Formulations 1, 3, 4, and 5 were reserved for "t0" analysis.

Two 0.3mL tubes were placed in a dark 40 °C incubator for analysis after 2d and 5d. The 2d samples were assayed after 40h (1.7d). One 0.3mL tube was subjected to "3X F/T." 1X F/T comprised freezing the material at -20 °C for at least 8h, thawing the material at room temperature (rt) in the dark (~1h), then gently mixing, inverting, and spinning to bring the material back to the bottom of the tube. This cycle was performed a total of three times. The remaining 0.3mL tube was reserved at 5 °C. One of the 0.5 mL tubes was placed in the dark at room temperature and the other 0.5 mL tube was placed in the light at room temperature. For light exposure, the tubes were placed on their side and exposed to ambient light. A volume of 0.5 mL was chosen for light exposure, as previous work had shown 0.3mL samples are prone to precipitation and degradation after light exposure in this configuration.

Stability samples were assayed by visual inspection, centrifugation, RP-HPLC, A₇₈₆, pH, and, in some cases, SDS-PAGE. Samples were held in the dark at 5 °C when not in use. Samples were examined visually for clarity, color, and visible particulates. Samples were inverted three times and examined again. Samples were gently mixed, then centrifuged ~10,000 rpm for ~2 min. Samples were examined visually for a pellet. Remaining assays were performed on the supernatant.

Samples were prepared per the manufacturer's recommendations and then heated at 70°C for 10 min. 2.5µg in 10µL was loaded onto the SDS-PAGE gels and then run at 165 - 200V. After electrophoresis, the gels were washed in water for 5 min at room temperature, 3 times. Gels were stained at room temperature for 1h, destained in water overnight, and then destained again with water and imaged on the same day.

Formulations were produced by dissolving Compound 16 at 10 mg/mL in water, diluting into the buffer and osmolyte, adjusting the pH, adding final additives and water as needed (Table 27).

**Table 27: Formulations Tested at 3 mg/mL Compound 16 at pH 6.8**

| **#** | **Buffer** | **Osmolyte** | **[Osm]** | **Other** | **[Other]** | **Parameter examined** |
|---|---|---|---|---|---|---|
| 1 | 10 mM Tris | mannitol | 5% | -- | | Buffer |
| 3 | 10 mM His | mannitol | 5% | -- | | Buffer |
| 4 | 10 mM His | sucrose | 9.5% | -- | | Osmolyte |
| 5 | 10 mM His | trehalose | 10.5% | -- | | Osmolyte |
| 6 | 10 mM His | NaCl | 140 mM | -- | | Ionic strength |
| 7 | 10 mM His | mannitol | 5% | NaCl | 10 mM | Ionic strength |
| 8 | 10 mM His | mannitol | 5% | Met | 10 mM | Antioxidant |
| 9 | 10 mM His | mannitol | 5% | BHT | 0.01% | Antioxidant |
| 10 | 10 mM His | mannitol | 5% | BHA | 0.01% | Antioxidant |
| 11 | 10 mM His | mannitol | 5% | propyl gallate | 0.01% | Antioxidant |
| 12 | 10 mM His | mannitol | 5% | EDTA | 1 mM | Antioxidant synergist (metal chelator) |
| 15 | 10 mM His | mannitol | 5% | Gly | 20 mM | Amino acid |
| 16 | 10 mM His | mannitol | 5% | PS80 | 0.02% | Surfactant |
| 17 | 10 mM His | mannitol | 5% | PS20 | 0.02% | Surfactant |
| 19 | 10 mM His | mannitol | 5% | HPCD | 5% | Cyclodextrin |

Formulations #6 (140mM NaCI), #12 (1mM EDTA) and #18 (0.2% Nal) had immediate gross precipitation and were discarded. In all other formulations, the material was clear, green, and had no significant visible particle formation at all timepoints tested (Table 28).

**Table 28: Visual Inspection. C = clear; G = emerald green; N = essentially no visible particles; F = very few particles or fibers visible; P = pellet, nt = not tested.**

| **Formulation** | | | | | **Visual** | | | | | |
|---|---|---|---|---|---|---|---|---|---|---|
| **#** | **Buffer** | **Osmolvte** | **Other** | **[Other]** | **t0** | **2d 40C** | **5d 40C** | **3d rt dk** | **3d rt lt** | **3X FT** |
| 1 | Tris | mannitol | -- | | CGF | CGN | CGF | CGN | CGN | CGN |
| 3 | His | mannitol | -- | | CGN | CGN | CGN | CGN | CGN | CGN |
| 4 | His | sucrose | -- | | CGN | CGN | CGN | CGN | CGN | CGN |
| 5 | His | trehalose | -- | | CGN | CGN | CGN | CGN | CGN | CGN |
| 6 | His | 140 mM NaCl | -- | | insoluble | nt | nt | nt | nt | nt |
| 7 | His | mannitol | NaCl | 10 mM | nt | CGN | nt | nt | nt | CGN |
| 8 | His | mannitol | Met | 10 mM | nt | CGN | CGN | CGN | CGN | CGN |
| 9 | His | mannitol | BHT | 0.01% | nt | CGN | CGN | CGN | CGN | CGN |
| 10 | His | mannitol | BHA | 0.01% | nt | CGN | CGN | CGN | CGN | CGN |
| 11 | His | mannitol | propyl gallate | 0.01% | nt | CGN | CGN | CGN | CGN | CGN |
| 12 | His | mannitol | EDTA | 1 mM | insoluble | nt | nt | nt | nt | nt |
| 15 | His | mannitol | Gly | 20 mM | nt | CGN | CGN | CGF | CGN | CGN |
| 16 | His | mannitol | PS80 | 0.02% | nt | CGN | CGN | CGF | CGN | CGN |
| 17 | His | mannitol | PS20 | 0.02% | nt | CGN | CGN | CGN | CGN | CGN |
| 19 | His | mannitol | HPCD | 5% | nt | CGN | CGN | CGN | CGN | CGN |

All samples were at pH 6.8 +/- 0.1 (Table 29).

**Table 29: pH**

| **Formulation** | | | | | **pH** | | | | | |
|---|---|---|---|---|---|---|---|---|---|---|
| **#** | **Buffer** | **Osmolyte** | **Other** | **[Other]** | **t0** | **2d 40C** | **5d 40C** | **3d rt dk** | **3d rt lt** | **3X FT** |
| 1 | Tris | mannitol | -- | | 6.8 | 6.8 | 6.8 | 6.9 | 6.8 | 6.9 |
| 3 | His | mannitol | -- | | 6.8 | 6.8 | 6.8 | 6.9 | 6.8 | 6.8 |
| 4 | His | sucrose | -- | | 6.8 | 6.8 | 6.8 | 6.9 | 6.8 | 6.9 |
| 5 | His | trehalose | -- | | 6.8 | 6.8 | 6.8 | 6.8 | 6.8 | 6.8 |
| 7 | His | mannitol | NaCl | 10 mM | nt | 6.8 | nt | nt | nt | 6.8 |
| 8 | His | mannitol | Met | 10 mM | nt | 6.8 | 6.8 | 6.8 | 6.8 | 6.8 |
| 9 | His | mannitol | BHT | 0.01% | nt | 6.8 | 6.8 | 6.8 | 6.8 | 6.8 |
| 10 | His | mannitol | BHA | 0.01% | nt | 6.8 | 6.8 | 6.8 | 6.8 | 6.8 |
| 11 | His | mannitol | propyl gallate | 0.01% | nt | 6.8 | 6.7 | 6.9 | 6.8 | 6.8 |
| 15 | His | mannitol | Gly | 20 mM | nt | 6.8 | 6.8 | 6.8 | 6.8 | 6.8 |
| 16 | His | mannitol | PS80 | 0.02% | nt | 6.8 | 6.8 | 6.9 | 6.8 | 6.8 |
| 17 | His | mannitol | PS20 | 0.02% | nt | 6.8 | 6.8 | 6.8 | 6.8 | 6.8 |
| 19 | His | mannitol | HPCD | 5% | nt | 6.8 | 6.8 | 6.8 | 6.8 | 6.8 |

The % main peak by RP-HPLC is given in Table 30.

**Table 30: % Main Peak by RP-HPLC. Δ light vs. dark = % main peak after 3d room temperature in the light minus % main peak after 3d room temperature in the dark.**

| **Formulation** | | | | | **% Main Peak** | | | | |
|---|---|---|---|---|---|---|---|---|---|
| **#** | **Buffer** | **Osmolyte** | **Other** | **[Other]** | **t0** | **2d 40C** | **5d 40C** | **3d rt lt** | **3X FT** |
| 1 | Tris | mannitol | -- | | 97.5% | 93.9% | 85.1% | 73.9% | 97.4% |
| 3 | His | mannitol | -- | | 98.5% | 94.0% | 86.1% | 92.6% | 98.6% |
| 7 | His | mannitol | NaCl | 10 mM | nt | 95.3% | nt | nt | 98.3% |
| 8 | His | mannitol | Met | 10 mM | nt | 94.3% | 87.5% | 93.0% | 98.6% |
| 9 | His | mannitol | BHT | 0.01% | nt | 94.1% | 83.4% | 91.2% | 98.7% |
| 10 | His | mannitol | BHA | 0.01% | nt | 93.9% | 84.9% | 91.1% | 98.3% |
| 11 | His | mannitol | propyl gallate | 0.01% | nt | 88.7% | 72.7% | 89.7% | 98.2% |
| 15 | His | mannitol | Gly | 20 mM | nt | 94.4% | 83.9% | 90.9% | 97.7% |
| 16 | His | mannitol | PS980 | 0.02% | nt | 93.4% | 83.8% | 91.9% | 98.6% |
| 17 | His | mannitol | PS20 | 0.02% | nt | 94.4% | 84.3% | 90.3% | 98.3% |
| 19 | His | mannitol | HPCD | 5% | nt | 87.3% | 65.6% | 89.3% | 96.2% |

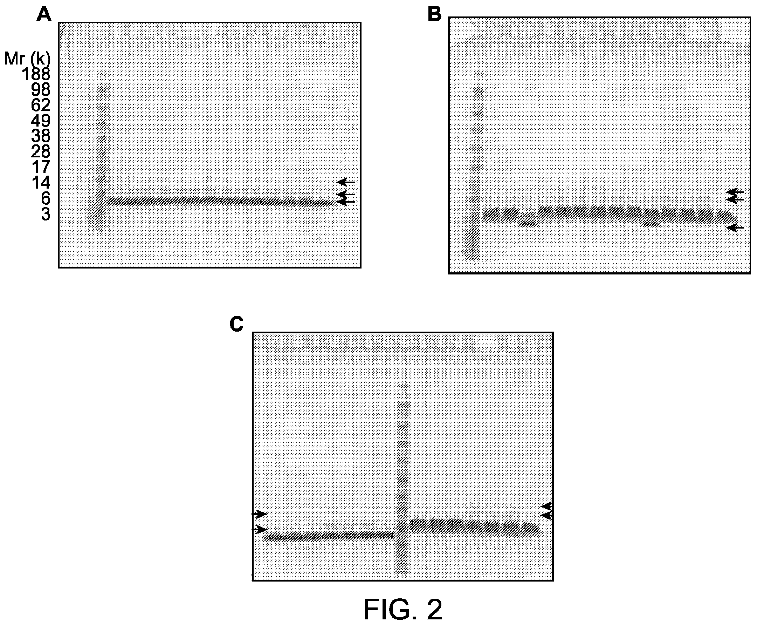

After 5 days at 40 °C, formation of significant amounts of higher molecular weight species (HMWS) were visible by reducing and nonreducing SDS-PAGE (FIG. 2). FIG. 2 shows SDS-PAGE of formulations after 5 days at 40 °C (and at time = 0). Samples are after 5 days at 40 °C unless marked as time = 0. Formulations from left to right. FIGS. 2A and 2B show SDS-PAGE analysis of, from left to right, (molecular weight marker) MWM, 3, 4, 5, 8, 9, 10, 11, 13, 14, 15, 16, 17, 19, reference. FIG. 2A was performed with a reducing agent and FIG. 2B was performed without a reducing agent. FIG. 2C shows SDS-PAGE analysis of, from left to right, 1 t0, 3 t0, 5 t0, 1, 2, 3, reference, MWM, 1 t0, 3 t0, 5 t0, 1, 2, 3, reference. For FIG. 2C, lanes to the left of MWM were performed with a reducing agent and lanes to the right of MWM were performed without a reducing agent. Reference = Pilot Lot, Sublot #2 (-20 °C). MWM (top to bottom): 188k, 98k, 62k, 49k, 38k, 28k, 17k, 14k, 6k, 3k. Black arrows point to higher molecular weight species, green arrow points to new band in formulation 19, and red arrow points to what may be reduced material in formulations 5 and 15.

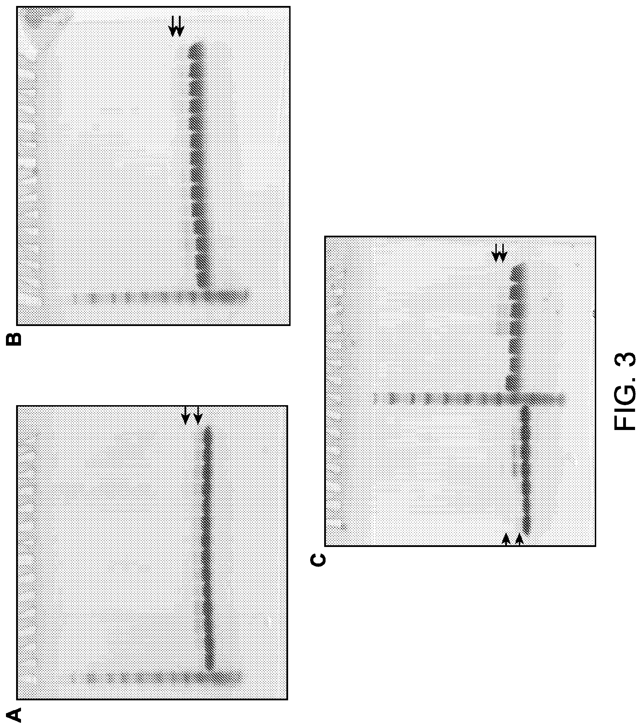

After 3 days at room temperature in the light, higher molecular weight species formation was evident (FIG. 3). Samples are from after 3 days at room temperature in the light unless marked as F/T. FIGS. 2A and 2B show SDS-PAGE analysis of, from left to right, (molecular weight marker) MWM, reference, 3, 4, 5, 8, 9, 10, 11, 13, 14, 15, 16, 17, 19. FIG. 3A was performed with a reducing agent and FIG. 3B was performed without a reducing agent. FIG. 3C shows SDS-PAGE analysis of, from left to right, 3 F/T, 13 F/T, 14 F/T, 1, 2, 3, reference, MWM, 3 F/T, 13 F/T, 14 F/T, 1, 2, 3, reference. For FIG. 3C, lanes to the left of MWM were performed with a reducing agent and lanes to the right of MWM were performed without a reducing agent. Reference = Compound 16, Pilot Lot, Sublot #2 (-20 °C). MWM (top to bottom): 188k, 98k, 62k, 49k, 38k, 28k, 17k, 14k, 6k, 3k. Black arrows point to HMWS.

The effect of various excipients on Compound 16 stability was tested with Compound 16 in a base buffer. Formulations were stored in microcentrifuge tubes with an air atmosphere and assayed after 2 days and 5 days at 40°C in the dark, after 3 days at room temperature (rt) in the dark or in ambient light, and after 3X freeze/thaw between -20 °C and room temperature in the dark.

### EXAMPLE 3

### Evaluation of Chlorotoxin Conjugate Stability in Various Vial Types and Atmospheres

This example describes the stability of Compound 16 in different vial types and in different atmospheric (headspace) environments.

The stability of KNTi-0303 (the active pharmaceutical ingredient of BLZ-100) was tested in amber glass, clear glass, CZ, and Type 1+ glass vials with air or N₂ headspace. The purity of samples was assessed by RP-HPLC, as shown in Table 31. All samples degraded significantly upon incubation at 40 °C. In some cases, the purity values after 8 d at 40 °C were similar or even higher than the values after 5 d at 40 °C. Both the 5 d and 8 d samples were reanalyzed together the following week, and the purity values as well as chromatogram shapes were comparable to the original analyses. The 2 d, 5 d, and 8 d vials were all in the 40 °C incubator on the same days, with the vials being removed after the set number of days, placed at 5 °C in the dark, and then analyzed within 1 d.

**Table 31: % purity by RP-HPLC of Compound 16 in stored in different vial types.**

| **Configuration** | | | **% purity RP-HPLC** | | | | | | | | | | | |
|---|---|---|---|---|---|---|---|---|---|---|---|---|---|---|
| **#** | **Vial** | **Atm** | **t0'** | **2d 40C** | **5d 40C** | **8d 40C** | **t0** | **1d rt lt** | **3d rt lt** | **1d rt dk** | **3d rt dk** | **1d rt shak e** | **3d rt shak e** | **3X F/T** |
| 1 | Amber | Air | 98.7 | 92.2 | 72.6 | 72.9 | 98.8 | 98.2 | 96.8 | 98.0 | 97.6 | nt | nt | nt |
| 2 | Amber | N₂ | 98.7 | 94.2 | 75.4 | 80.2 | 98.8 | 98.3 | 98.0 | 98.8 | 98.2 | 98.5 | 97.7 | 98.4 |
| 3 | Clear | Air | 98.7 | 91.1 | 74.3 | 70.6 | 98.8 | 97.8 | 95.8 | nt | nt | nt | nt | nt |
| 4 | Clear | N₂ | 98.7 | 95.5 | 85.5 | 84.7 | 98.8 | 98.4 | 97.6 | nt | nt | nt | 98.0 | 98.9 |
| 5 | CZ | Air | 98.7 | 93.3 | 74.3 | 72.4 | 98.8 | 97.6 | 95.5 | nt | nt | nt | nt | nt |
| 6 | CZ | N₂ | 98.7 | 95.9 | 83.1 | 79.6 | 98.8 | 98.3 | 96.7 | nt | nt | nt | 97.7 | 98.9 |
| 7 | Type 1+ | Air | 98.7 | 93.8 | 76.2 | 72.3 | 98.8 | 97.6 | 95.8 | nt | nt | nt | nt | nt |
| 8 | Type 1+ | N₂ | 98.7 | 95.5 | 85.8 | 80.8 | 98.8 | 98.1 | 97.5 | nt | nt | nt | 98.3 | 98.8 |

In all vials stored at 40 °C, purity loss by RP-HPLC was significantly slower when samples were stored under N₂ rather than air. Under N₂, purity loss was significantly more rapid in amber glass vials than in clear glass, Type 1+ glass, or CZ vials. Under air, however, there was not a clear difference between vial types, though degradation may have been slower in Type 1+ glass or CZ vials.

When exposed to light, purity loss by RP-HPLC in all vials was again significantly slower when samples were stored under N₂ rather than air. With light exposure, purity loss was slowed by storage in amber glass vials rather than the other vial types. Under N₂, purity loss after 3 d in the light (8 h of light exposure per day) was 0.8% in amber glass vials and 1.2% in clear glass vials. Under air, purity loss after 3 d in the light was 2.0% in amber glass vials and 3.0% in clear glass vials. Thus, the improved purity by storage in amber vials after 3 d in the light is 0.4% under N₂ and 1.0% in air compared to clear glass vials. Clear glass vials, Type 1+ vials, and CZ vials performed similarly with light exposure except that CZ vials under N₂ were less protective than other vials under N₂, likely due to the increased gas permeability of CZ vials.

Comparing data from samples at rt in the light versus in the dark shows that the formulation in amber vials undergoes very little degradation due to light exposure over 3 d. After 3 d, the additional purity loss from light exposure was 0.2% under N₂ and 0.8% in air.

By RP-HPLC, Compound 16 did not appear very sensitive to shaking in the formulations and configurations tested. Compared to rt storage in the dark without shaking, there was an additional purity loss of 0.5% due to shaking for 3 d in amber vials under N₂. There was no clear difference between vial types upon shaking under N2, but the stability may be slightly higher in clear glass or Type 1+ glass vials. Shaking provides agitation stress but also provides additional convection that could increase exposure to oxygen in the headspace or surface leaching.

There was no apparent degradation after 3X F/T in any vial type under N₂ as assessed by RP-HPLC. While the value appears slightly lower for the amber vials, the 1^{st} RP-HPLC injection yielded a value of 98.0% but the 2^{nd} injection yielded a value of 98.8%. The 2^{nd} injection was comparable to the other vial types; it is possible the 1^{st} injection was artificially low due to effect of the prior injected sample.

### EXAMPLE 4

### Development of Candidate Formulations for Lyophilized Conjugates

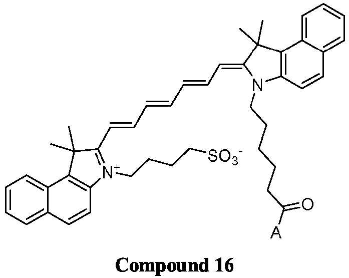

A = MCMPCFTTDHQMARRCDDCCGGRGRGKCYGPQCLCR (K-27 is point of attachment)

This example shows various lyophilization conditions for Compound 16.

Methods: Various strategies for lyophilization of Compound 16 were attempted for different formulations of Compound 16. For freezing techniques, 1.2mL of a formulation was added to 3mL glass vial and stoppered with single vent fluorotec coated stopper. For fast freezing, vials were placed on a shelf after equilibration of the shelf at -40°C. For slow freezing, the temperature was controlled to reduce from 4°C to -40°C at a rate of 1°C/min. The peptide-dye conjugate concentrations used were from 1, 3, 4, 6, and 10 mg/mL.

Lyophilized Compound 16 was reconstituted by addition of 1.2mL of water and followed with gentle mixing for <1 min. The reconstituted Compound 16 was a clear, emerald green solution with no visible particles and no pellet observed after centrifugation (15,000 x g, 5 min). The pH ranged from 6.8-6.95 and the moisture content was <1% as determined by KF titration.

Prior to lyophilization, the formulation volumes were each 1.2mL and lyophilized formulations were reconstituted with 1.1mL of water followed by gentle mixing for <1 min. No visible aggregates were observed during or after reconstitution.

Reconstituted samples were analyzed for oxidation/purity by RP-HPLC, mass determined by RP-HPLC and/or OD786, aggregation measured by pellet formation after centrifugation, MFI and/or SDS-PAGE, charge heterogeneity determined by cIEF, secondary structure indicated by FTIR, pH measurements as described herein (the pH ranged from 6.8-7.0) and the KF titration determined (residual moisture was <0.5%).

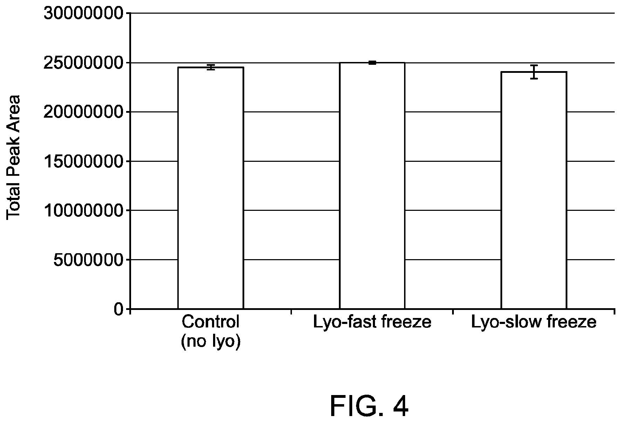

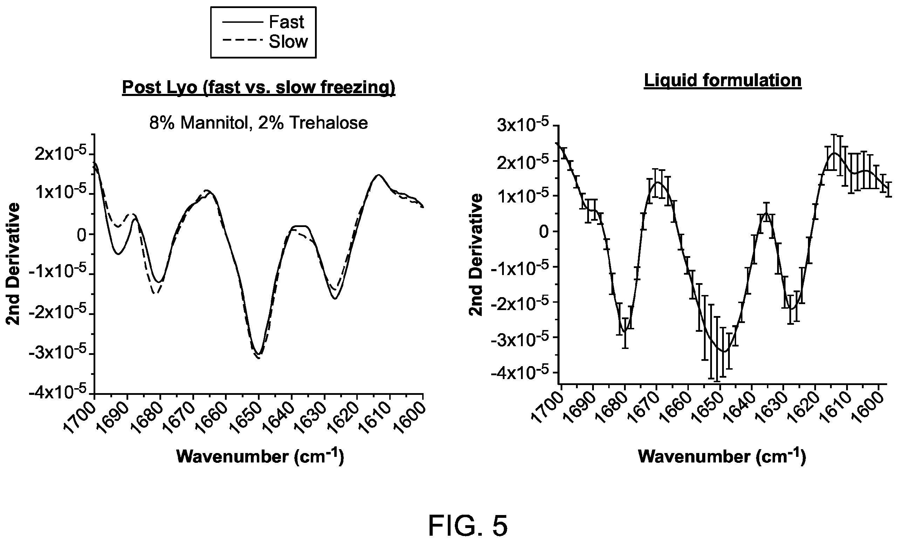

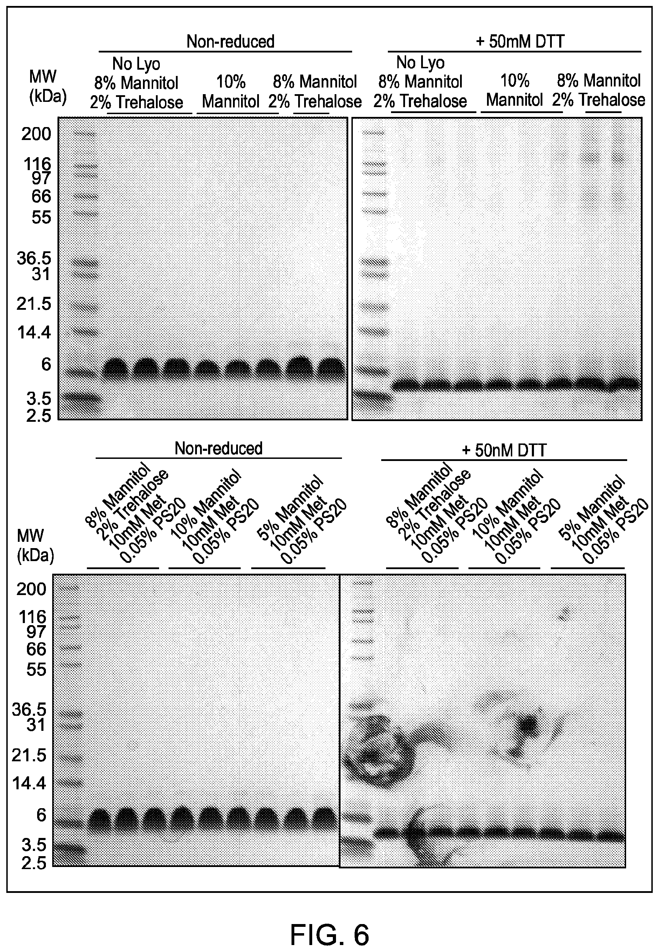

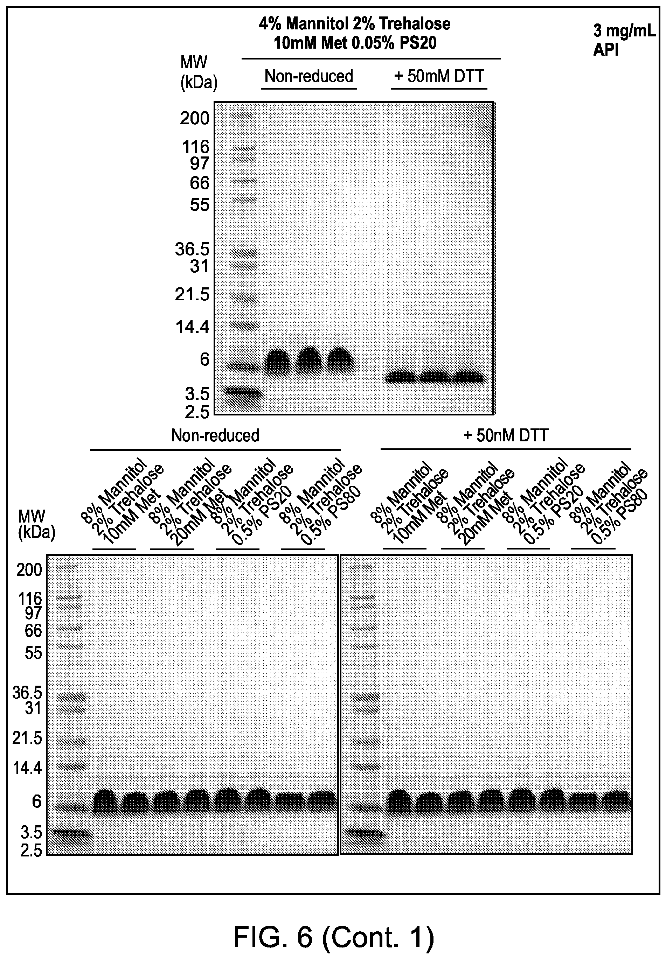

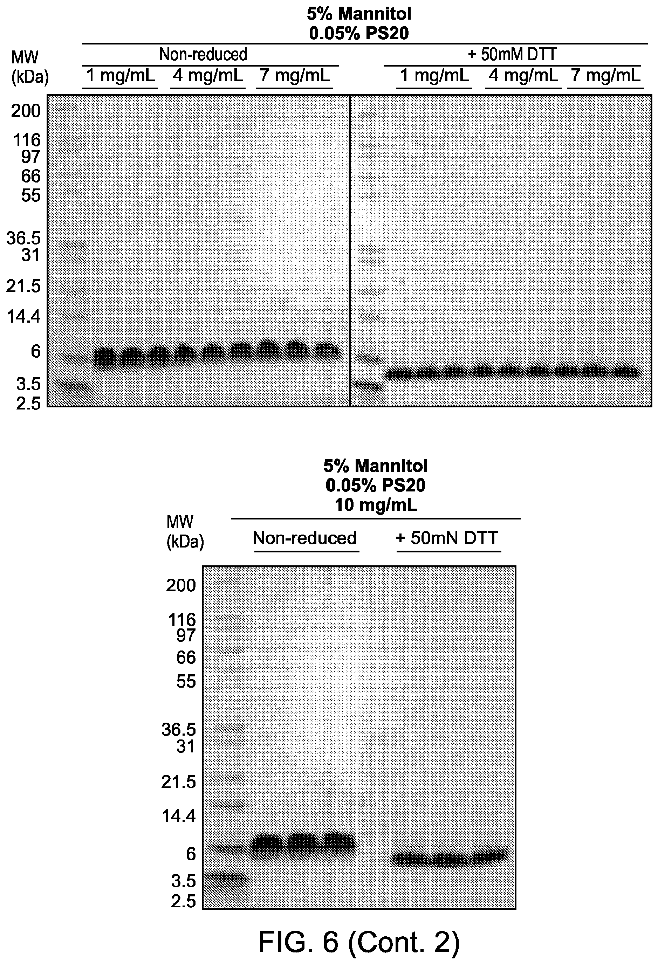

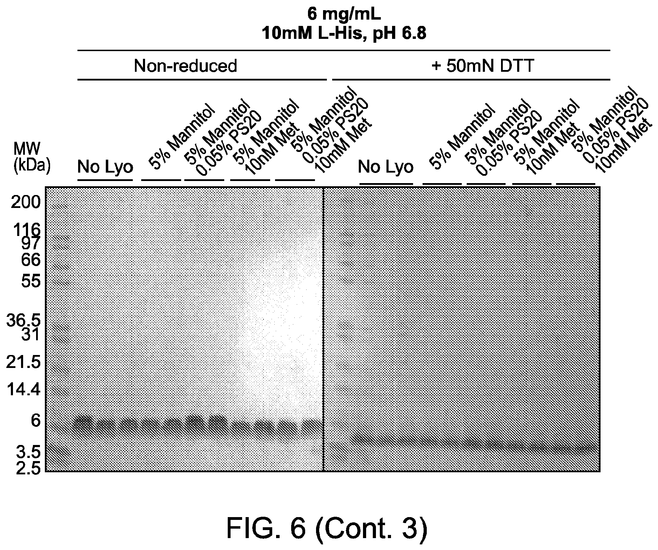

Results: Summary data for various lyophilized Compound 16 formulations are provided in Tables 32-33. As determined by RP-HPLC, lyophilization with fast and slow freezing produced no substantial differences in total peak area of chromatograms from formulations with 8% Mannitol and 2% trehalose (FIG. 4). The FTIR spectra of reconstituted samples were comparable to FTIR spectra of samples that had not been lyophilized (Table 33). Secondary structures indicated by FTIR spectra did not differ between formulations that underwent fast versus slow freezing (FIG. 5). No difference in particle size was detected by SDS-PAGE of various lyophilized formulations (FIG. 6). There also was no significant difference in charge heterogeniety between samples (isoelectric point, pI), as determined by cIEF.

**Table 32: Conservative Lyophilization Cycle**

| | **Freeze** | **Primary/Secondary Drying** | | | | | | | **Final** |
|---|---|---|---|---|---|---|---|---|---|
| Step | 1 | 1 | 2 | 3 | 4 | 5 | 6 | 7 | 1 |
| Shelf Temp (°C) | -40 | -40 | -30 | -35 | -20 | -10 | 0 | 20 | 4 |
| Ramp Rate (°C/min) | - | 0.1 | 0.1 | 0.1 | 0.1 | 0.1 | 0.1 | 0.1 | 1.0 |
| Time (min) | 540 | 270 | 400 | 920 | 600 | 60 | 60 | 60 | HOLD |
| Vacuum (mT) | - | 100 | 100 | 100 | 100 | 100 | 100 | 100 | 100 |

**Table 33: Properties of Formulations. Intr-FL = Intrinsic fluorescence; N/A = Not applicable; ND = Not Done; NC = No change from control.**

| **#** | **API Conc (mg/mL)** | **Formulation** | **Cake Appearanc e** | **pH** | **FTIR** | **Intr-FL** | **Pellet Obs (Y/N)** | **SDS Page** | **OD 786 (%)** | **cIEF Area (%)** | **RP-HPLC Purity (%)** |
|---|---|---|---|---|---|---|---|---|---|---|---|
| 1 | 3 | No Lyo | N/A | 6.96 | NC | NC | N | NC | 98.1 | 95.0 | 98.9 |
| 2 | 3 | 8% Mannitol 2% Trehalose | Intact | 6.75 | NC | NC | N | NC | 112.8 | 95.3 | 98.2 |
| 3 | 3 | 8% Mannitol 2% Trehalose 10mM Met | Intact | 6.69 | NC | NC | N | NC | 118.5 | 95.6 | 98.4 |
| 4 | 3 | 8% Mannitol 2% Trehalose 20mM Met | Intact | 6.93 | NC | NC | N | NC | 113.0 | 93.8 | 98.4 |
| 5 | 3 | 8% Mannitol 2% Trehalose 0.05% PS20 | Intact | 6.97 | NC | NC | N | NC | 116.0 | 92.8 | 96.8 |
| 6 | 3 | 8% Mannitol 2% Trehalose 0.05% PS80 | Intact | 6.91 | NC | NC | N | NC | 115.1 | 94.6 | 96.8 |
| 7 | 3 | 8% Mannitol 2% Trehalose 10mM Met, 0.05% PS20 | Intact | 6.84 | NC | NC | N | NC | 126.4 | 93.7 | 97.2 |
| 8 | 3 | 10% Mannitol 10mM Met, 0.05% PS20 | Intact | 6.77 | NC | NC | N | NC | 119.4 | 91.8 | 97.1 |
| 9 | 3 | 5% Mannitol 10mM Met, 0.05% PS20 | Intact | 6.78 | NC | NC | N | NC | 106.7 | 91.3 | 97.2 |
| 10 | 3 | 4% Mannitol 2% Trehalose 10mM Met, 0.05% PS20 | Intact | 6.90 | NC | NC | N | NC | 114.4 | 90.0 | 97.3 |
| 11 | 3 | 10% Mannitol | Intact | 7.08 | NC | NC | N | NC | 80.6 | 95.5 | 98.5 |
| 12 | 1 | 5% Mannitol 0.05% PS20 1 mg/mL | Intact | 7.03 | NC | NC | Y | NC | 99.0 | 88.9 | 92.1 |
| 13 | 4 | 5% Mannitol 0.05% PS20 4 mg/mL | Intact | 6.96 | NC | NC | N | NC | 83.0 | 91.4 | 97.1 |
| 14 | 7 | 5% Mannitol 0.05% PS20 7 mg/mL | Intact | 6.94 | NC | NC | N | NC | 87.0 | 91.4 | 97.8 |
| 15 | 10 | 5% Mannitol 0.05% PS20 10 mg/mL | Intact | 6.73 | NC | NC | N | NC | 84.0 | 90.4 | 98.1 |
| 16 | 6 | 5% Mannitol No Lyo | N/A | 6.91 | NC | NC | N | NC | 92.0 | 92.4 | 98.9 |
| 17 | 6 | 5% Mannitol 0.05% PS20 No Lyo | N/A | 7.08 | NC | NC | N | NC | 156.0 | 92.2 | 98.9 |
| 18 | 6 | 5% Mannitol 10%mM Met No Lyo | N/A | 7.15 | NC | NC | N | NC | 104.0 | 92.2 | 98.9 |
| 19 | 6 | 5% Mannitol 10mM Met 0.05% PS20, No Lyo | N/A | 7.12 | NC | NC | N | NC | 100.0 | 92.4 | 98.9 |
| 20 | 6 | 5% Mannitol | Intact | 7.08 | NC | NC | N | NC | 113.5 | 90.5 | 98.9 |
| 21 | 6 | 5% Mannitol 0.05% PS20 | Intact | 7.06 | NC | NC | N | NC | 135.0 | 89.9 | 98.9 |
| 22 | 6 | 5% Mannitol 10mM Met | Intact | 7.13 | NC | NC | N | NC | 97.0 | 90.2 | 98.8 |
| 23 | 6 | 5% Mannitol 10mM Met 0.05% PS20 | Intact | 7.04 | NC | NC | N | NC | 97.5 | 87.5 | 98.9 |
| 24 | 3 | 7% Mannitol 3% Trehalose | Intact | 6.81 | ND | ND | N | ND | 82.0 | ND | ND |
| 25 | 3 | 9% Mannitol 1% Trehalose | Intact | 6.93 | ND | ND | N | ND | 81.0 | ND | ND |
| 26 | 3 | 10% Trehalose | Phase separation | ND | ND | ND | ND | ND | ND | ND | ND |
| 27 | 3 | 10% Sucrose | Collapse and Phase Separation | ND | ND | ND | ND | ND | ND | ND | ND |
| 28 | 3 | 10% Trehalose 1% Dextran T40 | Collapse and Phase Separation | ND | ND | ND | ND | ND | ND | ND | ND |
| 29 | 3 | 8% Trehalose 1% Glycine | Collapse and Phase Separation | ND | ND | ND | ND | ND | ND | ND | ND |
| 30 | 3 | 10% Trehalose, 1% HSA | Aggregation upon HSA addition | ND | ND | ND | ND | ND | ND | ND | ND |
| 31 | 3 | 2% Mannitol 8% Trehalose | Phase Separation | ND | ND | ND | ND | ND | ND | ND | ND |

### EXAMPLE 5

### Preparation and Use of Compound for Targeting of Glioma Tumor Tissues

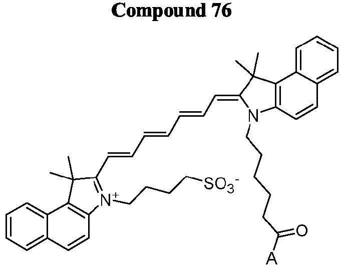

A = (PEGrMCMPCFTTDHQMARACDDCCGGAGRGKCYGPQCLCR (K-27 is point of attachment)

This example shows the performance of ICG in targeting tumor tissue compared to normal tissue, often as the signal/noise ratio and the biodistribution of ICG conjugates 24 hours after injection into a subject.

N-terminal PEGylation of chlorotoxin (CTX) and Compound 76 prior to conjugation with ICG minimizes product heterogeneity resulting from potential ICG conjugation to the N-terminus in addition to the K27 site (the peptide sequence of Compound 76 having a sequence of H-Met-Cys-Met-Pro-Cys-Phe-Thr-Thr-Asp-His-Gln-Met-Ala-Arg-Ala-Cys-Asp-Asp-Cys-Cys-Gly-Gly-Ala-Gly-Arg-Gly-Lys-Cys-Tyr-Gly-Pro-Gln-Cys-Leu-Cys-Arg-OH). PEGylation often improves solubility in aqueous solutions and the signal to noise ratio during imaging of cancerous tissue.

Three derivatives of Compound 76 were prepared, i.e., PEGaldehyde derivatives of 2kD, 5kD, and 40kD. These were obtained by PEGylating Compound 76 using 2 kD, 5 kD and 40 kD PEGaldehyde derivatives, respectively. 5 kD-PEGylated modified chlorotoxin (Compound 76-5kD) was obtained by PEGylating Compound 76 using a 5 kD polydisperse PEGaldehyde derivative.

Mice bearing U87 flank xenografts were injected with 2 nmol of each chlorotoxin based conjugate through the tail vein (IV). Twenty four hours after injection, the mice were euthanized and the tumor and leg muscle were resected or the brain, heart, liver, kidney, spleen, and blood were collected. Tumor and leg muscle resected tissue was imaged with the IVIS Spectrum equipped with a 745nm excitation filter and an 820nm emission filter. All tissue was frozen in OCT and stored.

Tissues were sliced using a cryostat into 12µm thick sections and scanned using the Li-Cor Odyssey set to 21µm resolution, high quality, intensity 7.0, channel 800nm (excitation = 785 nm). The images were quantified using the Li-Cor Odyssey software using regions of interest (ROI's) of identical size and expressed in units of Integrated Intensity (counts per mm²). Tissue was submitted to the FHCRC Experimental Histopathology core for H&E staining.

Free dye experiments were performed using Cardiogreen (ICG) (Sigma, product # I 2633). A 200 µM solution was prepared in H₂O and filter sterilized. A 40µM dilution was prepared in 1X PBS. The ICG free dye remained in solution after this procedure and was used within 1h after reconstitution.

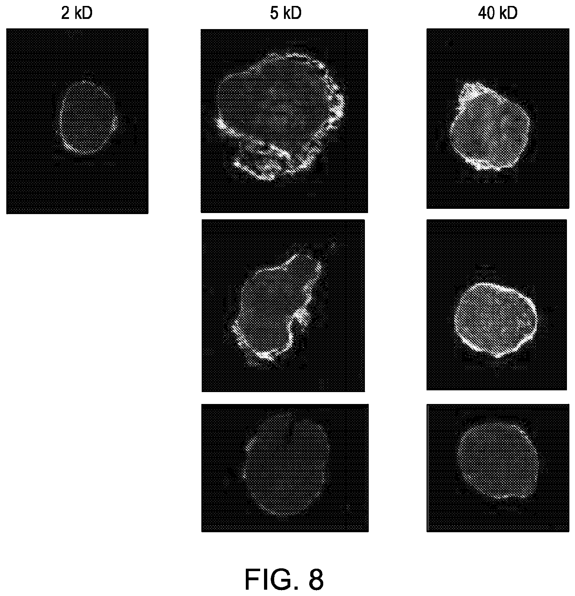

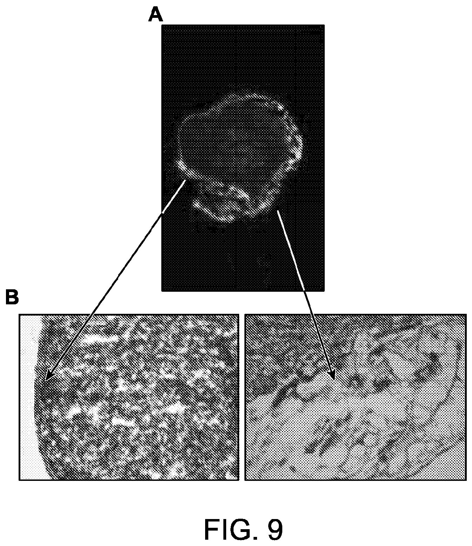

Results. The Odyssey analysis showed signal/noise for each conjugate as follows: Compound 76-2kD, 6.6+/-3.7 (n = 8); Compound 76-5kD, 8.8+/- 1.6 (n = 7); Compound 76-40kD, 10.3 +/-4.4 (n = 8). Representative fluorescent images from each of the conjugates are shown (FIG. 8). The capsule surrounding the tumor tissue has a high level of signal compared to the tumor cells (FIG. 9).

Compound 76-2kD, Compound 76-5kD, Compound 76-40kD targeted the tumor tissue. The free dye is not detected in the tumor 24 hours after injection. PEGylated chlorotoxin conjugated to the fluorophore ICG binds to tumor tissue and is detected 24h after injection. Tumor binding is specific to the properties of PEGylated chlorotoxin as the free dye was not detectable in tumors after 24h.

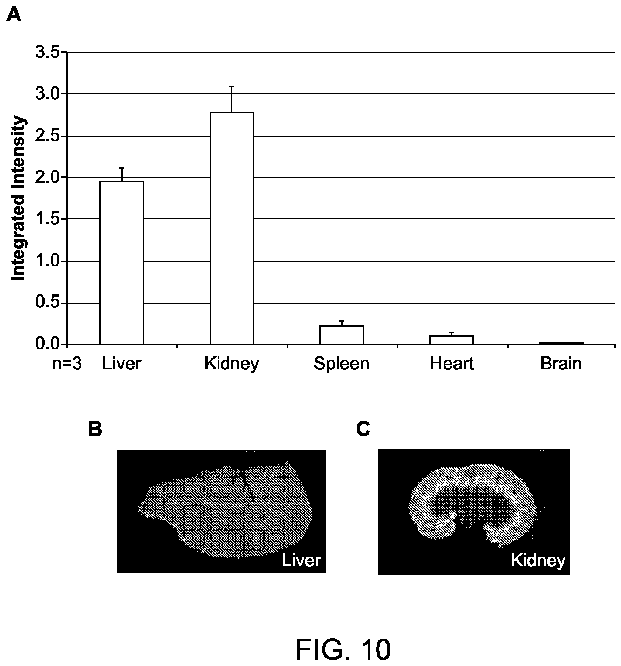

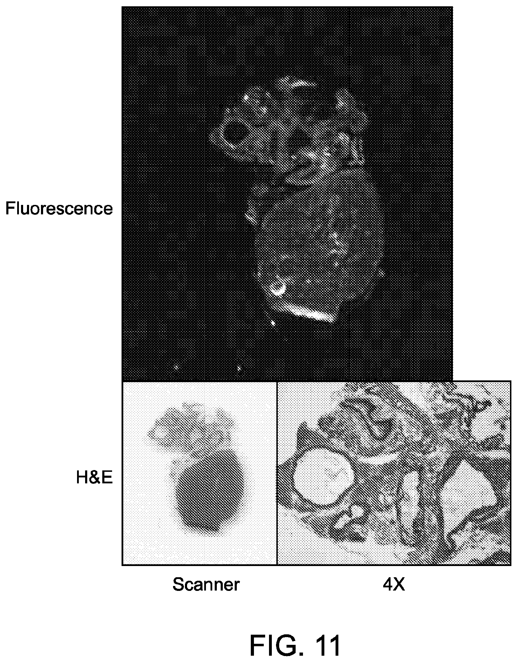

Biodistribution patterns 24h after injection show that Compound 76-5kD distributes mainly to the liver and kidney with a low signal detected in the spleen and heart. Little or no signal was detected in normal brain (FIG. 10). Additionally, significant signal was not detected in the great vessels of the heart, which has been observed with the IR800 and Cy5.5 conjugates in past experiments (FIG. 11).

ICG free dye is not detectable in liver, kidney, spleen, or brain. Free dye is detectable in the fecal matter indicating that it is cleared from the blood by the liver in into the bile. The biodistribution patterns of the conjugates therefore more closely resembles that which is seen for other CTX conjugates while the free dye biodistribution is similar to what is reported for non-conjugated ICG.

### EXAMPLE 6

### Administration of Other Chlorotoxin Conjugate Compounds and Targeting of Glioma Tumor Tissues

This example shows the use of Compounds 1-720 in targeting tumor tissue compared to normal tissue, as the signal/noise ratio and the biodistribution of chlorotoxin conjugate compounds 1 to 720 at 24 hours after injection into a subject.

Materials and methods are used as described in Example 5 but with Compounds 1-720.

Results. Compounds 1-720 preferentially target and label tumor tissue and capsules surrounding tumors. Biodistribution patterns 24h after injection show that Compounds 1-720 distribute mainly to the liver and kidney with a low signal detected in the spleen and heart. Little or no signal is detected in normal brain or in great vessels of the heart.

### EXAMPLE 7

### Dose Intervals and Imaging Intervals Using Compound 16 for Detection of Glioma Tumors in Models

This example shows the optimal imaging time and dose of Compound 16 in mice and further shows that Compound 16 targets U87 human glioma cells implanted into the brains of mice. Compound 16 signal in tumor compared to normal brain (signal-to-noise ratio, SNR) was compared to the SNR calculated in subcutaneous U87 flank xenografts using both whole tissue imaging and sliced tissue analysis.

Materials. Compound 16 in 10mM Tris and 5% Mannitol at concentrations of 0.03 mg/ml or 6µM (0.6nmole/100µl); 0.1 mg/ml or 20 µM (2nmole/100µl); 0.3 mg/ml or 60µM (6nmole/100µl); 0.5 mg/ml or 100µM (10nmole/100µl); and 1 mg/ml or 200µM (20nmole/100µl); 10nmole/100µl (0.5 mg/ml).

Methods. Nu/Nu female mice bearing U87 (human glioma cell line cultured using standard culture conditions in DMEM (Invitrogen), 10% Fetal Bovine Serum (Qualified #26140, Invitrogen), Pen/Strep (Invitrogen) xenografts in the flank were injected through the tail vein with 0.6, 2, 6, 10, or 20nmol of Compound 16 in a total volume of 100 µl. Mice were euthanized 1, 2, or 3 days after injection. Tumor, muscle, and skin were collected for all mice. Brain, heart, liver, and kidney were dissected for a subset of mice. The tissues were imaged using the IVIS Spectrum (Perkin Elmer) and quantified using Living Image software (Perkin Elmer). The tumor tissue was frozen on dry ice in OCT, sliced into 12µm sections, and scanned on the Odyssey CLx near- infrared imaging system (Li-Cor Biosciences) using the 800 nm channel (785nm excitation). The tissue was scanned using the "auto" intensity setting and 21 µm resolution. Images were analyzed using the Image Studio software (Li-Cor) by measuring the fluorescent signal within a region of interest (ROI) drawn in each tissue image.

Orthotopic xenograft implants were generated using five week old female Nu/Nu mice (Harlan Laboratories) anesthetized with isoflurane. The scalp was swabbed with providone-iodine and alcohol. Using a scalpel, an incision was made in the scalp down the midline in the area of the cerebral cortex. A burr hole was drilled through the skull using a micro-drill fitted with a 0.9mm bit. The burr hole was placed into the right cerebral hemisphere approximately 1 mm lateral (right) of the sagittal suture, 2 mm anterior to the lambdoid suture, and 2 mm deep. Using a p20 pipet and tip, 100,000 U87 cells suspended in serum-free DMEM (Invitrogen) was injected into the brain. The burr hole was covered with a Gelfoam Sponge (Pfizer) fragment and the incision closed with Vetbond tissue adhesive (3M). The mouse received bupivacaine at the injection site for pain relief. Mice developed tumors approximately 4 weeks after implantation.

Three mice with orthotopic U87 brain tumors and three mice with U87 flank tumors received tail vein injections of 100µl of a 10nmol/100µl dose of Compound 16. One day after injection the mice were euthanized using CO₂ inhalation. The brains bearing orthotopic xenografts, flank tumors, and normal brain were excised and imaged on the Ivis Spectrum (Perkin Elmer) using the 745nm excitation and 820 nm emission filters. The whole tissues were then imaged on the Odyssey CLx near- infrared imaging system (Li-Cor Biosciences) using the 800nm channel (785 nm excitation). The tumor tissue was frozen on dry ice in Optimal Cutting Tissue medium (OCT) (Tissue Tek), sliced into 12µm sections, placed on charged slides (Fisherbrand) and scanned on the Odyssey. The tissue was scanned using the "auto" intensity setting and 21µm resolution. Images were analyzed using the Image Studio software (Li-Cor) by measuring the fluorescent signal within a region of interest (ROI) drawn in each tissue image. Slides were stained with Hematoxylin and Eosin (H&E) using standard histological protocols.

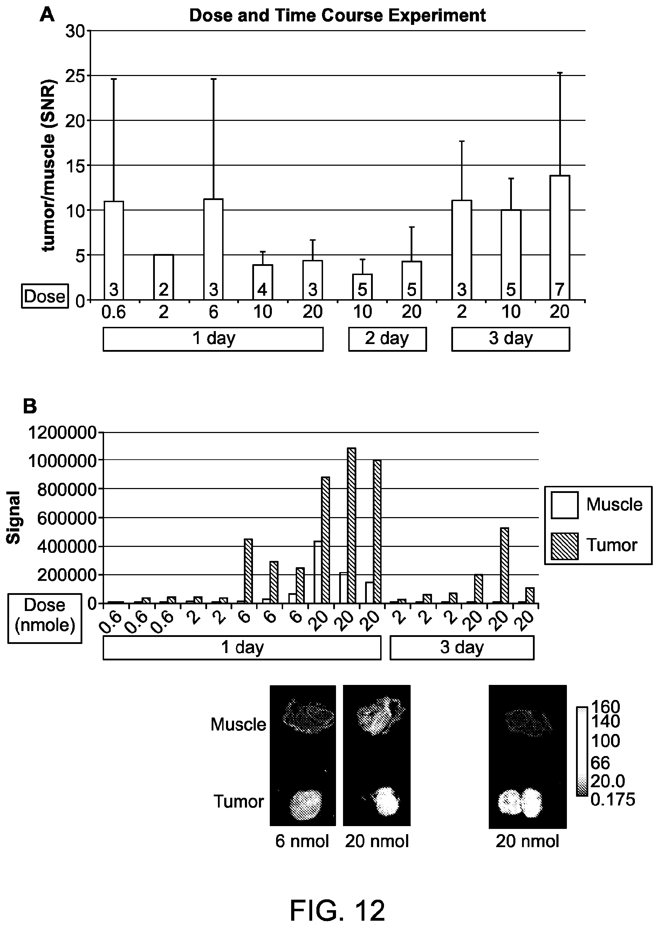

Results. Signal in tumor compared to muscle (SNR) was analyzed 1-3 days after Compound 16 injections. In the flank model, SNR at the one day time point was greatest with a 6nmol dose while SNR was highest at the three day time point with a 20nmol dose (FIG. 12). SNR was driven by the lower signal in normal muscle which was lower in the 6nmol cohort than the 20nmol group one day after injection (FIG. 12). The signal in muscle decreased considerably three days after injection. Signal in the tumor decreased after three days while the ratio of signal in tumor to muscle improved.

Normal brain, skin, heart, liver, and kidney were evaluated in a subset of animals. The tissues were excised one or three days after injection and imaged *ex vivo* using the IVIS Spectrum (FIG 57). Signal increased with increased dose for all tissues. Signal was highest in the skin, liver, and kidney.

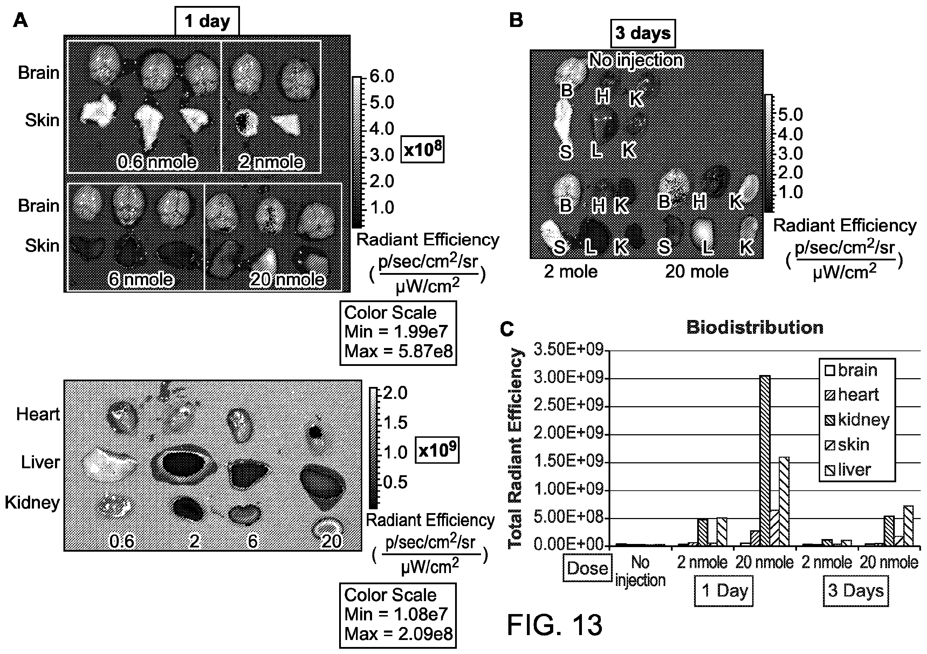

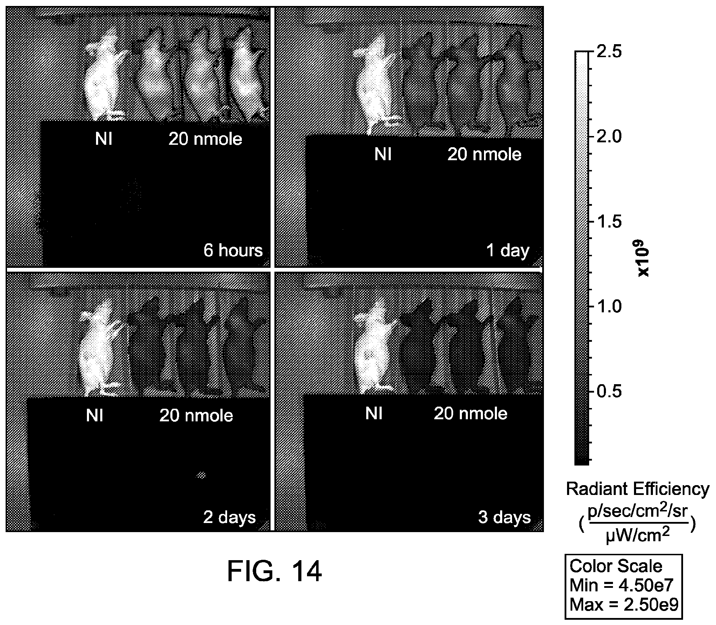

Signal declined in all tissues by the three day time point (FIG. 13). Signal in the kidney declined 5.6 fold in the 20nmol group while signal in the skin declined by 3.7 fold. Whole body live animal imaging shows rapid distribution of Compound 16 six hours after injection then a decline signal over the next three days (FIG. 14).

In order to standardize tissue thickness, the tissue was sliced in 12µm sections, scanned, and analyzed. SNR in the orthotopic samples was 12.7-200 while the SNR for the flank xenograft tissue was 246-344. The higher SNR ratios between whole tissue and sliced tissue analysis is most likely due to the very low levels of signal detected in normal brain tissue which were often below the level of detection in 12 µm sections.

Signal in tumor tissue compared to normal muscle was dose and time dependent. The optimal dose for a one day imaging time point was determined to be 6nmol while the optimal dose for a three day time point was 20nmol. Signal in normal tissues distributes rapidly after injection with the highest accumulation of Compound 16 in the kidney, liver, and skin one day after injection. Compound 16 cleared out of normal tissues with residual amounts remaining in the kidney, liver, and skin after three days.

Optimal imaging time after Compound 16 injection was assessed for doses between 0.6-and 20nmol. Mice with U87 flank xenografts were imaged 1, 2 or 3 days after injection. For one day imaging, signal in tumor compared to muscle (signal to noise ratio, SNR) was greatest at 6nmol doses. For three day imaging, SNR was greatest at 20nmol doses.

Tumor targeting and imaging efficacy of Compound 16 was assessed in the U87 orthotopic brain xenograft mouse model of glioma. Mice with U87 human glioma cells implanted in either the brain or the flank were injected with 10nmole of Compound 16. Brain and tumor tissue was excised and imaged 1 day after injection. Signal in tumor compared to normal brain was assessed on both whole tissue images and frozen tissue sliced in 12µm sections. Signal in tumor compared to normal brain (SNR) was 11.6 - 60 for the orthotopic xenograft samples and 131-138 for the flank xenograft samples (FIG. 13). Because most of the tumor tissue adhered to the skull, orthotopic sample #1 was not included in the quantitative analysis. Residual tumor cells were detected in the brain (FIG. 13).

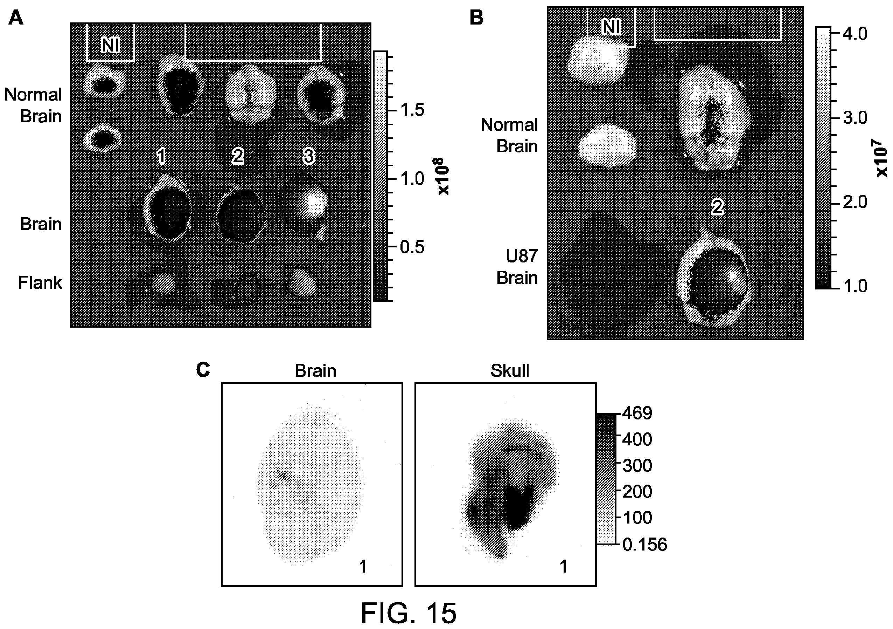

Compound 16 was detected in tumors from two out of three U87 orthotopic brain tumors using the IVIS Spectrum (FIG. 15). The brain tumor from orthotopic #1 did not have a signal on the Ivis Spectrum. The absence of signal was due to the tumor tissue adhering to the underside of the skull which was subsequently pulled out of the brain during necropsy. Compound 16 signal was detected in the skull during whole tissue imaging on the Odyssey (FIG. 15).

### EXAMPLE 8

### Dose Intervals and Imaging Intervals Using Other Chlorotoxin Conjugate Compounds for Detection of Glioma Tumors in Models

This example evaluates the optimal imaging time and dose of Compounds 1-720 in mice and further shows that Compounds 1-720 target U87 human glioma cells implanted into the brains of mice. Compounds 1-720 signal in tumor compared to normal brain (SNR) is compared to the SNR calculated in subcutaneous U87 flank xenografts using both whole tissue imaging and sliced tissue analysis.

Materials and methods are as described in Example 7.

Results. Signal in tumor compared to muscle (SNR) is analyzed 1-3 days after Compounds 1-720 injections. SNR at the one day time point is greatest with a 6nmol dose while SNR is highest at the three day time point with a 20nmol dose. SNR is driven by the lower signal in normal muscle which is lower in the 6nmol cohort than the 20nmol group one day after injection. The signal in muscle decreased considerably three days after injection. Signal in the tumor decreases after three days while the ratio of signal in tumor to muscle improves.

Normal brain, skin, heart, liver, and kidney are evaluated. Signal increases with increased dose for all tissues. Signal is highest in the skin, liver, and kidney. Histopathology analysis of mice, rats, and non-human primates treated with doses as high as 100X standard imaging dose of Compounds 1-720 finds no test article related toxicity.

Signal declines in all tissues by the three day time point. Whole body live animal imaging shows rapid distribution of Compounds 1-720 six hours after injection then a decline signal over the next three days.

Signal in tumor tissue compared to normal muscle was dose and time dependent. The optimal dose for a one day imaging time point was determined to be 6nmol while the optimal dose for a three day time point was 20nmol. Signal in normal tissues distributes rapidly after injection with the highest accumulation of Compounds 1-720 in the kidney, liver, and skin one day after injection. Compounds 1-720 clear out of normal tissues with residual amounts remaining in the kidney, liver, and skin after three days.

Optimal imaging time after injection of Compounds 1-720 is assessed for doses between 0.6-and 20nmol. Mice with U87 flank xenografts are imaged 1, 2 or 3 days after injection. For one day imaging, signal in tumor compared to muscle (signal to noise ratio, SNR) is greatest at a lower dose than the optimal dose for high SNR when imaging after three days.

Tumor targeting and imaging efficacy of Compounds 1-720 is assessed in the U87 orthotopic brain xenograft mouse model of glioma. Mice with U87 human glioma cells implanted in either the brain or the flank are injected with 10nmole of Compounds 1-720. Brain and tumor tissue is excised and imaged 1 day after injection. Signal in tumor compared to normal brain is assessed on both whole tissue images and frozen tissue sliced in 12µm sections. Signal in orthotropic brain tumors is higher than normal brain tissue using whole tissue imaging and sliced tissue analysis. Signal in flank tumors is higher than normal brain tissue using whole tissue imaging and sliced tissue analysis.

### EXAMPLE 9

### Ex vivo Image Analysis and Determination of Optimal Dose for Imaging in Dogs

This example describes the determination of optimal BLZ-100 imaging dose in dogs with naturally occurring tumors. This analysis was conducted using tissues from dogs enrolled in the study described in Example 19. The optimal clinical imaging dose is a function of the overall fluorescence intensity of the tumor, which impacts ease of detection, and the ratio of signal in the tumor tissue compared with the normal tissue in which it resides. Comparison of tumor intensity and signal to background were performed using *ex vivo* imaging on gross tumors and sections.

BLZ-100 was given intravenously at fixed doses of 0.1 - 1.5 mg in a dose escalation scheme, followed by expansion at the apparent optimal dose. To normalize for variation in body size, doses were computed in mg/m² for all dogs. Doses ( mg/m²) were 0.25 - 0.8 (7 dogs), 0.8 - 1.2 (16) and 1.2 - 1.6 (5).

The Odyssey near-infrared scanner (Li-Cor) is a flat-bed scanner that is optimized for detection of 800 nm fluorescence. It has 21 micron resolution and gives quantitative data for up to 9 orders of magnitude of intensity. This instrument was able to measure fluorescence in even the very early samples from patients treated with the lowest doses. Data from the gross tumor Odyssey scans was used to compare overall fluorescence across all doses administered. The analysis shows that at the low doses, tumor intensity is correlated with dose level. At doses up to 0.8 mg/m², signal in gross tumor samples increased as a function of dose. At doses above 0.8 mg/m², no further gain in fluorescence was apparent under these conditions. Therefore this is the lower limit of the optimal dose range for imaging canine tumors under at least these conditions.

Tumor type was the most important variable in imaging intensity at doses greater than 0.8 mg/m². A total of 21 dogs were treated with doses above this threshold. In order to compare gross fluorescence intensity across samples, a region of interest analysis was conducted using the Odyssey scans of the gross tumors. A subset of the soft tissue sarcomas had highest overall uptake, followed by the carcinomas (adenocarcinoma and squamous cell carcinoma). Oral fibrosarcomas had the lowest overall uptake, but it was unclear whether this was due to the histologic subtype or to anatomic location. There were no associations between signal and other study variables, such as breed and body mass. The data show that soft tissue sarcomas, as a class, have the highest fluorescence, with a median intensity almost threefold that of the carcinomas, and a maximum more than 7 fold higher.

In dogs treated with 0.8 mg/m² or higher (21 dogs in total), ratios of fluorescence in tumor to normal surrounding tissue ranged from <1 (no specific signal, 3 dogs) to >200, with good differentiation in several tumor types including meningioma, carcinomas (lung, thyroid, and mammary), and sarcomas. Highest signals and gross tumor to background ratios were seen in a subset of soft tissue sarcomas, suggesting preferential uptake of the conjugate in these tumor types.

The soft-tissue sarcomas showed the most intense fluorescence on gross imaging, so these tumors were selected to perform a histopathologic analysis of tumor to background ratio for determination of the upper limit of the optimal dose range. It is presumed that when tumor uptake is maximal, further dose administration will result in higher background staining and reduction in tumor to background ratio (TBR). The analysis of soft-tissue sarcomas showed that this was indeed the case.

Four soft tissue sarcoma cases were available for this analysis (Patients 11, 12, 13, and 19). Tissues were sectioned on a cryostat, and 30 micron sections were imaged on the Odyssey scanner. These sections or serial sections were stained with H&E and read by an expert histopathologist who was blinded to the fluorescence data. A grid was overlaid on the fluorescence image, and total fluorescence in each grid square was measured using Image Studio (Li-Cor) software provided with the Odyssey scanner. Overlay of the fluorescence image with the scored H&E image enabled calling of tumor vs. non-tumor for each grid square. The average of the fluorescence intensity across all tumor and non-tumor grid squares in a section was used to compute TBR for each patient. The TBR declined with increasing dose across these four tumor samples, indicating that higher doses may contribute to increased background staining and loss of specificity. The time between dose and imaging may also influence the TBR.

Dog 13 (TBR 207) had surgery 48 hours after dose administration, and dogs 11 (TBR 0.44), 12 (TBR 16), and 19 (TBR 4) had surgery 24 hours after dose administration. Excluding dog 13, comparison of the TBR for the 24 hour cases shows the same trend, supporting the overall conclusion.

Taken together, these data suggest that the optimal imaging dose for tumor imaging in dogs is in the 0.8 - 1.2 mg/m² range.

### EXAMPLE 10

### Ex vivo Image Analysis and Determination of Optimal Dose for Imaging Using Compound 16

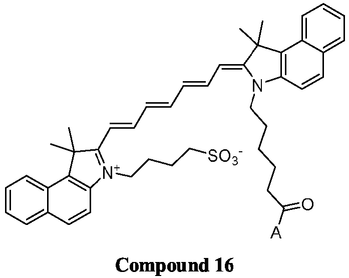

A = MCMPCFTTDHQMARRCDDCCGGRGRGKCYGPQCLCR (K-27 is point of attachment)

This example describes the determination of optimal chlorotoxin conjugate Compound 16 imaging dose in dogs with naturally occurring tumors.

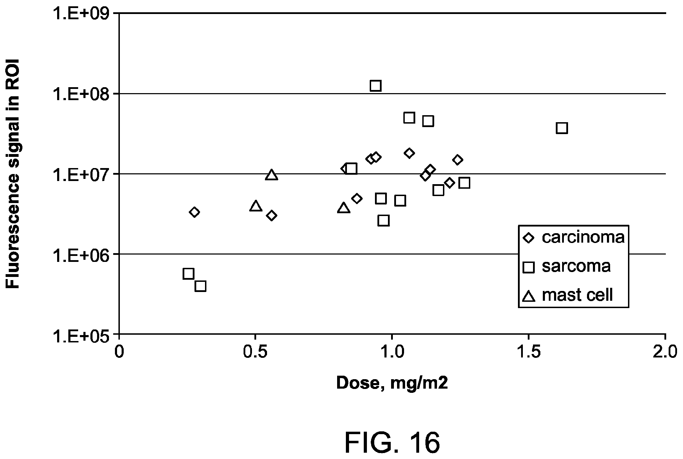

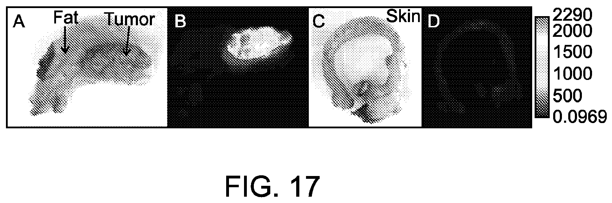

Chlorotoxin conjugate Compound 16 was given intravenously at fixed doses of 0.1 - 1.5 mg in a dose escalation scheme, followed by expansion at the apparent optimal dose. The total fluorescence within each region of interest (ROI) was plotted as a function of dose (FIG. 16). A subset of the soft tissue sarcomas had highest overall uptake, followed by the carcinomas (adenocarcinoma and squamous cell carcinoma). Oral fibrosarcomas had the lowest overall uptake, but it was unclear whether this was due to the histologic subtype or to anatomic location. Highest signals and gross tumor to background ratios were seen in a subset of soft tissue sarcomas (FIG. 17), suggesting preferential uptake of the conjugate in these tumor types.

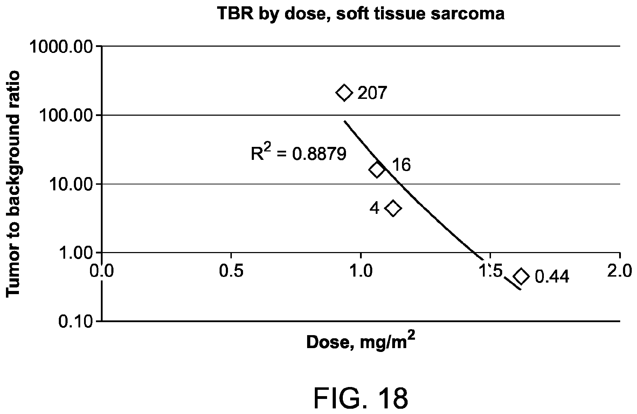

The soft-tissue sarcomas showed the most intense fluorescence on gross imaging, so these tumors were selected to perform a histopathologic analysis of tumor to background ratio for determination of the upper limit of the optimal dose range. The average of the fluorescence intensity across all tumor and non-tumor grid squares in a section was used to compute TBR for each patient. The results are shown in FIG. 18. The TBR declined with increasing dose, indicating that higher doses may contribute to increased background staining and loss of specificity.

Taken together, these data suggest that the optimal imaging dose for tumor imaging in dogs with Compound 16 is in the 0.8 - 1.1 mg/m² range.

### EXAMPLE 11

### Ex vivo Image Analysis and Determination of Optimal Dose for Imaging Using Other Chlorotoxin Conjugate Compounds

This example describes the determination of optimal imaging dose of Compounds 1-720 in dogs with naturally occurring tumors.

Compounds 1-720 are given intravenously at fixed doses, followed by expansion at the apparent optimal dose.

Materials and methods are as in Example 9, but with Compounds 1-720.

The analysis shows that at the low doses, tumor intensity is correlated with dose level. Tumor type is the most important variable in imaging intensity at higher doses. There are no associations between signal and other study variables, such as breed and body mass.

Soft-tissue sarcomas are selected to perform a histopathologic analysis of tumor to background ratio for determination of the upper limit of the optimal dose range. When tumor uptake is maximal, further dose administration results in higher background staining and reduction in tumor to background ratio (TBR).

TBR declines with increasing dose for tumor samples, indicating that higher doses may contribute to increased background staining and loss of specificity. The time between dose and imaging may also influence the TBR.

Taken together, these data establish an optimal imaging dose for tumor imaging in dogs with Compounds 1-720.

### EXAMPLE 12

### Quantitation of Fluorescence by Type of Tumor

This example describes a method for quantitating the fluorescence for various tumor types labeled with BLZ-100. Multiple canine tumor types were explored in this study in order to determine whether specific tumor types are more amenable to imaging with a chlorotoxin conjugate than others, and to gain broad experience with tumors arising in various anatomic locations and tissue types.

As an initial study of gross fluorescence intensity, the highest pixel intensity for each gross tumor sample was plotted as described above. All tumors except the meningioma (see below) were available for this analysis. Grouping the tumors by anatomic location showed that for most tumors, overall signal was related more to dose than to anatomic location. The exception was for tumors arising in bone; these were consistently low at each dose range, suggesting that there may be some tissue-specific influence on BLZ-100 uptake.

Grouping the tumors by tumor type shows that the soft-tissue sarcomas as a group show the highest fluorescence and the most variability in gross imaging. The soft-tissue sarcomas include a wide variety of histologic types and grades, so the variability seen in this class may be due to variation in histology and/or grade. They also can have high intratumor variability (see below), so some of the variation in the Odyssey data could be due to the sections of tumor that were sampled. Two patients with fibrosarcoma in the group treated with effective imaging doses had tumors arising in the jaw. Since tumors arising in the jaw generally had poor uptake, it is unclear whether the anatomic site played a role in the limited uptake and specificity in these tumors.

The intensity values for soft-tissue sarcomas (all subtypes) and the carcinomas (adenocarcinomas and squamous cell carcinomas) were compared for all cases treated with doses at or above 0.8 mg/m². This analysis revealed a high level of variability among soft-tissue sarcomas, and weaker but more consistent intensities among the carcinomas.

In dogs treated with 0.8 mg/m² or higher (21 dogs in total), ratios of fluorescence in tumor to normal surrounding tissue ranged from <1 (no specific signal) to >200.

Taken together, the gross tissue imaging data suggest that soft-tissue sarcomas are a tumor type with very high potential for the clinical utility of a chlorotoxin.

### EXAMPLE 13

### Quantitation of Fluorescence by Type of Tumor Using Chlorotoxin Conjugate Compound 16

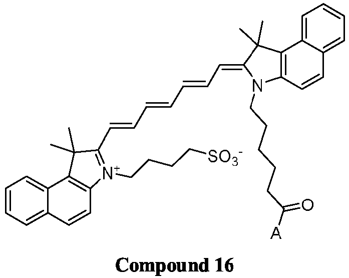

A = MCMPCFTTDHQMARRCDDCCGGRGRGKCYGPQCLCR (K-27 is point of attachment)

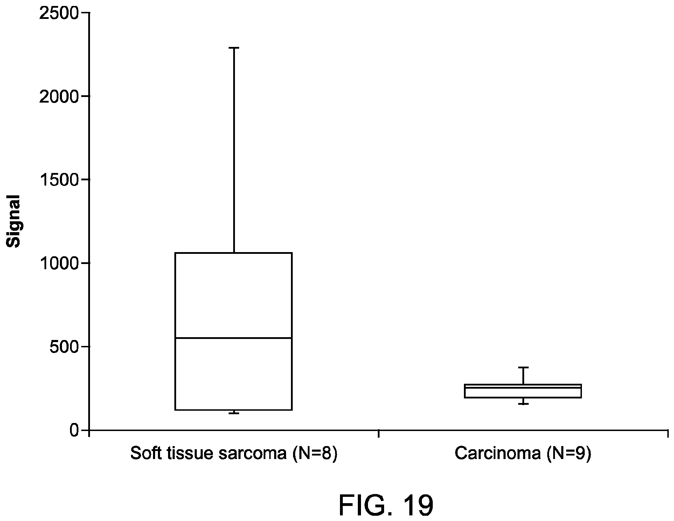

This example describes a method for quantitating the fluorescence for various tumor types labeled with chlorotoxin conjugate Compound 16. Multiple canine tumor types were explored. The intensity values for soft-tissue sarcomas (all subtypes) and the carcinomas (adenocarcinomas and squamous cell carcinomas) were compared for all cases treated with doses at or above 0.8 mg/m². This analysis shows the variability among soft-tissue sarcomas, and the lower but relatively consistent intensities among the carcinomas (FIG. 19).

Ratios of fluorescence in tumor to normal surrounding tissue ranged from <1 (no specific signal) to >200 as shown in Table 34.

**Table 34: Summary of gross imaging data for dogs treated with 0.8 mg/m² or higher (N = 21). TBR: tumor to background ratio**

| **Tumor type** | **Summary of canine tumors** |
|---|---|
| Brain tumors | Meningioma (1): TBR 2.5 |
| Head & Neck cancer | Oral squamous cell carcinoma (1): TBR 2-3 |
| Lung cancer | Adenocarcinoma (1): TBR 3 |
| Breast cancer | Mammary carcinoma (3): TBR 2.5 - 9 |
| | Mammary sarcoma (1): TBR could not be calculated due to lack of adequate normal tissue. Signal in gross tumor was very high. |
| Skin cancer | Cutaneous squamous cell carcinoma (2): TBR 2.5 - 5 |
| Soft-tissue sarcoma | Haemangiopericytoma (1): TBR 33 - 89 vs. skin, 73 - 257 vs. fat |
| | Soft-tissue sarcoma, subtype not specified (3): TBR 5 - 17 |
| | Spindle cell (1): TBR 2 |
| | Fibrosarcoma, jaw (2): TBR 0.5 - 3 (low uptake in tumors, and high background in oral mucosa) |
| | Hemangiosarcoma, vertebral body (1): TBR <1 Chondrosarcoma (1): non-specific; patient had radiation therapy prior to treatment. Tumor was necrotic and nasal mucosa had high background. |
| Other | Thyroid carcinoma (2): TBR 2 - 3 |
| | Mastocytoma (1): TBR 1.5 |

### EXAMPLE 14

### Quantitation of Fluorescence by Type of Tumor Using Other Chlorotoxin Conjugate Compounds

This example describes a method for quantitating the fluorescence for various tumor types labeled with Compounds 1-720. Multiple tumor types are explored in this study in order to determine whether specific tumor types are more amenable to imaging with a chlorotoxin conjugate than others, and to gain broad experience with tumors arising in various anatomic locations and tissue types.

Materials and methods used are as in Example 12 but with Compounds 1-720. Grouping the tumors by anatomic location shows that for most tumors, overall signal is related more to dose than to anatomic location.

Grouping the tumors by tumor type shows that the soft-tissue sarcomas as a group show the highest fluorescence and the most variability in gross imaging. The soft-tissue sarcomas include a wide variety of histologic types and grades, so the variability seen in this class may be due to variation in histology and/or grade. They also can have high intratumor variability, so some of the variation in the Odyssey data could be due to the sections of tumor that were sampled.

The intensity values for soft-tissue sarcomas (all subtypes) and the carcinomas (adenocarcinomas and squamous cell carcinomas) are compared for all cases treated with doses at or above 0.8 mg/m². This analysis shows the variability among soft-tissue sarcomas, and the lower but relatively consistent intensities among the carcinomas.

Taken together, the gross tissue imaging data suggest that soft-tissue sarcomas are a tumor type with very high potential for the clinical utility of Compounds 1-720.

### EXAMPLE 15

### Tolerability of Compound 16 in Normal Mice

This example shows experimental analysis of tolerability of Compound 16 in normal CD-1 mice.

Methods. Compound 16 was formulated in 10mM Histidine, 5% Dextrose at concentrations of 5, 0.5, 0.05 mg/ml. Chlorotoxin free peptide (KNT-01) was manufactured by Alamone Laboratory and formulated in 10mM Histidine, 5% Dextrose at a concentration of 1mM (200nmole/200µl). Compound 76 free peptide (KNT-02) was manufactured by American Peptide Company and formulated in 10 mM Histidine, 5% Dextrose at a concentration of 1mM (200nmole/200µl) (the peptide component of Compound 76 having a sequence of H-Met-Cys-Met-Pro-Cys-Phe-Thr-Thr-Asp-His-Gln-Met-Ala-Arg-Ala-Cys-Asp-Asp-Cys-Cys-Gly-Gly-Ala-Gly-Arg-Gly-Lys-Cys-Tyr-Gly-Pro-Gln-Cys-Leu-Cys-Arg-OH). Compound 16 free peptide was manufactured by Bachem and formulated in 10mM Histidine, 5% Dextrose at concentrations of 1mM, 0.1mM and 0.01mM (the peptide component of Compound 16 having a sequence of H-Met-Cys-Met-Pro-Cys-Phe-Thr-Thr-Asp-His-Gln-Met-Ala-Arg-Arg-Cys-Asp-Asp-Cys-Cys-Gly-Gly-Arg-Gly-Arg-Gly-Lys-Cys-Tyr-Gly-Pro-Gln-Cys-Leu-Cys-Arg-OH).

6-10 week old female CD-1 mice were injected with 2, 20, or 200nmol of Compound 16 diluted in 200µl of 10mM Histidine, 5% Dextrose in the tail vein. These dose levels are equivalent to 0.01, 0.1 and 1 mg of conjugate, respectively. Vehicle only was used as the control group. Mice were observed 10min, 1 hour, and 4 hours after injection and then daily until euthanasia at 3 or 14 days post dose. Mice were scored using a modified Body Condition Score (BCS) and activity level by visual inspection. Body weight was measured every 3 days. Blood was collected using terminal cardiac puncture and placed in serum separating tubes (SST microtainer). General chemistry screens were performed by Phoenix Central Laboratory. Major organs (brain, heart, kidney, liver, lungs, intestines, skin, and spleen) were dissected, placed in 10% buffered formalin, paraffin embedded, stained with hematoxylin and eosin, and evaluated by a board certified veterinary pathologist.

6-10 week female CD-1 mice were injected with 0.008, 0.08, or 0.8 mg (molar equivalent of conjugate) of native chlorotoxin (KNT-01), Compound 76 (KNT-02), or Compound 16 (KNT-03) free peptide diluted in 200µl of 10mM Histidine, 5% Dextrose in the tail vein. The mice were observed for 1 hour post injection for activity level.

Results. Mice were evaluated 10 minutes, 1 hour, and 4 hours after injection then once daily until euthanasia 3 or 14 days post treatment. Mice in the vehicle control and the 0.01 mg dose group were normal at all observation time points. Four out of six mice in the 0.1 mg group and six out of six animals in the 1 mg dose cohort exhibited a decrease in spontaneous motor activity, somnolence, and prostration approximately 1-3 minutes after the injection. Ptosis was noted in some mice. The hypoactive behavior lasted 30-60 minutes and all mice had completely recovered by the 4 hour observation time point. The mice were not unconscious or paralyzed. Breathing remained normal. Coloration remained normal without signs of cyanosis, red eyes, or lacrimation. The decrease in motor activity behavior lasted approximately 30-60 minutes after injection.

In order to ascertain if the decrease in motor activity level was due to the Compound 16 conjugate, additional mice were injected with the KNT-03 free peptide, or the related KNT-01 or KNT-02 free peptides. Similar to the Compound 16 conjugate, all of the mice injected with free peptides in the high dose (200nmol) showed a decrease in activity level, somnolence, and prostration starting 3 minutes post injection. This indicated that the hypoactive effect resulted from the peptide backbone. Because the effect was observed in the free peptide with the native sequence, the transient hypoactivity was not a novel property created by the mutated Compound 16 peptide and/or conjugation to the dye. In addition, this transient behavior was only observed in mice. Rats and non-human primates injected with similar high doses of Compound 16 did not exhibit abnormal activity levels.

With the exception of the transient low activity immediately after injection all mice were clinically normal by visual inspection for the duration of the study. All mice had BCS3 scores at each health observation indicating the mice were healthy. Mice were weighed every three days until euthanasia. No dose related changes in body weight were observed (FIG. 67).

Blood was collected from each mouse after euthanasia, at 3 and 14 days post injection. The serum was analyzed with a general chemistry screen. No dose related changes were observed. Table 35 shows serum values for kidney and liver function tests. None of the mice had BUN levels greater than 40 mg/dl. Compound 16 was detected in the liver and kidney where it was eliminated in the urine. These tests indicate that Compound 16 did not damage the kidney and liver, even at high doses.

**Table 35: Liver and Kidney Serum Chemistry Analysis**

| **Animal ID** | **Group** | **BUN ( mg/dl)** | **Creatinine ( mg/dl)** | **ALT (U/L)** | **AST (U/L)** | **GGT (U/L)** |
|---|---|---|---|---|---|---|
| **3 Days** | Control | 19 | 0.3 | 27 | 75 | 0 |
| | Control | 22 | 0.3 | 23 | 72 | 0 |
| | Control | 22 | 0.3 | 25 | 65 | 0 |
| | 0.01 mg | 24 | 0.2 | 30 | 86 | 0 |
| | 0.01 mg | 28 | 0.2 | 26 | 86 | 0 |
| | 0.01 mg | 23 | 0.4 | 29 | 77 | 0 |
| | 0.1 mg | 23 | 0.4 | 26 | 76 | 0 |
| | 0.1 mg | 25 | 0.3 | 25 | 83 | 0 |
| | 0.1 mg | 29 | 0.4 | 28 | 80 | 0 |
| | 1 mg | 22 | 0.3 | 29 | 76 | 0 |
| | 1 mg | 20 | 0.2 | 28 | 124 | 0 |
| | 1 mg | 31 | 0.4 | 35 | 74 | 0 |
| | | | | | | |
| **14 Days** | Control | 22 | 0.3 | 35 | 83 | 0 |
| | Control | 31 | 0.3 | 30 | 120 | 0 |
| | Control | 19 | 0.2 | 30 | 89 | 0 |
| | 0.01 mg | 21 | 0.2 | 26 | 80 | 0 |
| | 0.01 mg | 17 | 0.2 | 52 | 97 | 0 |
| | 0.01 mg | 24 | 0.2 | 32 | 109 | 0 |
| | 0.1 mg | 23 | 0.2 | 34 | 107 | 0 |
| | 0.1 mg | 19 | 0.2 | 30 | 117 | 0 |
| | 0.1 mg | 23 | 0.3 | 59 | 175 | 0 |
| | 1 mg | 31 | 0.2 | 40 | 81 | 0 |
| | 1 mg | 23 | 0.1 | 39 | 97 | 0 |
| | 1 mg | 20 | 0.2 | 36 | 206 | 0 |
| | | | | | | |
| **mean** | | 16 | 0.3 | 42 | 84 | 3 |
| **low** | | 9 | 0 | 18 | 45 | 0 |
| **high** | | 24 | 0.4 | 71 | 182 | 19 |

Major organs were dissected 3 and 14 days after injection. All tissue was analyzed by a certified veterinary pathologist. There were no histologic findings attributed to administration of Compound 16 on day 3 or on day 14.

### EXAMPLE 16

### Tolerability of Other Chlorotoxin Conjugate Compounds in Normal Mice

This example shows experimental analysis of tolerability of Compounds 1-720 in normal CD-1 mice.

Materials and methods are as in Example 15, but with Compounds 1-720.

Results. Mice are evaluated 10 minutes, 1 hour, and 4 hours after injection then once daily until euthanasia 3 or 14 days post treatment. Some mice exhibit a decrease in spontaneous motor activity, somnolence, and prostration approximately 1-3 minutes after the injection. In order to ascertain if the decrease in motor activity level is due to Compounds 1-720, additional mice are injected with the free peptides. All of the mice injected with free peptides show a decrease in activity level, somnolence, and prostration starting 3 minutes post injection. This indicates that the hypoactive effect results from the peptide backbone. Because the effect is observed in the free peptide with the native sequence, the transient hypoactivity is not a novel property created by Compounds 1-720 and/or conjugation to the dye. In addition, this transient behavior is only observed in mice. Rats and non-human primates injected with similar high doses of Compounds 1-720 do not exhibit abnormal activity levels.

With the exception of the transient low activity immediately after injection all mice are clinically normal by visual inspection for the duration of the study. All mice have BCS3 scores at each health observation indicating the mice are healthy. Mice are weighed every three days until euthanasia. No dose related changes in body weight are observed.

Blood is collected from each mouse after euthanasia, at 3 and 14 days post injection. The serum is analyzed with a general chemistry screen. No dose related changes are observed. None of the mice have BUN levels greater than 40 mg/dl. Compounds 1-720 are detected in the liver and kidney where they are eliminated in the urine. These tests indicate that Compounds 1-720 do not damage the kidney and liver, even at high doses.

Major organs are dissected 3 and 14 days after injection. All tissue was analyzed by a certified veterinary pathologist. There are no histologic findings attributed to administration of Compounds 1-720 on day 3 or on day 14.

### EXAMPLE 17

### Imaging of Compound 16 in ND2:SmoA1 mice with Medulloblastoma Tumors

This example shows targeting and illumination of medulloblastoma tumors and cells in a ND2:SmoA1 mouse model using Compound 16 and further shows clinical and pathological effects of high doses of Compound 16.

Medulloblastoma is the most common malignant solid tumor in children. Current therapy includes maximal safe surgical resection, irradiation, and chemotherapy. Complete surgical resection of the tumor heavily influences the prognosis of patients with medulloblastoma by conferring a 30% survival improvement over patients with residual disease. Patients with residual disease are considered high-risk for tumor progression and are treated with higher doses of radiation and chemotherapy. These patients have a greater risk of suffering from the deleterious side-effects of aggressive treatments while facing a lower chance of surviving their disease. The goal of near- complete surgical resection must be balanced with surgically accurate tumor removal because damaging healthy brain tissue could severely impair normal neurological functions. Strategies, such as Compound 16 guided surgery, are needed to improve complete and accurate tumor resection in patients with brain cancer increasing patient survival and reducing morbidity.

The ND2:SmoA1 (abbreviated SmoA1) mouse model of medulloblastoma on a C57BL/6 background was used to evaluate binding of Compound 16 to medulloblastoma tumor. These mice develop spontaneous medulloblastoma tumors in the cerebellum that closely resemble the human disease. These genetically engineered transgenic mice express a constitutively active smoothened mutant protein (SmoA1) driven by a 1-kb fragment of the neuroD2 promoter. This promoter is activated mainly in the cerebellar granule neuron precursors in the brain. This mouse model mimics the sonic-hedgehog pathway subtype of medulloblastoma. Symptomatic mice that were homozygous for the transgene were selected for enrollment in these studies. Clinical symptoms of brain cancer were detected using an open field cage evaluation. Symptoms include head tilt, hunched posture, ataxia, protruding skull, and weight loss.

To evaluate Compound 16 signal in normal brain, Nu/Nu mice received an intravenous injection of 6nmol of Compound 16. These mice were administered 60µL of 0.5 mg/ml (10nmol/100µI) through the tail vein (n = 6). One day after injection, the mice were euthanized using CO₂ inhalation. The normal brain was then removed and imaged using the IVIS Spectrum (Perkin Elmer) with the 745nm/820 nm excitation and emission filter set. Signal was analyzed using the Living Image software (Perkin Elmer) by drawing equal sized "regions of interest" (ROI's) within the brain tissue. SNR (signal to noise ratio) was calculated using the non-injected ROI and the average of the normal injected brains.

SmoA1 mice with moderate symptoms of medulloblastoma received an intravenous injection of 10nmol of Compound 16. Mice were administered 100µl of 0.5 mg/ml (10nmol/100µl) Compound 16 through the tail vein. One day after injection the mice were euthanized using CO2 inhalation. *Ex vivo* whole brain imaging was performed with the IVIS Spectrum (Perkin Elmer) using the 745 nm/820 nm excitation and emission filter set, and on the Odyssey CLx (Li-Cor Biosciences) near-infrared scanner using the 800nm setting (785nm excitation laser). The tissue was then frozen in Optimal Cutting Temperature medium (OCT, Tissue Tek) on dry ice or fixed in 10% neutral buffered formalin. The frozen tissue was sliced into 12µm sections. Tissues were either scanned using the Odyssey CLx scanner or stained with hematoxylin and eosin (H&E) according to standard histological protocols. For brains that were fixed in 10% neutral buffered formalin, the tissue was processed, embedded, sliced, and H&E stained according to standard protocols at Histology Consultation Services. Slides were scanned on the Aperio Scanscope AT (Leica Biosystems) at 20x magnification.Images were analyzed using either Living Image software (Perkin Elmer) or Image Studio software (Li-Cor Biosciences) by measuring the fluorescent signal within a region of interest (ROI) drawn in each tissue image. Signal in tumor compared to normal tissue (SNR) was calculated using tumor and cortex measurements from the same brain. Analysis was performed in both the dorsal and ventral orientations due to variations in tumor location within the brain. Signal measurements from whole tissue ROI's were used for statistical analysis. Significance values were calculated using a one-tailed T-test of unequal variance.

SmoA1 mice with medulloblastoma were injected with Compound 16 and imaged one day later. Compound 16 was higher in tumor tissue compared to normal brain in all eight of the SmoA1 mice that were enrolled in the study. Signal in tumor tissue compared to normal brain was between 5.9 and 47. Four out of eight samples had considerably lower signal in the tumor which was near the lower level of detection for the IVIS Spectrum and the Odyssey scanner. Signal in unaffected cortex was 2.7-3.6 fold higher and signal in normal brains from Nu/Nu mice was the same as non-injected animals. Small foci of metastatic leptomeningeal spread were detected using Compound 16.

Background signal in normal brain due to non-specific binding or incomplete clearance was evaluated in mice that had been injected with 6nmol of Compound 16. The non-injected animal had a background signal of 3.17 x10⁷ as expressed by radiant efficiency. One day after Compound 16 administration, the average radiant efficiency was 3.26 x 10⁷ +/-2.5 x10⁶ (n = 6) which was not appreciably higher than the brain sample that did not receive an injection with a SNR of 1.03. Compound 16 was sufficiently cleared from normal brain tissue without non-specific binding one day after injection.

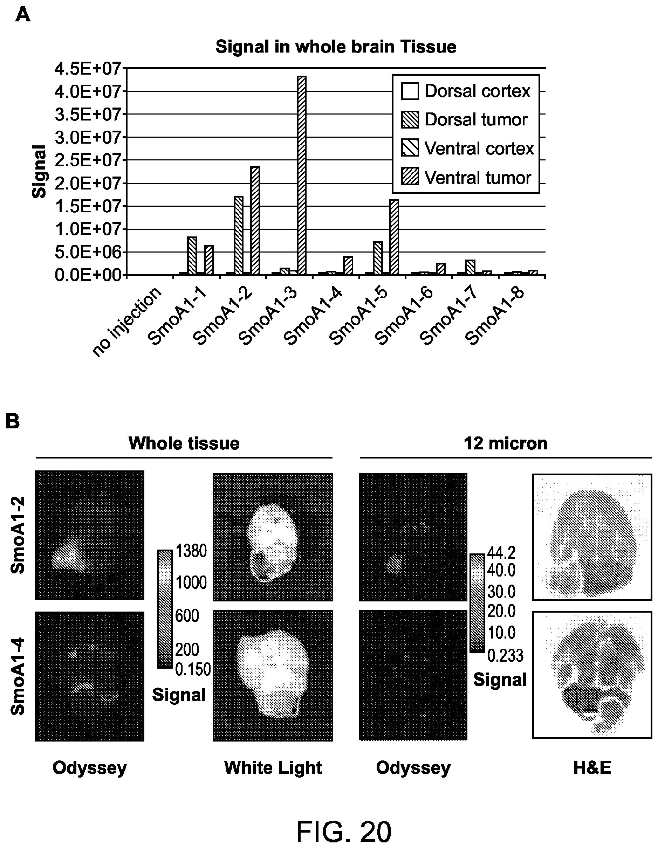

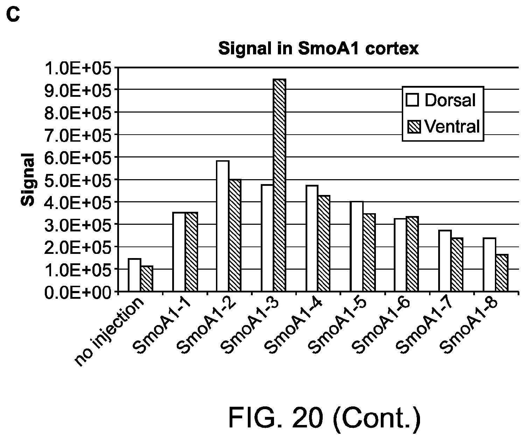

Compound 16 was analyzed in tumor bearing SmoA1 mice (n = 8) with a 10nmol dose one day after injection. Using whole brain analysis on the Odyssey, all eight samples had a higher signal in the tumor than the normal cortex (p = 0.03). The fluorescent signal was noticeably lower in four out of eight tumors and near the lower limit of detection on both the IVIS Spectrum and the Odyssey scanner (FIG. 20). Signal in tumor compared to normal brain (SNR) was between 5.9 and 47.3 (FIG. 20). Signal in the cortex of the SmoA1 brain was 2.7-3.6 fold higher than the non-injected animal whereas the signal in the Nu/Nu normal brain was the same as the non-injected animal at the same time point (FIG. 20). The higher signal was possibly caused by the higher dose of Compound 16 that was used in SmoA1 mice or abnormalities that affect the entire SmoA1 brain. The SmoA1 mice often have mild to severe hydrocephalus which often affects clearance of Compound 16 from non-tumor tissue.

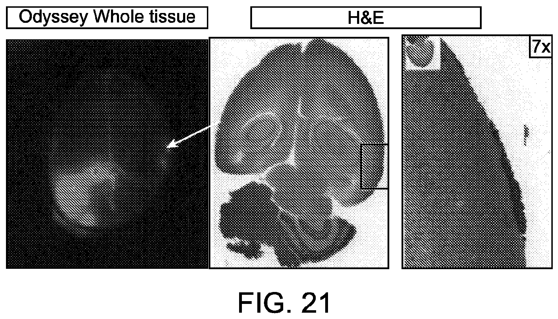

Leptomeningeal spread in SmoA1 mice was occasionally observed. These mice, like 30-35% of human patients, develop small foci of tumor cells in the meningeal membrane. Leptomeningeal spread was detected in one mouse using Compound 16. A whole brain fluorescent scan of SmoA1-1 is shown (FIG. 21). The scan illuminates small foci of fluorescence that corresponds to small clusters of tumor cells highlighted in the H&E stained slide (FIG. 21).

All mice with medulloblastoma had a higher signal in the tumor than normal cortex with SNR's ranging from 5.9-47 after administration of Compound 16. While the signal in tumor was higher than normal in all mice, four of the samples had noticeably lower signal in the tissue. Compound 16 signal was not detected in mice with normal brain tissue (Nu/Nu mice) while signal in unaffected SmoA1 cortex was 2.7-3.6 fold higher than the non-injected animals. The residual background signal in non-tumor tissue is possibly caused by hydrocephalus which occurs often in these mice. Small foci of metastatic cells were detected using Compound 16 in one mouse emphasizing Compound 16's capability to detect even small clusters of cancerous tissue.

### EXAMPLE 18

### Imaging of Other Chlorotoxin Conjugate Compounds in ND2:SmoA1 mice with Medulloblastoma Tumors

This example shows targeting and illumination of medulloblastoma tumors and cells in a ND2:SmoA1 mouse model using Compounds 1-720 and further shows clinical and pathological effects of high doses of Compounds 1-720.

The ND2:SmoA1 (abbreviated SmoA1) mouse model of medulloblastoma on a C57BL/6 background is used to evaluate binding of Compounds 1-720 to medulloblastoma tumor.

Materials and methods are as in Example 17.

SmoA1 mice with medulloblastoma are injected with Compounds 1-720 and imaged one day later. Compounds 1-720 is higher in tumor tissue compared to normal brain in all eight of the SmoA1 mice that are enrolled in the study. Background signal in normal brain due to non-specific binding or incomplete clearance is evaluated in mice that have been injected with 6nmol of Compounds 1-720. Compounds 1-720 is sufficiently cleared from normal brain tissue without non-specific binding one day after injection.

Compounds 1-720 analyzed in tumor bearing SmoA1 mice has a higher signal in the tumor than the normal cortex.

Leptomeningeal spread in SmoA1 mice is occasionally observed and is detected using Compounds 1-720.

Mice with medulloblastoma have a higher signal in the tumor than normal cortex. Compounds 1-720 signal is not detected in mice with normal brain tissue (Nu/Nu mice) while signal

Small foci of metastatic cells are detected using Compounds 1-720 emphasizing the capability of Compounds 1-720 to detect even small clusters of cancerous tissue.

### EXAMPLE 19

### Detection of Naturally Occurring Solid Tumors in Dogs

This example describes methods for detecting naturally occurring solid tumors in dogs using the fluorescently labeled chlorotoxin conjugate, BLZ-100.

Many types of canine tumors resemble human disease, including sarcomas, breast and lung cancers, mucosal squamous cell cancers, and gliomas. The diversity of these spontaneously occurring tumors in size and type, surrounding tissue, and patient body mass provides a model that is superior to the mouse in predicting the clinical characteristics of a chlorotoxin conjugate, such as BLZ-100, including tumor penetration, background staining, and effective imaging dose.

### Methods:

Twenty-eight dogs undergoing planned solid tumor resection were enrolled in the study. All options were discussed and client consent was obtained under an approved IACUC protocol. Dogs received standard of care including tumor resection with intent to control or cure local disease. Dogs received BLZ-100 intravenously 24-48 hours before surgery, and tissues were imaged *ex vivo* after surgery. *Ex vivo* imaging was performed on gross tissue specimens using the IVIS Spectrum (PerkinElmer) and the Odyssey NIR scanner (Li-Cor) to determine overall signal in tumor and gross signal to background. Tissues were embedded in OCT, sectioned on a cryostat, and scanned on the Odyssey. Serial sections were stained with H&E, and comparison with the fluorescence scans was used to validate the specificity of BLZ-100 for tumor tissue. Intraoperative imaging was conducted in several cases, using a prototype open NIR imaging device.

### Dose preparation:

BLZ-100 was prepared in a formulation buffer (100 mM histidine/5% dextrose, 10 mM Tris/5% dextrose, or 10 mM Tris/5% mannitol). For all dose batches, lyophilized conjugate was suspended in formulation buffer. The material was drawn up into a sterile syringe, and then aliquoted through a sterile 0.44 micron filter into pre-capped sterile amber glass vials. The vials were stored at -20°C, and were shipped on dry ice to the study site.

### Patients and dose administration:

Twenty-eight dogs with spontaneously-occurring solid tumors were enrolled in the study. Tumor types included several subtypes of sarcoma; oral and cutaneous squamous cell carcinomas; mast cell tumors; adenocarcinomas including lung, mammary, and thyroid; and a brain meningioma.

BLZ-100 was given intravenously 24-48 hours prior to surgery. Complete blood count, serum chemistry, and urinalysis data from prior to, and 24-48 hours post injection, were evaluated for changes that might indicate toxicity from the CTX. There were small declines in serum BUN, calcium, and potassium levels and in urine pH that reached statistical significance (two-tailed t-test); however, they remained well within the normal ranges and were not considered clinically significant. There were no other significant differences, and no overt safety concerns noted.

Nearly all dogs experienced an immediate pseudoallergy/hypersensitivity reaction within 10 minutes of dosing, characterized by erythema, mild to severe pruritus, and less commonly swelling of the muzzle and distal extremities. The severity of the reactions was not related to dose level or rate of administration. The reactions were self-limiting, ameliorated by diphenhydramine, and were completely resolved within 4 hours in all cases. These observations are consistent with a systemic release of histamine, which has been reported for a wide variety of drugs. Some dogs (mast cell tumor and CNS tumor patients) were receiving corticosteroids as part of their standard management, but corticosteroids were not required for management of reactions in any patient. No other systemic changes were identified. All dogs tolerated anesthesia and surgery normally. There were no apparent surgical complications associated with BLZ-100.

Patients 13 - 17 had surgery 2 days after BLZ-100 administration. This was done in order to evaluate the impact of time on tumor to background ratio. Since no consistent improvement was seen, the remainder of the patients had surgery 1 day after BLZ-100 administration.

### Pharmacokinetics:

Serum samples were collected at time points following dose administration. A fluorescence assay was used to calculate the serum concentration of BLZ-100. The data show a rapid distribution into tissues, followed by a slower elimination phase. These data are similar to those obtained from laboratory mice, rats, dogs, and non-human primates.

### EXAMPLE 20

### Detection of Naturally Occurring Solid Tumors in Dogs Using Chlorotoxin Conjugate Compounds

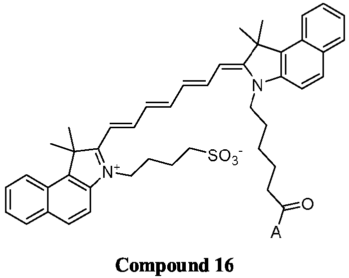

A = MCMPCFTTDHQMARRCDDCCGGRGRGKCYGPQCLCR (K-27 is point of attachment)

This example describes methods for detecting naturally occurring solid tumors in dogs using Compound 16.

Materials and methods used were as in Example 19. Patient characteristics, tumor type and site, body weight, and dose information are summarized in Table 36.

**Table 36: Summary of tumor characteristics, patient data and dose administered**

| **Patient** | **Tumor Type** | **Site** | **Breed** | **Sex** | **Age (vr)** | **Weight (kg)** | **Dose ( mg)** |
|---|---|---|---|---|---|---|---|
| 1 | Soft-tissue sarcoma | Subcutaneous | Labrador mix | M | 11.7 | 24 | 0.375 |
| 2 | Adenocarcinoma | Lymph node | Poodle mix | M | 6.5 | 6.7 | 0.1 |
| 3 | Fibrosarcoma | Subcutaneous | Rhodesian Ridgeback | F | 11.7 | 39.1 | 0.3 |
| 4 | Hemangiosarcoma | Jaw | Labrador Retriever | M | 10.7 | 30.9 | 0.3 |
| 5 | Mastocytoma | Cutaneous | Labrador Retriever | F | 10 | 30.5 | 0.5 |
| 6 | Mastocytoma | Cutaneous | Pit Bull Terrier | F | 5.3 | 26 | 0.5 |
| 7 | Adenocarcinoma | Lung | American Eskimo | F | 11 | 14.3 | 0.5 |
| 8 | Squamous cell | Jaw | English Springer Spaniel | M | 5 | 25.8 | 0.5 |
| 9 | Chondrosarcoma | Nasal | Labrador mix | F | 8.4 | 29.4 | 1 |
| 10 | Adenosquamous carcinoma | Mammary | Pit Bull Terrier mix | F | 7 | 22.1 | 0.9 |
| 11 | Soft-tissue sarcoma | Mammary | Yorkshire Terrier | F | 7 | 3.8 | 0.4 |
| 12 | Soft-tissue sarcoma | Subcutaneous | Border Collie mix | F | 3.9 | 27.9 | 1 |
| 13 | Hemangiopericyto ma | Subcutaneous | Standard Poodle | F | 7 | 33.7 | 1 |
| 14 | Squamous cell carcinoma | Cutaneous | Pit Bull Terrier | F | 11.8 | 28 | 1 |
| 15 | Squamous cell carcinoma | Jaw | Springer Spaniel | M | 8.3 | 23 | 1 |
| 16 | Fibrosarcoma | Jaw | Golden Retriever | F | 10.7 | 39.6 | 1.5 |
| 17 | Fibrosarcoma | Jaw | Chesapeak e Bay Retriever | M | 5 | 44.4 | 1.5 |
| 18 | Mastocytoma | Cutaneous | English Bulldog | F | 9 | 29.3 | 0.8 |
| 19 | Soft-tissue sarcoma | Subcutaneous | Labrador Retriever | F | 10.3 | 25.6 | 1 |
| 20 | Follicular carcinoma | Thyroid | Golden Retriever | F | 7 | 37.7 | 1 |
| 21 | Adenocarcinoma | Thyroid | Boxer | F | 6.3 | 25.2 | 1 |
| 22 | Adenocarcinoma | Mammary | Brittany Spaniel | F | 7 | 22.2 | 1 |
| 23 | Squamous cell carcinoma | Cutaneous | Golden Retriever | F | 9.1 | 34.4 | 1 |
| 24 | Adenocarcinoma | Mammary | Labrador mix | F | 13.3 | 21.8 | 0.75 |
| 25 | Soft-tissue sarcoma | Subcutaneous | Golden Retriever | F | 13.1 | 39 | 1 |
| 26 | Soft-tissue sarcoma | Subcutaneous | Chow mix | F | 6.1 | 33 | 1 |
| 27 | Meningioma | Brain | Border Collie | F | 13.2 | 17.3 | 1 |
| 28 | Hemangiosarcoma | Vertebral bodv | Golden Retriever | M | 10.8 | 32 | |

Serum samples were collected at time points following dose administration. A fluorescence assay was used to calculate the serum concentration of Compound 16. The data show a rapid distribution into tissues, followed by a slower elimination phase (Table 37).

**Table 37: Serum Compound 16 levels in dogs, measured by standard curve analysis of fluorescence at time points following dosing. Serum samples from each time point were diluted 1:1 in formulation buffer. A standard curve of Compound 16 (10 mcg/ml to 4 ng/ml) in 50% serum/50% formulation buffer was prepared. Fluorescence was measured on the Odyssey scanner (765 nm excitation/800 nm emission). Serum concentrations of the product were back-calculated using the standard curve.**

| **Nominal time point (hr):** | | **0.25** | **1** | **4** | **24** | **48** |
|---|---|---|---|---|---|---|
| **Patient** | **Dose ( mg/m²)** | ***Calculated serum BLZ-100 concentration, ng*/*ml*** | | | | |
| 1 | 0.44 | 520.49 | 31.51 | 2.21 | 1.30 | |
| 2 | 0.28 | 91.78 | 332.41 | 28.63 | 1.53 | |
| 3 | 0.25 | 1358.79 | 105.62 | 61.22 | 11.87 | |
| 4 | 0.30 | 140.20 | 48.15 | 11.42 | 2.91 | |
| 5 | 0.50 | 140.70 | 42.22 | 14.96 | 2.31 | |
| 6 | 0.56 | 161.12 | 42.53 | 6.93 | 1.20 | |
| 7 | 0.83 | 262.08 | 56.84 | 14.04 | 1.37 | |
| 9 | 1.03 | 2277.50 | 103.09 | 84.62 | 1.14 | |
| 10 | 1.12 | 634.11 | 206.93 | 12.78 | 2.92 | |
| 11 | 1.62 | 1685.95 | | 620.00 | 1.31 | |
| 12 | 1.06 | 8243.41 | 716.46 | 17.08 | 4.92 | |
| 13 | 0.94 | 862.19 | 149.60 | 14.84 | | 0.69 |
| 14 | 1.06 | 1561.45 | 193.46 | 20.00 | | 0.53 |
| 15 | 1.21 | 1668.98 | 121.85 | 20.70 | | 0.95 |
| 16 | 1.26 | 405.91 | 93.91 | 20.39 | | 1.16 |
| 17 | 1.17 | 160.69 | 164.74 | 2.89 | | 0.71 |
| 18 | 0.82 | 2084.98 | 324.36 | 29.74 | 0.81 | |
| 19 | 1.13 | 268.25 | 121.42 | 15.08 | 1.77 | |
| 20 | 0.87 | 1896.58 | 232.80 | 195.14 | 1.31 | |
| 21 | 1.14 | 928.75 | 23.97 | 3.28 | 1.02 | |
| 22 | 1.24 | 1069.51 | 129.34 | 17.07 | 0.74 | |
| 23 | 0.93 | 19698.61 | 1921.64 | 214.52 | 12.96 | |
| 24 | 0.94 | 18009.88 | 514.70 | 173.52 | 6.44 | |
| 25 | 0.85 | 666.06 | 902.32 | 60.58 | 8.33 | |
| 26 | 0.95 | 17754.16 | 1516.42 | 231.49 | 8.48 | |
| 27 | 1.47 | 3008.52 | 1183.35 | 107.49 | 11.18 | |

### EXAMPLE 21

### Detection of Naturally Occurring Solid Tumors in Dogs Using Other Chlorotoxin Conjugate Compounds

This example describes methods for detecting naturally occurring solid tumors in dogs using Compounds 1-720.

Materials and methods are as in Example 19 but with Compounds 1-720.

Serum samples are collected at time points following dose administration. A fluorescence assay is used to calculate the serum concentration of Compounds 1-720. The data show a rapid distribution into tissues, followed by a slower elimination phase. These data are similar for laboratory mice, rats, dogs, and non-human primates.

### EXAMPLE 22

### Pharmacokinetics and Tolerability of Compound 16

This example demonstrates the pharmacokinetic (PK) profile of Compound 16 following a single intravenous (IV) injection in mice, rats, dogs, and monkeys. Pilot studies performed in all four species were used to estimate exposure and inform the study design of definitive GLP studies. PK samples from these studies were analyzed using research-based methods. The PK of Compound 16 following IV administration was evaluated as part of GLP single dose toxicology studies in rats and monkeys. In the GLP studies, serum Compound 16 concentrations were determined using validated LC/MS-based methods.

In this example, Compound 16 was intravenously administered as a single-use intraoperative fluorescent imaging agent to specifically label tumor tissue. Compound 16 was sterilely formulated in a liquid at 2 mg/mL in Tris/mannitol buffer at neutral pH. The dye was chemically linked via a single lysine residue on the CTX peptide. Compound 16 contained no novel excipients or linker molecules. A tabular listing of the single-dose nonclinical toxicity studies conducted to support initiation of first-in-human (FIH) clinical trials of Compound 16 is provided in Table 38.

**Table 38: Tabular Listing of Single-Dose Toxicity Studies of Compound 16**

| **Study No.** | **Study Title** | **Testing Facility** | **GLP** | **Compound 16 Dose and Route** | **Status** |
|---|---|---|---|---|---|
| RPT0031 | Pilot Tolerability of Compound 16 and Unconjugated KNT-03 in CD1 Mice | Blaze Bioscience Seattle, WA | No | 0.01, 0.1, 1 mg IV | Completed |
| 10777 | Toxicology and Toxicokinetic Study of Compound 16 in Male Sprague Dawley Rats | Xenometrics Stilwell, KS | No | 0.03, 0.3, 1.5 mg IV | Completed |
| 10865 | Single-dose Intravenous Toxicity and Toxicokinetic Study of Compound 16 in Sprague Dawley Rats | Xenometrics Stilwell, KS | Yes | 0, 0.07, 0.7, 7 mg IV | Completed |
| BRT 20140605 | Evaluation fo the Effect of Compound 16 on Complement Activiation and Basophill/Mast Cell Activation using Canine *In vitro* Test Systems | Burleson Research Technologies Morrisville, NC | No | 0, 100, 1000, 10,000 ng/mL | Results pending |
| 10803 | Pilot Pharmacokinetics Study of Compound 16 Following IV Administration to Male Beagle Dogs. This study includes safety evaluation endpoints. | Xenometrics Stilwell, KS | No | 1 mg IV | Completed |
| 11265 | Mechanistic Study Of Pseudoallergy Response In Male Beagle Dogs From a Single IV Dose Of Compound 16 | Xenometrics Stilwell, KS | No | 1 mg IV | Results available, draft report in preparation |
| 10822 | Pilot Pharmacokinetics Study of Compound 16 Following IV Administration to Male Non-human Primates (Cynomolgus). This study includes safety evaluation endpoints. | Xenometrics Stilwell, KS | No | 0.6 mg IV | Completed |
| 814.02 | A Single Dose 14-Day Intravenous Toxicity Study of Compound 16 in Cynomolgus Monkeys | SNBL Seattle, WA | Yes | 0, 0.6, 6, 60 mg IV | Draft Report |

All species used for nonclinical safety evaluations were pharmacologically relevant for Compound 16 based on a high degree of sequence homology to the CTX peptide target. The rat and monkey were selected as the primary species for GLP toxicology studies based on the sequence homology to the CTX peptide target (97 and 100% respectively), suitability of the species for safety assessment (i.e., preference for rat over mouse), and lack of potential confounding effects (pseudoallergy/hypersensitivity reactions have been observed in the dog but not in the monkey). Toxicology studies were performed in accordance with Good Laboratory Practice (GLP) regulations. Applicable International Conference on Harmonization (ICH) and Food and Drug Administration (FDA) guidance documents were referred to during the development of the compound, most notably ICH S6(R1), S9 and M3(R2) and the FDA Guidance for Industry document "Developing Medical Imaging Drug and Biological Products Part 1: Conducting Safety Assessments".

The selection of dose levels and method of administration were based on initial mouse pharmacology models in which a dose level of 0.01 mg produced consistent tumor imaging. Since the preferred method of dose administration in the clinical trials is on a fixed basis (i.e., not adjusted for body weight or surface area), dose levels in the nonclinical safety studies were converted to fixed dose levels using estimated body surface area for each species. The dose level for humans was estimated using imaging data in mice and dogs (Table 39).

**Table 39: Estimated Imaging Dose Levels Across Species**

| **Species (~Body Surface Area (BSA) m²: kg body wt)** | **Dose ( mg)** | **Dose ( mg/m²)** | **Dose ( mg/kg)** |
|---|---|---|---|
| Mouse (0.008 m²: 0.02 kg) | 0.01 | 1.3 | 0.5 |
| Rat (0.039 m²: 0.25 kg) | 0.07 | 1.8 | 0.28 |
| Dog (1 m²: 30 kg) | 1 | 1 | 0.03 |
| Monkey (0.25 m²: 3kg) | 0.6 | 2.4 | 0.2 |
| Human | 3 | 1.9 | 0.05 |
| (1.6 m²: 60 kg) | | | |

In single-dose non-GLP pilot studies, mice displayed transiently decreased spontaneous motor activity, somnolence and prostration following intraveneous (IV) administration of 0.1 and 1 mg of Compound 16. Transient salivation was the only finding in the pilot study in male rats following IV administration of 0.03, 0.3, or 1.5 mg Compound 16 0 IV. In male dogs, notable findings in the pilot study included pseudoallergy/hypersensitivity reactions (i.e., itching/scratching, warm ears, etc.) in 2 of 2 treated dogs during or immediately after IV administration of 1 mg Compound 16. The mechanism of the pseudoallergy has been further explored in 3 male beagle dogs following IV administration of 1 mg of Compound 16. A rapid rise in plasma histamine levels was noted in all dogs, coincident with the appearance of clinical signs. No changes in complement level were seen, suggesting Compound 16 acted directly on mast cells/basophils in the dog. In the pilot study in male Cynomolgus monkeys, there were no abnormal clinical observations following IV administration of 0.6 mg Compound 16.

In single-dose GLP toxicology studies, Compound 16 was well tolerated in rats and monkeys. There were no Compound 16 -related adverse findings. The no observed adverse effect level (NOAEL) was the high dose of 7 mg, approximately 28 mg/kg, in rats and the high dose of 60 mg, approximately 20 mg/kg, in monkeys.

In safety pharmacology studies, there were minimal effects on hERG current amplitude in human embryonic kidney cells following treatment with 0.2, 0.6 or 2.0µM Compound 16 and no effects on cardiovascular parameters or respiratory rate in conscious cynomolgus monkeys in response to administration of 0.6, 6 and 60 mg Compound 16. In a tumor imaging pharmacology study with safety evaluation endpoints, most dogs with spontaneous tumors exhibited pseudoallergy/hypersensitivity reactions after IV administration of 0.1 to 1.5 mg Compound 16.

### Single-Dose Toxicity:

Pilot Tolerability of Compound 16 and Unconjugated Compound 16 in CD1 Mice. The objective of this example was to assess the tolerability of Compound 16 in groups of female CD-1. Mice (n = 3 mice/group/time point) were given a single IV bolus injection of Compound 16 at fixed dose levels of 0.01, 0.1 or 1 mg which was the equivalent to 1, 10 and 100 times the tumor imaging dose in mice. Tolerability was assessed by clinical observations, serum chemistry and histopathology of several major organs. Animals were euthanized on study days 3 and 14.

At the dose of 0.01 mg, mice were clinically normal during all observation time points. A subset of mice at 0.1 mg and all mice at 1 mg exhibited a decrease in spontaneous motor activity, somnolence, and prostration occuring as rapidly as 1 minute post-dose. The hypoactivity lasted approximately 30 to 60 minutes. All mice returned to normal by the 4 hour observation time point and remained normal for the duration of the study. Similar clinical signs were seen when mice were injected with equimolar amounts of the peptide backbone of Compound 16.

### Toxicology and Toxicokinetic Study of Compound 16 in Male Sprague Dawley Rats.

Male rats (n = 3/group) were intraveneously administered a single bolus injection of Compound 16 at fixed dose levels of either 0.03, 0.3, or 1.5 mg. Rats were observed up to 48 hours post-dose and were euthanized on day 3 of the study. Tolerability was assessed by clinical observations, hematology, serum chemistry and gross pathology.

Transient treatment-related clinical signs were limited to salivation, which was observed at the 0.03 mg dose in 2 rats at 20 minutes and 1 hour post-dose, at the 0.3 mg dose in 2 rats at 20 minutes post-dose, and at the 1.5 mg dose in 1 rat at 20 minutes, 1 hour, and 2 hours post-dose. At the 0.03 mg dose (n = 3 rats) and the 0.3 mg dose (n = 2 rats), clear oral stain was observed 1 or 2 hours post-dose. No clear dose-response relationship was evident and clinical signs were resolved at 2 hours and before 4 hours post-dose. There were no apparent test article-related hematology, clinical chemistry, or gross pathology findings at any dose level.

Single Dose Intravenous Toxicity and Toxicokinetic Study of Compound 16 in Sprague Dawley Rats. Rats (n = 10/sex/group) were intravenously administered a single bolus injection of Compound 16 at fixed dose levels of 0, 0.07, 0.7, or 7 mg. Half of the animals were observed up to day 3 of the study and were then euthanized. The remaining animals were observed up to day 15 of the study and were then euthanized (Table 40).

**Table 40: Dose Groups for Single-Dose Toxicology Study in Rats**

| **Group No.** | **No. Animals (M/F)** | **Test Article** | **Fixed Dose ( mg)** | **Dose Volume (mL/rat)** | **Dose Conc. ( mg/mL)** | **Sacri fice** | |
|---|---|---|---|---|---|---|---|
| | | | | | | **Day 3** | **Day 15** |
| A | 10/10 | Vehicle | 0 | 1.4 | 0 | 5/sex/group | 5/sex/group |
| B | 10/10 | Cmpd 16 | 0.07 | 0.35 | 0.2 | 5/sex/group | 5/sex/group |
| C | 10/10 | Cmpd 16 | 0.7 | 0.35 | 2 | 5/sex/group | 5/sex/group |
| D | 10/10 | Cmpd 16 | 7 | 1.4 | 5 | 5/sex/group | 5/sex/group |

Single Dose Intravenous Toxicity and Toxicokinetic Study of Compound 16 in Sprague Dawley Rats. There were no Compound 16-related adverse findings at any dose level based on clinical observations (e.g., including detailed open field assessments), mortality, body weights, food consumption, ophthalmology, clinical pathology (e.g., including hematology, coagulation, clinical chemistry and urinalysis), organ weights and histopathology (e.g., including the injection site). At 0.07 mg dose, a slight, transient salivation was seen in 1 male rat during open field assessment approximately 15 minutes post-dose. This observation was most likely related to Compound 16; however, no clear dose-response relationship was evident.

At the 7 mg dose, the gross pathology findings were limited to green discoloration of the kidneys on days 3 and 15 of the study. As noted above, there were no Compound 16-related findings on organ weights or microscopic findings at any dose level. The findings of green discoloration in kidneys are not considered adverse. As such, the NOAEL was considered the high dose of 7 mg, or approximately 28 mg/kg.

Evaluation of the Effect of Compound 16 on Complement Activation and Basophill/Mast Cell Activation using Canine *In vitro* Test Systems. Blood from beagle dogs (n = 5) was used to prepare high complement container serum. Compound 16, at concentrations of 0 (negative control) 100, 1000, and 10000 ng/mL, was incubated in the dog serum for 30, 60 and 120 minutes at 37 °C in 96 well plate format. Cobra venom factor, which contains native CTX, was used as a positive control. Complement activation was measured by converting intact dog C3 to the stable end product, human sC5b-9, which was quantified by ELISA. Activation of complement in dog serum by Compound 16was assessed by subtracting the total amount of complement at baseline, prior to addition of Compound 16 from residual C3 at the various timepoints.

Basophil activation was measured in whole blood samples (n = 3) isolated from dogs. Compound 16 at concentrations of 100, 1000, 10000 ng/mL was incubated with the whole blood samples for 10 or 60 minutes. Anti-canine IgE and N-formyl-methionyl-leucyl-phenylalanine (fMLP) were used as positive controls. The presence of histamine, as measured by ELISA, in the blood samples was used to assess basophil activation.

Pilot Pharmacokinetics Study of Compound 16 Following IV Administration to Male Non-Human Primates (Cynomolgus). Cynomolgus monkeys (n = 2 males/group) were intravenously administered a single bolus injection of Compound 16 at a fixed dose level of 0.6 mg. Blood samples were collected for pharmacokinetics at pre-administration, 0.083, 0.25, 0.5, 1, 2, 4, 6, 24, 48, 72 and 96 hours post-dose. Clinical observations were made at each time point. There were no abnormal clinical observations up to 96 hours post-dose

A Single Dose 14-Day Intravenous Toxicity Study of Compound 16 in Cynomolgus Monkeys. Cynomolgus monkeys (n = 3/sex/group) were intravenously administered a single bolus of Compound 16 at fixed dose levels of 0, 0.6, 6, or 60 mg and were observed for 14 days (Table 41). Ophthalmic examinations were performed prior to dosing at study day -7 and on study day 11. The animals were euthanized on study day 15 and gross post-mortem examinations were performed.

There were no Compound 16-related adverse findings at any dose level based on mortality, clinical observations, body weights, ophthalmology, clinical pathology (e.g, including hematology, coagulation, serum chemistry and urinalysis), organ weights and histopathology (e.g., including the injection site). Neurological and musculoskeletal assessments were additional parameters added to the study and no test-article related changes were noted after once weekly assessments.

**Table 41: Dose Groups for Single-Dose Toxicology Study in Monkeys**

| **Group No.** | **No. Animals\| (M/F)** | **Test Article** | **Fixed Dose ( mg)** | **Dose Volume (mL)** | **Dose Conc. ( mg/mL)** |
|---|---|---|---|---|---|
| 1 | 3/3 | Vehicle | 0 | 12 | 0 |
| 2 | 3/3 | Cmpd 16 | 0.6 | 3 | 0.2 |
| 3 | 3/3 | Cmpd 16 | 6 | 3 | 2 |
| 4 | 3/3 | Cmpd 16 | 60 | 12 | 5 |

At 60 mg, green-colored urine was noted by gross examination in one male and three females at day 3 of the study day with no corresponding impact on urinary parameters or renal pathology. Urine from all animals was subjected to an exploratory fluorescence assay, which revealed a dose dependent increase in fluorescent signal intensity on day 3 of the study in all groups treated with Compound 16 compared to the control group which was not treated with Compound 16. On day 15 of the study, the fluorescent signal was lower compared to day 3, but still detected in most mid- and high-dose animals, but not the low-dose animals when compared to the control group. These results suggest drug was present in the urine, which possibly accounted for the green appearance of urine in the high dose animals. Therefore the NOAEL was considered to be the high dose of 60 mg or approximately 20 mg/kg.

Single bolus doses of Compound 16 administered intravenously were well-tolerated in mice, rats and monkeys. Treatment-related changes observed in mice included decreased spontaneous motor activity, somnolence and prostration at doses of 0.1 and 1 mg Compound 16. The hypoactivity was transient and occurred from 30 to 60 minutes post-dose. All mice were normal by 4 hours. These changes were not observed to-date in the clinical trials or in species other than mice. Clinical signs of transient salivation were observed in a pilot study in male rats at doses of 0.03, 0.3, or 1.5 mg Compound 16 following intravenous administration. However, salivation was not observed in the subsequent single-dose study performed in male and female rats at doses up to 7 mg of Compound 16 by intravenous administration, nor was salivation observed in any other species. In addition to the toxicity evaluations, no effects on heart rate, blood pressure, respiration rate or ECG tracings were observed in a safety pharmacology study in conscious monkeys.

*In vitro* hemolysis and local tolerance studies have not been conducted. Compound 16 is a biotechnology-derived pharmaceutical candidate that is formulated with commonly-used excipients, such as Tris buffer and D-mannitol. Importantly, (1) no treatment-related hematological findings were identified in the completed nonclinical safety studies or in the preliminary data from the on-going phase 1 clinical safety study in subjects with skin cancer and (2) no irritation or lesions at the injection site were identified by macroscopic examination or by histopathology in the single-dose, intravenous administration, or toxicology studies in rats and monkeys.

Genetic toxicology studies were not conducted with Compound 16 since it is not expected to react with DNA and the formulation does not contain any novel excipients. The dye is attached to the peptide via a stable, covalent amide bond. Nothing in the structure of the peptide, dye or attachment suggests the potential of mutagenicity. In addition, typical clinical use and exposure to Compound 16 will be of short duration, consisting of a single injection per subject, perhaps a single injection during a lifetime, with an estimated human plasma terminal half-life of approximately 1-2 days, into subjects clinically diagnosed with cancer. Examination of the chromatograms from the LC/MS pharmacokinetic methods has not revealed the presence of any major metabolites of Compound 16.

The peptide component of Compound 16 is similar to native CTX. A synthetic version of the CTX peptide (TM-601) has been studied in mice and marmosets and it was well-tolerated. The NOAEL for TM-601 after a single IV dose in the mouse was 6.4 mg/kg (highest dose tested) and 2.0 mg/kg in the marmoset (highest dose tested). Repeated-dosing for 7 weeks in mice at doses of 2 and 5 mg/kg by intravenous administration resulted in clinical signs of transient ptosis and hypoactivity within 1 hour post-dose. No effects on hematology or tissue pathology were observed.

The typical doses of ICG vary by indication, but generally range from 25 to 50 mg. By comparison, Compound 16 contains roughly 0.15 mg of dye per mg of drug product. Compound 16 imaging doses are currently estimated to range from 3 to 12 mg, or 0.45 to 1.8 mg equivalents of ICG.

In the pilot studies, Compound 16 PK profiles following IV administration demonstrated a bi-exponential decline with a rapid initial phase and a longer terminal phase in all species. In mice and rats, the terminal phase could not be well defined due to low concentrations and study design/assay limitations. However, overall systemic exposure appeared to be well characterized since the majority of the systemic exposure was accounted for in the first 4 to 8 hours following IV administration. In the pilot studies of dogs and monkeys, the apparent t_{1/2} was approximately 55 hours in both species.

Following IV administration of Compound 16 in the rat and monkey single-dose GLP toxicology studies, exposure based on Cₘₐₓ and C₀ increased in an approximately dose-proportional manner across the tested ranges. AUC₀₋ₜ values increased in a dose-proportional or higher than dose-proportional manner. These observations suggested that Compound 16 clearance is reduced at higher doses.

At the highest dose group of 60 mg Compound 16 in monkeys, the serum concentration versus time profiles were adequately defined to estimate additional PK parameters dependent on characterization of the terminal phase. The overall mean t_{1/2}, CL, and Vₛₛ values were 33.7hr, 50.6mL/hr, and 211mL, respectively.

### Methods of Analysis:

Analytical Method for Quantitation of Compound 16 in Preclinical Species. Two main approaches have been used to quantify Compound 16 in serum. A fluorescence-based method was used in support of non-GLP studies in the mouse, dog, and monkey. This method was intended for research purposes only and was not subjected to method validation. Samples were analyzed in a 96-well format and quantification was achieved by comparison of measured fluorescence in the sample to a standard curve. An Odyssey CLx near infrared scanner (Li-Cor Biosciences) was used to measure signal from samples and standards, using the 800nm channel (785nm excitation). The studies in which this method was used are listed in Table 42.

**Table 42: Listing of Studies for the Analytical Method of Quantitation of Compound 16**

| **Species (Method of Administration)** | **Study Reference Number** | **Lowest Standard Used (ng/mL)** |
|---|---|---|
| CD-1 mouse (IV) | Research RPT0030 v02 | 14.6 |
| beagle dog (IV) | Xenometrics 10803 | 0.5 |
| cynomolgus monkey (IV) | Xenometrics 10822 | 0.5 |

LC/MS methods were developed and used to analyze serum from a non-GLP rat study and from GLP studies in rats and cynomolgus monkeys. The LC/MS methods were validated prior to GLP study sample analyses. The Lowest Level of Quantitation (LLOQ) for Compound 16 was 10ng/mL and 5ng/mL in rat and monkey serum, respectively. The studies in which these methods were used are listed in Table 43.

**Table 43: Listing of Studies for the LC/MS Analysis of Compound 16 in Serum**

| **Species (Method of Administration)** | **Study Reference Number** | **LLOQ (ng/mL)** |
|---|---|---|
| Sprague Dawley rat (IV) | Xenometrics 10777 | 10.0 |
| Sprague Dawley rat (IV) | Xenometrics 10865 | 10.0 |
| cynomolgus monkey (IV) | SNBL.814.02 | 5.0 |

The stability of rat serum samples stored at -70°C was at least 9 months. Stability data from the monkey support serum sample storage up to 1 month at -70°C. Incurred sample reanalysis (ISR) was performed on samples from the rat and monkey GLP studies. The rat ISR assessment passed, however the monkey ISR did not. Follow-up investigations of the ISR failure in the monkey study were conducted in an attempt to identify a root cause and assess impact of the ISR failure on the study data. No single root cause was identified, however issues with sample stability and variability from the various lots of control serum and matrix likely contributed. It was noted that the failure rate was highest in samples with relatively low concentrations of Compound 16.

Analytical Method for Quantitation of Compound 16 in Humans. Based on the methods used to detect Compound 16 in rat and monkey serum, an LC/MS assay to detect Compound 16 in human serum was developed. This method differs from the validated preclinical methods used in rat and monkey in a few aspects. First, the internal standard used in the human method is an isotope-labeled version of Compound 16 which is approximately 30Da heavier than the preclinical standard, Compound 76 (the peptide component of Compound 76 having a sequence of H-Met-Cys-Met-Pro-Cys-Phe-Thr-Thr-Asp-His-Gln-Met-Ala-Arg-Ala-Cys-Asp-Asp-Cys-Cys-Gly-Gly-Ala-Gly-Arg-Gly-Lys-Cys-Tyr-Gly-Pro-Gln-Cys-Leu-Cys-Arg-OH). Second, bovine serum albumin has been added to the standards and quality control solutions to improve stability. Third, an AB Sciex QTrap 5500 mass spectrometer was used in place of the Sciex API 5000. The LLOQ for the human assay is 10ng/mL.

Pharmacokinetic Methods. Compound 16 serum concentration versus time data were downloaded into Phoenix WinNonlin 6.3 (Pharsight, Cary, NC) for analyses using standard noncompartmental methods of intravenous bolus, intravenous infusion, or extravascular input as appropriate. Mouse and rat PK data were analyzed using the mean serum concentration versus time data. Dog and monkey PK data were analyzed by individual animal and then group summary statistics were calculated. Samples that were not analyzed for Compound 16 concentration were due to insufficient sample volume or concentration values below the limit of quantitation of the assay. Such samples were treated as missing for the purpose of calculating toxicokinetic (TK) parameters, and were not included in the calculation of group means. Nominal dose and sample collection times were used in estimating parameters.

Absorption. Compound 16 was administered IV as the intended clinical route of administration is IV.

### Distribution:

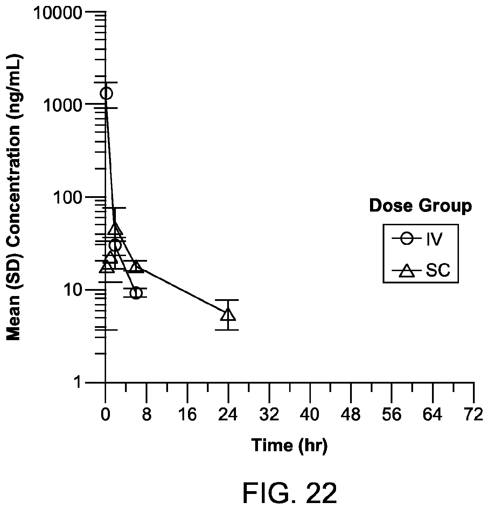

Pharmacokinetics of Intravenous Administration of Compound 16 in Female CD-1 Mice. Female mice were intravenously or subcutaneously administered a single fixed dose of 0.02 mg of Compound 16. Compound 16 was formulated in 10mM Tris/5% dextrose. Serum was collected from mice at 0.25, 2, 6 and 24 hours following administration (FIG. 22). A fluorescence-based method was used to measure Compound 16 serum concentrations.

Tolerability and Toxicokinetic Study of Compound 16 in Male Sprague Dawley Rats. Toxicokinetics (TK) were evaluated in male Sprague Dawley rats as part of a single dose tolerability study. Rats were intravenously administered a single fixed bolus dose of Compound 16 at one of two nominal dose levels, either 0.03 or 0.3 mg. Compound 16 was formulated in 10mM Tris, 5% Mannitol, pH 7.2. Animals (n = 3 per timepoint) were bled on a staggered sampling scheme at 0.083, 0.33, 1, 2, 4, 8, 24, and 48 hours after injection. An LC/MS method was used to measure Compound 16 serum concentrations.

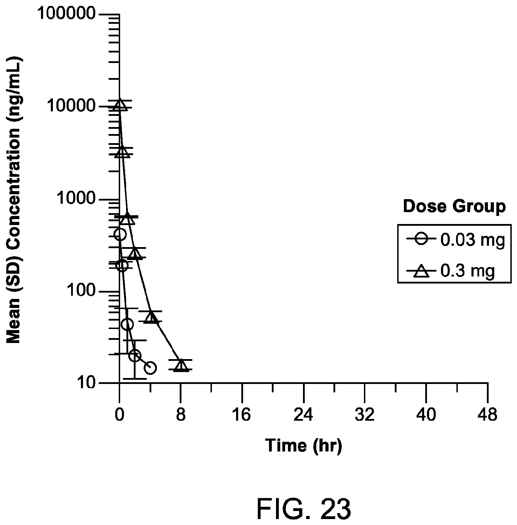

Following a single IV bolus, serum concentrations were measurable up to 4 hours in the 0.03 mg group and up to 8 hours in the 0.3 mg group (FIG. 23). Compound 16 exposure based on C₀ and AUC₀₋ₜ increased in an approximately dose-proportional manner. Due to the limited measurable concentrations available in the PK profile, parameters based on the terminal phase could not be calculated.

Biodistribution was evaluated by measuring fluorescence intensity in tissue sections from the organs collected at euthanasia 48 hours after intravenous administration. Kidney, liver, heart, and aorta were collected and fixed in 10% formalin. Tissue was stored at 4°C in 10% formalin until processed. The tissue was washed twice in PBS then processed through a sucrose gradient of 10% sucrose/PBS for 5 hours followed by 20% sucrose/PBS overnight at 4°C for cryoprotection. The tissue was then gross sectioned and frozen in OCT on dry ice. The frozen tissue blocks were sliced into 12µm sections and placed on gelatin coated slides. Slides were scanned using the Odyssey CLx imaging system (Li-Cor Biosciences) using the 800nm channel (785nm excitation). Images were analyzed using Image Studio software (Li-Cor Biosciences) by measuring the signal within a region of interest (ROI) for each sample. An average signal for three to four sections for each animal was calculated.

The signal emitted from Compound 16 was highest in the kidney which was consistent with renal clearance of the product. Less intense fluorescence was observed in liver and aorta. Signal was low in the heart. Signal increased with dose escalation in all of the tissues that were tested; however, the signal in the heart remained low even at high doses. Signal in the aorta and the great vessels of the heart was relatively high compared to the heart and increased with dose escalation. Distribution of Compound 16 to the kidney, liver, heart, and aorta did not seem to have toxicological significance. There were no corresponding changes in serum chemistry or histopathology.

Pilot Pharmacokinetics of Compound 16 Following Intravenous Administration to Male Beagle Dogs. Two male beagle dogs were intravenously administered a nominal dose of 1 mg of Compound 16. The first dog received the dose as an IV bolus and the second dog received the dose as a 15 minute IV infusion. Compound 16 was formulated in 10mM Tris, 5% Mannitol, pH 7.2. Animals were bled for PK analysis at pre-dose, 0.083, 0.25, 0.5, 1, 2, 4, 6, 24, 48, 72, and 96 hour post-dose (bolus) or post-start of infusion. A fluorescence-based method was used to measure Compound 16 serum concentrations. (Table 44).

Following a single IV bolus to Dog X1, the C₀ value was consistent with an administered dose of 0.36 mg (0.36 mg/515mL plasma volume in a dog. The t_{1/2} was approximately 54 hours, CL was 1100 mL/hr, and Vₛₛ was 29900 mL which was approximately twice total body water (Davies and Morris 1993). (Table 44).

**Table 44: Serum concentrations of Compound 16 (ng/ml) following a single intravenous dose (bolus or infusion) of 1 mg to male dogs, calculated by comparison to standard curve. BLQ = below limit of quantification. Time = 0 is pre-dose.**

| **Time (hr)** | **Dog X1 (IV bolus)** | **Dog X2 (15-min IV infusion)** |
|---|---|---|
| 0 | BLQ | BLQ |
| 0.083 | 391.89 | 54.61 |
| 0.25 | 209.5 | 225.74 |
| 0.5 | 73.74 | 36.84 |
| 1 | 68.24 | 22.89 |
| 2 | 10.43 | 6.18 |
| 4 | 2.63 | 1.61 |
| 6 | 1.58 | 1.13 |
| 24 | 1.32 | 0.56 |
| 48 | 0.81 | 0.32 |
| 72 | 0.52 | 0.21 |
| 96 | 0.44 | 0.18 |

Following a 15 minute IV infusion to Dog X2, the t_{1/2} at 57 hours was consistent with that of Dog X1. However, Cₘₐₓ was approximately 40% lower relative to the IV bolus Cₘₐₓ. Overall exposure based on AUC following the infusion was approximately 55% lower than that observed following the IV bolus dose. This corresponded to faster CL and higher Vₛₛ values relative to the IV bolus dose. It is unknown if this observed difference in CL and Vₛₛ is due to a true PK difference between the animals or dosing routes, or an artifact of issues with the dosing solution and a potentially incomplete priming of the infusion line, leading to a lower than expected administered dose. (Table 44).

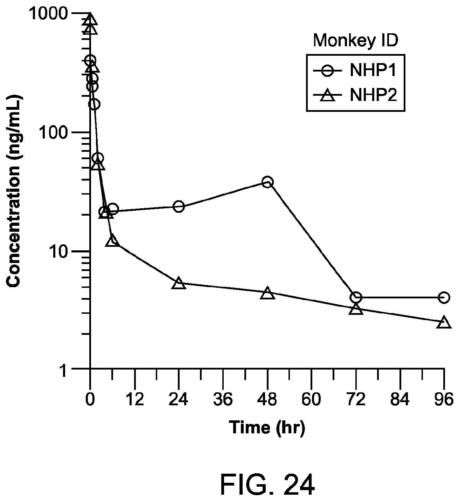

Pilot Pharmacokinetics of Compound 16 Following IV Administration to Male Non-Human Primates. Two cynomolgus monkeys were administered a nominal dose of 0.6 mg of Compound 16 as an IV bolus. Compound 16 was formulated in 10mM Tris, 5% Mannitol, pH 7.2. Animals were bled for PK analysis at predose, 0.083, 0.25, 0.5, 1, 2, 4, 6, 24, 48, 72, and 96 hours postdose. A fluorescence-based method was used to measure Compound 16 serum concentrations (FIG. 24).

The shape of monkey NHP1's serum concentration versus time profile was not what would be expected following a single IV bolus dose (Table 44). The reason is unknown and these data could not be used to calculate PK parameters that require definition of the terminal phase of the concentration versus time profile. The t_{1/2} for NHP2 was approximately 55 hours, CL was 170 mL/hr, and Vₛₛ was 6580 mL.

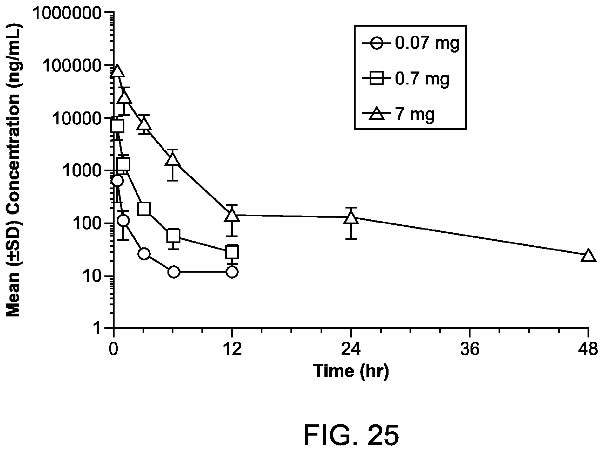

Single Dose Intravenous Toxicity and Toxicokinetic Study of Compound 16 in Sprague Dawley Rats. Male and female Sprague Dawley rats were administered a single IV bolus dose of Compound 16 at fixed doses of 0.07, 0.7, and 7 mg. Compound 16 was formulated in 10mM Tris, 5% Mannitol, pH 7.2. PK samples were obtained using a staggered sampling scheme of 3 males and 3 females per timepoint per group at pre-dose, 0.25, 1, 3, 6, 12, 24, and 48 hours post-dose (FIG. 25). Serum samples were analyzed for Compound 16 concentration via a validated LC/MS based procedure and the resulting concentration versus time data were used to estimate TK parameters using non-compartmental analysis.

Following a single fixed IV bolus dose of Compound 16, mean serum concentrations were measurable out to 12 hours post-dose with an LLOQ of10ng/mL in the 0.07 and 0.7 mg dose groups and out to 48 hours post-dose in the 7 mg dose group. Exposure based on Cₘₐₓ and C₀ increased in an approximately dose-proportional manner across the tested dose range. AUC₀₋ₜ was approximately dose-proportional between the 0.07 and 0.7 mg dose levels, and increased in a higher than dose-proportional manner between the lower dose groups versus the 7 mg dose level (see Table 45). Although Cₘₐₓ or C₀ values were variable between males and females, there was no obvious overall trend based on these parameters that would indicate an effect of sex. AUC₀₋ₜ was consistent between males and females across all dose groups.

**Table 45: Mean non-compartmental Compound 16 PK Parameters Following a Single IV Bolus Dose to Male and Female Sprague Dawley Rats. Note: Due to the unequal number of samples with measurable Compound 16 concentrations between males and females at certain timepoints, some overall dose group (male + female) exposure parameters do not equal the average of the corresponding male and female exposure values.**

| **Dose ( mg)** | **Sex** | **Cₘₐₓ (ng/mL)** | **C₀ (ng/mL)** | **AUC₀₋ₜ (hr*ng/mL)** |
|---|---|---|---|---|
| 0.07 | Male | 789 | 1500 | 821 |
| | Female | 541 | 921 | 700 |
| | **Male + Female** | **665** | **1200** | **794** |
| 0.7 | Male | 8130 | 16400 | 8130 |
| | Female | 6880 | 11000 | 7850 |
| | **Male** + **Female** | **7510** | **13400** | **8070** |
| 7 | Male | 83300 | 117000 | 127000 |
| | Female | 95400 | 155000 | 134000 |
| | **Male + Female** | **89400** | **136000** | **130000** |

A Single Dose 14-Day Intravenous Toxicity Study of Compound 16 in Cynomolgus Monkeys. Male and female cynomolgus monkeys (n = 3 males and 3 females per group) were administered a single IV bolus dose of Compound 16 at fixed doses of 0.6, 6, and 60 mg. Compound 16 was formulated in 10mM Tris, 5% Mannitol, pH 7.2. PK samples were obtained pre-dose, 0.083, 0.25, 1, 2, 4, 8, 12, 24, 36, 48, 72, 96, and 120 hours post-dose. Serum samples were analyzed for Compound 16 concentration using a validated LC/MS based procedure and the resulting concentration versus time data were used to estimate TK parameters using non-compartmental analysis.

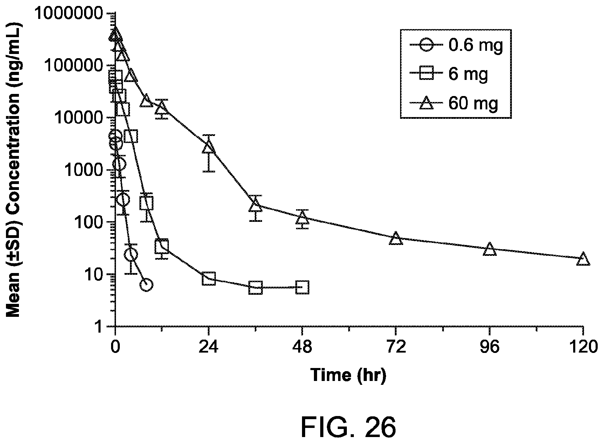

Mean serum concentrations of greater than 5 ng/mL were measured at 8, 48, and 120 hours post-dose in the 0.6, 6, and 60 mg dose groups, respectively (FIG. 26). Exposure based on C₀ and AUC₀₋ₜ was 6 to 29% higher in females relative to males across the tested dose levels (see Table 46). This is potentially due to the female monkeys' smaller size relative to the male monkeys since Compound 16 was administered as a fixed dose, rather than based on body weight. However, all dose-dependent trends in exposure were consistent between the sexes.

**Table 46: Mean Non-compartmental Compound 16 PK Parameters Following a Single IV Bolus Dose to Male and Female Cynomolgus Monkeys. ND: not determinable due to limited concentration versus time data in the terminal phase.**

| **Dose ( mg)** | **Sex** | **Cₘₐₓ (ng/mL)** | **C₀ (ng/mL)** | **AUC₀₋ₜ (hr*ng/mL)** | **t1/2 (hr)** | **CL (mL/hr)** | **Vss (mL)** |
|---|---|---|---|---|---|---|---|
| 0.6 | Male | 3930 | 4760 | 3310 | ND | ND | ND |
| | Female | 4950 | 5770 | 4280 | ND | ND | ND |
| | **Male + Female** | **4440** | **5260** | **3800** | **ND** | **ND** | **ND** |
| 6 | Male | 55400 | 67700 | 85800 | ND | ND | ND |
| | Female | 63700 | 78700 | 90000 | ND | ND | ND |
| | **Male + Female** | **60300** | **74300** | **88300** | **ND** | **ND** | **ND** |
| 60 | Male | 414000 | 433000 | 1120000 | 36.8 | 54.5 | 230 |
| | Female | 457000 | 460000 | 1360000 | 30.6 | 46.7 | 192 |
| | **Male + Female** | **436000** | **447000** | **1240000** | **33.7** | **50.6** | **211** |

Exposure based on Cₘₐₓ and C₀ generally increased in a dose-proportional manner, although the 6 mg dose level had higher than expected Cₘₐₓ and C₀ values. Exposure based on AUC₀₋ₜ values increased in a greater than dose-proportional manner across all dose groups, suggesting that Compound 16 clearance is reduced at higher doses (see Table 46). This also might be due, in part, to an incomplete characterization of the TK profile at the lowest dose level due to assay limitations. There also appeared to be a slower rate of decline in Compound 16 concentrations between 0.25 and 4 hours post-dose in the 6 mg dose group relative to the 0.6 mg dose group.

Additional non-compartmental TK parameters were able to be estimated for the 60 mg dose group. There were no substantial differences in the TK parameters between males and females. The overall mean t_{1/2}, CL, and Vₛₛ were 33.7 hour, 50.6 mL/hr, and 211mL, respectively.

Metabolism (Interspecies Comparison). No metabolism studies have been conducted with Compound 16. The ICG portion of the molecule is known to be mainly excreted unchanged into the bile, and does not undergo any appreciable metabolism.

Excretion. Urine excretion has not been formally assessed. Based on biodistribution data in select normal tissues from single dose mouse tumor models and a non-GLP single dose rat study, the kidney appears to be an important organ involved in the clearance and elimination of Compound 16. This is further supported by data from the single-dose GLP rat study in which green discolored kidneys were noted in the highest dose group 2 days after injection, and the appearance of green colored urine in the single-dose GLP monkey study.

Pharmacokinetic Drug Interaction. No drug interaction studies have been conducted with Compound 16.

Compound 16 PK profiles following IV administration demonstrated a bi-exponential decline with a rapid initial phase and a longer terminal phase in all species. Based on PK data from the rat and monkey single-dose GLP studies, Compound 16 exposure based on C₀ or Cₘₐₓ values appeared to be approximately dose proportional over the tested dose ranges. In contrast, AUC values increased in a dose-proportional or higher than dose-proportional manner, suggesting that Compound 16 clearance is reduced at higher doses. The terminal phase could be characterized in the high dose group (60 mg) in cynomolgus monkeys, allowing estimation of t_{1/2}, CL, and Vₛₛ parameters. The mean t_{1/2}, CL, and Vₛₛ values were 33.7 hours, 50.6 mL/hr, and 211mL, respectively.

There was no obvious effect of gender on Compound 16 PK in the rat, but exposure was consistently higher in female monkeys compared to male monkeys. This finding is perhaps due to the size difference in males and females since Compound 16 was administered at fixed dose levels.

### EXAMPLE 23

### Pharmacokinetics of Other Chlorotoxin Conjugate Compounds

This example demonstrates the pharmacokinetic (PK) profile of Compounds 1-720 following a single intravenous (IV) injection in mice, rats, dogs, and monkeys.

Materials and methods are as described in Example 22 but with Compounds 1-720.

Pilot Pharmacokinetics Study of Compounds 1-720 Following IV Administration to Male Non-Human Primates (Cynomolgus). Cynomolgus monkeys are intravenously administered a single bolus injection of Compounds 1-720 at a fixed dose level. Clinical observations are made at each time point.

In the pilot studies, pharmocokinetic (PK) profiles of Compounds 1-720 following IV administration demonstrate a bi-exponential decline with a rapid initial phase and a longer terminal phase in all species.

Following IV administration of Compounds 1-720 in the rat and monkey single-dose GLP toxicology studies, exposure based on Cₘₐₓ and C₀ increases in an approximately dose-proportional manner across the tested ranges. AUC₀₋ₜ values increase in a dose-proportional or higher than dose-proportional manner. These observations suggest that conjugate Compounds 1 to 720_clearance is reduced at higher doses.

Pharmacokinetics of Intravenous Administration of Compounds 1-720. In separate experiments, mice, rats, dogs and monkeys are intravenously administered as an IV bolus or IV infusion of Compounds 1-720. Serum is collected at multiple time points following administration. A fluorescence-based method is used to measure Compounds 1-720 serum concentrations. Cₘₐₓ, C₀, t_{1/2} and AUC₀₋ₜ values are obtained from the resulting data. Biodistribution is evaluated by measuring fluorescence intensity in tissue sections from the organs collected at euthanasia at time-points following intravenous administration.
Distribution of Compounds 1-720 to the organs typically does not seem to have toxicological significance, with no corresponding changes in serum chemistry or histopathology.

### EXAMPLE 24

### Pharmacokinetics of Compound 16 in Humans

This example demonstrates the pharmacokinetics of Compound 16 in human subjects with nonmelanotic skin cancer. The primary objective of the study was to evaluate the safety and tolerability of a single IV administration of Compound 16.

At the time of filing, the study was ongoing. Interim data from the first cohorts evaluated are summarized in this example.

Following a single IV administration, the maximum serum Compound 16 concentration was observed at the end of the infusion. Drug levels were detectable hours after infusion. Exposure based on Cₘₐₓ and AUC₀₋ₜ increased in a dose-dependent manner. The results indicate that pharmacokinetic data obtained using animal models is predictive of pharmacokinetics in human patients.

Overall, single IV administrations of Compound 16 were well tolerated. No significant or clear pattern of toxicities has been observed.

### EXAMPLE 25

### Pharmacokinetics of Chlorotoxin Conjugate Compounds in Humans

This example demonstrates the pharmacokinetics of a chlorotoxin conjugate in human subjects.

### Study Design:

Subjects are given intravenous (IV) bolus injections of 1 mg, 3 mg, 12 mg or 30 mg of a chlorotoxin conjugate such as Compound 16. Blood samples are collected before injection (time = 0 hours) and at 30, 60, 90, 120, 180 and 240 minutes post-injection. Samples are analyzed with fluoresence-based methods and with liquid chromatography/mass spectrometry (LC/MS) method to determine pharmacokinetic profiles of chlorotoxin conjugate in humans (FIG. 27).

Initial blood serum concentration and area under the curve data are consistent with predictions from animal models (Table 47, Table 48).

**Table 47: Initial blood serum concentration (C₀) and area under curve (AUC) percentile values at different time points following a 1 mg IV bolus dosing of Compound 16 into human patients.**

| **Parameter** | **Estimate** |
|---|---|
| C₀ | 172.00 ng/mL |
| AUC_{Last} | 85.27 hr*ng/mL |
| AUC₂₅ | 21.37 hr*ng/mL |
| AUC₅₀ | 42.67 hr*ng/mL |
| AUC₇₅ | 64.07 hr*ng/mL |

**Table 48: Times when different area under curve (AUC) percentiles are reached following a 1 mg IV bolus dosing of Compound 16 into human patients.**

| **Start Time (hr)** | **End Time (hr)** | **Label** | **Time (min)** |
|---|---|---|---|
| 0 | 0.143 | **AUC25** | **8.6** ± 5.0 |
| 0 | 0.344 | **AUC50** | **20.6** ± 7.8 |
| 0 | 0.690 | **AUC75** | **41.4** ± 15.1 |

### EXAMPLE 26

### Intraoperative Imaging and Tumor Identification

This example describes the tumor-binding specificity of BLZ-100 and the ratio between tumor and background binding by BLZ-100.

### Intraoperative imaging

A prototype intraoperative imaging system was utilized for patients 17 through 28 from the study described in Example 9. Its use during surgeries enabled imaging of tumor beds as well as tumors *in situ* and immediately following excision. The data show generally good discrimination between gross tumor and surrounding tissues. Peritumoral skin tended to have background fluorescence, while uninvolved skin had lower fluorescence. Mucosal tissues also showed background fluorescence, resulting in lack of specificity and residual non-tumor fluorescence in patient 17. Tumor bed imaging showed little or no background staining in "internal" tissues such as trachea, muscle, and fat. The intraoperative imaging showed good subjective concordance with the quantitative *ex vivo* image analysis conducted using the Odyssey scanner. The tumors that had overall high intensity and good tumor to normal ratios *ex vivo* also showed high contrast and were easy to detect intraoperatively (Table 49).

**Table 49: Summary of intraoperative imaging observations. Imaging was not performed on patient 25 due to technical issues with the instrument. TBR was calculated when both tumor and normal tissue were imaged simultaneously. ND = not determined.**

| **Patient** | **Tumor type** | **TBR (gross, *ex vivo*)** | **TBR (Intraoperative)** | **Delineation of tumor during surgery** | **Residual fluorescence in tumor bed** | **Clean surgical margins** |
|---|---|---|---|---|---|---|
| **17** | Fibrosarcoma | <1 | <1 | no | yes | yes |
| **18** | Mastocytoma | 1.5 | 1.5 | yes | no | yes |
| **19** | Soft tissue sarcoma | 5 | 19 | yes | no | yes |
| **20** | Follicular carcinoma | 2 | ND | yes | no | yes |
| **21** | Adenocarcinoma | 3 | ND | yes | no | no- invasion into vessels |
| **22** | Adenocarcinoma | 2.5 | 5 | yes, multiple lesions | no | yes |
| **23** | Squamous cell carcinoma | 5 | 8 | yes | no | yes |
| **24** | Adenocarcinoma | 2 | 3 | yes | no | yes |
| **26** | Soft tissue sarcoma | 2 | 2 | yes, multiple lesions new and previously irradiated | yes | no |
| **27** | Meningioma | 2.5 | 2.5 | yes | yes | no |
| **28** | Hemangiosarcoma | <1 | <1 | no | yes | no |

Several cases are presented as examples of the clinical utility of a chlorotoxin conjugate with intraoperative imaging.

### Patient 19, soft-tissue sarcoma

Patient 19 had a grade II soft-tissue sarcoma on her foreleg. The tumor had been clinically evident for several months without treatment. The peritumoral skin was swollen and ulcerated (FIG. 28A). Intraoperative imaging of the tumor *in situ* showed variable fluorescence in the tumor, some fluorescence in the swollen and ulcerated peritumoral skin, and little or no background fluorescence in other areas (FIGS. 28B, 28C). Imaging of the tumor immediately post excision showed roughly 8-fold variability of fluorescence intensity within the tumor (FIGS. 28D, 28E). The variation within the tumor is consistent with the pathology report that approximately 50% of the mass is replaced by eosinophilic debris (necrosis). Imaging of the tumor bed showed fluorescence in peritumoral skin; a sample of this skin was resected and sent for further imaging and histopathology. There was no residual fluorescence in the tumor bed or in the surrounding uninvolved skin (FIG. 28F). A sample of resected peritumoral skin was sent for further imaging and histopathology, which confirmed the absence of neoplastic invasion.

### Patient 22, mammary carcinoma

Patient 22 had a recurrent mammary adenocarcinoma. The tumor was removed en bloc with overlying skin and surrounding fatty tissue. Imaging of the tumor from the bottom showed that the mass is detectable through ~0.5 cm of normal fatty tissue (FIG. 29A). The diffuse appearance of the fluorescence is due to tissue scattering of the emitted light. A slice for further imaging was removed from the skin side, leaving the bulk of the mass and surrounding tissue exposed. The contrast between the tumor and surrounding tissue is improved due to the absence of intervening tissue (FIGS. 29B, 29C).

The tissue pieces collected for further imaging contained gross tumor (white areas, FIG. 29D) and adjacent tissue. Fluorescence imaging shows about 2.5-fold brighter fluorescence in the gross tumor areas compared with the adjacent tissue (FIG. 29E). The tumor bed showed no residual fluorescence (FIG. 29F). Note that the fluorescence in the skin appears brighter in panel F than in panel C; this is due to the increased sensitivity used in the survey of the tumor bed, to ensure that any residual fluorescence would be detected.

### Patient 23, cutaneous squamous cell carcinoma

Patient 23 had a cutaneous squamous cell carcinoma of the tail. The lesion had penetrated the skin, which was grossly swollen and ulcerated (FIG. 30A). The lesion was covered by a serocellular crust. Preoperative fluorescence imaging showed very little fluorescence penetrating the serocellular crust, while the peritumoral skin showed relatively bright staining (FIG. 30B). Two "fingers" of fluorescence were noted, which extended to the opposite side of the tail (FIG. 30C).

Following removal of the tail, tissues were imaged and sections were removed for further imaging. A section of central tumor (FIGS. 30D, 30E, at right) showed relatively intense fluorescence. The remaining central tumor, viewed from the side rather than through the serocellular crust, showed fluorescence intensity similar to that of the central tumor. The peritumoral skin was less intense than the tumor itself (FIG. 30F), but was about 3-fold more intense than uninvolved skin. Samples of skin from the fluorescent areas on the opposite side of the tumor were submitted for histopathology, and they did not contain tumor.

### Patient 20, thyroid carcinoma

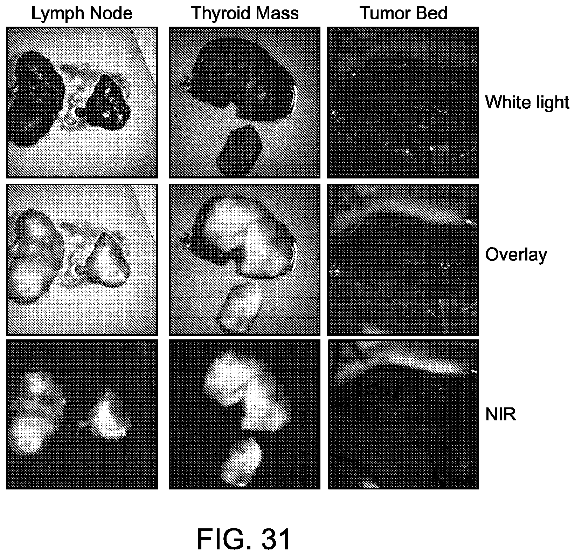

Patient 20 had a thyroid carcinoma. The entire thyroid gland was removed, along with an enlarged lymph node. Intraoperative imaging of the thyroid showed most of the gland was fluorescent, with about 2-fold variation in signal intensity throughout (FIG. 31). The lymph node had regions of fluorescence that were comparable to the primary tumor. The tumor bed had no residual fluorescence. Additionally, no significant non-specific background fluorescence was observed in the internal structures including the trachea, nerves, and arteries. Histopathology showed that the thyroid was 95% effaced by the tumor, which had large areas of blood filled spaces and necrosis. These may account for the variability in staining seen in the primary tumor. The lymph node was confirmed to contain metastatic disease.

### EXAMPLE 27

### Specificity and Tumor to Background Ratio of Other Chlorotoxin Conjugate Compounds

This example describes the tumor-binding specificity of Compounds 1-720 and the ratio between tumor and background binding by Compounds 1-720.

### Intraoperative imaging

Methods and materials used are as described in Example 26, but with Compounds 1-720. A prototype intraoperative imaging system is utilized. Its use during surgeries enables imaging of tumor beds as well as tumors *in situ* and immediately following excision. The data show generally good discrimination between gross tumor and surrounding tissues. Peritumoral skin tends to have background fluorescence, while uninvolved skin has lower fluorescence. Tumor bed imaging shows little or no background staining in "internal" tissues such as trachea, muscle, and fat. The intraoperative imaging shows good subjective concordance with the quantitative *ex vivo* image analysis conducted using the Odyssey scanner. The tumors that have overall high intensity and good tumor to normal ratios *ex vivo* also show high contrast and are easy to detect intraoperatively.

### EXAMPLE 28

### Histopathologic Scoring, Sensitivity and Specificity

This example describes the evaluation of tumor and adjacent tissues at the cellular level in order to assess sensitivity and specificity of a chlorotoxin conjugate, such as BLZ-100 for cancer cells.

Evaluation of canine tumor and adjacent tissues at the cellular level was performed in order to assess sensitivity and specificity of BLZ-100 for cancer cells. For this analysis, two cutaneous squamous cell carcinomas, three mammary cancers, and four subcutaneous soft tissue sarcomas were included. Sensitivity and specificity was calculated using a grid analysis on 30 micron frozen sections. An overlay of each fluorescence image with the corresponding H&E stained image that was scored as tumor or normal by a histopathologist was analyzed. This analysis was performed separately for each case.

The data were grouped by individual section and plotted for each patient. The subcutaneous soft tissue sarcomas showed highly specific tumor fluorescence. A logistic regression analysis was used to determine a reasonable threshold intensity for detecting tumor in the subcutaneous soft-tissue sarcomas. Non-skin tissues were used to compute sensitivity and specificity, with a threshold intensity of 30,000 used as a cutoff value. Grid squares were called tumor or no tumor based on fluorescence intensity and based on pathologist call. Concordant and discordant calls are used to calculate sensitivity and specificity. Sensitivity (95%) and specificity (85%) were very good using a threshold grid square fluorescence of 30,000. Peritumoral skin in patient 19 was above this threshold in all grid squares; as discussed in Example 26, this patient had an ulcerated tumor and grossly edematous skin immediately adjacent to the mass. The elevated signal in this patient's skin sample accounted for all above-threshold data points in this analysis.

The cutaneous squamous cell carcinomas had fluorescence signal coming both from the tumor and from the underlying dermis. In most cases, the signal was brighter in the underlying dermis, leading to the "inverted" tumor vs. normal intensity. Although histologic specificity in these tumors is low, truly uninvolved skin seen during intraoperative imaging in patient 23 and during *ex vivo* imaging in patient 14 was not fluorescent. Analysis of the mammary tumors shows that, like skin, mammary tissue adjacent to tumor takes up BLZ-100. In both tumor types, gross tumors had higher fluorescence than uninvolved tissue.

For this analysis, two cutaneous squamous cell carcinomas, three mammary cancers, and three subcutaneous soft-tissue sarcomas were included. Tissues were sectioned on a cryostat, and 30 micron sections were imaged on the Odyssey scanner. These sections or serial sections were stained with H&E and read by an expert histopathologist who was blinded to the fluorescence data. A grid was overlaid on the fluorescence image, and total fluorescence in each grid square was measured using Image Studio (Li-Cor) software provided with the Odyssey scanner. Overlay of the fluorescence image with the scored H&E image enabled calling of tumor vs. non-tumor for each grid square.

For each tumor, grid analysis was done on sections from different areas of tumor and adjacent non-tumor tissue, as well as samples of uninvolved tissue when available. As discussed above, background staining in the swollen, ulcerated skin in patient 19 caused suspicion of tumor infiltration during the surgery. The fluorescence analysis in sections of this skin would lead to the same conclusion. Note that the uninvolved skin samples from patients 12 and 13 are well below the threshold for being incorrectly identified as tumor tissue.

### EXAMPLE 29

### Histopathologic Scoring, Sensitivity and Specificity of Chlorotoxin Conjugate Compounds

This example describes the evaluation of tumor and adjacent tissues at the cellular level in order to assess sensitivity and specificity of a chlorotoxin conjugate, such as Compound 16 for cancer cells.

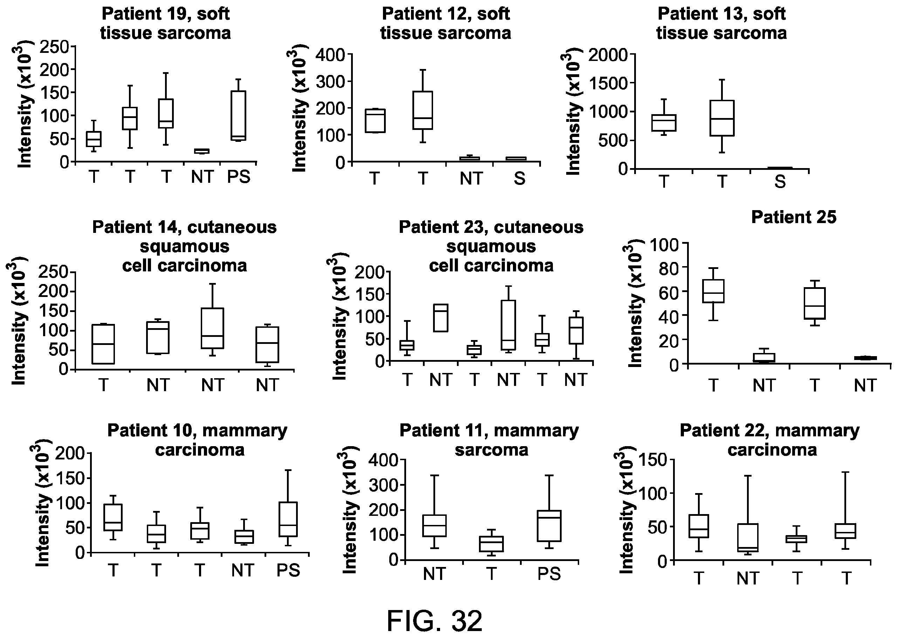

Materials and methods used were as described in Example 28. FIG. 32 shows box & whiskers plots of fluorescence intensity in grid squares from multiple patients and for each tissue section analyzed (T, tumor. NT, adjacent non-tumor tissue. PS, peritumoral skin. S, uninvolved skin). Sensitivity (95%) and specificity (85%) were very good using a threshold of 30,000 (arbitrary units). Peritumoral skin in patient 19 was above this threshold in all grid squares. This patient had an ulcerated tumor and grossly edematous skin immediately adjacent to the mass. The elevated signal in this patient's skin sample accounted for all above-threshold data points in this analysis.

Grid squares were called tumor or no tumor based on fluorescence intensity and based on pathologist call. Concordant and discordant calls are used to calculate sensitivity and specificity. The results are shown in Table 50.

**Table 50: Sensitivity and specificity analysis for subcutaneous soft-tissue sarcoma samples. Kappa coefficient (95% CI): 0.730 (0.543, 0.917); Sensitivity (95% CI): 94.1% (88.2%, 97.6%); Specificity (95% CI): 100.0% (71.5%, 100.0%).**

| | **Tumor (pathologist)** | **No Tumor (pathologist)** | **Total** |
|---|---|---|---|
| Tumor (intensity) | 111 | 0 | 111 |
| No Tumor (intensity) | 7 | 11 | 18 |
| Total | 118 | 11 | 129 |

### EXAMPLE 30

### Histopathologic Scoring, Sensitivity and Specificity of Other Chlorotoxin Conjugate Compounds

This example describes the evaluation of tumor and adjacent tissues at the cellular level in order to assess sensitivity and specificity of a chlorotoxin conjugate, such as Compounds 1-720 for cancer cells.

Evaluation of canine tumor and adjacent tissues at the cellular level is performed in order to assess sensitivity and specificity of Compounds 1-720 for cancer cells. Sensitivity and specificity are calculated using a grid analysis on 30 micron frozen sections. An overlay of each fluorescence image with the corresponding H&E stained image that was scored as tumor or normal by a histopathologist is analyzed. This analysis is performed separately for each case. Cutaneous squamous cell carcinomas, mammary cancers, and subcutaneous soft-tissue sarcomas are evaluated. Tumor tissue and tissue adjacent to tumors has higher mean fluorescence than does normal tissue.

### EXAMPLE 31

### Labeling of Additional Types of Tumors

This example describes the use of BLZ-100 for the labeling of other miscellaneous tumor types not described in the preceding examples. Besides the above-described tumor types, there were several tumor types for which the number of patients was insufficient to conduct meaningful sensitivity and specificity analysis. These include mast cell tumors (N = 1 at effective dose), lung cancer (N = 1), and meningioma (N = 1). There were two thyroid carcinomas in which signal in 30 micron sections was too low to permit the analysis. The oral tumors were determined to be non-specific on gross imaging.

Lung cancer is of potential interest clinically. The results of gross imaging in the canine lung cancer suggest specific tumor uptake, with 3:1 TBR compared with adjacent lung or with uninvolved skin. There was a small suspected metastasis seen in the adjacent lung tissue. On histopathologic analysis, the signal intensity was low but measurable, and was specific for tumor in the primary mass. The suspected metastasis could not be confirmed due to frozen section artifact.

Brain tumors are of very high interest for clinical and commercial development of a chlorotoxin conjugate. There was one brain tumor case enrolled in the study, a meningioma. Meningiomas are extra-axial tumors with typically low histologic grade in dogs as well as in humans. Intraoperative imaging showed signal in the chlorotoxin conjugate-labeled tumor with a 2.5-fold tumor to background (normal brain) ratio. The surgical approach was through the sinus, so nasal mucosa was present in the image. The mucosal tissues are a known source of background, and in this case provided a positive control for fluorescence intensity. Tumor was removed in fragments; pieces to be analyzed were embedded in OCT and snap-frozen. Imaging of 30 micron sections showed low but detectable signal. The optimal dose for CNS tumors arising within the blood-brain barrier will have to be determined with additional subjects, including cases with malignant tumors such as glioma. However, this case did provide an opportunity for imaging of normal brain tissue, and it demonstrated that a low-grade brain tumor can be successfully imaged. This is significant, since complete resection in low-grade tumors can be curative.

### EXAMPLE 32

### Labeling of Additional Types of Tumors Using Chlorotoxin Conjugate Compounds

This example describes the use of Compound 16 for the labeling of other tumor types.

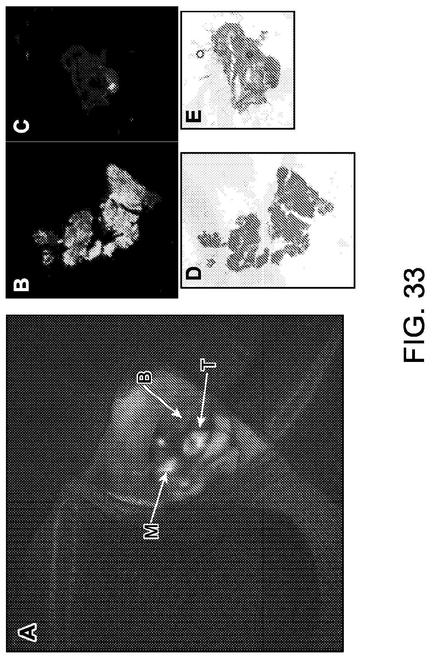

Intraoperative imaging of a canine meningioma showed signal in the Compound 16-labeled tumor (FIG. 33A), with a 2.5-fold tumor to background (normal brain) ratio. The surgical approach was through the sinus, so nasal mucosa was present in the image. The mucosal tissues are a known source of background, and in this case provided a positive control for fluorescence intensity. Tumor was removed in fragments; pieces to be analyzed were embedded in OCT and snap-frozen. Imaging of 30 micron sections showed low but detectable signal (FIGS. 33B, 33C). H&E stained sections are shown for comparison (FIGS. 33D, 33E). This case demonstrated that a low-grade brain tumor can be successfully imaged.

### EXAMPLE 33

### Labeling of Additional Types of Tumors Using Other Chlorotoxin Conjugate Compounds

This example describes the use of Compounds 1-720 for the labeling of miscellaneous tumor types, such as mast cell tumors, lung cancer, meningioma, thyroid carcinomas, and oral tumors.

Intraoperative tumor imaging shows signals emitted by Compounds 1-720. The tumors are removed in fragments; pieces to be analyzed are embedded in OCT and snap-frozen. Imaging of 30 micron sections shows low but detectable signal. H&E stained sections are used for comparison. Tumors are successfully identified using Compounds 1-720 and optimal doses are determined.

From the foregoing, it will be appreciated that, although specific embodiments of the invention have been described herein for purposes of illustration, various modifications may be made without deviating from the claimed invention. Accordingly, the invention is not limited except as by the appended claims.

### References

1. Deroose, J. P., Burger, J. W. A., van Geel, A. N., den Bakker, M. A., de Jong, J. S., Eggermont, A. M. M., and Verhoef, C. (2011) Radiotherapy for soft-tissue sarcomas after isolated limb perfusion and surgical resection: Essential for local control in all patients? Annals of Surgical Oncology 18, 321-327.
2. Paoloni, M. C., and Khanna, C. (2007) Comparative oncology today. Vet Clin North Am Small Anim Pract 37, 1023-v
3. Gordon, I., Paoloni, M., Mazcko, C., and Khanna, C. (2009) The Comparative Oncology Trials Consortium: Using spontaneously occurring cancers in dogs to inform the cancer drug development pathway. PLoS Med 6, e1000161
4. Goodman & Gilman's The Pharmacological Basis of Therapeutics. , McGraw-Hill
5. Hargis, A., and Thomassen, R. (1979) Animal model: Solar dermatosis (keratosis) and solar dermatosis with squamous cell carcinoma. Am J Pathol 94, 193-196
6. Culard, J.-F., Basset-Seguin, N., Calas, B., Guilhou, J.-J., and Martin, F. (1992) Characterization and subcellular localization of calcium-dependent phospholipid binding proteins (annexins) in normal human skin and reconstituted epidermis. J Invest Dermatol 98, 436-441
7. Munz, B., Gerke, V., Gillitzer, R., and Werner, S. (1997) Differential expression of the calpactin I subunits annexin II and p11 in cultured keratinocytes and during wound repair. J Invest Dermatol 108, 307-312
8. Vail, D. M. (2004) Veterinary Co-operative oncology group. Vet Comp Oncol 2, 194-213
9. Dernell, W. S., Withrow, S. J., Kuntz, C. A., and Powers, B. E. (1998) Principles of treatment for soft-tissue sarcoma. Clinical Techniques in Small Animal Practice 13, 59-64
10. Motta, L., Mandara, M. T., and Skerritt, G. C. (2012) Canine and feline intracranial meningiomas: An updated review. The Veterinary Journal 192, 153-165
11. Lyons, S. A., O'Neal, J., and Sontheimer, H. (2002) Chlorotoxin, a scorpion-derived peptide, specifically binds to gliomas and tumors of neuroectodermal origin. Glia 39, 162-173
12. Stroud, M. R., Hansen, S. J., and Olson, J. M. (2011) In vivo bio-imaging using chlorotoxin-based conjugates. Curr. Pharm. Des. 17, 4362-4371
13. Veiseh, M., Gabikian, P., Bahrami, S.-B., Veiseh, O., Zhang, M., Hackman, R. C., Ravanpay, A. C., Stroud, M. R., Kusuma, Y., Hansen, S. J., Kwok, D., Munoz, N. M., Sze, R. W., Grady, W. M., Greenberg, N. M., Ellenbogen, R. G., and Olson, J. M. (2007) Tumor Paint: A chlorotoxin:Cy5.5 bioconjugate for intraoperative visualization of cancer foci. Cancer Res. 67, 6882-6888
14. Veiseh, O., Sun, C., Fang, C., Bhattarai, N., Gunn, J., Kievit, F., Du, K., Pullar, B., Lee, D., Ellenbogen, R. G., Olson, J., and Zhang, M. (2009) Specific targeting of brain tumors with an optical/magnetic resonance imaging nanoprobe across the blood-brain barrier. Cancer Res. 69, 6200- 6207
15. Hockaday, D. C., Shen, S., Fiveash, J., Raubitschek, A., Colcher, D., Liu, A., Alvarez, V., and Mamelak, A. N. (2005) Imaging glioma extent with 131I-TM-601. The Journal of Nuclear Medicine 46, 580-586
16. Mamelak, A. N., Rosenfeld, S., Bucholz, R., Raubitschek, A., Nabors, L. B., Fiveash, J. B., Shen, S., Khazaeli, M. B., Colcher, D., Liu, A., Osman, M., Guthrie, B., Schade-Bijur, S., Hablitz, D. M., Alvarez, V. L., and Gonda, M. A. (2006) Phase I single-dose study of intracavitary-administered iodine-131-TM-601 in adults with recurrent high-grade glioma. Journal of Clinical Oncology 24, 3644-3650

## Claims

1. A compound having the structure of Formula (III), or a pharmaceutically acceptable salt thereof: 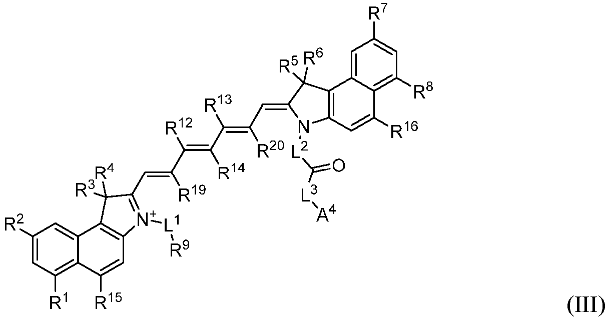 wherein:
R¹, R², R³, R⁴, R⁵, R⁶, R⁷, R⁸ , R¹⁵, and R¹⁶ are each independently selected from hydrogen, C₁-C₆ alkyl, C₁-C₆ alkylene-COOH, sulfonate, -COOH, -SO₂-NH₂, or C₁-C₆ alkoxy;
R⁹ is sulfonate;
L¹ is C₃-C₆ alkylene;
L² is C₁-C₁₀ alkylene;
L³ is a bond, -O-, -NR¹⁰-, -NR¹⁰-C₁-C₆ alkylene-, -O-NR¹⁰-, -NR¹⁰-C₁-C₆ alkylene- (O-C₁-C₆ alkylene)ₙ-, -NR¹⁰-L⁴-, -NR¹⁰-C₁-C₆ alkylene-NR¹¹- (C (= O) -C₁-C₆ alkylene-O-)ₘ-, or -NR¹⁰-C₁-C₆ alkylene--NR¹⁰-C₁-C₆ alkylene--NR¹⁰-C₁-C₆ alkylene-;
L⁴ is a bond, -heterocyclyl-, or -heterocyclyl-C₁-C₆ alkylene-;
R¹⁰ is hydrogen or C₁-C₆ alkyl;
R¹¹ is hydrogen or C₁-C₆ alkyl;
R¹² and R¹³ are independently selected from hydrogen, C₁-C₆ alkyl, or R¹² and R¹³ are joined together along with the other atoms to which they are attached to form a 5-membered or 6-membered carbocyclic or heterocyclic ring;
R¹⁴ is hydrogen or C₁-C₆ alkylene, -(L⁵)-aryl, -(L⁵)-heteroaryl, -NR¹⁷ R¹⁸, R¹⁴ and R¹⁹ are joined together along with the other atoms to which they are attached to form a 5-membered or 6-membered carbocyclic or heterocyclic ring, or R¹⁴ and R²⁰ are joined together along with the other atoms to which they are attached to form a 5-membered or 6-membered carbocyclic or heterocyclic ring;
L⁵ is a bond, C₁-C₁₀ alkylene, -O-, or -NR¹⁰-;
R¹⁷ and R¹⁸ are each independently hydrogen or aryl;
R¹⁹ and R²⁰ are independently selected from hydrogen, C₁-C₆ alkyl, R¹⁴ and R¹⁹ are joined together along with the other atoms to which they are attached to form a 5-membered or 6-membered carbocyclic or heterocyclic ring, or R¹⁴ and R²⁰ are joined together along with the other atoms to which they are attached to form a 5-membered or 6-membered carbocyclic or heterocyclic ring;
n is 0, 1, 2, or 3;
m is 0, 1, 2, or 3;
p is 0, 1, 2, or 3;
q is 0, 1, 2, or 3; and
A⁴ is a polypeptide having at least 90% sequence identity with SEQ ID NO: 9 or a fragment thereof, wherein the fragment of A⁴ comprises at least 25 amino acid residues.

2. The compound of claim 1,
wherein:
R³, R⁴, R⁵, and R⁶ are each independently methyl;
R¹, R², R⁷, R⁸, R¹⁵, and R¹⁶ are each independently hydrogen;
R¹⁰ is hydrogen; and
R¹², R¹³, R¹⁴, R¹⁹, and R²⁰ are each independently hydrogen;
wherein:
L¹ is butylene and L² is pentylene;
or combinations thereof.

3. The compound of any one of claims 1 or 2 having the structure of any one of Formulas (VII), (VIII), (IX), (XI), (XIII), or (XIV):

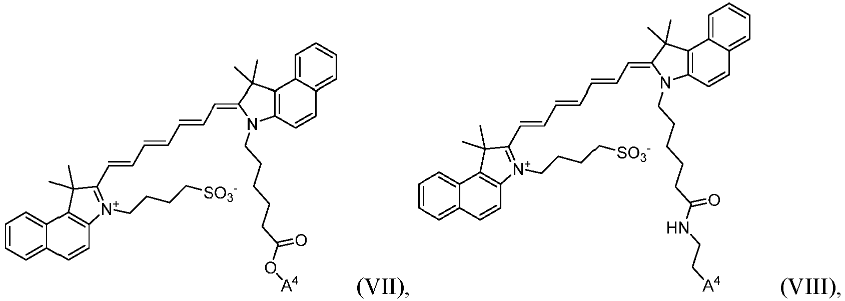

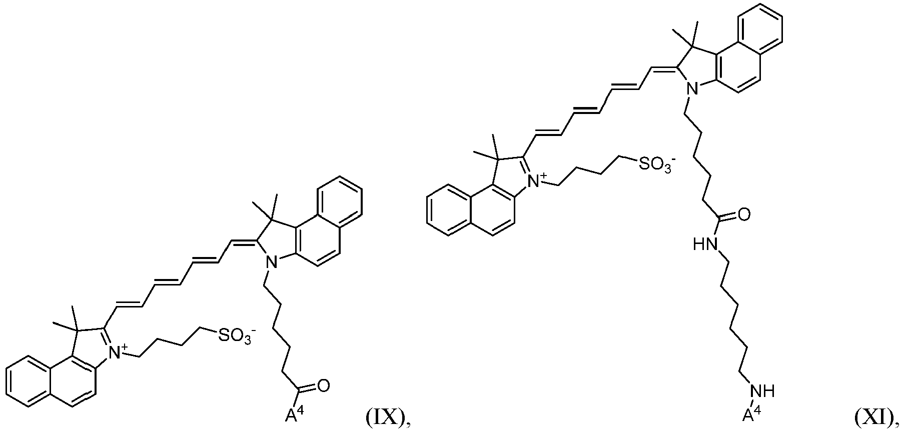

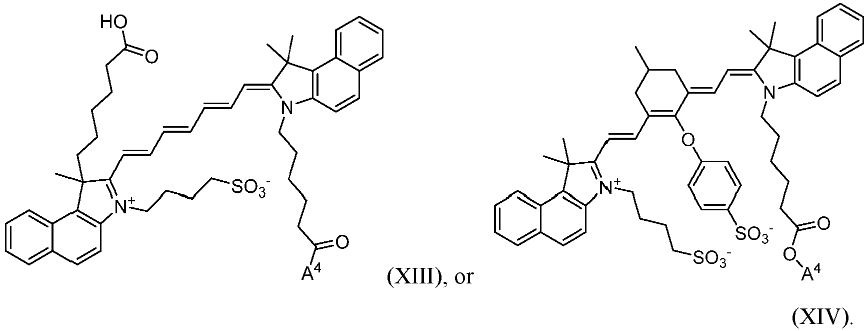

4. The compound of any one of claims 1-3, wherein:
the polypeptide comprises an isoelectric point of greater than 7.5 and three or four disulfide bonds;
a lysine is replaced with a nonnaturally occurring amino acid;
the fragment of A⁴ comprises at least 31 amino acid residues;
one or more methionine residues of the polypeptide is replaced with an amino acid residue selected from isoleucine, threonine, valine, leucine, serine, glycine, alanine, or a combination thereof;
the polypeptide comprises a lysine residue at the position corresponding to K-27 of native chlorotoxin, K-23 of native chlorotoxin, K-15 of native chlorotoxin, or a combination thereof; or
combinations thereof.

5. The compound of any one of claims 1-4, wherein L³ is attached to A⁴ at a lysine amino acid residue of the polypeptide.

6. The compound of any one of claims 1-5, wherein the compound is conjugated to polyethylene glycol (PEG), hydroxyethyl starch, polyvinyl alcohol, a water soluble polymer, a zwitterionic water soluble polymer, a water soluble poly(amino acid), an albumin, or a fatty acid.

7. The compound of any one of claims 1-6, further comprising a therapeutic agent attached to A⁴, wherein the therapeutic agent is selected from a radioisotope, toxin, cytotoxic agent, enzyme, sensitizing drug, nucleic acid, interfering RNA, antibody, anti-angiogenic agent, cisplatin, anti-metabolite, mitotic inhibitor, growth factor inhibitor, paclitaxel, temozolomide, topotecan, fluorouracil, vincristine, vinblastine, procarbazine, dacarbazine, altretamine, methotrexate, mercaptopurine, thioguanine, fludarabine phosphate, cladribine, pentostatin, cytarabine, azacitidine, etoposide, teniposide, irinotecan, docetaxel, doxorubicin, daunorubicin, dactinomycin, idarubicin, plicamycin, mitomycin, bleomycin, tamoxifen, flutamide, leuprolide, goserelin, aminogluthimide, anastrozole, amsacrine, asparaginase, mitoxantrone, mitotane, amifostine , or a combination thereof.

8. A compound according to any one of claims 1-7 further comprising a therapeutic agent for use in a method of treating a subject in need thereof, the method comprising administering to the subject the compound according to any one of claims 1-7 further comprising the therapeutic agent
in an amount sufficient to treat cancer in the subject wherein the cancer is selected from glioma, astrocytoma, medulloblastoma, choroids plexus carcinoma, ependymoma, neuroblastoma, carcinoma, head and neck cancer, lung cancer, small cell lung cancer, non-small cell lung cancer, breast cancer, intestinal cancer, pancreatic cancer, liver cancer, kidney cancer, sarcoma, osteosarcoma, rhabdomyosarcoma, Ewing's sarcoma, gastrointestinal stromal tumors, melanoma, ovarian cancer, cervical cancer, lymphoma, thyroid cancer, anal cancer, colorectal cancer, endometrial cancer, laryngeal cancer, multiple myeloma, prostate cancer, retinoblastoma, gastric cancer, testicular cancer, or Wilm's tumor.

9. A compound according to any one of claims 1-7 for use in a method of diagnosis *in vivo* of cancer, the method comprising:
administering to a subject the compound according to any one of claims 1-7;
and
detecting the presence or absence of the compound according to any one of claims 1-7 in a tissue or cell, wherein the presence of said
compound in the tissue or cell indicates the presence of a cancerous tissue or cancer cell.

10. The compound for use according to claim 9, further comprising surgically removing from the subject the cancerous tissue or cancer cell that is detected.

11. An *ex vivo* method for detecting a cancerous tissue or cancer cell, the method comprising:
detecting the presence or absence of a compound according to any one of claims 1-7 in an *ex vivo* tissue or cell that has been obtained from a subject that has been administered the compound, wherein the presence of the compound in the tissue or cell indicates the presence of a cancerous tissue or cancer cell.

12. The compound for use according to claim 9 or 10, or the *ex vivo* method according to claim 11, wherein the detecting comprises fluorescence imaging.

13. A composition comprising the compound of any one of claims 1-7 and a pharmaceutically acceptable carrier.

14. The composition of claim 13, wherein the composition is formulated for intravenous administration, intravenous bolus administration, intravenous infusion administration, intramuscular administration, subcutaneous administration, intraperitoneal administration, oral administration, topical administration, transdermal administration using electroporation, intralesional administration, or a combination thereof ,
and/or wherein:
the composition comprises a pH from 6 to 7.5;
the ionic strength of the composition is less than 50 mM;
the composition is stored as a lyophilized solid; or
combinations thereof.

15. The composition of claim 13 or 14, further comprising:
a buffer, wherein the buffer is selected from histidine, tris, HEPES, ethylene diamine, or a combination thereof;
a sugar alcohol;
10 to 100 mM histidine;
2% to 10% (wt/vol %) mannitol; or
combinations thereof.

## Patentansprüche

1. Verbindung mit der Struktur der Formel (III) oder ein pharmazeutisch verträgliches Salz davon: 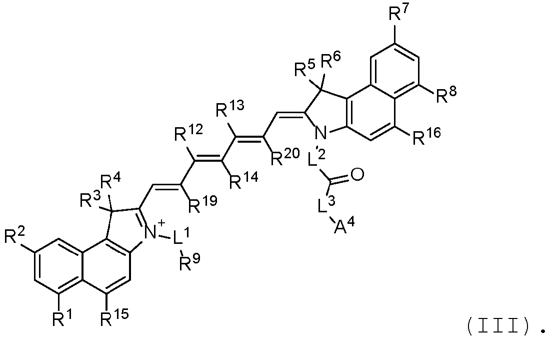 wobei:
R¹, R², R³, R⁴, R⁵, R⁶, R⁷, R⁸, R¹⁵, R¹⁶ sind jeweils unabhängig ausgewählt aus Wasserstoff, C₁-C₆ Alkyl, C₁-C₆ Alkylen-COOH, Sulfonat, -COOH, -SO₂-NH₂, C₁-C₆ Alkoxy;
R⁹ für Sulfonat steht;
L¹ C₃-C₆ -Alkylen;
L² C₁-C₁₀ Alkylen;
L³ ist eine Bindung, -O-, -NR¹⁰-, -NR¹⁰-C₁-C₆ Alkylen-, -O-NR¹⁰-, -NR¹⁰-C₁-C₆ Alkylen- (O-C₁-C₆ Alkylen)ₙ-, -NR¹⁰-L⁴-, -NR¹⁰-C₁-C₆ Alkylen-NR¹¹ -(C(=O)-C₁-C₆ Alkylen-O-)ₘ-, or -NR¹⁰-C₁-C₆ Alkylen--NR¹⁰-C₁-C₆ Alkylen--NR¹⁰-C₁-C₆ Alkylen-;
L⁴ eine Bindung, -Heterocyclyl-, oder -Heterocyclyl-C₁-C₆-alkylen-;
R¹⁰ Wasserstoff oder C₁-C₆ Alkyl;
R¹¹ Wasserstoff oder C₁-C₆ Alkyl;
R¹² und R¹³ sind unabhängig voneinander ausgewählt aus Wasserstoff, C₁-C₆ Alkyl, oder R¹² und R¹³ sind zusammen mit den anderen Atomen, an die sie gebunden sind, zu einem 5- oder 6-gliedrigen carbocyclischen oder heterocyclischen Ring verbunden;
R¹⁴ Wasserstoff oder C₁-C₆ Alkylen, -(L⁵) aryl, -L⁵-heteroaryl, -NR¹⁷R¹⁸, R¹⁴ und R¹⁹ sind zusammen mit den anderen Atomen, an die sie gebunden sind, zu einem 5- oder 6-gliedrigen carbocyclischen oder heterocyclischen Ring verbunden, oder R¹⁴ und R²⁰ sind zusammen mit den anderen Atomen, an die sie gebunden sind, zu einem 5- oder 6-gliedrigen carbocyclischen oder heterocyclischen Ring verbunden;
L⁵ eine Bindung, C₁-C₁₀ Alkylen, -O- oder -NR¹⁰-;
R¹⁷ und R¹⁸ unabhängig voneinander Wasserstoff oder Aryl bedeuten;
R¹⁹ und R²⁰ sind unabhängig voneinander ausgewählt aus Wasserstoff, C₁-C₆ alkyl, R¹⁴ und R¹⁹ sind zusammen mit den anderen Atomen, an die sie gebunden sind, zu einem 5- oder 6-gliedrigen carbocyclischen oder heterocyclischen Ring verbunden, oder R¹⁴ und R²⁰ sind zusammen mit den anderen Atomen, an die sie gebunden sind, zu einem 5- oder 6-gliedrigen carbocyclischen oder heterocyclischen Ring verbunden;
n für 0, 1, 2 oder 3 steht;
m für 0, 1, 2 oder 3 steht;
p für 0, 1, 2 oder 3 steht;
q für 0, 1, 2 oder 3 steht; und
A⁴ ist ein Polypeptid mit mindestens 90% Sequenzidentität mit SEQ ID NO: 9 oder ein Fragment davon, wobei das Fragment von A⁴ mindestens 25 Aminosäurereste umfasst.

2. Verbindung gemäß Anspruch 1,
wobei:
R³, R⁴, R⁵ und R⁶ unabhängig voneinander Methyl;
R¹, R², R⁷, R⁸, R¹⁵ und R¹⁶ sind jeweils unabhängig voneinander Wasserstoff;
R¹⁰ ist Wasserstoff; und
R¹², R¹³, R¹⁴, R¹⁹ und R²⁰ unabhängig voneinander Wasserstoff sind;
wobei:
L¹ ist Butylen und L² ist Pentylen;
oder Kombinationen davon.

3. Verbindung nach einem der Ansprüche 1 oder 2 mit der Struktur einer der Formeln (VII), (VIII), (IX), (XI), (XIII) oder (XIV): 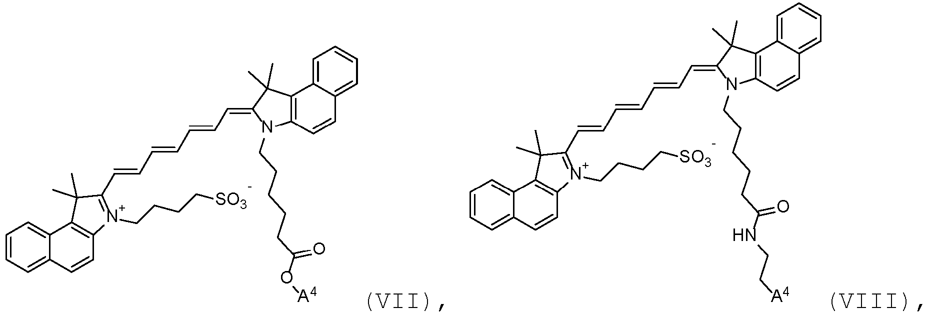 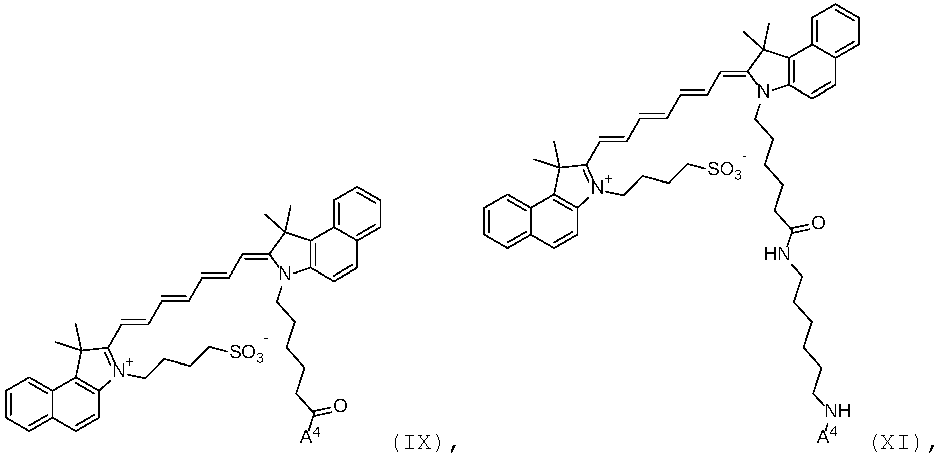 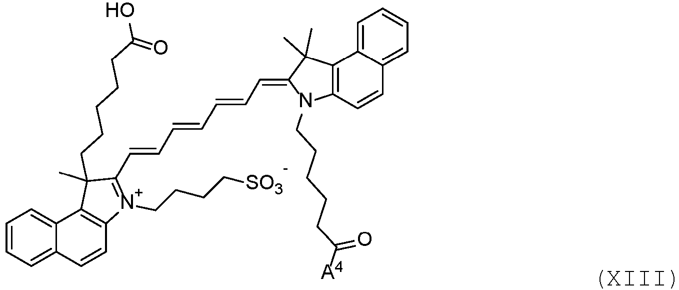 oder

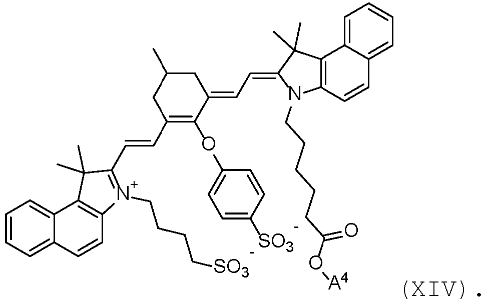

4. Verbindung nach einem der Ansprüche 1 bis 3, wobei:
das Polypeptid einen isoelektrischen Punkt von größer als 7,5 und drei oder vier Disulfidbindungen aufweist;
ein Lysin durch eine nicht natürlich vorkommende Aminosäure ersetzt wird;
das Fragment von A⁴ mindestens 31 Aminosäurereste umfasst;
ein oder mehrere Methioninreste des Polypeptids durch einen Aminosäurerest, ausgewählt aus Isoleucin, Threonin, Valin, Leucin, Serin, Glycin, Alanin oder einer Kombination davon, ersetzt wird;
das Polypeptid einen Lysinrest an der Position umfasst, die K-27 von nativem Chlortoxin, K-23 von nativem Chlortoxin, K-15 von nativem Chlortoxin oder einer Kombination davon entspricht; oder
Kombinationen davon.

5. Verbindung nach einem der Ansprüche 1 bis 4, wobei L ³ an einen Lysinaminosäurerest des Polypeptids an A ⁴ gebunden ist.

6. Verbindung nach einem der Ansprüche 1 bis 5, wobei die Verbindung an Polyethylenglykol (PEG), Hydroxyethylstärke, Polyvinylalkohol, ein wasserlösliches Polymer, ein zwitterionisches wasserlösliches Polymer, eine wasserlösliche Poly(aminosäure), ein Albumin oder eine Fettsäure konjugiert ist.

7. Verbindung nach einem der Ansprüche 1 bis 6, ferner umfassend ein an A⁴ gebundenes Therapeutikum, wobei das Therapeutikum ausgewählt ist aus einem Radioisotop, Toxin, zytotoxischen Mittel, Enzym, sensibilisierendem Medikament, Nukleinsäure, interferierender RNA, Antikörper, anti-angiogenes Mittel, Cisplatin, Antimetabolit, Mitoseinhibitor, Wachstumsfaktor-Inhibitor, Paclitaxel, Temozolomid, Topotecan, Fluorouracil, Vincristin, Vinblastin, Procarbazin, Dacarbazin, Altretamin, Methotrexat, Mercaptopurin, Thioguanin, Fludarabinphosphat, Cladribin, Pentostatin, Cytarabin, Azacitidin, Etoposid, Teniposid, Irinotecan, Docetaxel, Doxorubicin, Daunorubicin, Dactinomycin, Idarubicin, Plicamycin, Mitomycin, Bleomycin, Tamoxifen, Flutamid, Leuprolid, Goserelin, Aminogluthimid, Anastrozol, Amsacrin, Asparaginase, Mitoxantron, Mitotan, Amifostin oder eine Kombination davon.

8. Verbindung nach einem der Ansprüche 1 bis 7, ferner umfassend ein therapeutisches Mittel zur Verwendung in einem Verfahren zur Behandlung eines Patienten, der es benötigt, wobei das Verfahren umfasst, dass dem Patienten die Verbindung nach einem der Ansprüche 1 bis 7, ferner umfassend das therapeutische Mittel in einer Menge, die ausreicht, um Krebs bei dem Patienten zu behandeln, wobei der Krebs ausgewählt ist aus Gliom, Astrozytom, Medulloblastom, Choroiden-Plexus-Karzinom, Ependymom, Neuroblastom, Karzinom, Kopf- und Halskrebs, Lungenkrebs, kleinzelligem Lungenkrebs, Brustkrebs, Darmkrebs, Pankrebs, Leberkrebs, Sarkom, Osteosarkom, Rhabdomyosarkom, Ewing-Sarkom, gastrointestinale Stromatumoren, Melanom, Ovarialkarzinom, Zervixkarzinom, Lymphom, Schilddrüsenkarzinom, Analkarzinom, kolorektales Karzinom, Endometriumkarzinom, Larynxkarzinom, multiples Myelom, Prostatakarzinom, Retinoblastom, Magenkarzinom, Hodenkarzinom oder Wilm-Tumor.

9. Verbindung nach einem der Ansprüche 1 bis 7 zur Verwendung in einem Verfahren zur Diagnose von Krebs in *vivo,* wobei das Verfahren umfasst:
Verabreichen der Verbindung nach einem der Ansprüche 1-7 an einen Probanden; und
Nachweis der Anwesenheit oder Abwesenheit der Verbindung nach einem der Ansprüche 1 bis 7 in einem Gewebe oder einer Zelle, wobei die Anwesenheit der Verbindung in dem Gewebe oder der Zelle die Anwesenheit eines Krebsgewebes oder einer Krebszelle anzeigt.

10. Verwendung nach Anspruch 9, **dadurch gekennzeichnet, dass** das detektierte Krebsgewebe bzw. die detektierten Krebszellen chirurgisch vom Probanden entfernt werden.

11. Ex *vivo*-Verfahren zum Nachweis eines Krebsgewebes oder einer Krebszelle, wobei das Verfahren umfasst:
Nachweis der Anwesenheit oder Abwesenheit einer Verbindung nach einem der Ansprüche 1 bis 7 in einem *ex vivo* Gewebe oder einer Zelle, die von einem Subjekt erhalten wurde, dem die Verbindung verabreicht wurde, wobei die Anwesenheit der Verbindung in dem Gewebe oder der Zelle die Anwesenheit eines Krebsgewebes oder einer Krebszelle anzeigt.

12. Verbindung zur Verwendung nach Anspruch 9 oder 10, oder dasex *vivo* Verfahren nach Anspruch 11, wobei das Nachweisen eine Fluoreszenzbildgebung umfasst.

13. Eine Zusammensetzung, umfassend die Verbindung nach einem der Ansprüche 1 bis 7 und einen pharmazeutisch annehmbaren Träger.

14. Die Zusammensetzung nach Anspruch 13, wobei die Zusammensetzung zur intravenösen Verabreichung, zur intravenösen Bolusgabe, zur intravenösen Infusionsverabreichung, zur intramuskulären Verabreichung, zur subkutanen Verabreichung , zur intraperitonealen Verabreichung, zur oralen Verabreichung, zur topischen Verabreichung, zur transdermalen Verabreichung mittels Elektroporation, zur intraläsionalen Verabreichung oder zu einer Kombination davon formuliert ist,
wobei:
die Zusammensetzung einen pH-Wert von 6 bis 7,5 umfasst;
die Ionenstärke der Zusammensetzung kleiner als 50 mM ist;
die Zusammensetzung als lyophilisierter Feststoff gelagert wird; oder
Kombinationen davon.

15. Die Zusammensetzung nach Anspruch 13 oder 14, ferner umfassend:
einen Puffer, wobei der Puffer ausgewählt ist aus Histidin, Tris, HEPES, Ethylendiamin oder einer Kombination davon;
einen Zuckeralkohol;
10 bis 100 mM Histidin;
2 bis 10 Gew.-/Vol.-% Mannit oder
Kombinationen davon.

## Revendications

1. Composé ayant la structure de formule (III), ou un sel pharmaceutiquement acceptable de celui-ci : 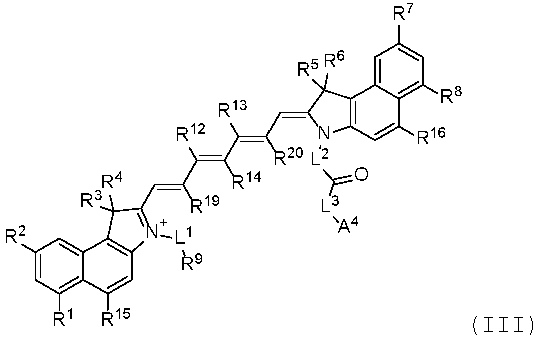 dans lequel :
R¹, R², R³, R⁴, R⁵, R⁶, R⁷, R⁸, R¹⁵ et R¹⁶ sont chacun indépendamment choisis parmi l'hydrogène, un alkyle en C₁-C₆, un alkylène en C₁-C₆ -COOH, un sulfonate, -COOH, -SO₂-NH₂ ou un alcoxy en C₁-C₆ ;
R⁹ est un sulfonate ;
L¹ est un alkylène en C₃-C₆ ;
L² est un alkylène en C₁-C₁₀ ;
L³ est une liaison, -O-, -NR¹⁰, -NR¹⁰-alkylène en C₁-C₆-, - O-NR¹⁰-, -NR¹⁰-alkylène en C₁-C₆- (O-alkylène en C₁-C₆)ₙ-, -NR¹⁰-L⁴-, -NR¹⁰-alkylène en C₁-C₆ -NR¹¹ - (C (=O) -alkylène en C₁-C₆-O-)ₘ-, ou -NR¹⁰-alkylène en C₁-C₆ --NR¹⁰-alkylène en C₁-C₆--NR¹⁰ -alkylène en C₁-C₆- ;
L⁴ est une liaison, -hétérocyclyle- ou -hétérocyclyle-alkylène en C₁-C₆ ;
R¹⁰ est l'hydrogène ou un groupe alkyle en C₁-C₆ ;
R¹¹ est l'hydrogène ou un groupe alkyle en C₁-C₆ ;
R¹² et R¹³ sont indépendamment choisis parmi l'hydrogène, un alkyle en C₁-C₆ ou R¹² et R¹³ sont liés ensemble avec les autres atomes auxquels ils sont attachés pour former un cycle carbocyclique ou hétérocyclique à 5 ou 6 chaînons ;
R¹⁴ est un hydrogène ou un alkylène en C₁-C₆, -(L⁵)-aryle, - (L⁵) -hétéroaryle, -NR¹⁷ R¹⁸, R¹⁴ et R¹⁹ sont liés entre eux avec les autres atomes auxquels ils sont attachés pour former un cycle carbocyclique ou hétérocyclique à 5 ou 6 chaînons, ou R¹⁴ et R²⁰ sont liés entre eux avec les autres atomes auxquels ils sont attachés pour former un cycle carbocyclique ou hétérocyclique à 5 ou 6 chaînons ;
L⁵ est une liaison, un alkylène en C₁-C₁₀, -O- ou -NR¹⁰- ;
R¹⁷ et R¹⁸ sont chacun indépendamment l'hydrogène ou un aryle ;
R¹⁹ et R²⁰ sont indépendamment choisi parmi l'hydrogène, un alkyle en C₁-C₆, R¹⁴ et R¹⁹ sont liés entre eux avec les autres atomes auxquels ils sont attachés pour former un cycle carbocyclique ou hétérocyclique à 5 ou 6 chaînons, ou R¹⁴ et R²⁰ sont liés entre eux avec les autres atomes auxquels ils sont attachés pour former un cycle carbocyclique ou hétérocyclique à 5 ou 6 chaînons ;
n est 0, 1, 2 ou 3 ;
m est 0, 1, 2 ou 3 ;
p est 0, 1, 2 ou 3 ;
q est 0, 1, 2 ou 3 ; et
A⁴ est un polypeptide ayant au moins 90 % d'identité de séquence avec SEQ ID NO : 9, ou un fragment de celui-ci dans lequel le fragment de A⁴ comprend au moins 25 résidus d'acides aminés.

2. Composé selon la revendication 1,
dans lequel :
R³, R⁴, R⁵ et R⁶ sont chacun indépendamment un méthyle ;
R¹, R², R⁷, R⁸, R¹⁵ et R¹⁶ sont chacun indépendamment l'hydrogène ;
R¹⁰ est l'hydrogène ; et
R¹², R¹³, R¹⁴, R¹⁹ et R²⁰ sont chacun indépendamment
l'hydrogène ;
dans lequel :
L¹ est un butylène et L² est un pentylène ;
ou des combinaisons de ceux-ci.

3. Composé selon l'une quelconque des revendications 1 ou 2 ayant la structure de l'une quelconque des formules (VII), (VIII), (IX), (XI), (XIII) ou (XIV) : 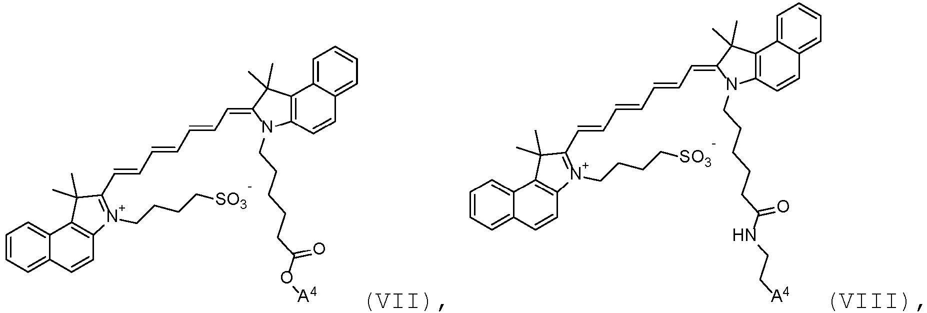 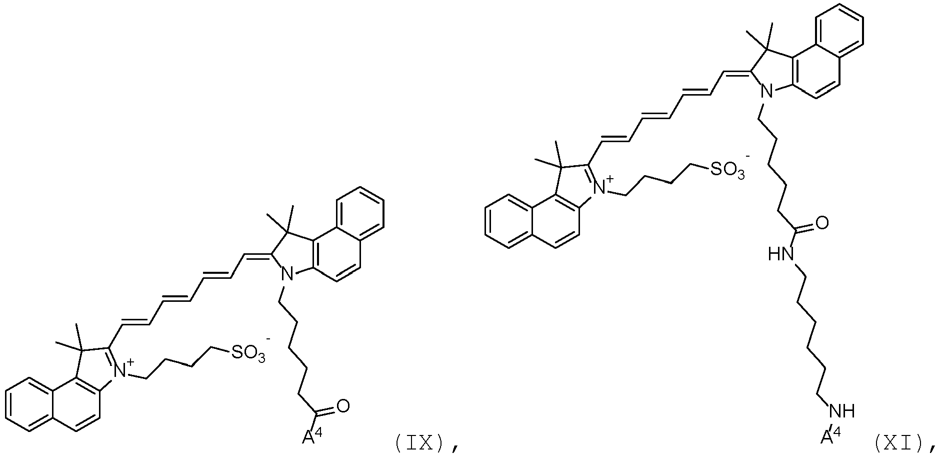 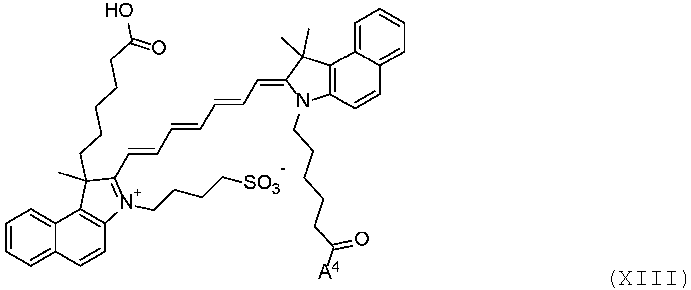 ou

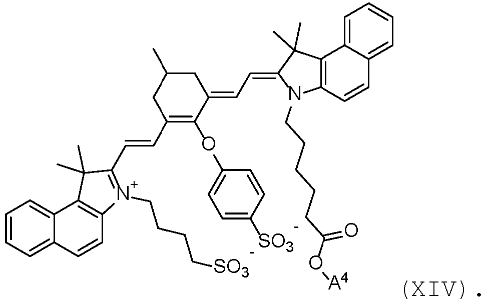

4. Composé selon l'une quelconque des revendications 1 à 3, dans lequel :
le polypeptide comprend un point isoélectrique supérieur à 7,5 et trois ou quatre liaisons disulfures ;
une lysine est remplacée par un acide aminé non naturel ;
le fragment de A⁴ comprend au moins 31 résidus d'acides aminés ;
un ou plusieurs résidus de méthionine du polypeptide sont remplacés par un résidu d'acide aminé choisi parmi l'isoleucine, la thréonine, la valine, la leucine, la sérine, la glycine, l'alanine ou une combinaison de celles-ci ;
le polypeptide comprend un résidu de lysine à la position correspondant à K-27 de la chlorotoxine native, K-23 de la chlorotoxine native, K-15 de la chlorotoxine native, ou une combinaison de ceux-ci ; ou
des combinaisons de ceux-ci.

5. Composé selon l'une quelconque des revendications 1 à 4, dans lequel L³ est attaché à A⁴ au niveau d'un résidu d'acide aminé de lysine du polypeptide.

6. Composé selon l'une quelconque des revendications 1 à 5, dans lequel le composé est conjugué à du polyéthylène glycol (PEG), de l'hydroxyéthylamidon, du poly(alcool vinylique), un polymère hydrosoluble, un polymère zwitterionique hydrosoluble, un poly(acide aminé) hydrosoluble, une albumine ou un acide gras.

7. Composé selon l'une quelconque des revendications 1 à 6, comprenant également un agent thérapeutique lié à A⁴, dans lequel l'agent thérapeutique est choisi parmi un radio-isotope, une toxine, un agent cytotoxique, une enzyme, un médicament sensibilisant,un acide nucléique, un ARN interférent, un anticorps, un agent anti-angiogénique, le cisplatine, un antimétabolite, un inhibiteur mitotique, un inhibiteur du facteur de croissance, le paclitaxel, le témozolomide, le topotécan, le fluorouracile, la vincristine, la vinblastine, la procarbazine, la dacarbazine, l'altrétamine, le méthotrexate, la mercaptopurine, la thioguanine, le phosphate de fludarabine, la cladribine, la pentostatine, la cytarabine, l'azacitidine, l'étoposide, le téniposide, l'irinotécan, le docétaxel, la doxorubicine, la daunorubicine, la dactinomycine, l'idarubicine, la plicamycine, la mitomycine, la bléomycine, le tamoxifène, le flutamide, le leuprolide, la goséréline, l'aminogluthimide, l'anastrozole, l'amsacrine, l'asparaginase, la mitoxantrone, le mitotane, l'amifostine ou une combinaison de ceux-ci.

8. Composé selon l'une quelconque des revendications 1 à 7, comprenant également un agent thérapeutique destiné à être utilisé dans un procédé de traitement d'un sujet en ayant besoin, le procédé comprenant l'administration au sujet le composé selon l'une quelconque des revendications 1 à 7, comprenant également l'agent thérapeutique en une quantité suffisante pour traiter un cancer chez le sujet, dans lequel le cancer est choisi parmi le gliome, l'astrocytome, le médulloblastome, le carcinome du plexus choroïde, l'épendymome, le neuroblastome, le carcinome, le cancer de la tête et du cou, le cancer du poumon, le cancer du poumon à petites cellules, le cancer du poumon non à petites cellules, le cancer du sein, le cancer de l'intestin, le cancer du pancréas, le cancer du foie, le cancer du rein, le sarcome, l'ostéosarcome, le rhabdomyosarcome, le sarcome d'Ewing, les tumeurs stromales gastro-intestinales, le mélanome, le cancer de l'ovaire, le cancer du col de l'utérus, le lymphome, le cancer de la thyroïde, le cancer anal, le cancer colorectal, le cancer de l'endomètre, le cancer du larynx, le myélome multiple, le cancer de la prostate, le rétinoblastome, le cancer gastrique, le cancer des testicules ou la tumeur de Wilm.

9. Composé selon l'une quelconque des revendications 1 à 7 pour une utilisation dans un procédé de diagnostic *in vivo* du cancer, le procédé comprenant :
l'administration à un sujet le composé selon l'une quelconque des revendications 1 à 7 ; et
la détection de la présence ou de l'absence du composé selon l'une quelconque des revendications 1 à 7 dans un tissu ou une cellule, dans lequel la présence dudit composé dans le tissu ou la cellule indique la présence d'un tissu cancéreux ou d'une cellule cancéreuse.

10. Composé pour une utilisation selon la revendication 9, comprenant également l'élimination chirurgicale du tissu cancéreux ou de la cellule cancéreuse détectée chez le sujet.

11. Procédé *ex vivo* de détection d'un tissu cancéreux ou d'une cellule cancéreuse, le procédé comprenant :
la détection de la présence ou de l'absence d'un composé selon l'une quelconque des revendications 1 à 7 dans un tissu ou une cellule *ex vivo* qui a été obtenu auprès d'un sujet auquel le composé a été administré, dans lequel la présence du composé dans le tissu ou la cellule indique la présence d'un tissu cancéreux ou d'une cellule cancéreuse.

12. Composé pour une utilisation selon la revendication 9 ou 10, ou procédé *ex vivo* selon la revendication 11, dans lequel la détection comprend l'imagerie par fluorescence.

13. Composition comprenant le composé selon l'une quelconque des revendications 1 à 7 et un support pharmaceutiquement acceptable.

14. Composition selon la revendication 13, dans laquelle la composition est formulée pour une administration intraveineuse, une administration en bolus intraveineux, une administration par perfusion intraveineuse, une administration intramusculaire, une administration sous-cutanée, une administration intrapéritonéale, une administration orale, une administration topique, une administration transdermique par électroporation, une administration intralésionnelle, ou une combinaison de celles-ci,
et/ou dans laquelle :
la composition comprend un pH de 6 à 7,5 ;
la force ionique de la composition est inférieure à 50 mM ;
la composition est stockée sous forme d'un solide lyophilisé ; ou
des combinaisons de ceux-ci.

15. Composition selon la revendication 13 ou 14, comprenant également :
un tampon, dans lequel le tampon est choisi parmi l'histidine, le tris, l'HEPES, l'éthylène diamine ou une combinaison de ceux-ci ;
un alcool de sucre ;
10 à 100 mM d'histidine ;
2 % à 10 % (% en poids/volume) de mannitol ; ou
des combinaisons de ceux-ci.
